# EUROPEAN PATENT SPECIFICATION

(11) **EP 3 277 276 B1**
(45) Date of publication and mention of the grant of the patent: **04.11.2020**
(21) Application number: 16774100.8
(22) Date of filing: 30.03.2016
(51) Int. Cl.: A61K 31/433, A61K 31/501, A61K 31/4245, A61P 31/12, A61P 35/00, A61P 37/00

(54) **METHODS OF ADMINISTERING GLUTAMINASE INHIBITORS**
VERFAHREN ZUR VERABREICHUNG VON GLUTAMINASEINHIBITOREN
PROCÉDÉS D'ADMINISTRATION D'INHIBITEURS DE GLUTAMINASE

(30) Priority: 30.03.2015 US 201562139928 P; 29.05.2015 US 201562168112 P
(43) Date of publication of application: 07.02.2018
(73) Proprietor: Calithera Biosciences, Inc., South San Francisco, CA 94080 (US)
(72) Inventor: GROSS, Matthew I., Walnut Creek, California 94598 (US); BENNETT, Mark, K., Brooklyn, NY 11201 (US); MOLINEAUX, Christopher, San Francisco, CA 94114 (US)
(74) Representative: HGF
(86) International application number: PCT/US2016/024998
(87) International publication number: WO 2016/160980

(56) References cited:
- WO-A1-2013/078123
- WO-A1-2013/078123
- WO-A1-2014/089048
- WO-A1-2015/061432
- WO-A1-2015/138902
- WO-A1-2015/192014
- WO-A1-2016/004418
- WO-A1-2016/014890
- WO-A1-2016/022969
- WO-A1-2016/054388
- WO-A2-2016/077632
- US-A1- 2014 142 081
- US-A1- 2014 142 146
- US-A1- 2014 194 421
- US-A1- 2016 010 158
- KRUPA SHUKLA ET AL: "Design, Synthesis, and Pharmacological Evaluation of Bis-2-(5-phenylacetamido-1,2,4-thiadiazol- 2-yl)ethyl Sulfide 3 (BPTES) Analogs as Glutaminase Inhibitors", JOURNAL OF MEDICINAL CHEMISTRY, vol. 55, no. 23, 13 December 2012 (2012-12-13), pages 10551-10563, XP055205094, ISSN: 0022-2623, DOI: 10.1021/jm301191p
- M. I. GROSS ET AL: "Antitumor Activity of the Glutaminase Inhibitor CB-839 in Triple-Negative Breast Cancer", MOLECULAR CANCER THERAPEUTICS, vol. 13, no. 4, 12 February 2014 (2014-02-12), pages 890-901, XP055243589, US ISSN: 1535-7163, DOI: 10.1158/1535-7163.MCT-13-0870
- GROSS, M. I. ET AL.: 'Antitumor Activity of the Glutaminase Inhibitor CB-839 in Triple-Negative Breast Cancer' MOLECULAR CANCER THERAPEUTICS, [Online] vol. 13, no. 4, pages 890 - 901, XP055243589 Retrieved from the Internet: <URL:http://mct.aacrjoumals.org/content/13/ 4/890. full>
- BROMLEY-DULFANO, S.: 'Antitumor Activity Of The Glutaminase Inhibitor CB-839 In Hematological Malignances' BLOOD vol. 122, no. 21, 05 December 2013, page 4226, XP055318327
- ZIMMERMANN, S. C. ET AL.: 'Allosteric Glutaminase Inhibitors Based on a 1,4-Di(5-amino-1,3,4 thiadiazol-2-yl)butane Scaffold' ACS MED. CHEM. LETT. vol. 7, no. 5, 13 March 2016, pages 520 - 52, XP055318330
- JACQUE, N. ET AL.: 'Targeting glutaminolysis has antileukemic activity in acute myeloid leukemia and synergizes with BCL-2 inhibition' BLOOD vol. 126, no. 11, 17 July 2015, pages 1346 - 1356., XP055318334

## Description

The present invention is defined by the appended claims.

### Background

Glutamine supports cell survival, growth and proliferation through metabolic and non-metabolic mechanisms. In actively proliferating cells, the metabolism of glutamine to lactate, also referred to as "glutaminolysis" is a major source of energy in the form of NADPH. The first step in glutaminolysis is the deamination of glutamine to form glutamate and ammonia, which is catalyzed by the glutaminase enzyme. Thus, deamination via glutaminase is a control point for glutamine metabolism.

Ever since the observation that ascites tumor cells exhibited high rates of glucose consumption and lactate secretion in the presence of oxygen, researchers have been exploring how cancer cells utilize metabolic pathways to be able to continue actively proliferating. Subsequent research has demonstrated how glutamine metabolism supports macromolecular synthesis necessary for cells to replicate.

Thus, glutaminase has been theorized to be a potential therapeutic target for the treatment of diseases characterized by actively proliferating cells, such as cancer. Therefore, compositions and methods for administering glutaminase inhibitors to prevent or treat disease are desirable.

### Summary

In some aspects, the invention relates to compounds of formula I, formula II or formula III for use in a method of treating cancer, comprising orally administering a compound of formula I, formula II, or formula III wherein the compound is administered with a meal (*e.g*., with food as defined herein) or in fed mode.

### Brief Description of the Drawings

**Figure 1** shows steady-state pharmacokinetics results for human clinical trials with various oral doses of the compound of formula III, either administered two times per day ("BID") or three times per day ("TID").
**Figure 2** shows pharmacokinetics results for human clinical trials with various oral doses of the compound of formula III administered three times per day either with meals ("fed") or in a fasted state ("fasted").

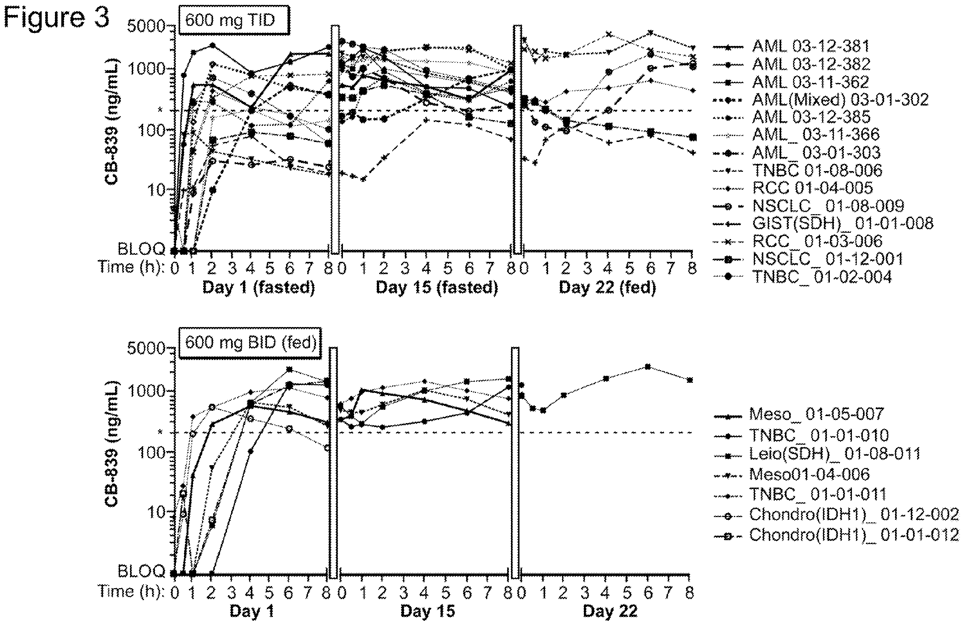

**Figure 3** shows pharmacokinetics profiles for human clinical trials with 600 mg doses of the compound of formula III administered two times per day ("BID"; 2 doses of 600 mg each) or three times per day ("TID"; 3 doses of 600 mg each).
**Figure 4** shows pharmacokinetics profiles for human clinical trials with 600 mg doses of the compound of formula III administered two times per day (Squares; 2 doses of 600 mg each) or three times per day (Circles; 3 doses of 600 mg each).
**Figure 5** shows pharmacokinetics profiles for human clinical trials with 600 mg doses of the compound of formula III administered two times per day (Squares; 2 doses of 600 mg each) or three times per day (Circles; 3 doses of 600 mg each).
**Figure 6** is a table that describes the dosing regimen for CB-839. The findings suggest that the BID Fed dosing regimen provides consistent exposure to CB-839.
**Figure 7** shows pharmacokinetics profiles for human clinical trials with 600 mg doses of the compound of formula III administered two times per day ("BID"; 2 doses of 600 mg each) or three times per day ("TID"; 3 doses of 600 mg each).
**Figure 8** are graphs plotting the dosage level of the compound of formula III against PK parameters AUC, Cₘₐₓ, and Cₘᵢₙ when the compound of formula III was administered two times per day (triangles) or three times per day (circles) in human subjects.

### Detailed Description

### Definitions

As used herein, the phrase "conjoint administration" refers to any form of administration of two or more different therapeutic compounds such that the second compound is administered while the previously administered therapeutic compound is still effective in the body (*e.g.,* the two compounds are simultaneously effective in the patient, which may include synergistic effects of the two compounds). For example, the different therapeutic compounds can be administered either in the same formulation or in a separate formulation, either concomitantly or sequentially. In certain embodiments, the different therapeutic compounds can be administered within one hour, 12 hours, 24 hours, 36 hours, 48 hours, 72 hours, or a week of one another. Thus, an individual who receives such treatment can benefit from a combined effect of different therapeutic compounds.

The term "fed mode," as used herein, refers to a state which is induced by the presence of food in the stomach. In the normal digestive process, the passage of matter through the stomach is delayed by the physiological condition referred to as the fed mode herein. Between fed modes, the stomach is in the interdigestive or "fasting" mode. The fed mode is typically initiated by nutritive materials entering the stomach upon the ingestion of food, and it persists for approximately 4 to 6 hours. The fed mode can also be induced pharmacologically by the administration of a pharmacological agent that has an effect that is the same or similar to that of a meal. These fed-mode inducing agents may be administered separately or they may be included in the dosage form as an ingredient dispersed in the dosage form or in an outer release coating. Examples of pharmacological fed-mode inducing agents are disclosed in U.S. Pat. No. 7,405,238.

The term "healthcare providers" refers to individuals or organizations that provide healthcare services to a person, community, etc. Examples of "healthcare providers" include doctors, hospitals, continuing care retirement communities, skilled nursing facilities, subacute care facilities, clinics, multispecialty clinics, freestanding ambulatory centers, home health agencies, and HMO's.

As used herein, a therapeutic that "prevents" a disorder or condition refers to a compound that, in a statistical sample, reduces the occurrence of the disorder or condition in the treated sample relative to an untreated control sample, or delays the onset or reduces the severity of one or more symptoms of the disorder or condition relative to the untreated control sample.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, solvent or encapsulating material. Each carrier must be "acceptable" in the sense of being compatible with the other ingredients of the formulation and not injurious to the patient. Some examples of materials which can serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The term "prodrug" is intended to encompass compounds which, under physiologic conditions, are converted into the therapeutically active agents of the present invention (*e.g.,* a compound of formulas I-VI). A common method for making a prodrug is to include one or more selected moieties which are hydrolyzed under physiologic conditions to reveal the desired molecule. In other embodiments, the prodrug is converted by an enzymatic activity of the host animal. For example, esters or carbonates (*e.g.,* esters or carbonates of alcohols or carboxylic acids) are preferred prodrugs of the present invention. In certain embodiments, some or all of the compounds of formulas I-VI in a formulation can be replaced with the corresponding suitable prodrug, *e.g.,* wherein a hydroxyl in the parent compound is presented as an ester or a carbonate or carboxylic acid present in the parent compound is presented as an ester.

The term "therapeutically effective amount" relates to the concentration of a compound that is sufficient to elicit the desired therapeutic effect. It is generally understood that the effective amount of the compound will vary according to the weight, sex, age, and medical history of the patient. Other factors which influence the effective amount may include, but are not limited to, the severity of the patient's condition, the disorder being treated, the stability of the compound, and, if desired, another type of therapeutic agent being administered with the compound of the invention. A larger total dose can be delivered by multiple administrations of the agent. Methods to determine efficacy and dosage are known to those skilled in the art (Isselbacher et al. (1996) Harrison's Principles of Internal Medicine 13 ed., 1814-1882.

The term "treating" includes prophylactic and/or therapeutic treatments. The term "prophylactic or therapeutic" treatment is art-recognized and includes administration to the host of one or more of the subject compositions. If it is administered prior to clinical manifestation of the unwanted condition (*e.g.,* disease or other unwanted state of the host animal) then the treatment is prophylactic (i.e., it protects the host against developing the unwanted condition), whereas if it is administered after manifestation of the unwanted condition, the treatment is therapeutic, (i.e., it is intended to diminish, ameliorate, or stabilize the existing unwanted condition or side effects thereof).

The terms "with food", "with a meal", "with meals", "during a meal", "after a meal" refers to the administration of a compound in temporal proximity to (e.g., before, during, or after) the ingestion of food (*e.g.,* a meal), and more particularly refers to the administration of a compound within 1, 2, 3, 4, 5, 10, 15, 20, 25, or 30 minutes before ingesting food, during a meal, or within 1, 2, 3, 4, 5, 10, 15, 20, 25, 30, 45, 60, or 90 minutes after ingesting food. In preferred embodiments, the terms "with food" and "with a meal" refer to the administration of a compound with a meal, before the meal (*e.g.,* 30 minutes before ingesting the food or meal), and after the meal (*e.g.,* 90 minutes after ingesting the food or meal).

### Definitions of Functional Groups

The term "acyl" is art-recognized and refers to a group represented by the general formula hydrocarbylC(O)-, preferably alkylC(O)-.
The term "acylamino" is art-recognized and refers to an amino group substituted with an acyl group and may be represented, for example, by the formula hydrocarbylC(O)NH-.
The term "acyloxy" is art-recognized and refers to a group represented by the general formula hydrocarbylC(O)O-, preferably alkylC(O)O-.
The term "alkoxy" refers to an alkyl group, preferably a lower alkyl group, having an oxygen attached thereto. Representative alkoxy groups include methoxy, ethoxy, propoxy, tert-butoxy and the like.
The term "alkoxyalkyl" refers to an alkyl group substituted with an alkoxy group and may be represented by the general formula alkyl-O-alkyl.
The term "alkenyl", as used herein, refers to an aliphatic group containing at least one double bond and is intended to include both "unsubstituted alkenyls" and "substituted alkenyls", the latter of which refers to alkenyl moieties having substituents replacing a hydrogen on one or more carbons of the alkenyl group. Such substituents may occur on one or more carbons that are included or not included in one or more double bonds. Moreover, such substituents include all those contemplated for alkyl groups, as discussed below, except where stability is prohibitive. For example, substitution of alkenyl groups by one or more alkyl, carbocyclyl, aryl, heterocyclyl, or heteroaryl groups is contemplated.
An "alkyl" group or "alkane" is a straight chained or branched non-aromatic hydrocarbon which is completely saturated. Typically, a straight chained or branched alkyl group has from 1 to about 20 carbon atoms, preferably from 1 to about 10 unless otherwise defined. Examples of straight chained and branched alkyl groups include methyl, ethyl, n-propyl, iso-propyl, n-butyl, sec-butyl, tert-butyl, pentyl, hexyl, pentyl and octyl. A C₁-C₆ straight chained or branched alkyl group is also referred to as a "lower alkyl" group.
Moreover, the term "alkyl" (or "lower alkyl") as used throughout the specification, examples, and claims is intended to include both "unsubstituted alkyls" and "substituted alkyls", the latter of which refers to alkyl moieties having substituents replacing a hydrogen on one or more carbons of the hydrocarbon backbone. Such substituents, if not otherwise specified, can include, for example, a halogen, a hydroxyl, a carbonyl (such as a carboxyl, an alkoxycarbonyl, a formyl, or an acyl), a thiocarbonyl (such as a thioester, a thioacetate, or a thioformate), an alkoxyl, a phosphoryl, a phosphate, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. It will be understood by those skilled in the art that the moieties substituted on the hydrocarbon chain can themselves be substituted, if appropriate. For instance, the substituents of a substituted alkyl may include substituted and unsubstituted forms of amino, azido, imino, amido, phosphoryl (including phosphonate and phosphinate), sulfonyl (including sulfate, sulfonamido, sulfamoyl and sulfonate), and silyl groups, as well as ethers, alkylthios, carbonyls (including ketones, aldehydes, carboxylates, and esters), -CF₃, -CN and the like. Exemplary substituted alkyls are described below. Cycloalkyls can be further substituted with alkyls, alkenyls, alkoxys, alkylthios, aminoalkyls, carbonyl-substituted alkyls, -CF₃, -CN, and the like.
The term "C_{x-y}" when used in conjunction with a chemical moiety, such as, acyl, acyloxy, alkyl, alkenyl, alkynyl, or alkoxy is meant to include groups that contain from x to y carbons in the chain. For example, the term "C_{x-y}alkyl" refers to substituted or unsubstituted saturated hydrocarbon groups, including straight-chain alkyl and branched-chain alkyl groups that contain from x to y carbons in the chain, including haloalkyl groups such as trifluoromethyl and 2,2,2-tirfluoroethyl, etc. C₀ alkyl indicates a hydrogen where the group is in a terminal position, a bond if internal. The terms "C_{2-y}alkenyl" and "C_{2-y}alkynyl" refer to substituted or unsubstituted unsaturated aliphatic groups analogous in length and possible substitution to the alkyls described above, but that contain at least one double or triple bond respectively.
The term "alkylamino", as used herein, refers to an amino group substituted with at least one alkyl group.
The term "alkylthio", as used herein, refers to a thiol group substituted with an alkyl group and may be represented by the general formula alkylS-.
The term "alkynyl", as used herein, refers to an aliphatic group containing at least one triple bond and is intended to include both "unsubstituted alkynyls" and "substituted alkynyls", the latter of which refers to alkynyl moieties having substituents replacing a hydrogen on one or more carbons of the alkynyl group. Such substituents may occur on one or more carbons that are included or not included in one or more triple bonds. Moreover, such substituents include all those contemplated for alkyl groups, as discussed above, except where stability is prohibitive. For example, substitution of alkynyl groups by one or more alkyl, carbocyclyl, aryl, heterocyclyl, or heteroaryl groups is contemplated.
The term "amide", as used herein, refers to a group 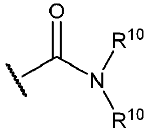 wherein each R¹⁰ independently represent a hydrogen or hydrocarbyl group, or two R¹⁰ are taken together with the N atom to which they are attached complete a heterocycle having from 4 to 8 atoms in the ring structure.
The terms "amine" and "amino" are art-recognized and refer to both unsubstituted and substituted amines and salts thereof, *e.g.,* a moiety that can be represented by 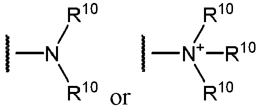 wherein each R¹⁰ independently represents a hydrogen or a hydrocarbyl group, or two R¹⁰ are taken together with the N atom to which they are attached complete a heterocycle having from 4 to 8 atoms in the ring structure.
The term "aminoalkyl", as used herein, refers to an alkyl group substituted with an amino group.
The term "aralkyl", as used herein, refers to an alkyl group substituted with an aryl group.
The term "aryl" as used herein include substituted or unsubstituted single-ring aromatic groups in which each atom of the ring is carbon. Preferably the ring is a 5- to 7-membered ring, more preferably a 6-membered ring. The term "aryl" also includes polycyclic ring systems having two or more cyclic rings in which two or more carbons are common to two adjoining rings wherein at least one of the rings is aromatic, *e.g.,* the other cyclic rings can be cycloalkyls, cycloalkenyls, cycloalkynyls, aryls, heteroaryls, and/or heterocyclyls. Aryl groups include benzene, naphthalene, phenanthrene, phenol, aniline, and the like.
The term "carbamate" is art-recognized and refers to a group 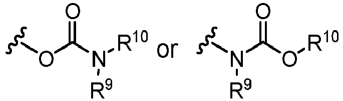 wherein R⁹ and R¹⁰ independently represent hydrogen or a hydrocarbyl group, such as an alkyl group, or R⁹ and R¹⁰ taken together with the intervening atom(s) complete a heterocycle having from 4 to 8 atoms in the ring structure.
The terms "carbocycle", and "carbocyclic", as used herein, refers to a saturated or unsaturated ring in which each atom of the ring is carbon. The term carbocycle includes both aromatic carbocycles and non-aromatic carbocycles. Non-aromatic carbocycles include both cycloalkane rings, in which all carbon atoms are saturated, and cycloalkene rings, which contain at least one double bond. "Carbocycle" includes 5-7 membered monocyclic and 8-12 membered bicyclic rings. Each ring of a bicyclic carbocycle may be selected from saturated, unsaturated and aromatic rings. Carbocycle includes bicyclic molecules in which one, two or three or more atoms are shared between the two rings. The term "fused carbocycle" refers to a bicyclic carbocycle in which each of the rings shares two adjacent atoms with the other ring. Each ring of a fused carbocycle may be selected from saturated, unsaturated and aromatic rings. In an exemplary embodiment, an aromatic ring, *e.g.,* phenyl, may be fused to a saturated or unsaturated ring, *e.g.,* cyclohexane, cyclopentane, or cyclohexene. Any combination of saturated, unsaturated and aromatic bicyclic rings, as valence permits, is included in the definition of carbocyclic. Exemplary "carbocycles" include cyclopentane, cyclohexane, bicyclo[2.2.1]heptane, 1,5-cyclooctadiene, 1,2,3,4-tetrahydronaphthalene, bicyclo[4.2.0]oct-3-ene, naphthalene and adamantane. Exemplary fused carbocycles include decalin, naphthalene, 1,2,3,4-tetrahydronaphthalene, bicyclo[4.2.0]octane, 4,5,6,7-tetrahydro-1H-indene and bicyclo[4.1.0]hept-3-ene. "Carbocycles" may be susbstituted at any one or more positions capable of bearing a hydrogen atom.
A "cycloalkyl" group is a cyclic hydrocarbon which is completely saturated. "Cycloalkyl" includes monocyclic and bicyclic rings. Typically, a monocyclic cycloalkyl group has from 3 to about 10 carbon atoms, more typically 3 to 8 carbon atoms unless otherwise defined. The second ring of a bicyclic cycloalkyl may be selected from saturated, unsaturated and aromatic rings. Cycloalkyl includes bicyclic molecules in which one, two or three or more atoms are shared between the two rings. The term "fused cycloalkyl" refers to a bicyclic cycloalkyl in which each of the rings shares two adjacent atoms with the other ring. The second ring of a fused bicyclic cycloalkyl may be selected from saturated, unsaturated and aromatic rings. A "cycloalkenyl" group is a cyclic hydrocarbon containing one or more double bonds.
The term "carbocyclylalkyl", as used herein, refers to an alkyl group substituted with a carbocycle group.
The term "carbonate" is art-recognized and refers to a group -OCO₂-R¹⁰, wherein R¹⁰ represents a hydrocarbyl group.
The term "carboxy", as used herein, refers to a group represented by the formula -CO₂H.
The term "ester", as used herein, refers to a group -C(O)OR¹⁰ wherein R¹⁰ represents a hydrocarbyl group.
The term "ether", as used herein, refers to a hydrocarbyl group linked through an oxygen to another hydrocarbyl group. Accordingly, an ether substituent of a hydrocarbyl group may be hydrocarbyl-O-. Ethers may be either symmetrical or unsymmetrical. Examples of ethers include, but are not limited to, heterocycle-O-heterocycle and aryl-O-heterocycle. Ethers include "alkoxyalkyl" groups, which may be represented by the general formula alkyl-O-alkyl.
The terms "halo" and "halogen" as used herein means halogen and includes chloro, fluoro, bromo, and iodo.
The terms "hetaralkyl" and "heteroaralkyl", as used herein, refers to an alkyl group substituted with a hetaryl group.
The term "heteroalkyl", as used herein, refers to a saturated or unsaturated chain of carbon atoms and at least one heteroatom, wherein no two heteroatoms are adjacent.
The terms "heteroaryl" and "hetaryl" include substituted or unsubstituted aromatic single ring structures, preferably 5- to 7-membered rings, more preferably 5- to 6-membered rings, whose ring structures include at least one heteroatom, preferably one to four heteroatoms, more preferably one or two heteroatoms. The terms "heteroaryl" and "hetaryl" also include polycyclic ring systems having two or more cyclic rings in which two or more carbons are common to two adjoining rings wherein at least one of the rings is heteroaromatic, *e.g.,* the other cyclic rings can be cycloalkyls, cycloalkenyls, cycloalkynyls, aryls, heteroaryls, and/or heterocyclyls. Heteroaryl groups include, for example, pyrrole, furan, thiophene, imidazole, oxazole, thiazole, pyrazole, pyridine, pyrazine, pyridazine, and pyrimidine, and the like.
The term "heteroatom" as used herein means an atom of any element other than carbon or hydrogen. Preferred heteroatoms are nitrogen, oxygen, and sulfur.
The terms "heterocyclyl", "heterocycle", and "heterocyclic" refer to substituted or unsubstituted non-aromatic ring structures, preferably 3- to 10-membered rings, more preferably 3- to 7-membered rings, whose ring structures include at least one heteroatom, preferably one to four heteroatoms, more preferably one or two heteroatoms. The terms "heterocyclyl" and "heterocyclic" also include polycyclic ring systems having two or more cyclic rings in which two or more carbons are common to two adjoining rings wherein at least one of the rings is heterocyclic, *e.g.,* the other cyclic rings can be cycloalkyls, cycloalkenyls, cycloalkynyls, aryls, heteroaryls, and/or heterocyclyls. Heterocyclyl groups include, for example, piperidine, piperazine, pyrrolidine, morpholine, lactones, lactams, and the like.
The term "heterocyclylalkyl", as used herein, refers to an alkyl group substituted with a heterocycle group.
The term "hydrocarbyl", as used herein, refers to a group that is bonded through a carbon atom that does not have a =O or =S substituent, and typically has at least one carbon-hydrogen bond and a primarily carbon backbone, but may optionally include heteroatoms. Thus, groups like methyl, ethoxyethyl, 2-pyridyl, and trifluoromethyl are considered to be hydrocarbyl for the purposes of this application, but substituents such as acetyl (which has a =O substituent on the linking carbon) and ethoxy (which is linked through oxygen, not carbon) are not. Hydrocarbyl groups include, but are not limited to aryl, heteroaryl, carbocycle, heterocyclyl, alkyl, alkenyl, alkynyl, and combinations thereof.
The term "hydroxyalkyl", as used herein, refers to an alkyl group substituted with a hydroxy group.
The term "lower" when used in conjunction with a chemical moiety, such as, acyl, acyloxy, alkyl, alkenyl, alkynyl, or alkoxy is meant to include groups where there are ten or fewer non-hydrogen atoms in the substituent, preferably six or fewer. A "lower alkyl", for example, refers to an alkyl group that contains ten or fewer carbon atoms, preferably six or fewer. In certain embodiments, acyl, acyloxy, alkyl, alkenyl, alkynyl, or alkoxy substituents defined herein are respectively lower acyl, lower acyloxy, lower alkyl, lower alkenyl, lower alkynyl, or lower alkoxy, whether they appear alone or in combination with other substituents, such as in the recitations hydroxyalkyl and aralkyl (in which case, for example, the atoms within the aryl group are not counted when counting the carbon atoms in the alkyl substituent).
The terms "polycyclyl", "polycycle", and "polycyclic" refer to two or more rings (*e.g.,* cycloalkyls, cycloalkenyls, cycloalkynyls, aryls, heteroaryls, and/or heterocyclyls) in which two or more atoms are common to two adjoining rings, *e.g.,* the rings are "fused rings". Each of the rings of the polycycle can be substituted or unsubstituted. In certain embodiments, each ring of the polycycle contains from 3 to 10 atoms in the ring, preferably from 5 to 7.
The term "silyl" refers to a silicon moiety with three hydrocarbyl moieties attached thereto.
The term "substituted" refers to moieties having substituents replacing a hydrogen on one or more carbons of the backbone. It will be understood that "substitution" or "substituted with" includes the implicit proviso that such substitution is in accordance with permitted valence of the substituted atom and the substituent, and that the substitution results in a stable compound, *e.g.,* which does not spontaneously undergo transformation such as by rearrangement, cyclization, elimination, etc. As used herein, the term "substituted" is contemplated to include all permissible substituents of organic compounds. In a broad aspect, the permissible substituents include acyclic and cyclic, branched and unbranched, carbocyclic and heterocyclic, aromatic and non-aromatic substituents of organic compounds. The permissible substituents can be one or more and the same or different for appropriate organic compounds. For purposes of this invention, the heteroatoms such as nitrogen may have hydrogen substituents and/or any permissible substituents of organic compounds described herein which satisfy the valences of the heteroatoms. Substituents can include any substituents described herein, for example, a halogen, a hydroxyl, a carbonyl (such as a carboxyl, an alkoxycarbonyl, a formyl, or an acyl), a thiocarbonyl (such as a thioester, a thioacetate, or a thioformate), an alkoxyl, a phosphoryl, a phosphate, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. It will be understood by those skilled in the art that substituents can themselves be substituted, if appropriate. Unless specifically stated as "unsubstituted," references to chemical moieties herein are understood to include substituted variants. For example, reference to an "aryl" group or moiety implicitly includes both substituted and unsubstituted variants.
The term "sulfate" is art-recognized and refers to the group -OSO₃H, or a pharmaceutically acceptable salt thereof.
The term "sulfonamide" is art-recognized and refers to the group represented by the general formulae 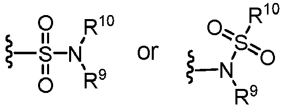 wherein R⁹ and R¹⁰ independently represents hydrogen or hydrocarbyl, such as alkyl, or R⁹ and R¹⁰ taken together with the intervening atom(s) complete a heterocycle having from 4 to 8 atoms in the ring structure.
The term "sulfoxide" is art-recognized and refers to the group -S(O)-R¹⁰, wherein R¹⁰ represents a hydrocarbyl.
The term "sulfonate" is art-recognized and refers to the group SO₃H, or a pharmaceutically acceptable salt thereof.
The term "sulfone" is art-recognized and refers to the group -S(O)₂-R¹⁰, wherein R¹⁰ represents a hydrocarbyl.
The term "thioalkyl", as used herein, refers to an alkyl group substituted with a thiol group.
The term "thioester", as used herein, refers to a group -C(O)SR¹⁰ or -SC(O)R¹⁰ wherein R¹⁰ represents a hydrocarbyl.
The term "thioether", as used herein, is equivalent to an ether, wherein the oxygen is replaced with a sulfur.
The term "urea" is art-recognized and may be represented by the general formula 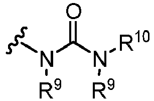 wherein R⁹ and R¹⁰ independently represent hydrogen or a hydrocarbyl, such as alkyl, or either occurrence of R⁹ taken together with R¹⁰ and the intervening atom(s) complete a heterocycle having from 4 to 8 atoms in the ring structure.
The term "protecting group" refers to a group of atoms that, when attached to a reactive functional group in a molecule, mask, reduce or prevent the reactivity of the functional group. Typically, a protecting group may be selectively removed as desired during the course of a synthesis. Examples of protecting groups can be found in Greene and Wuts, Protective Groups in Organic Chemistry, 3rdEd., 1999, John Wiley & Sons, NY and Harrison et al., Compendium of Synthetic Organic Methods, Vols. 1-8, 1971-1996, John Wiley & Sons, NY. Representative nitrogen protecting groups include, but are not limited to, formyl, acetyl, trifluoroacetyl, benzyl, benzyloxycarbonyl ("CBZ"), tert-butoxycarbonyl ("Boc"), trimethylsilyl ("TMS"), 2-trimethylsilyl-ethanesulfonyl ("TES"), trityl and substituted trityl groups, allyloxycarbonyl, 9-fluorenylmethyloxycarbonyl ("FMOC"), nitro-veratryloxycarbonyl ("NVOC") and the like. Representative hydroxylprotecting groups include, but are not limited to, those where the hydroxyl group is either acylated (esterified) or alkylated such as benzyl and trityl ethers, as well as alkyl ethers, tetrahydropyranyl ethers, trialkylsilyl ethers (*e.g.,* TMS or TIPS groups), glycol ethers, such as ethylene glycol and propylene glycol derivatives and allyl ethers.

### I. COMPOUNDS

The present invention relates to the claimed compounds for use in methods of treating cancer, comprising orally administering a compound of formula I, 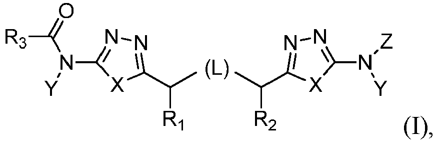 or a pharmaceutically acceptable salt thereof, wherein:
L represents CH₂SCH₂, CH₂CH₂, CH₂CH₂CH₂, CH₂, CH₂S, SCH₂, CH₂NHCH₂, CH=CH, or 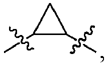 preferably CH₂CH₂, wherein any hydrogen atom of a CH or CH₂ unit may be replaced by alkyl or alkoxy, any hydrogen of an NH unit may be replaced by alkyl, and any hydrogen atom of a CH₂ unit of CH₂CH₂, CH₂CH₂CH₂ or CH₂ may be replaced by hydroxy;
X, independently for each occurrence, represents S, O or CH=CH, preferably S or CH=CH, wherein any hydrogen atom of a CH unit may be replaced by alkyl;
Y, independently for each occurrence, represents H or CH₂O(CO)R₇;
R₇, independently for each occurrence, represents H or substituted or unsubstituted alkyl, alkoxy, aminoalkyl, alkylaminoalkyl, heterocyclylalkyl, arylalkyl, or heterocyclylalkoxy;
Z represents H or R₃(CO);
R₁ and R₂ each independently represent H, alkyl, alkoxy or hydroxy;
R₃, independently for each occurrence, represents substituted or unsubstituted alkyl, hydroxyalkyl, aminoalkyl, acylaminoalkyl, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, heteroaryloxyalkyl or C(R₈)(R₉)(R₁₀), N(R₄)(R₅) or OR₆, wherein any free hydroxyl group may be acylated to form C(O)R₇;
R₄ and R₅ each independently represent H or substituted or unsubstituted alkyl, hydroxyalkyl, acyl, aminoalkyl, acylaminoalkyl, alkenyl, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, wherein any free hydroxyl group may be acylated to form C(O)R₇;
R₆, independently for each occurrence, represents substituted or unsubstituted alkyl, hydroxyalkyl, aminoalkyl, acylaminoalkyl, alkenyl, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, wherein any free hydroxyl group may be acylated to form C(O)R₇; and
R₈, R₉ and R₁₀ each independently represent H or substituted or unsubstituted alkyl, hydroxy, hydroxyalkyl, amino, acylamino, aminoalkyl, acylaminoalkyl, alkoxycarbonyl, alkoxycarbonylamino, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, or R₈ and R₉ together with the carbon to which they are attached, form a carbocyclic or heterocyclic ring system, wherein any free hydroxyl group may be acylated to form C(O)R₇;
wherein the compound is administered with a meal.
In some embodiments, at least two of R₈, R₉ and R₁₀ are not H.

In certain embodiments wherein alkyl, hydroxyalkyl, amino, acylamino, aminoalkyl, acylaminoalkyl, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl are substituted, they are substituted with one or more substituents selected from substituted or unsubstituted alkyl, such as perfluoroalkyl (*e.g.,* trifluoromethyl), alkenyl, alkoxy, alkoxyalkyl, aryl, aralkyl, arylalkoxy, aryloxy, aryloxyalkyl, hydroxyl, halo, alkoxy, such as perfluoroalkoxy (*e.g.,* trifluoromethoxy), alkoxyalkoxy, hydroxyalkyl, hydroxyalkylamino, hydroxyalkoxy, amino, aminoalkyl, alkylamino, aminoalkylalkoxy, aminoalkoxy, acylamino, acylaminoalkyl, such as perfluoro acylaminoalkyl (*e.g.,* trifluoromethylacylaminoalkyl), acyloxy, cycloalkyl, cycloalkylalkyl, cycloalkylalkoxy, heterocyclyl, heterocyclylalkyl, heterocyclyloxy, heterocyclylalkoxy, heteroaryl, heteroarylalkyl, heteroarylalkoxy, heteroaryloxy, heteroaryloxyalkyl, heterocyclylaminoalkyl, heterocyclylaminoalkoxy, amido, amidoalkyl, amidine, imine, oxo, carbonyl (such as carboxyl, alkoxycarbonyl, formyl, or acyl, including perfluoroacyl (*e.g.,* C(O)CF₃)), carbonylalkyl (such as carboxyalkyl, alkoxycarbonylalkyl, formylalkyl, or acylalkyl, including perfluoroacylalkyl (e*.g.,* -alkylC(O)CF₃)), carbamate, carbamatealkyl, urea, ureaalkyl, sulfate, sulfonate, sulfamoyl, sulfone, sulfonamide, sulfonamidealkyl, cyano, nitro, azido, sulfhydryl, alkylthio, thiocarbonyl (such as thioester, thioacetate, or thioformate), phosphoryl, phosphate, phosphonate or phosphinate.

In certain embodiments, L represents CH₂SCH₂, CH₂CH₂, CH₂CH₂CH₂, CH₂, CH₂S, SCH₂, or CH₂NHCH₂, wherein any hydrogen atom of a CH₂ unit may be replaced by alkyl or alkoxy, and any hydrogen atom of a CH₂ unit of CH₂CH₂, CH₂CH₂CH₂ or CH₂ may be replaced by hydroxyl. In certain embodiments, L represents CH₂SCH₂, CH₂CH₂, CH₂S or SCH₂. In certain embodiments, L represents CH₂CH₂. In certain embodiments, L is not CH₂SCH₂.

In certain embodiments, Y represents H.

In certain embodiments, X represents S or CH=CH. In certain embodiments, one or both X represents CH=CH. In certain embodiments, each X represents S. In certain embodiments, one X represents S and the other X represents CH=CH.

In certain embodiments, Z represents R₃(CO). In certain embodiments wherein Z is R₃(CO), each occurrence of R₃ is not identical (*e.g.,* the compound of formula I is not symmetrical).

In certain embodiments, R₁ and R₂ each represent H.

In certain embodiments, R₃ represents arylalkyl, heteroarylalkyl, cycloalkyl or heterocycloalkyl. In certain embodiments, R₃ represents C(R₈)(R₉)(R₁₀), wherein R₈ represents aryl, arylalkyl, heteroaryl or heteroaralkyl, such as aryl, arylalkyl or heteroaryl, R₉ represents H, and R₁₀ represents hydroxy, hydroxyalkyl, alkoxy or alkoxyalkyl, such as hydroxy, hydroxyalkyl or alkoxy.

In certain embodiments, L represents CH₂SCH₂, CH₂CH₂, CH₂S or SCH₂, such as CH₂CH₂, CH₂S or SCH₂, Y represents H, X represents S, Z represents R₃(CO), R₁ and R₂ each represent H, and each R₃ represents arylalkyl, heteroarylalkyl, cycloalkyl or heterocycloalkyl. In certain such embodiments, each occurrence of R₃ is identical.

In certain embodiments, L represents CH₂SCH₂, CH₂CH₂, CH₂S or SCH₂, Y represents H, X represents S, Z represents R₃(CO), R₁ and R₂ each represent H, and each R₃ represents C(R₈)(R₉)(R₁₀), wherein R₈ represents aryl, arylalkyl, heteroaryl or heteroaralkyl, such as aryl, arylalkyl or heteroaryl, R₉ represents H, and R₁₀ represents hydroxy, hydroxyalkyl, alkoxy or alkoxyalkyl, such as hydroxy, hydroxyalkyl or alkoxy. In certain such embodiments, each occurrence of R₃ is identical.

In certain embodiments, L represents CH₂CH₂, Y represents H, X represents S or CH=CH, Z represents R₃(CO), R₁ and R₂ each represent H, and each R₃ represents substituted or unsubstituted arylalkyl, heteroarylalkyl, cycloalkyl or heterocycloalkyl. In certain such embodiments, each X represents S. In other embodiments, one or both occurrences of X represents CH=CH, such as one occurrence of X represents S and the other occurrence of X represents CH=CH. In certain embodiments of the foregoing, each occurrence of R₃ is identical. In other embodiments of the foregoing wherein one occurrence of X represents S and the other occurrence of X represents CH=CH, the two occurrences of R₃ are not identical.

In certain embodiments, L represents CH₂CH₂, Y represents H, X represents S, Z represents R₃(CO), R₁ and R₂ each represent H, and each R₃ represents C(R₉)(R₉)(R₁₀), wherein R₈ represents aryl, arylalkyl or heteroaryl, R₉ represents H, and R₁₀ represents hydroxy, hydroxyalkyl or alkoxy. In certain such embodiments, R₈ represents aryl and R₁₀ represents hydroxyalkyl. In certain such embodiments, each occurrence of R₃ is identical.

In certain embodiments wherein L represents CH₂, CH₂CH₂CH₂ or CH₂CH₂, X represents O, and Z represents R₃(CO), both R₃ groups are not alkyl, such as methyl, or C(R₉)(R₉)(R₁₀), wherein R₈, R₉ and R₁₀ are each independently hydrogen or alkyl.

In certain embodiments wherein L represents CH₂CH₂, X represents S, and Z represents R₃(CO), both R₃ groups are not phenyl or heteroaryl, such as 2-furyl.

In certain embodiments wherein L represents CH₂CH₂, X represents O, and Z represents R₃(CO), both R₃ groups are not N(R₄)(R₅) wherein R₄ is aryl, such as phenyl, and R₅ is H.

In certain embodiments wherein L represents CH₂SCH₂, X represents S, and Z represents R₃(CO), both R₃ groups are not aryl, such as optionally substituted phenyl, aralkyl, such as benzyl, heteroaryl, such as 2-furyl, 2-thienyl or 1,2,4-trizole, substituted or unsubstituted alkyl, such as methyl, chloromethyl, dichloromethyl, n-propyl, n-butyl, t-butyl or hexyl, heterocyclyl, such as pyrimidine-2,4(1H,3H)-dione, or alkoxy, such as methoxy, pentyloxy or ethoxy.

In certain embodiments wherein L represents CH₂SCH₂, X represents S, and Z represents R₃(CO), both R₃ groups are not optionally substituted phenyl, aralkyl, heteroaryl, substituted or unsubstituted alkyl, or alkoxy.

In certain embodiments wherein L represents CH₂SCH₂, X represents S, and Z represents R₃(CO), both R₃ groups are not N(R₄)(R₅) wherein R₄ is aryl, such as substituted or unsubstituted phenyl (*e.g*., phenyl, 3-tolyl, 4-tolyl, 4-bromophenyl or 4-nitrophenyl), and R₅ is H.

In certain embodiments wherein L represents CH₂CH₂CH₂, X represents S, and Z represents R₃(CO), both R₃ groups are not alkyl, such as methyl, ethyl, or propyl, cycloalkyl, such as cyclohexyl, or C(R₉)(R₉)(R₁₀), wherein any of R₈, R₉ and R₁₀ together with the C to which they are attached, form any of the foregoing.

In certain embodiments, the compound is not one of the following: 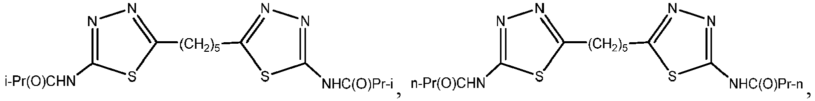 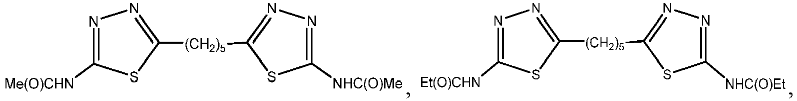 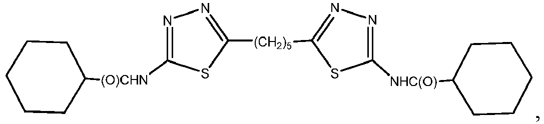 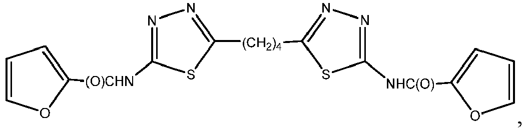 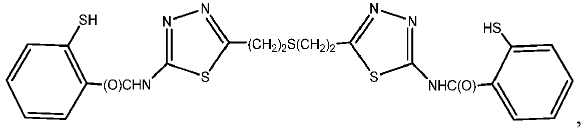 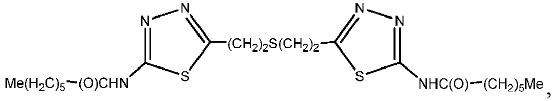 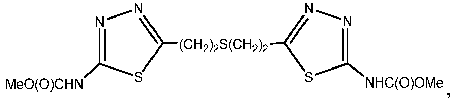 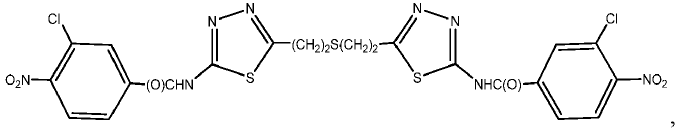 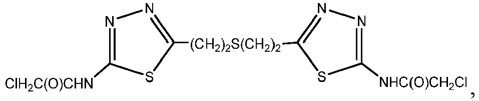 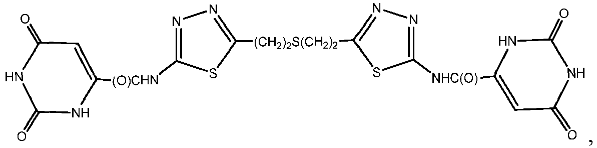 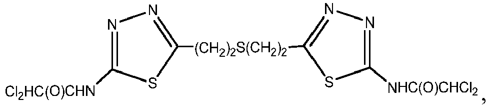 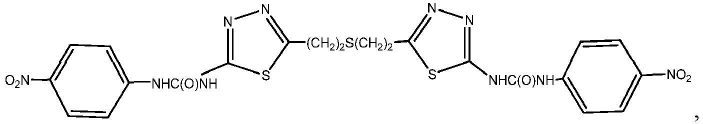 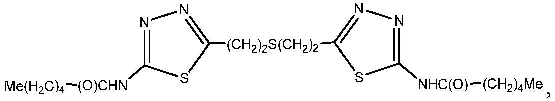 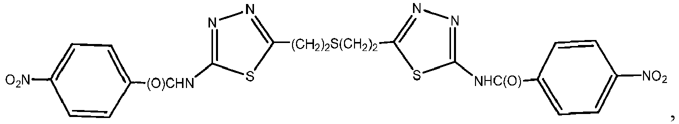 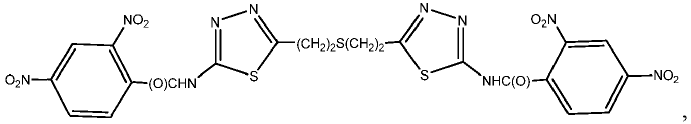 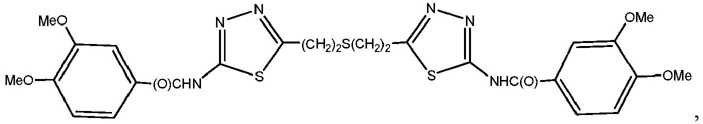 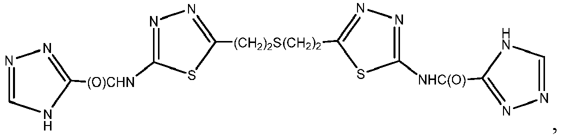 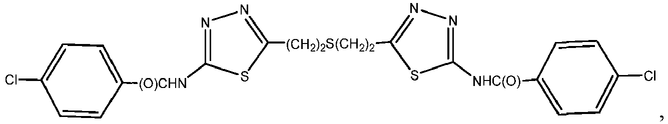 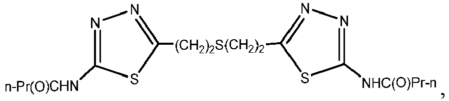 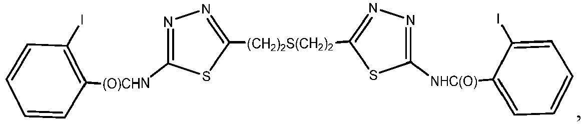 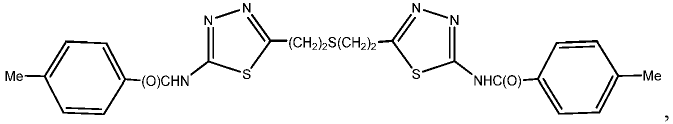 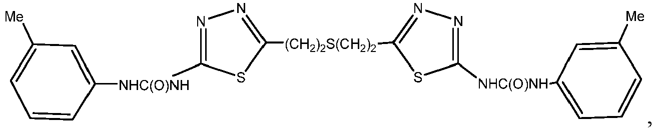 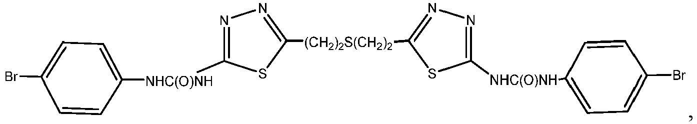 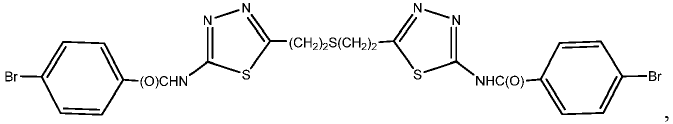 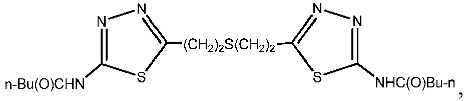 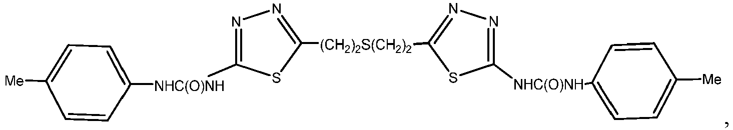 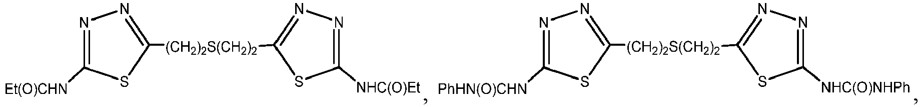 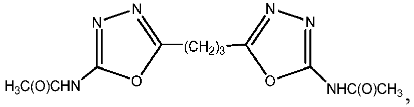 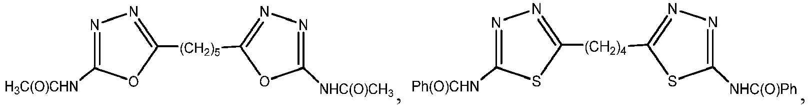 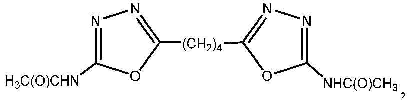 or

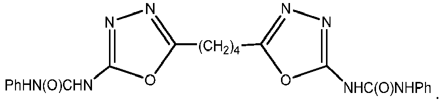

The present invention further provides the claimed compounds for use in methods of treating cancer, comprising orally administering a compound of formula la, 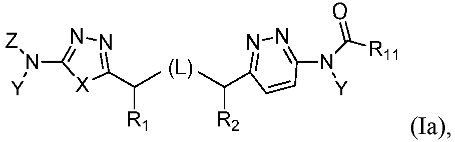 or a pharmaceutically acceptable salt thereof, wherein:
L represents CH₂SCH₂, CH₂CH₂, CH₂CH₂CH₂, CH₂, CH₂S, SCH₂, CH₂NHCH₂, CH=CH, or 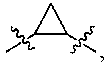 preferably CH₂CH₂, wherein any hydrogen atom of a CH or CH₂ unit may be replaced by alkyl or alkoxy, any hydrogen of an NH unit may be replaced by alkyl, and any hydrogen atom of a CH₂ unit of CH₂CH₂, CH₂CH₂CH₂ or CH₂ may be replaced by hydroxy;
X represents S, O or CH=CH, preferably S or CH=CH, wherein any hydrogen atom of a CH unit may be replaced by alkyl;
Y, independently for each occurrence, represents H or CH₂O(CO)R₇;
R₇, independently for each occurrence, represents H or substituted or unsubstituted alkyl, alkoxy, aminoalkyl, alkylaminoalkyl, heterocyclylalkyl, arylalkyl, or heterocyclylalkoxy;
Z represents H or R₃(CO);
R₁ and R₂ each independently represent H, alkyl, alkoxy or hydroxy, preferably H;
R₃ represents substituted or unsubstituted alkyl, hydroxyalkyl, aminoalkyl, acylaminoalkyl, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, heteroaryloxyalkyl or C(R₈)(R₉)(R₁₀), N(R₄)(R₅) or OR₆, wherein any free hydroxyl group may be acylated to form C(O)R₇;
R₄ and R₅ each independently represent H or substituted or unsubstituted alkyl, hydroxyalkyl, acyl, aminoalkyl, acylaminoalkyl, alkenyl, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, wherein any free hydroxyl group may be acylated to form C(O)R₇;
R₆, independently for each occurrence, represents substituted or unsubstituted alkyl, hydroxyalkyl, aminoalkyl, acylaminoalkyl, alkenyl, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, wherein any free hydroxyl group may be acylated to form C(O)R₇; and
R₈, R₉ and R₁₀ each independently represent H or substituted or unsubstituted alkyl, hydroxy, hydroxyalkyl, amino, acylamino, aminoalkyl, acylaminoalkyl, alkoxycarbonyl, alkoxycarbonylamino, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, or R₈ and R₉ together with the carbon to which they are attached, form a carbocyclic or heterocyclic ring system, wherein any free hydroxyl group may be acylated to form C(O)R₇, and wherein at least two of R₈, R₉ and R₁₀ are not H;
R₁₁ represents substituted or unsubstituted aryl, arylalkyl, aryloxy, aryloxyalkyl, heteroaryl, heteroarylalkyl, heteroaryloxy, or heteroaryloxyalkyl, or C(R₁₂)(R₁₃)(R₁₄), N(R₄)(R₁₄) or OR₁₄, wherein any free hydroxyl group may be acylated to form C(O)R₇;
R₁₂ and R₁₃ each independently respresent H or substituted or unsubstituted alkyl, hydroxy, hydroxyalkyl, amino, acylamino, aminoalkyl, acylaminoalkyl, alkoxycarbonyl, alkoxycarbonylamino, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, wherein any free hydroxyl group may be acylated to form C(O)R₇, and wherein both of R₁₂ and R₁₃ are not H; and
R₁₄ represents substituted or unsubstituted aryl, arylalkyl, aryloxy, aryloxyalkyl, heteroaryl, heteroarylalkyl, heteroaryloxy, or heteroaryloxyalkyl;
wherein the compound is administered with a meal.

In certain embodiments wherein alkyl, hydroxyalkyl, amino, acylamino, aminoalkyl, acylaminoalkyl, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl are substituted, they are substituted with one or more substituents selected from substituted or unsubstituted alkyl, such as perfluoroalkyl (*e.g.,* trifluoromethyl), alkenyl, alkoxy, alkoxyalkyl, aryl, aralkyl, arylalkoxy, aryloxy, aryloxyalkyl, hydroxyl, halo, alkoxy, such as perfluoroalkoxy (*e.g.,* trifluoromethylalkoxy), alkoxyalkoxy, hydroxyalkyl, hydroxyalkylamino, hydroxyalkoxy, amino, aminoalkyl, alkylamino, aminoalkylalkoxy, aminoalkoxy, acylamino, acylaminoalkyl, such as perfluoro acylaminoalkyl (*e.g.,* trifluoromethylacylaminoalkyl), acyloxy, cycloalkyl, cycloalkylalkyl, cycloalkylalkoxy, heterocyclyl, heterocyclylalkyl, heterocyclyloxy, heterocyclylalkoxy, heteroaryl, heteroarylalkyl, heteroarylalkoxy, heteroaryloxy, heteroaryloxyalkyl, heterocyclylaminoalkyl, heterocyclylaminoalkoxy, amido, amidoalkyl, amidine, imine, oxo, carbonyl (such as carboxyl, alkoxycarbonyl, formyl, or acyl, including perfluoroacyl (*e.g.,* C(O)CF₃)), carbonylalkyl (such as carboxyalkyl, alkoxycarbonylalkyl, formylalkyl, or acylalkyl, including perfluoroacylalkyl (*e.g.,* -alkylC(O)CF₃)), carbamate, carbamatealkyl, urea, ureaalkyl, sulfate, sulfonate, sulfamoyl, sulfone, sulfonamide, sulfonamidealkyl, cyano, nitro, azido, sulfhydryl, alkylthio, thiocarbonyl (such as thioester, thioacetate, or thioformate), phosphoryl, phosphate, phosphonate or phosphinate.

In certain embodiments, R₁₁ represents substituted or unsubstituted arylalkyl, such as substituted or unsubstituted benzyl.

In certain embodiments, L represents CH₂SCH₂, CH₂CH₂, CH₂CH₂CH₂, CH₂, CH₂S, SCH₂, or CH₂NHCH₂, wherein any hydrogen atom of a CH₂ unit may be replaced by alkyl or alkoxy, and any hydrogen atom of a CH₂ unit of CH₂CH₂, CH₂CH₂CH₂ or CH₂ may be replaced by hydroxyl. In certain embodiments, L represents CH₂SCH₂, CH₂CH₂, CH₂S or SCH₂, preferably CH₂CH₂. In certain embodiments, L is not CH₂SCH₂.

In certain embodiments, each Y represents H. In other embodiments, at least one Y is CH₂O(CO)R₇.

In certain embodiments, X represents S or CH=CH. In certain embodiments, X represents S.

In certain embodiments, R₁ and R₂ each represent H.

In certain embodiments, Z represents R₃(CO). In certain embodiments wherein Z is R₃(CO), R₃ and R₁₁ are not identical (*e.g.,* the compound of formula I is not symmetrical).

In certain embodiments, Z represents R₃(CO) and R₃ represents arylalkyl, heteroarylalkyl, cycloalkyl or heterocycloalkyl. In certain embodiments, Z represents R₃(CO) and R₃ represents C(R₈)(R₉)(R₁₀), wherein R₈ represents aryl, arylalkyl, heteroaryl or heteroaralkyl, such as aryl, arylalkyl or heteroaryl, R₉ represents H, and R₁₀ represents hydroxy, hydroxyalkyl, alkoxy or alkoxyalkyl, such as hydroxy, hydroxyalkyl or alkoxy. In certain embodiments, Z represents R₃(CO) and R₃ represents heteroarylalkyl.

In certain embodiments, L represents CH₂SCH₂, CH₂CH₂, CH₂S or SCH₂, such as CH₂CH₂, Y represents H, X represents S, Z represents R₃(CO), R₁ and R₂ each represent H, R₃ represents arylalkyl, heteroarylalkyl, cycloalkyl or heterocycloalkyl, and R₁₁ represents arylalkyl. In certain such embodiments, R₃ represents heteroarylalkyl.

In certain embodiments, L represents CH₂SCH₂, CH₂CH₂, CH₂S or SCH₂, such as CH₂CH₂, Y represents H, X represents S, Z represents R₃(CO), R₁ and R₂ each represent H, and each R₃ represents C(R₈)(R₉)(R₁₀), wherein R₈ represents aryl, arylalkyl, heteroaryl or heteroaralkyl, such as aryl, arylalkyl or heteroaryl, R₉ represents H, and R₁₀ represents hydroxy, hydroxyalkyl, alkoxy or alkoxyalkyl, such as hydroxy, hydroxyalkyl or alkoxy, and R₁₁ represents arylalkyl. In certain such embodiments, R₈ represents heteroaryl.

In certain embodiments, L represents CH₂CH₂, Y represents H, X represents S or CH=CH, such as S, Z represents R₃(CO), R₁ and R₂ each represent H, R₃ represents substituted or unsubstituted arylalkyl, heteroarylalkyl, cycloalkyl or heterocycloalkyl, and R₁₁ represents arylalkyl. In certain such embodiments, R₃ represents heteroarylalkyl.

In certain embodiments, L represents CH₂CH₂, Y represents H, X represents S, Z represents R₃(CO), R₁ and R₂ each represent H, R₃ represents C(R₈)(R₉)(R₁₀), wherein R₈ represents aryl, arylalkyl or heteroaryl, R₉ represents H, and R₁₀ represents hydroxy, hydroxyalkyl or alkoxy, and R₁₁ represents arylalkyl. In certain such embodiments, R₈ represents aryl and R₁₀ represents hydroxyalkyl. In certain other embodiments, R₈ represents heteroaryl.

In certain embodiments, the compound is selected from any one of the compounds disclosed in Table 1. Preferably, the compound is selected from compound 1, 2, 6, 7, 8, 11, 13, 14, 15, 16, 17, 18, 19, 20, 21, 24, 25, 26, 27, 28, 29, 30, 31, 32, 33, 35, 36, 38, 39, 40, 41, 43, 44, 47, 48, 50, 51, 52, 54, 55, 58, 63, 64, 65, 67, 68, 69, 70, 71, 72, 73, 77, 78, 79, 80, 81, 82, 83, 84, 85, 86, 87, 88, 92, 93, 94, 95, 97, 99, 100, 102, 105, 107, 111, 112, 114, 115, 116, 117, 118, 120, 121, 122, 123, 126, 127, 133, 135, 136, 138, 140, 141, 143, 146, 147, 148, 152, 153, 155, 156, 157, 158, 159, 160, 161, 162, 163, 164, 165, 166, 168, 169, 170, 172, 173, 174, 175, 176, 177, 178, 179, 180, 181, 182, 185, 186, 187, 188, 189, 190, 193, 194, 195, 196, 197, 198, 199, 200, 201, 202, 203, 204, 205, 208, 210, 211, 213, 214, 216, 217, 219, 220, 226, 227, 228, 229, 231, 232, 234, 235, 236, 237, 239, 240, 241, 242, 243, 244, 245, 246, 247, 248, 249, 250, 251, 252, 255, 256, 257, 258, 259, 260, 261, 262, 263, 264, 265, 266, 267, 268, 269, 270, 271, 273, 274, 275, 276, 278, 279, 280, 281, 282, 283, 285, 286, 287, 288, 290, 291, 292, 293, 294, 295, 296, 297, 298, 299, 300, 302, 304, 1038, 306, 307, 308, 309, 310, 311, 313, 314, 315, 316, 317, 318, 319, 320, 321, 322, 323, 324, 325, 327, 329, 332, 333, 334, 335, 336, 337, 338, 339, 340, 341, 342, 343, 344, 345, 346, 527, 347, 348, 349, 350, 351, 352, 353, 354, 355, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 516, 517, 518, 519, 520, 521, 522, 523, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 638, 639, 640, 641, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 692, 693, 694, 695, 696, 697, 698, 699, 700, 701, 702, 703, 704, 705, 707, 708, or 709.

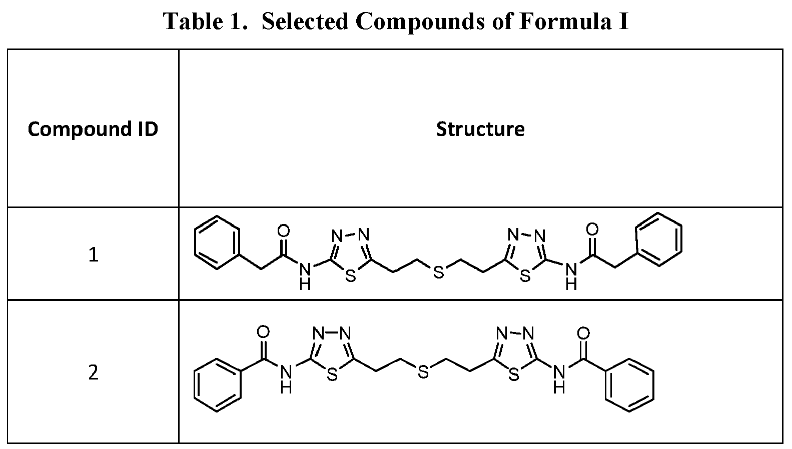

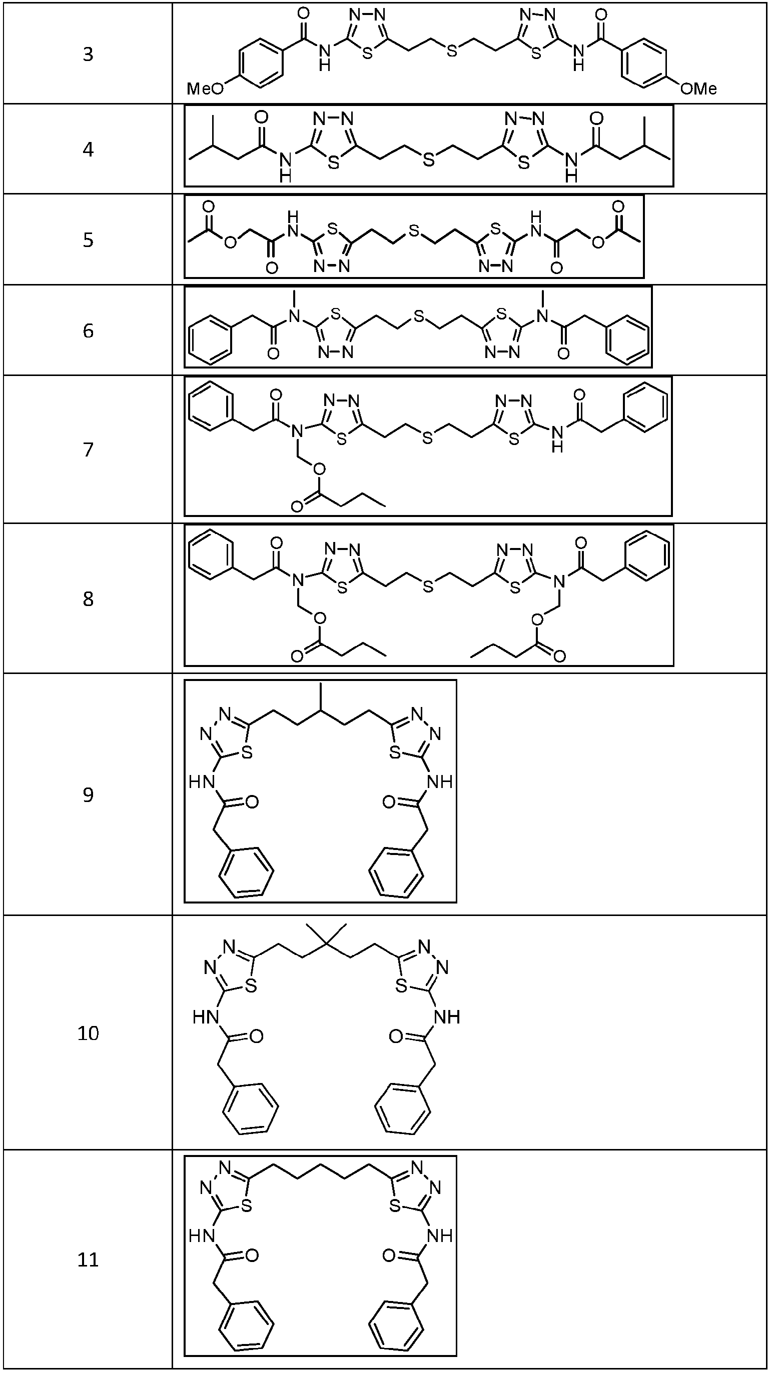

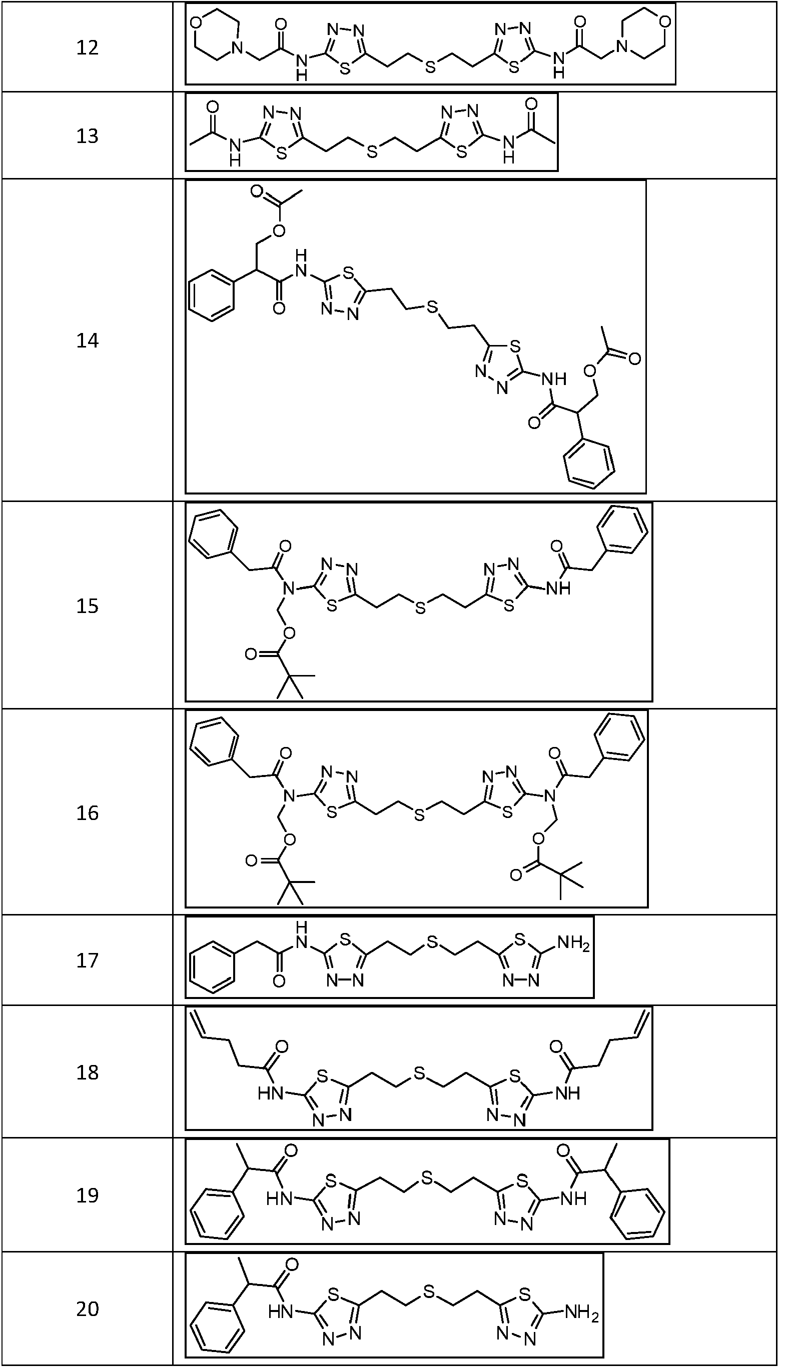

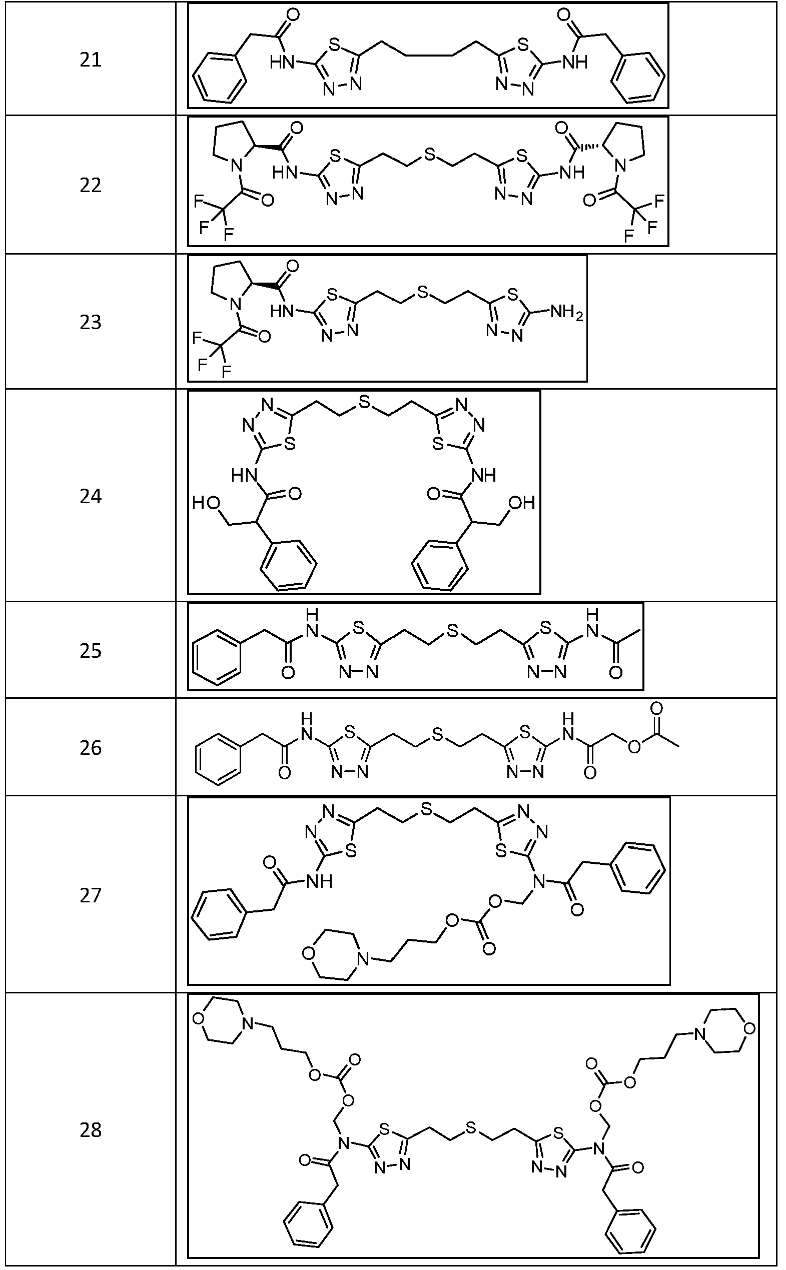

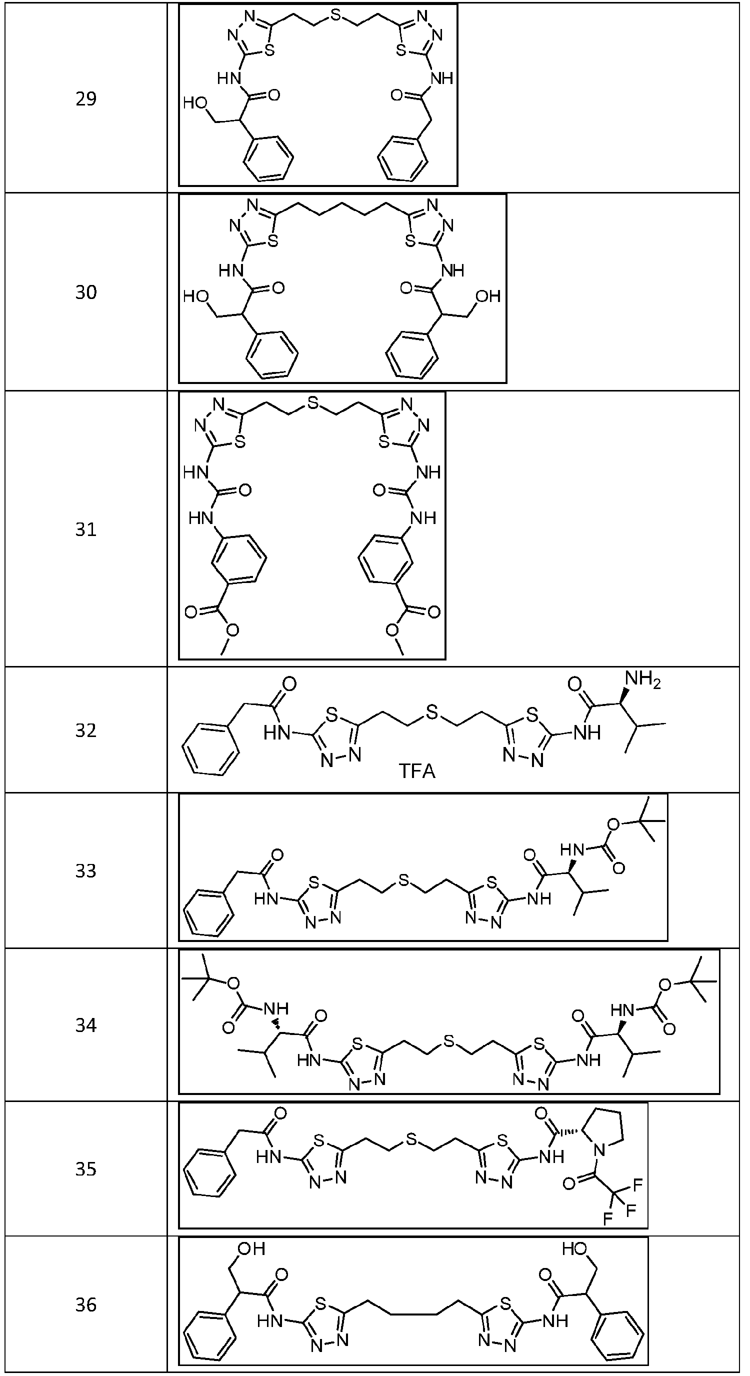

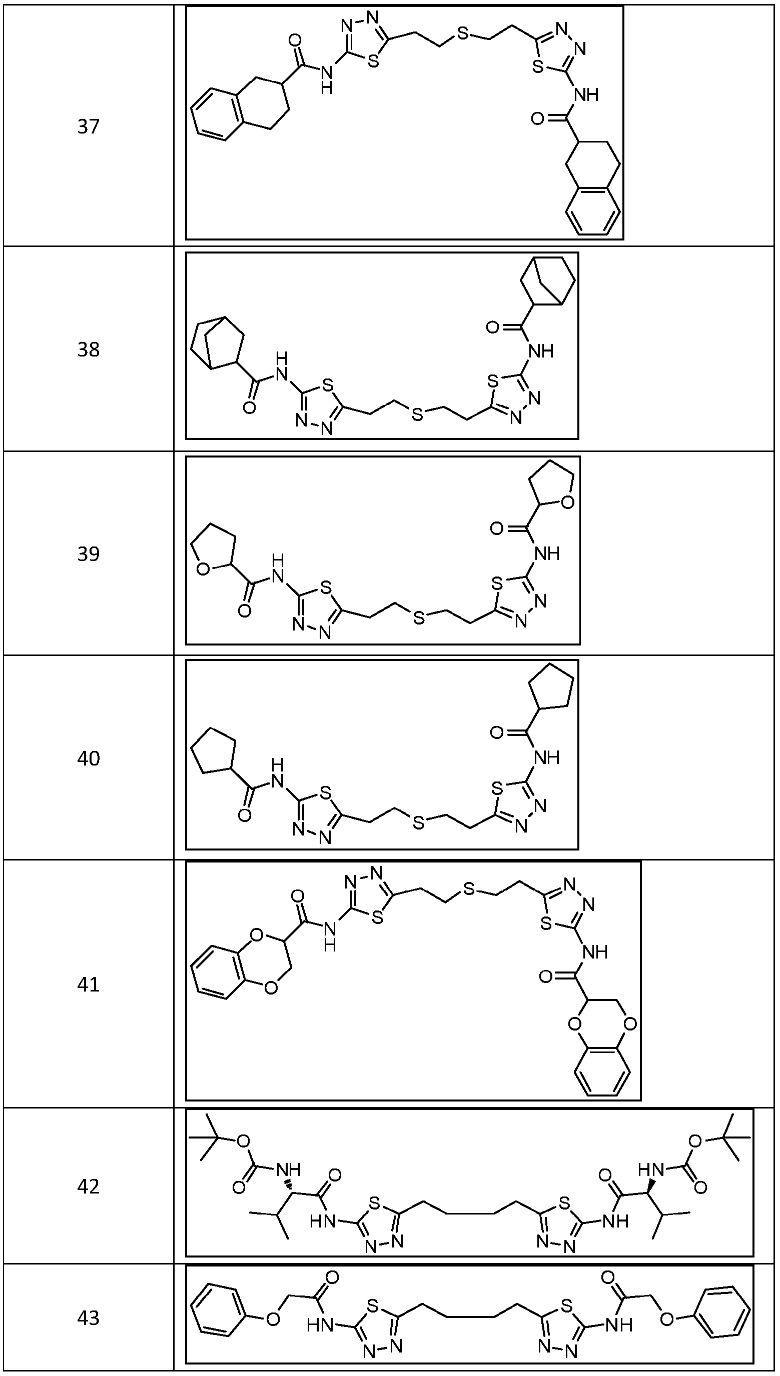

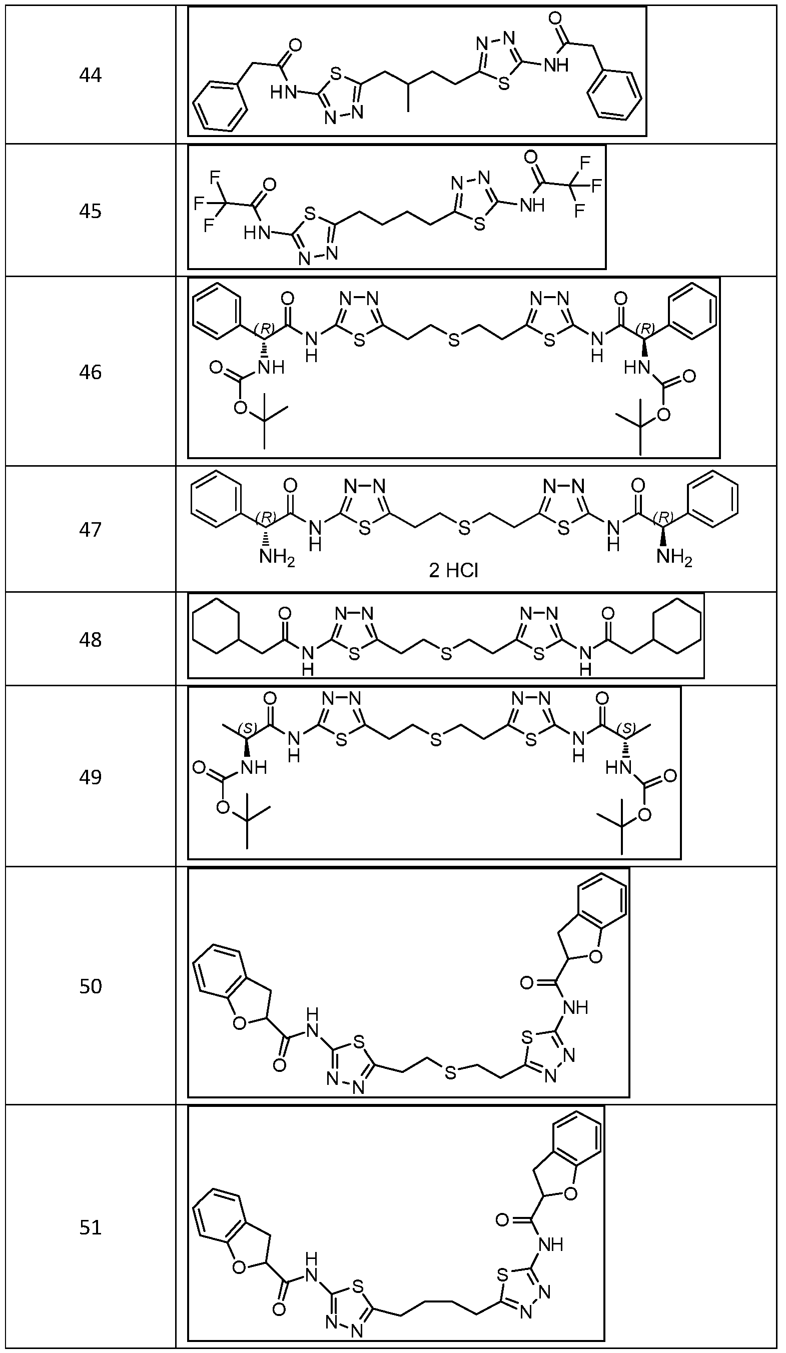

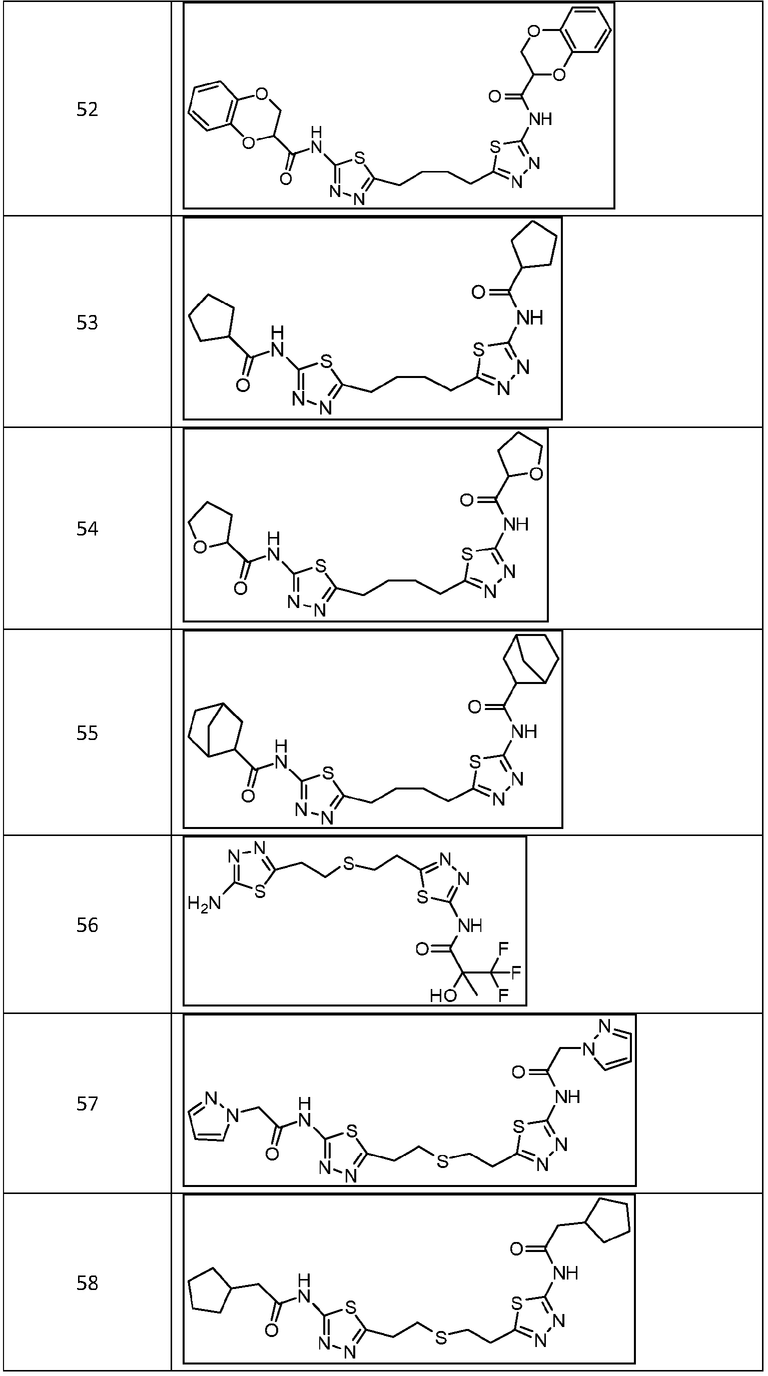

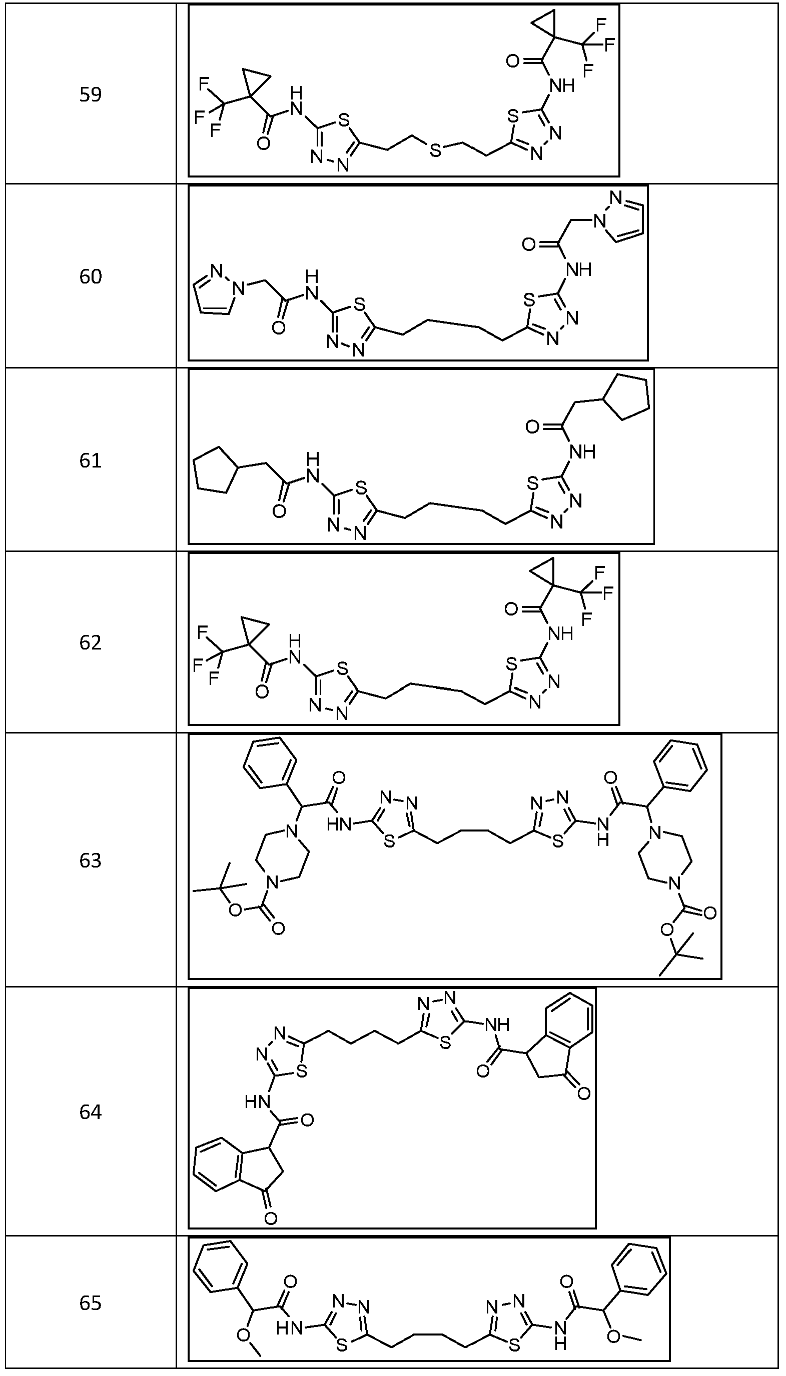

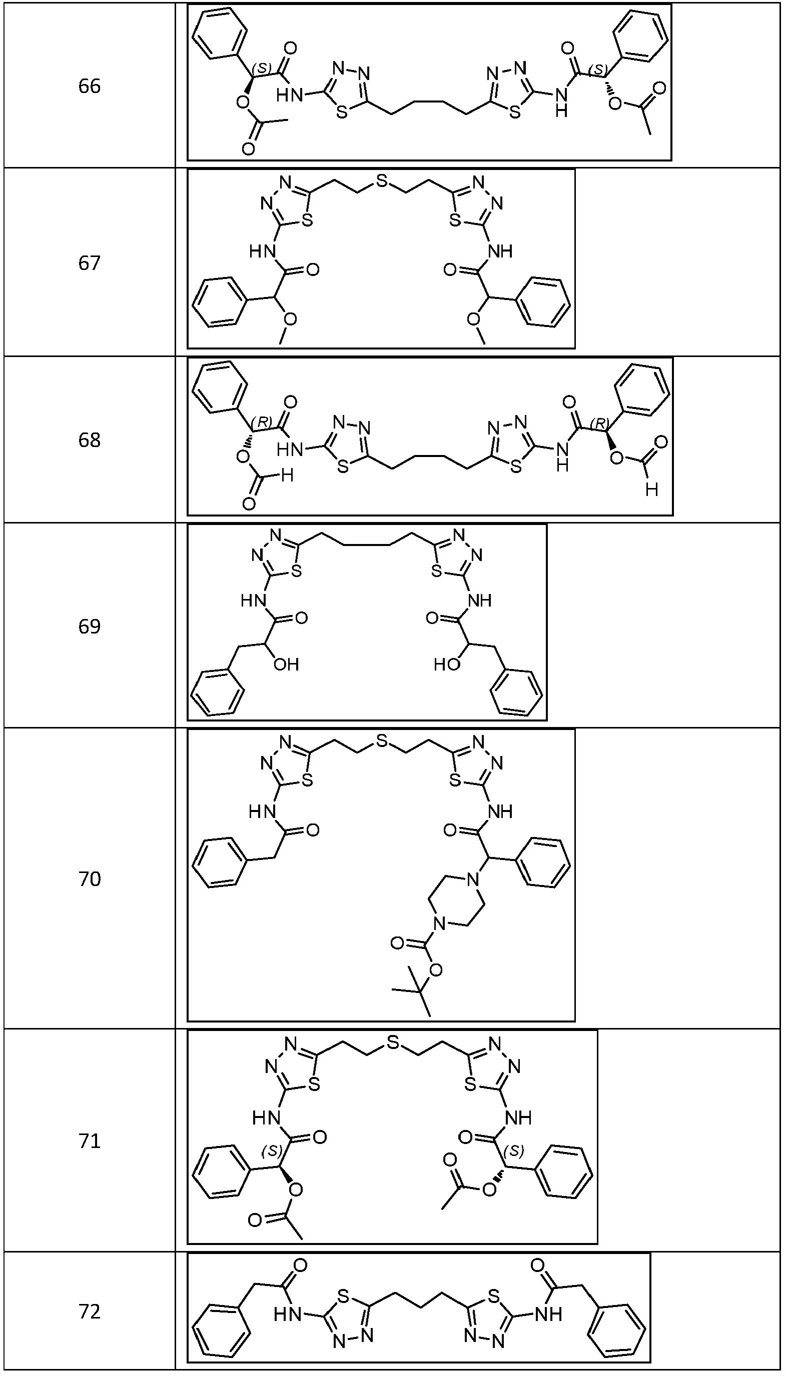

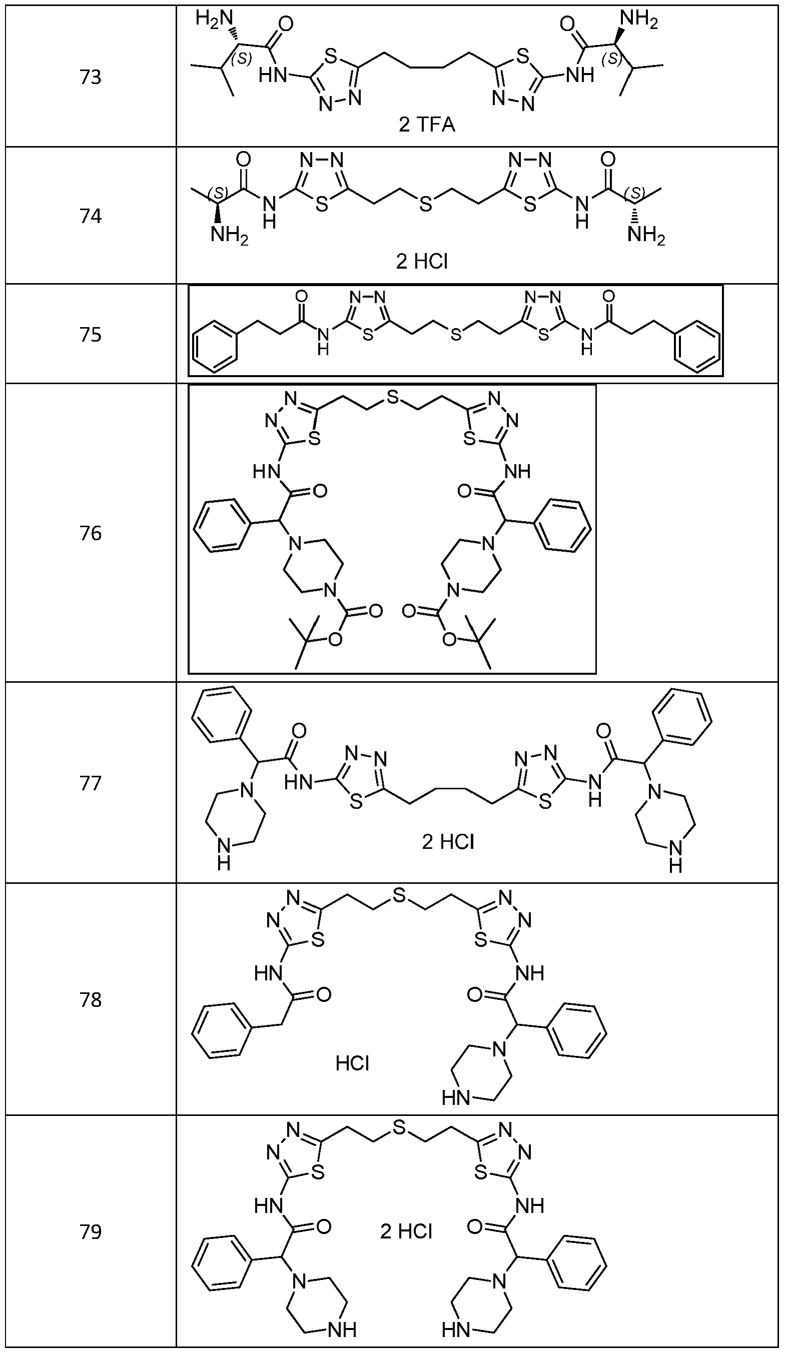

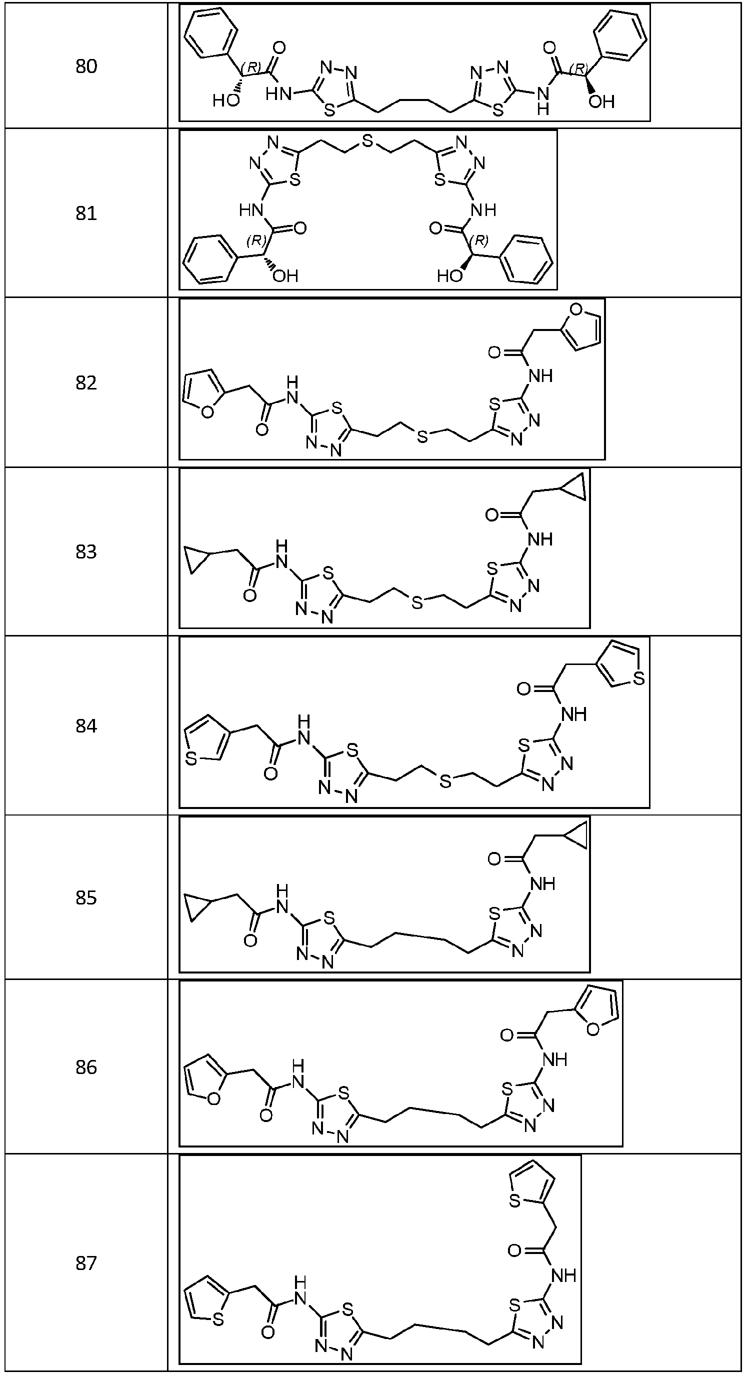

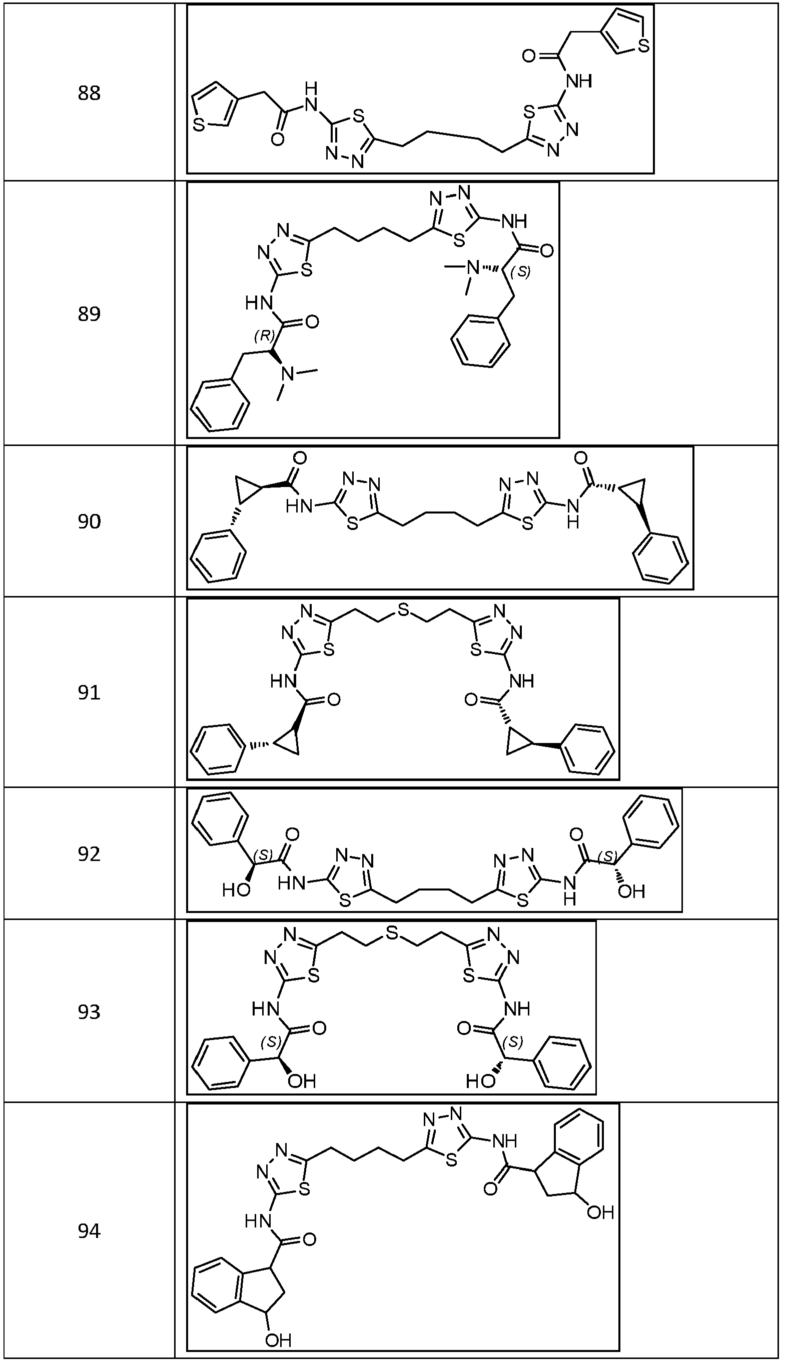

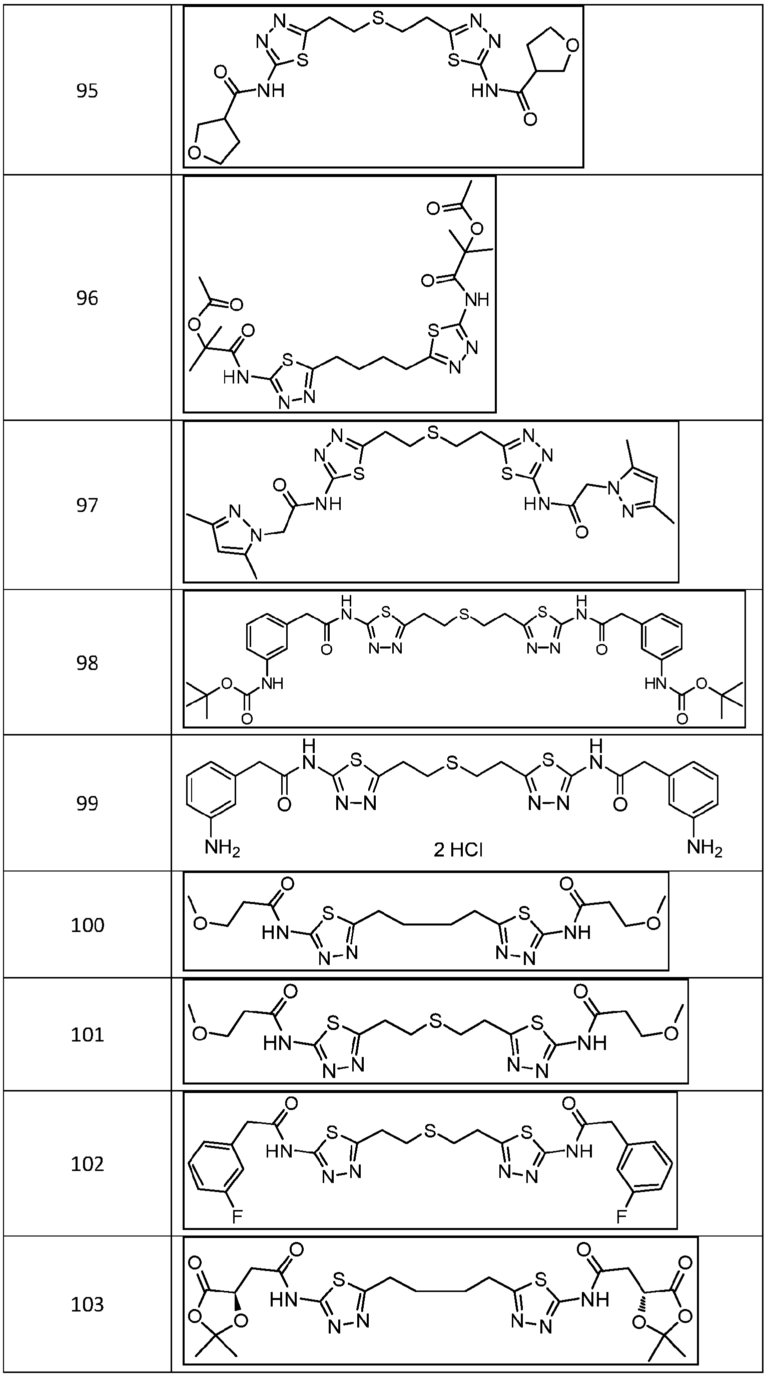

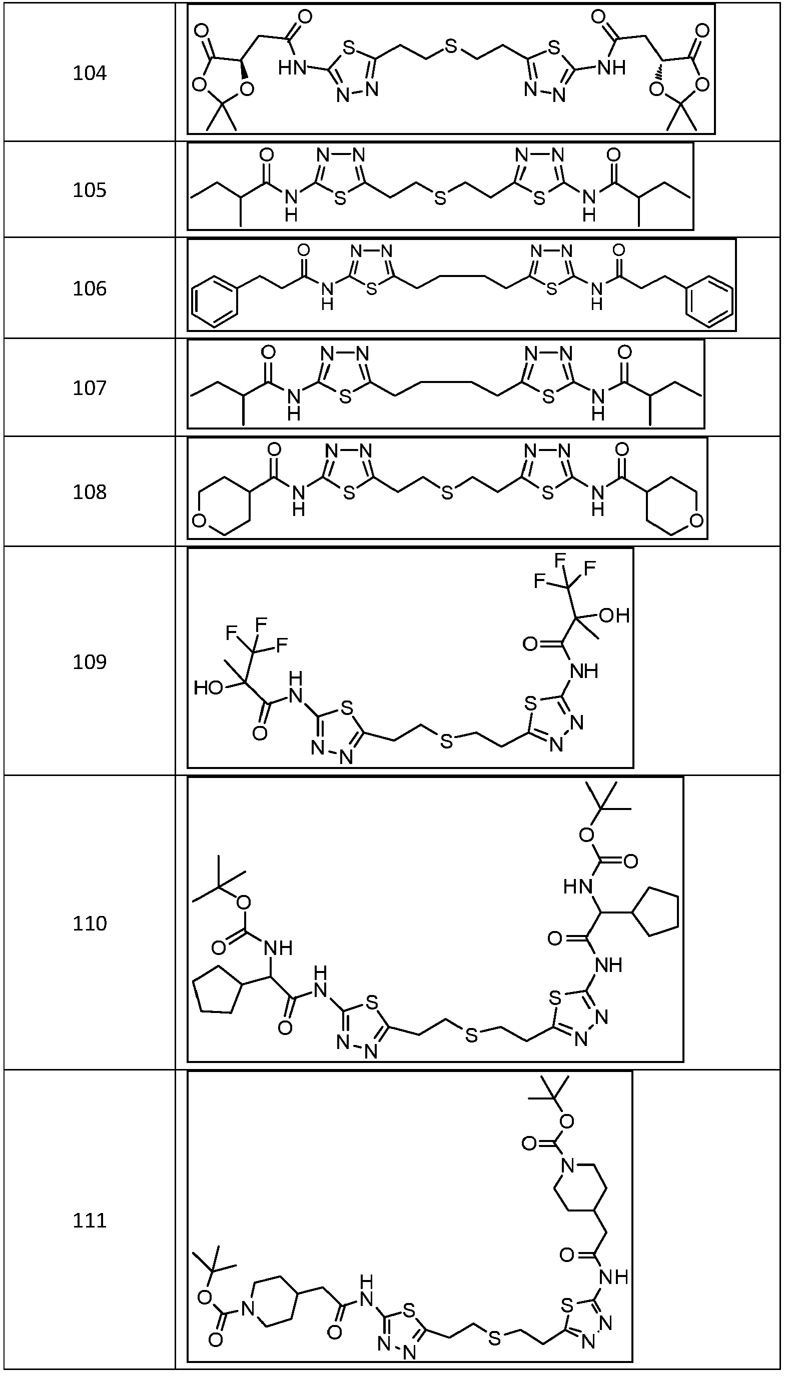

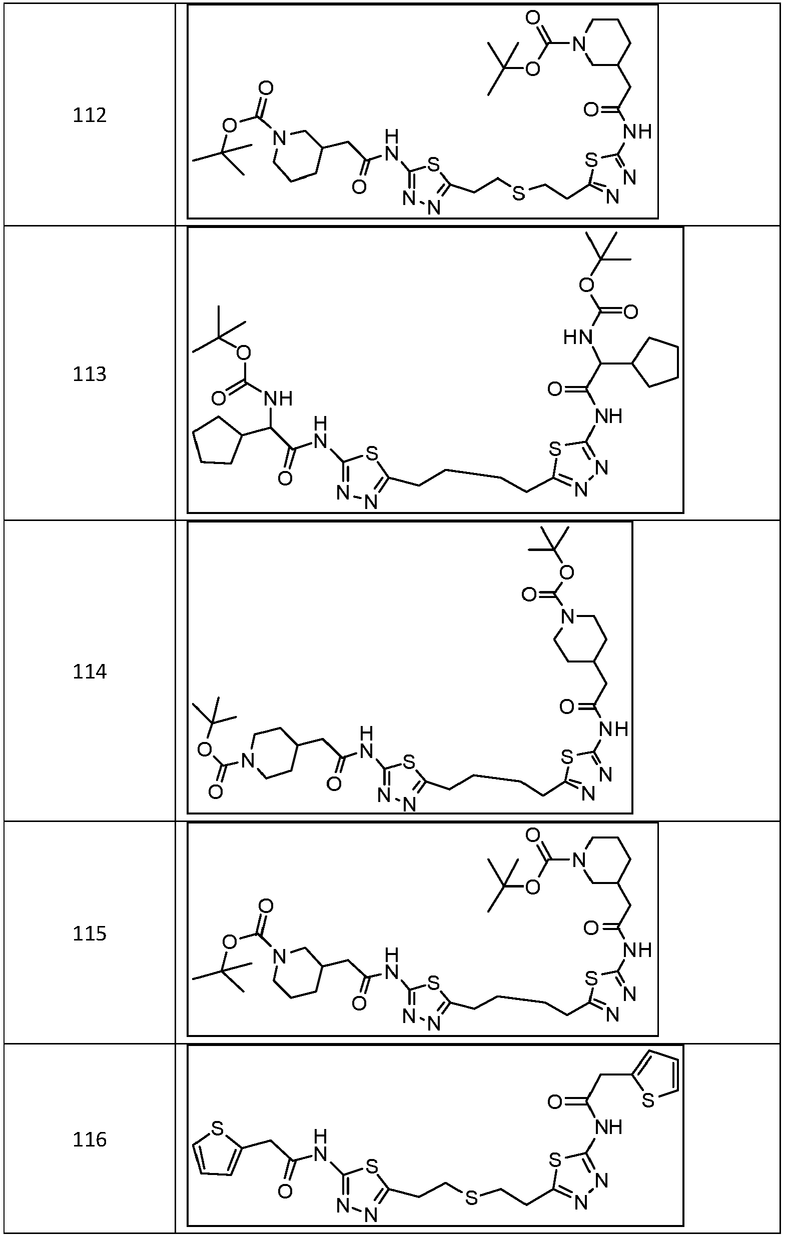

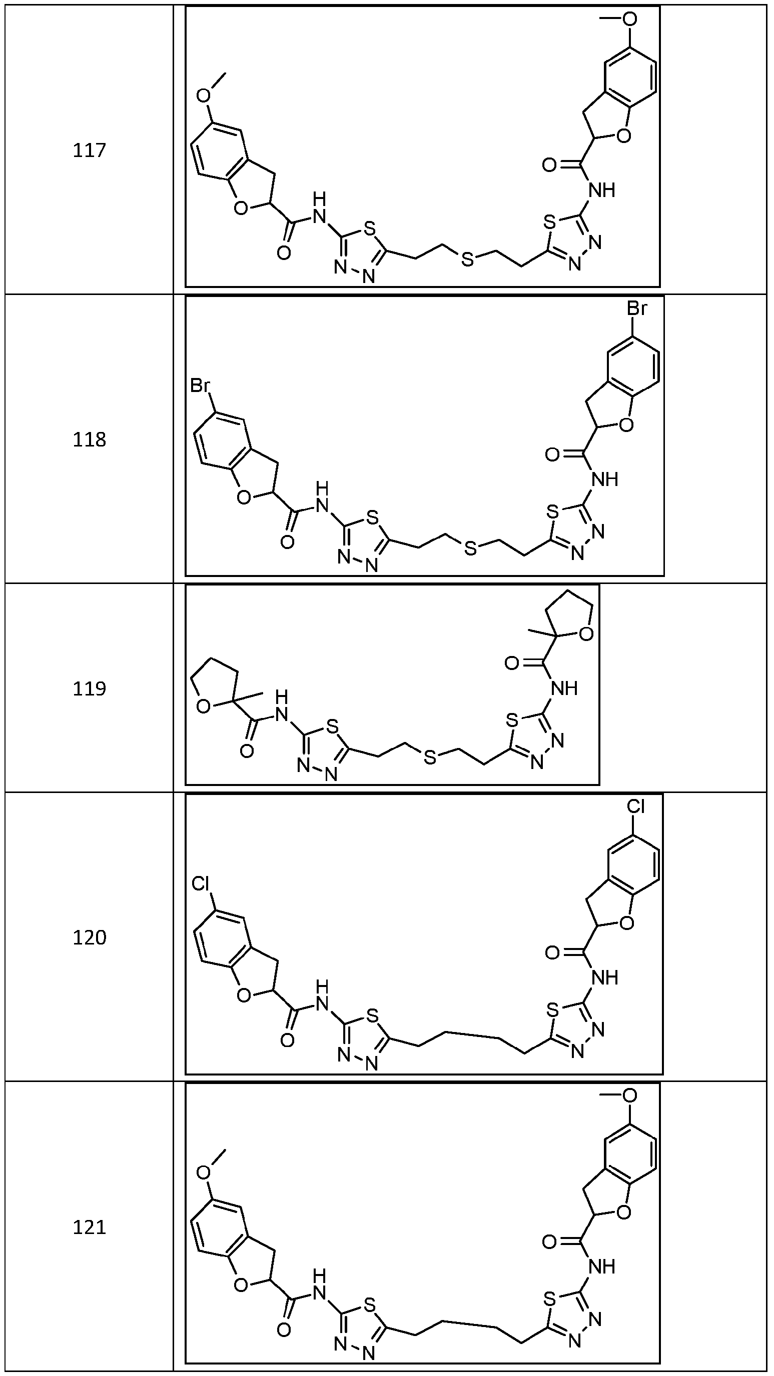

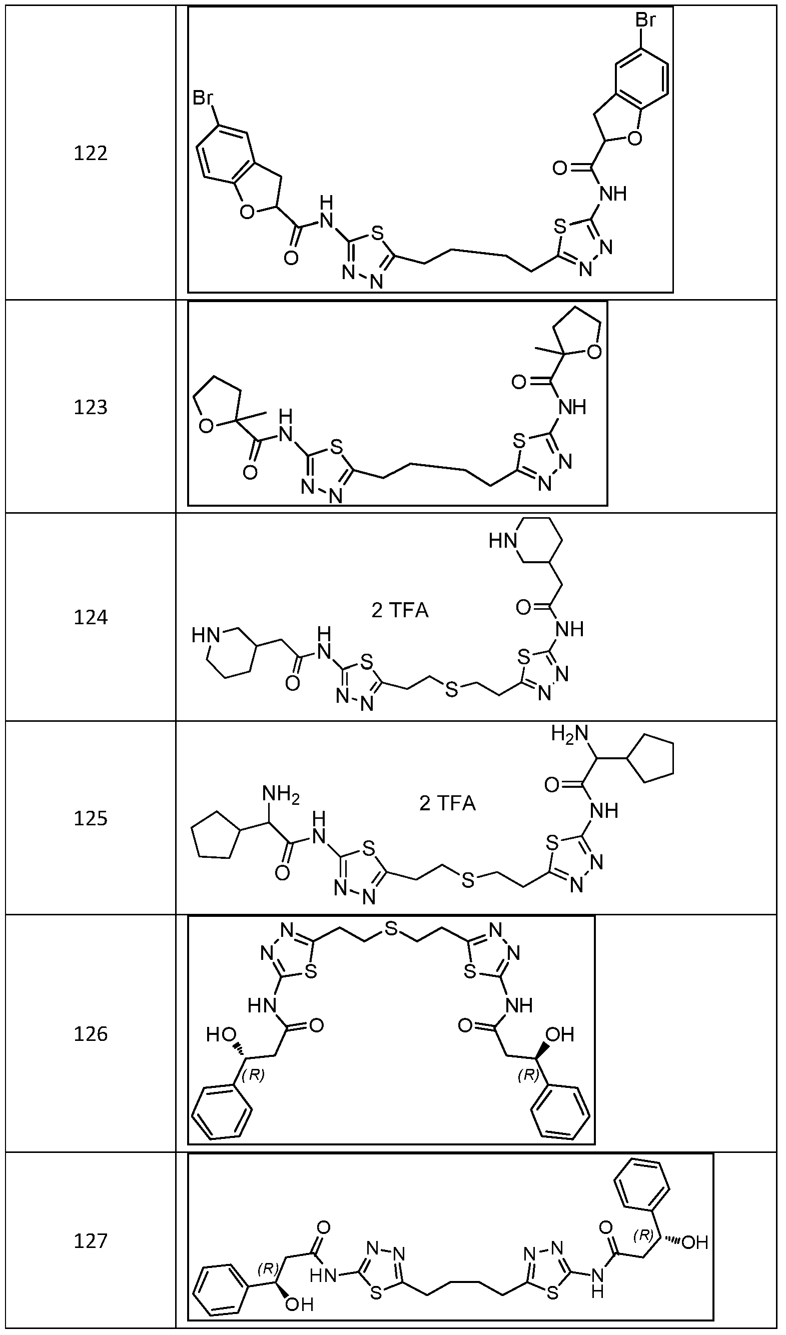

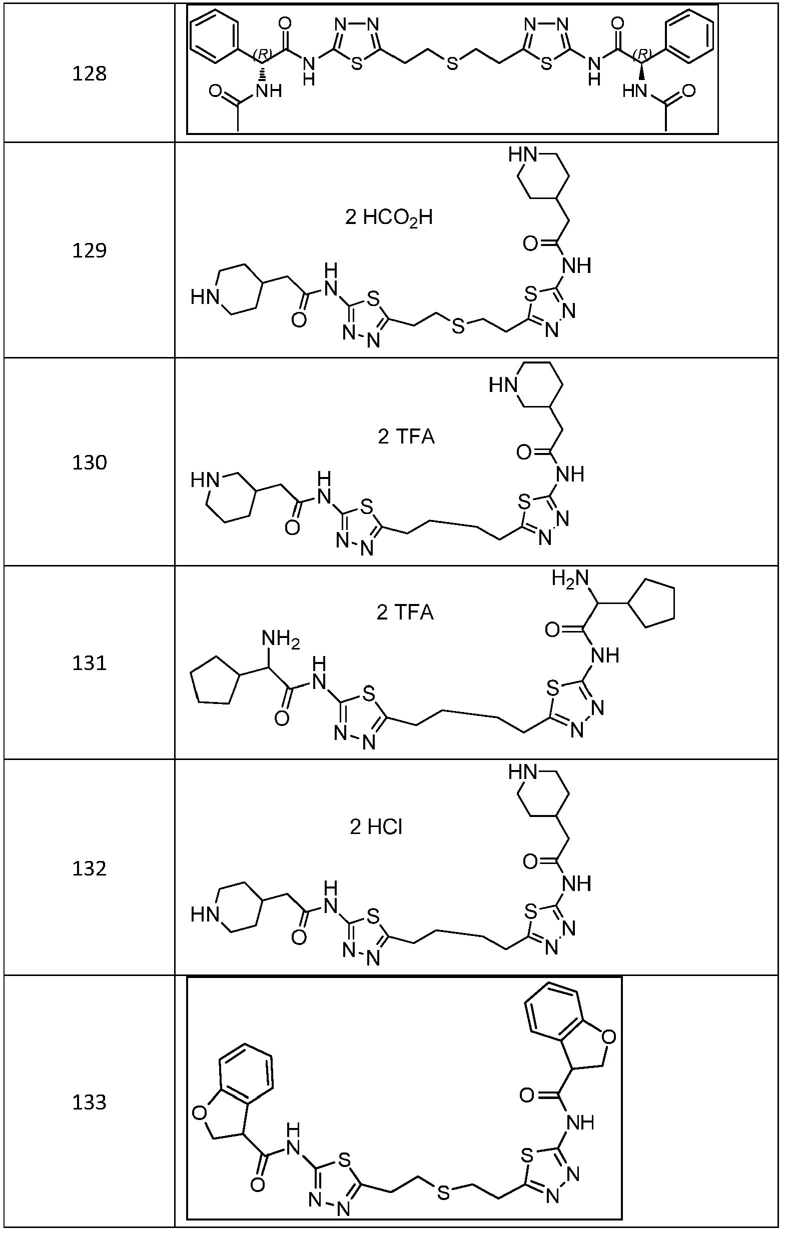

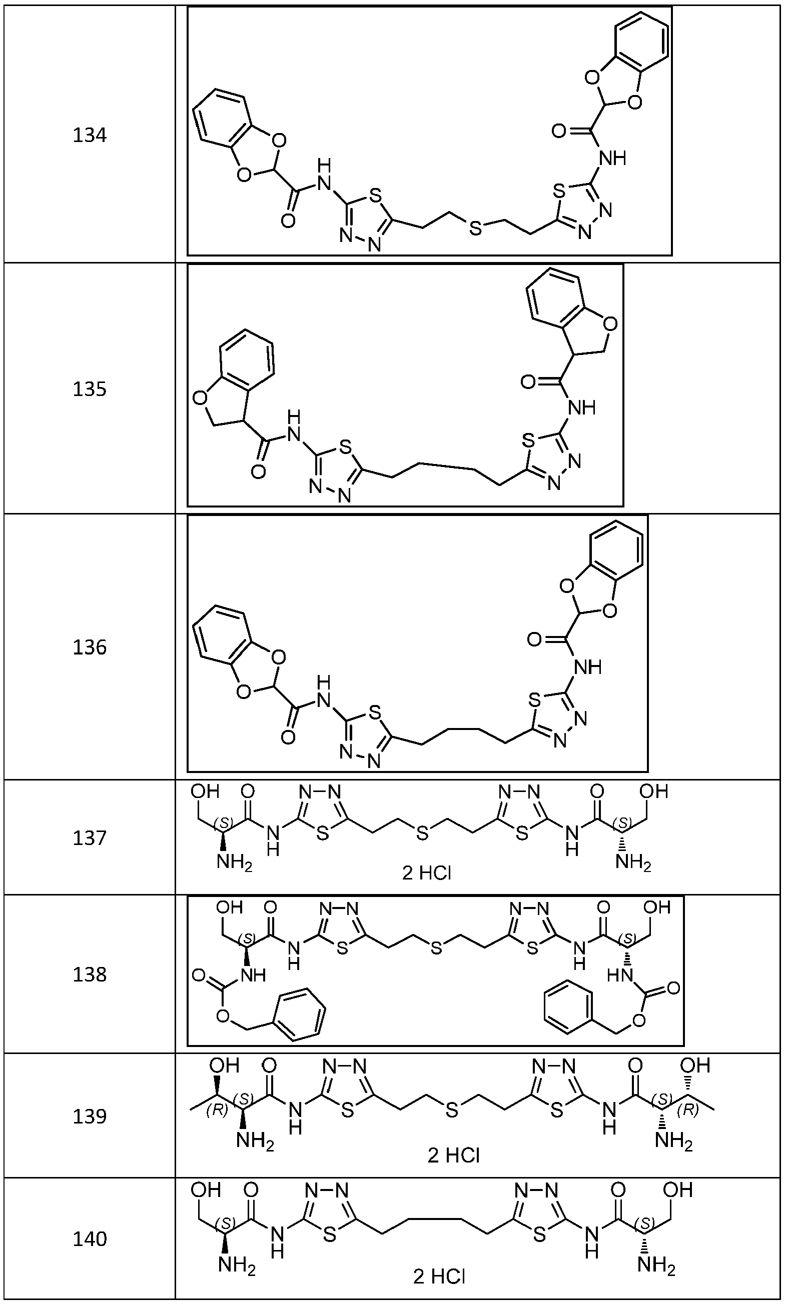

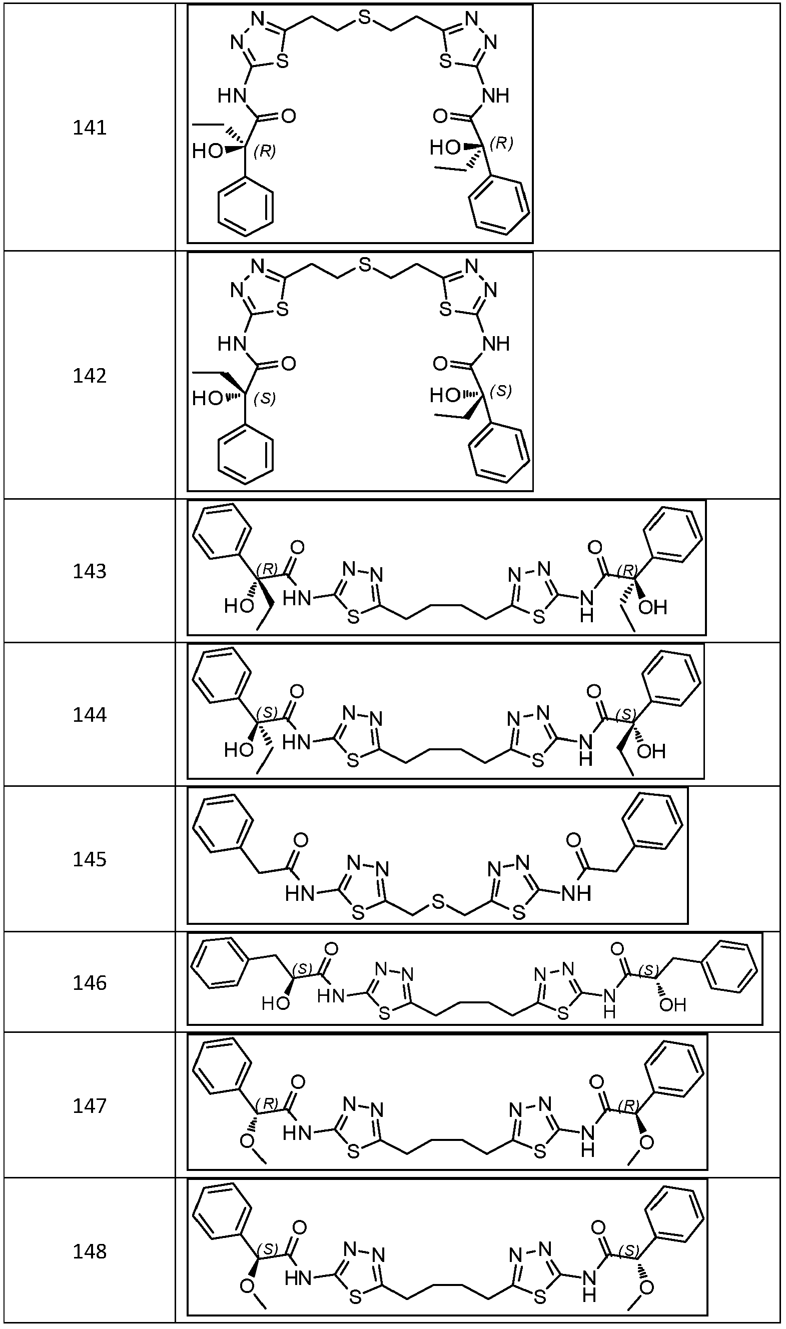

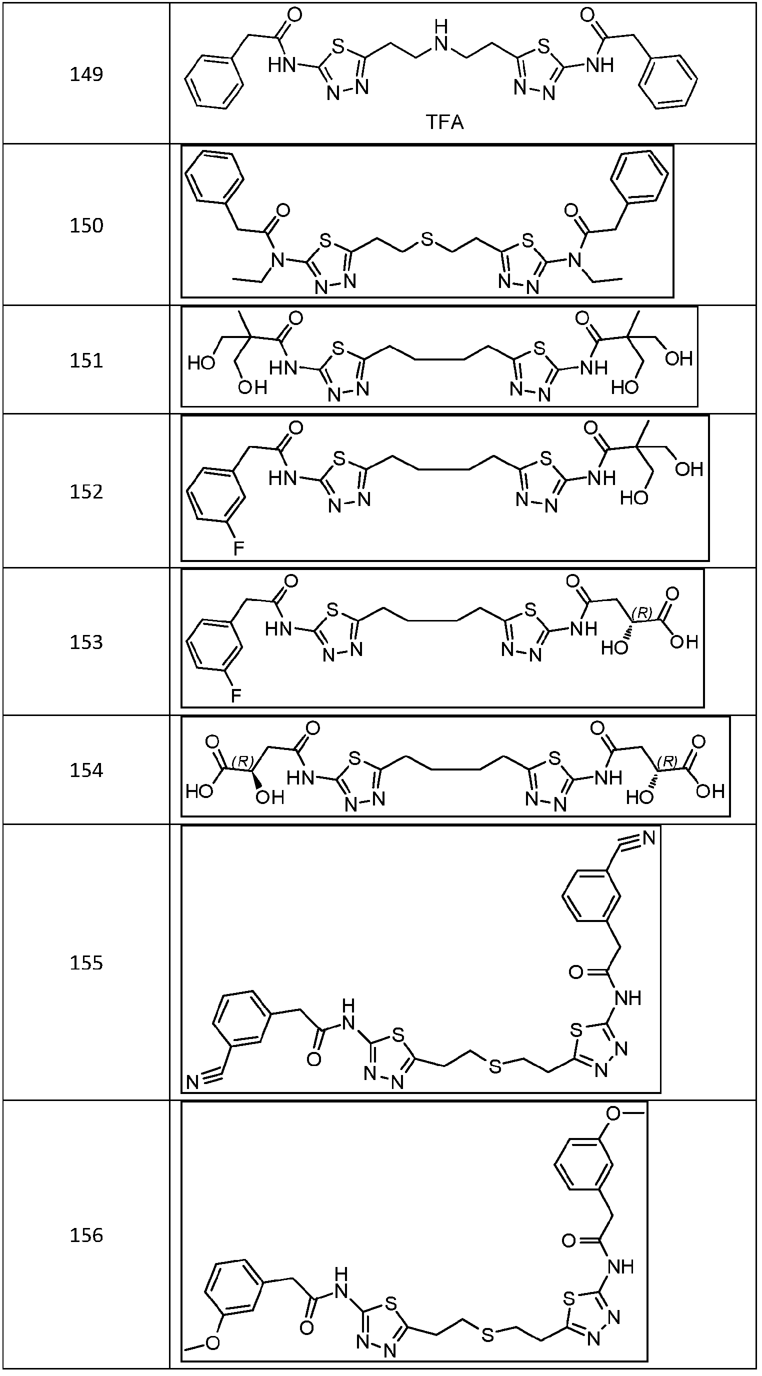

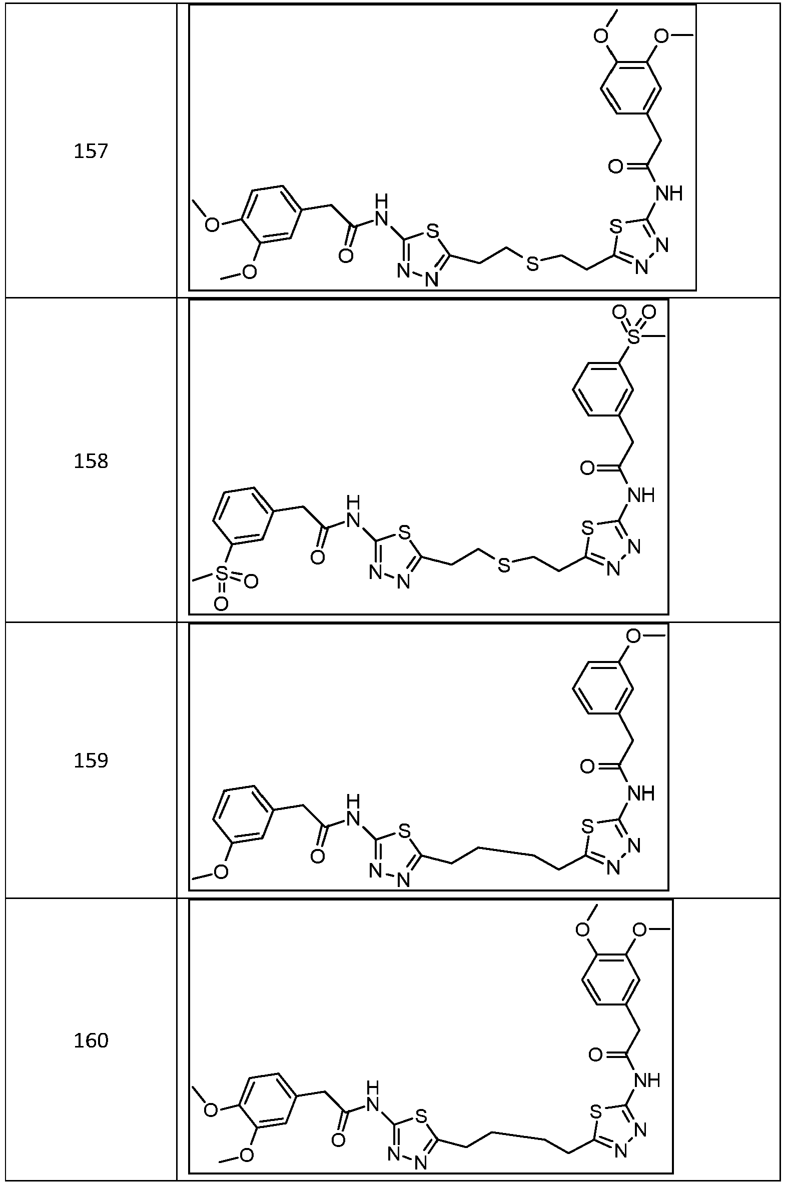

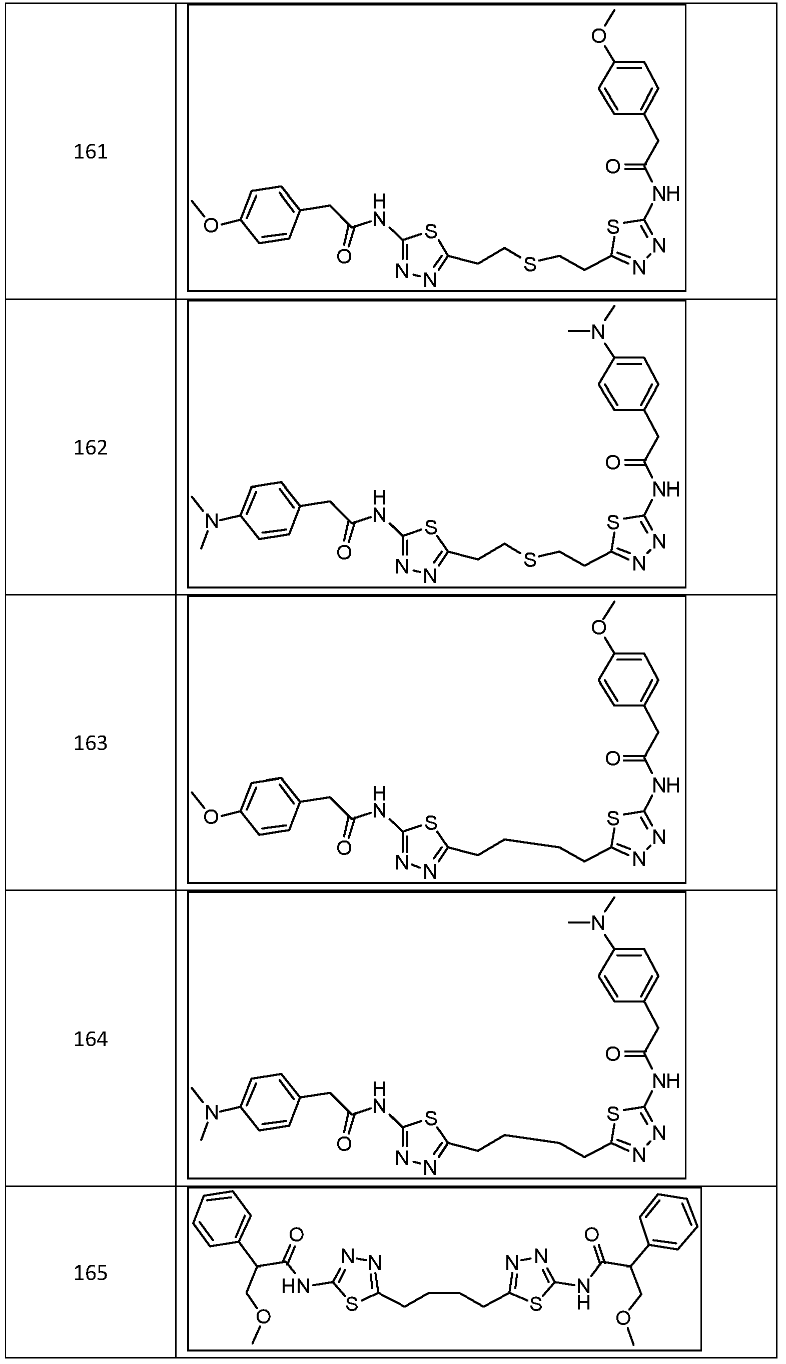

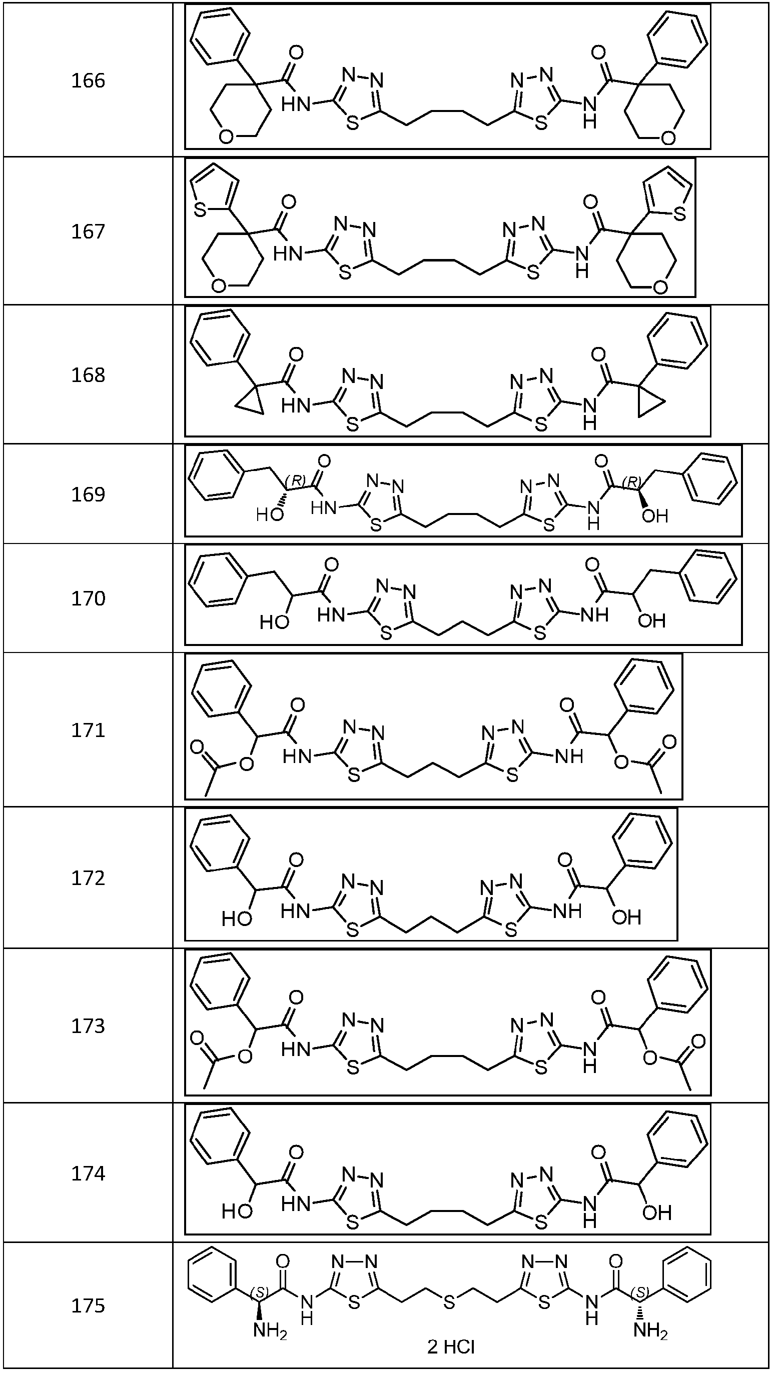

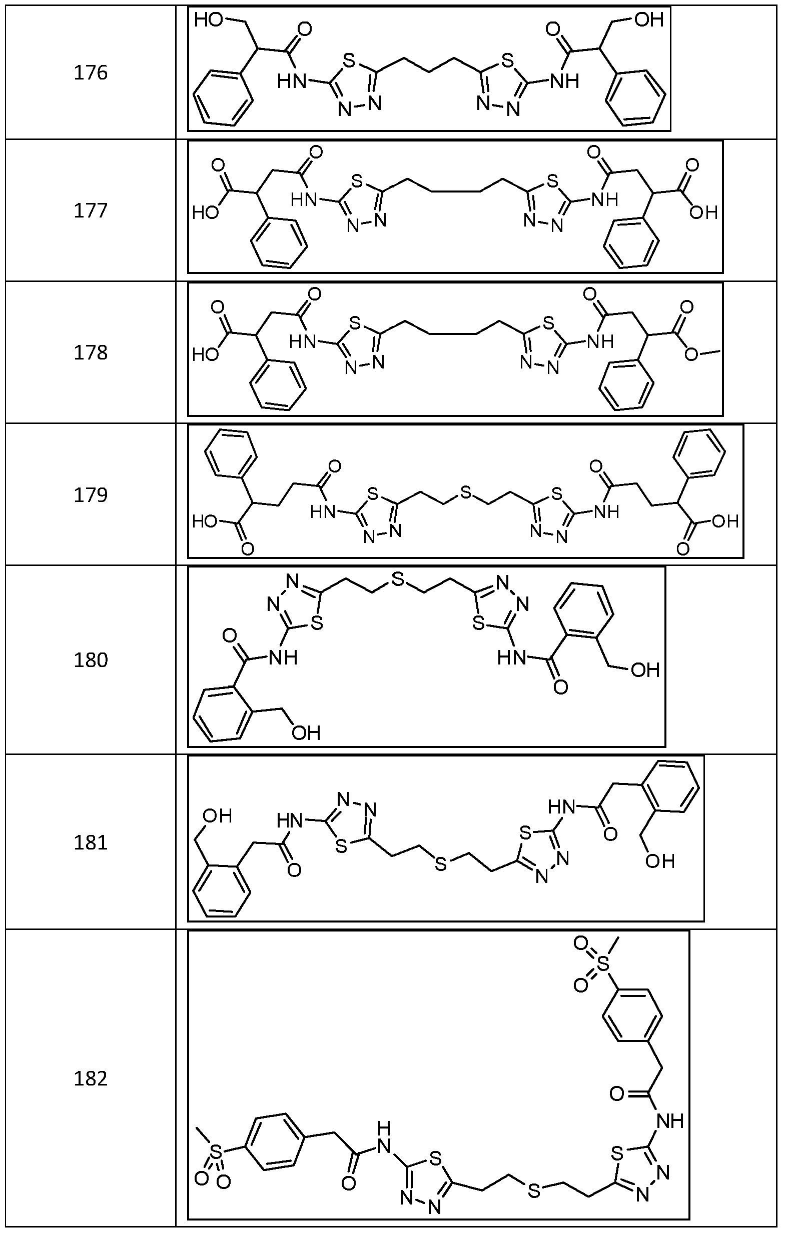

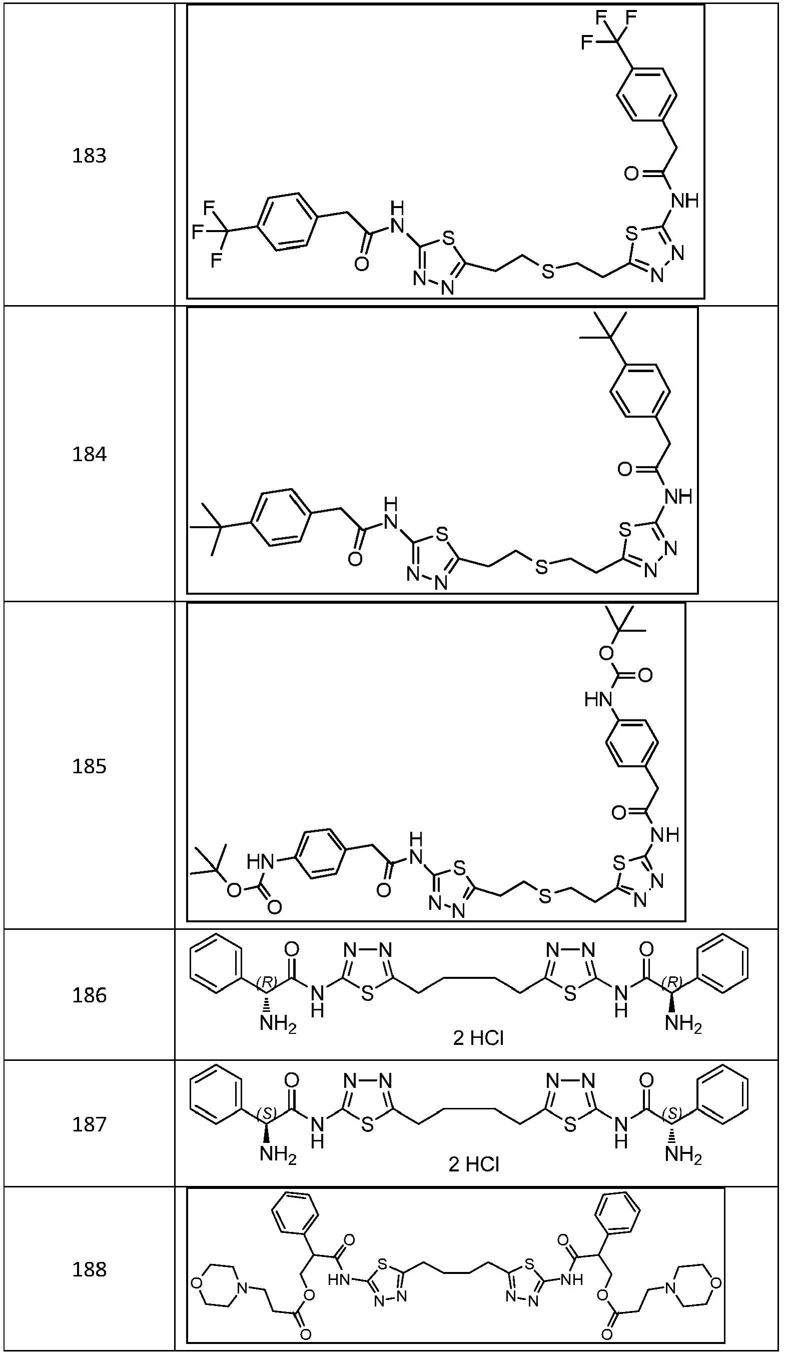

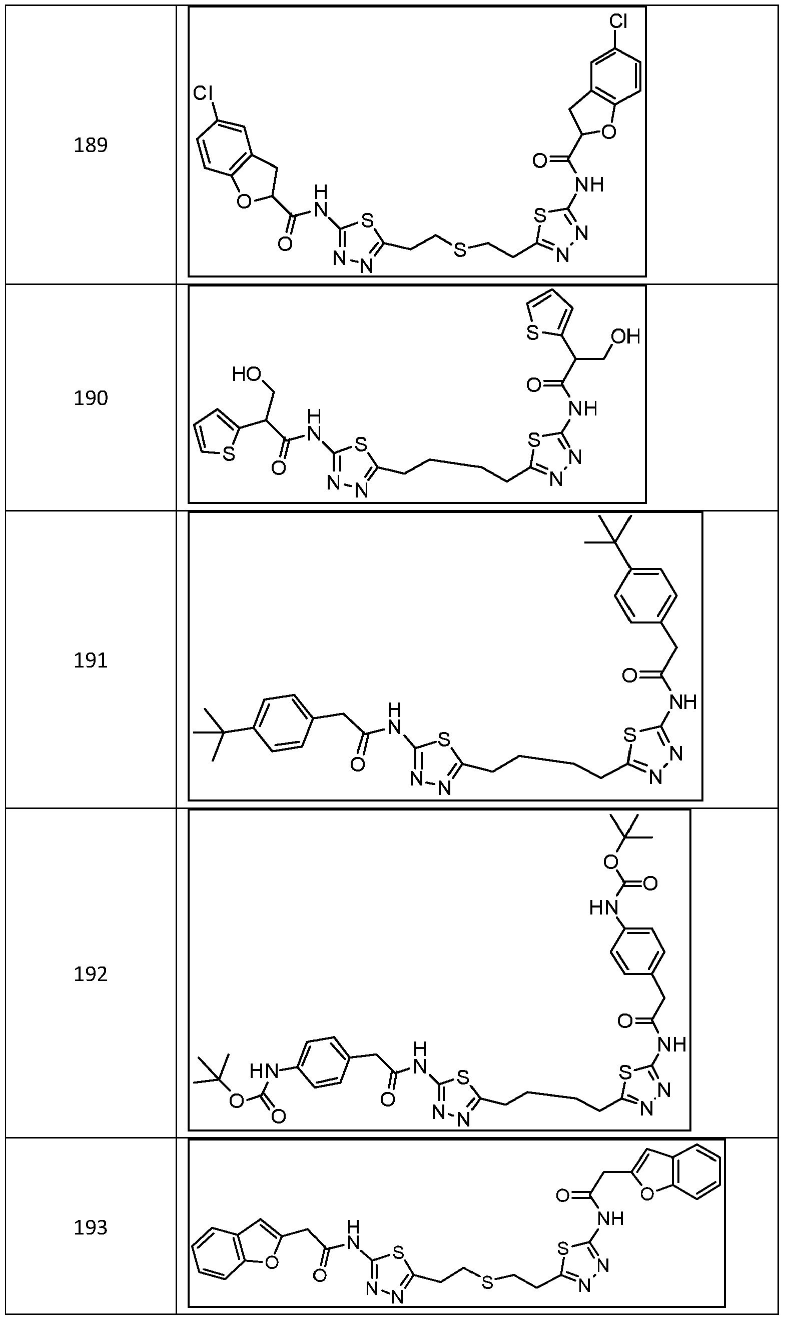

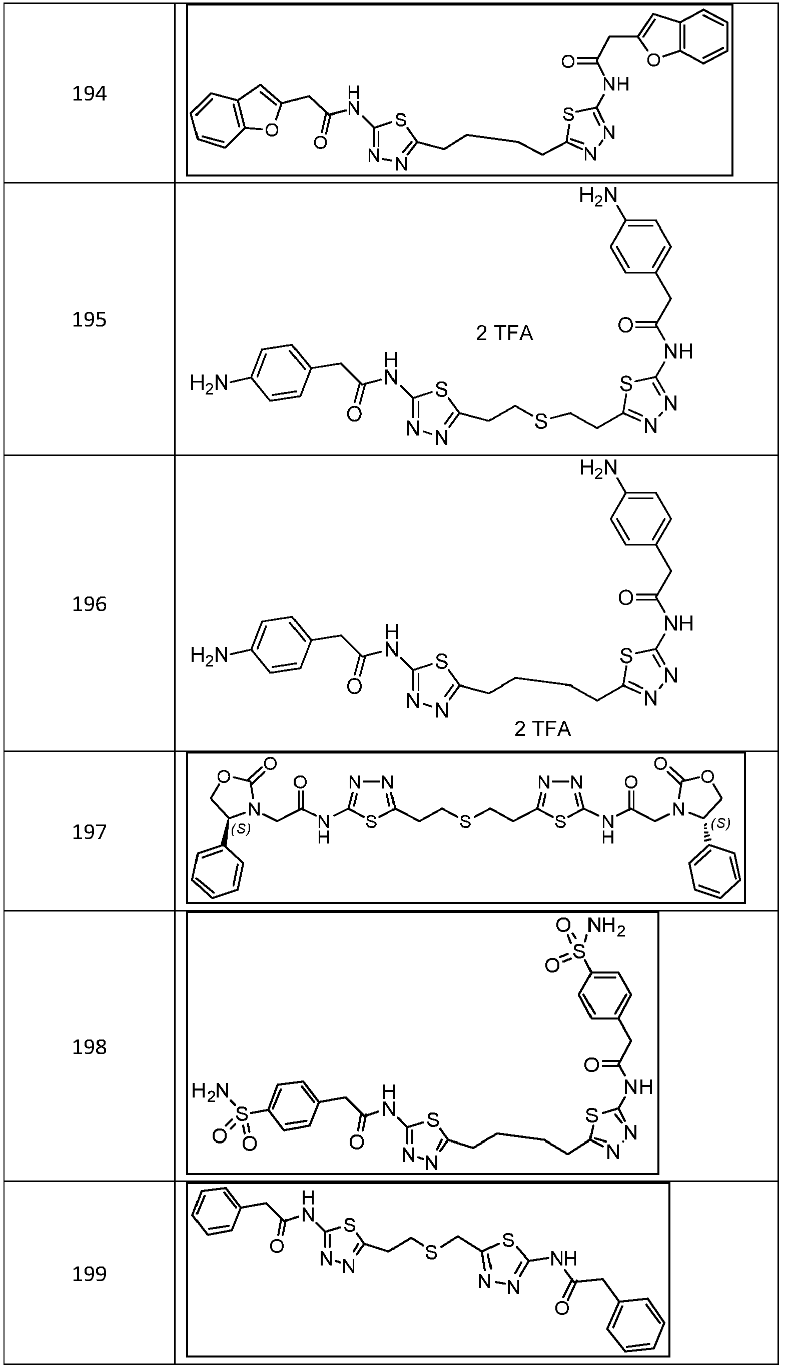

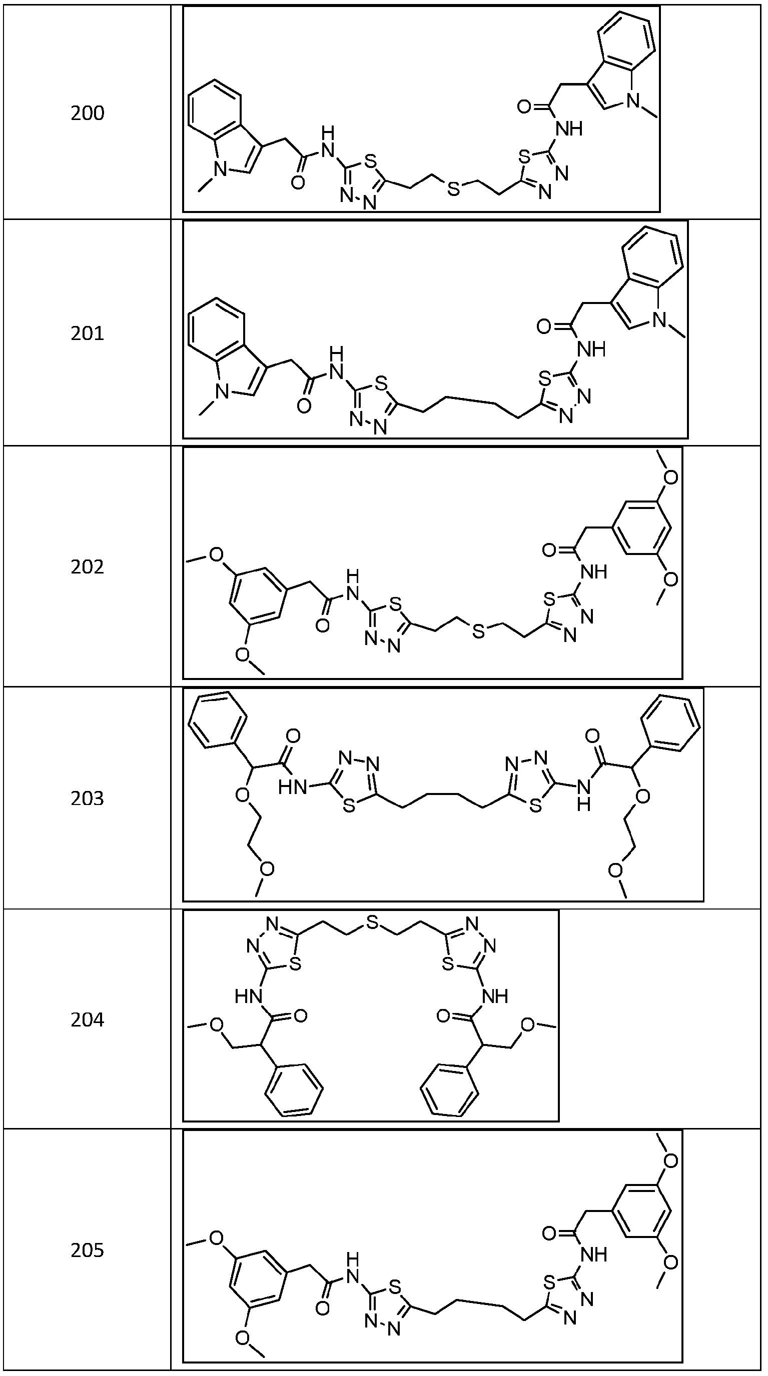

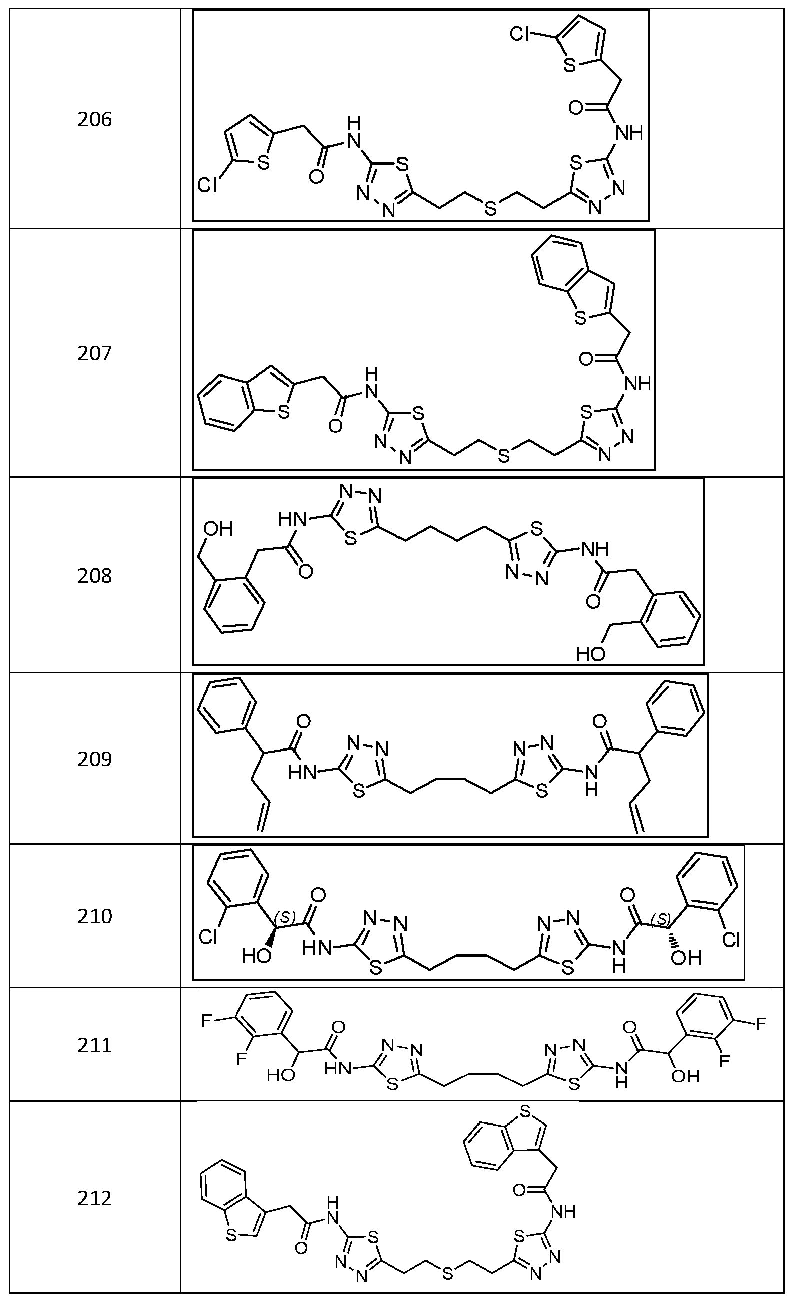

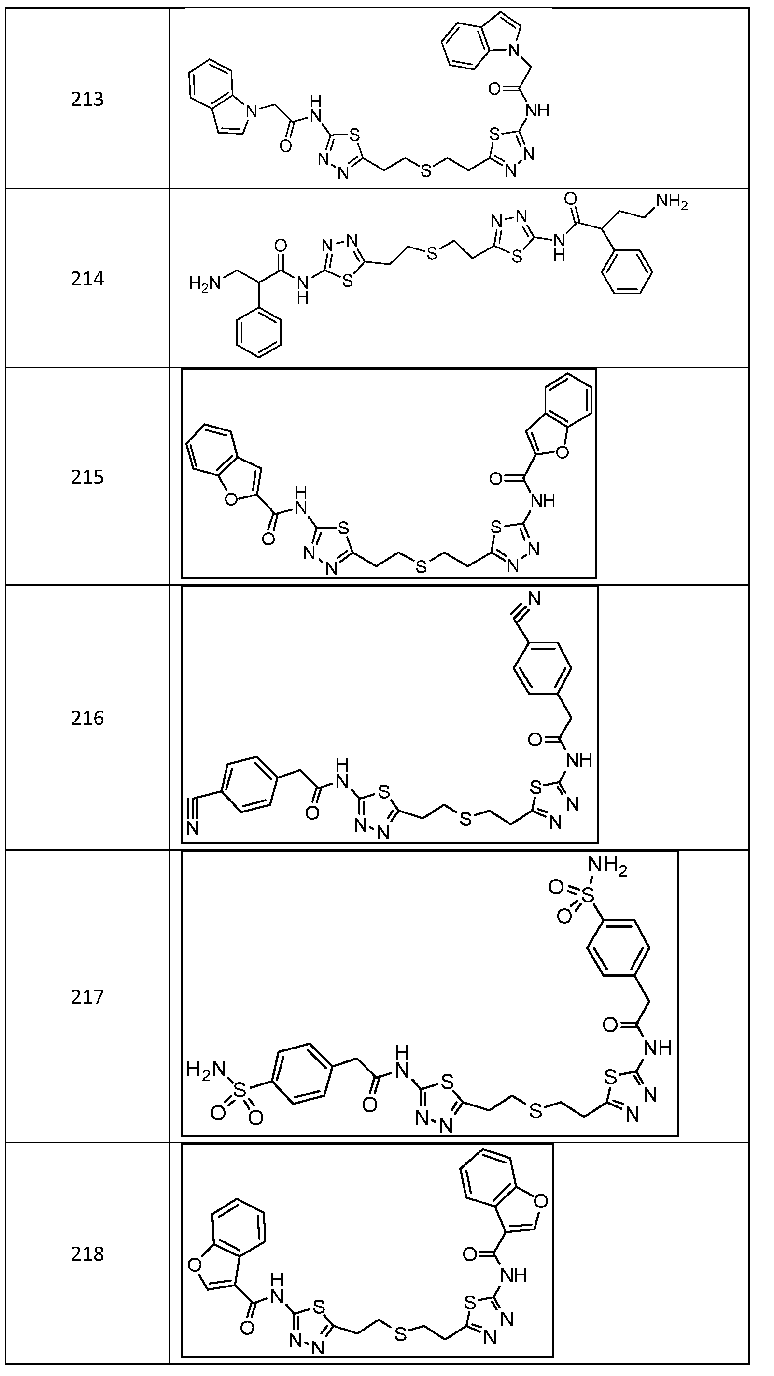

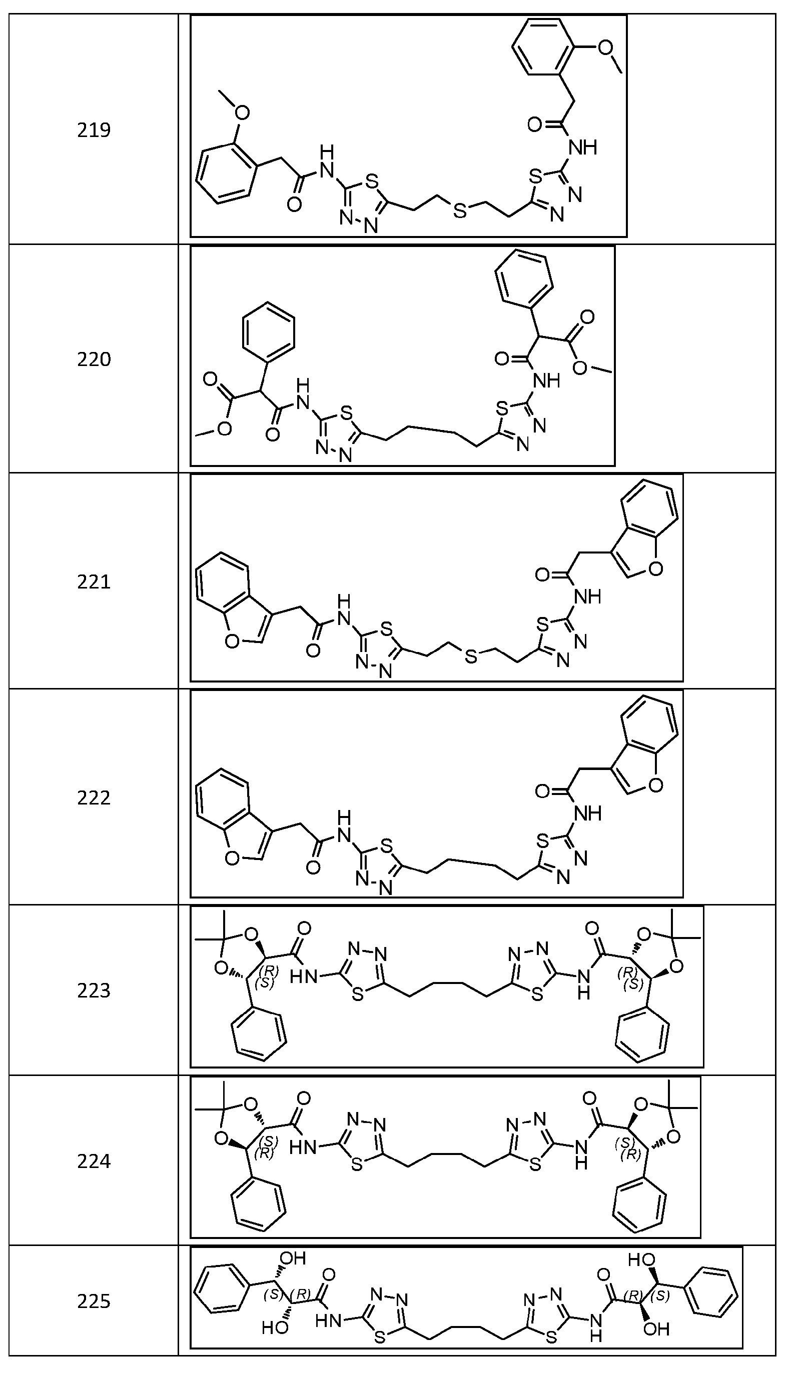

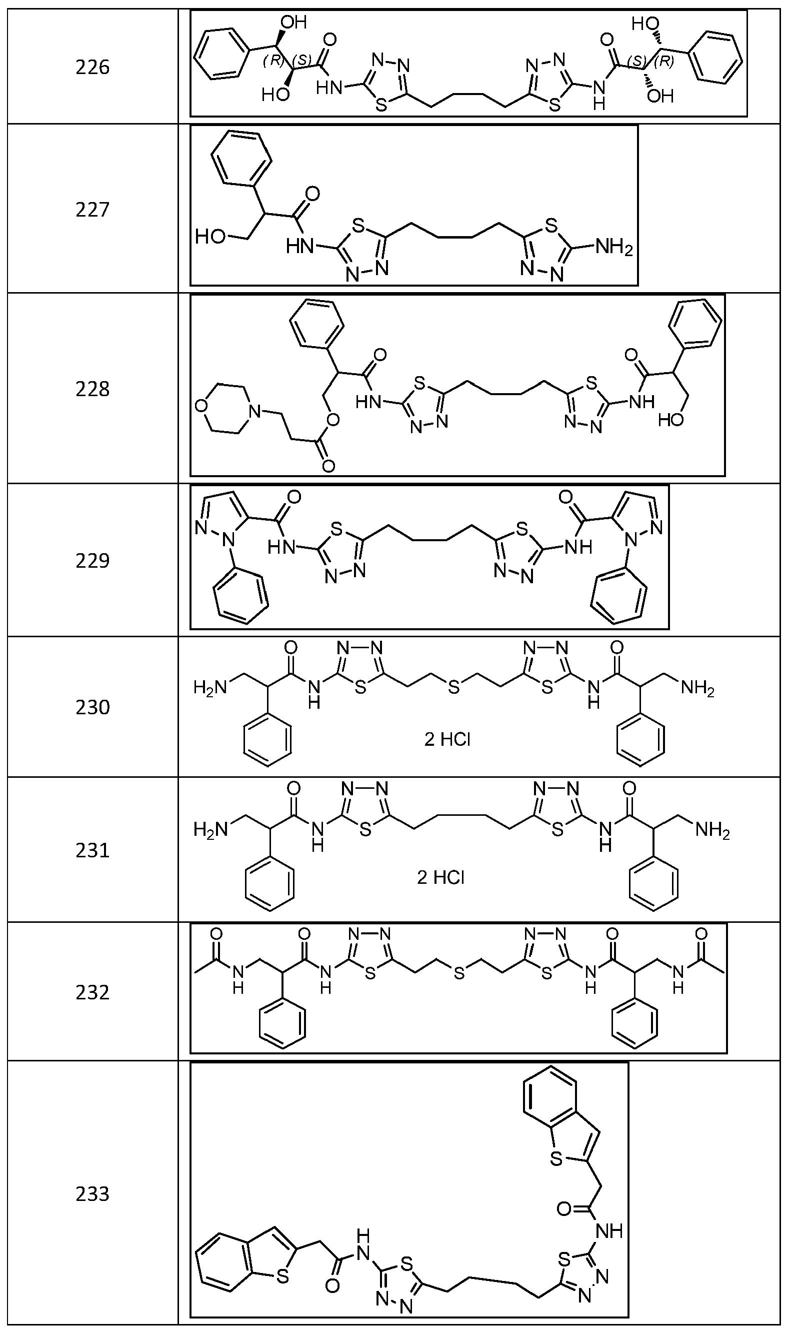

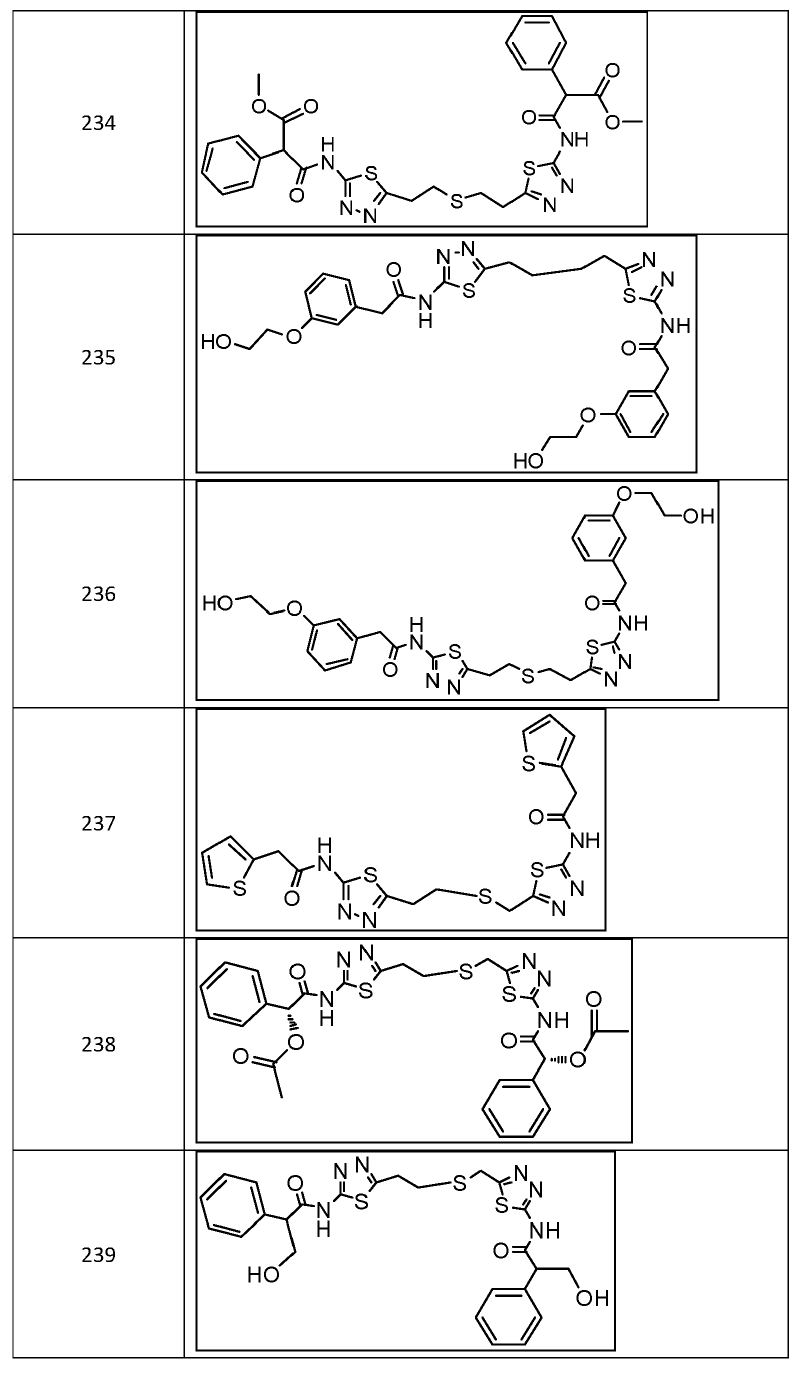

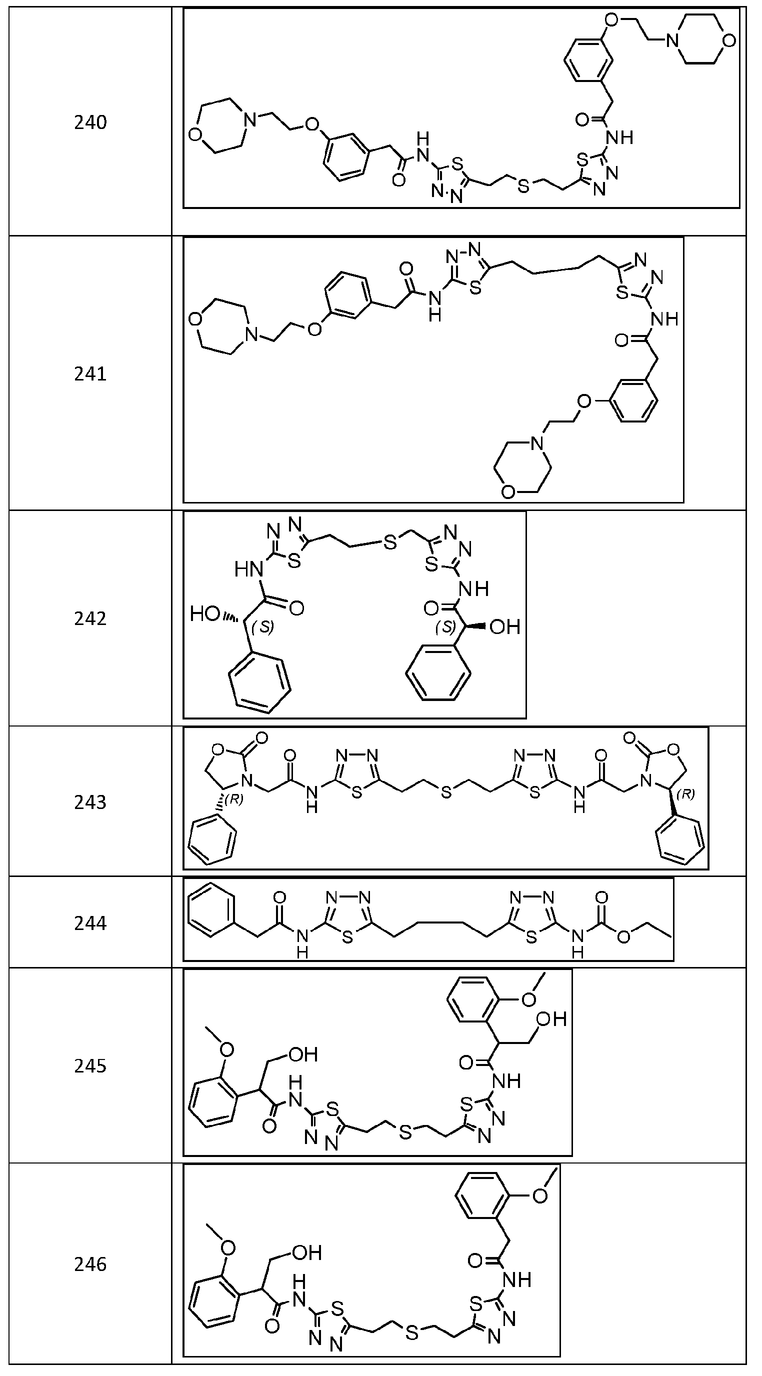

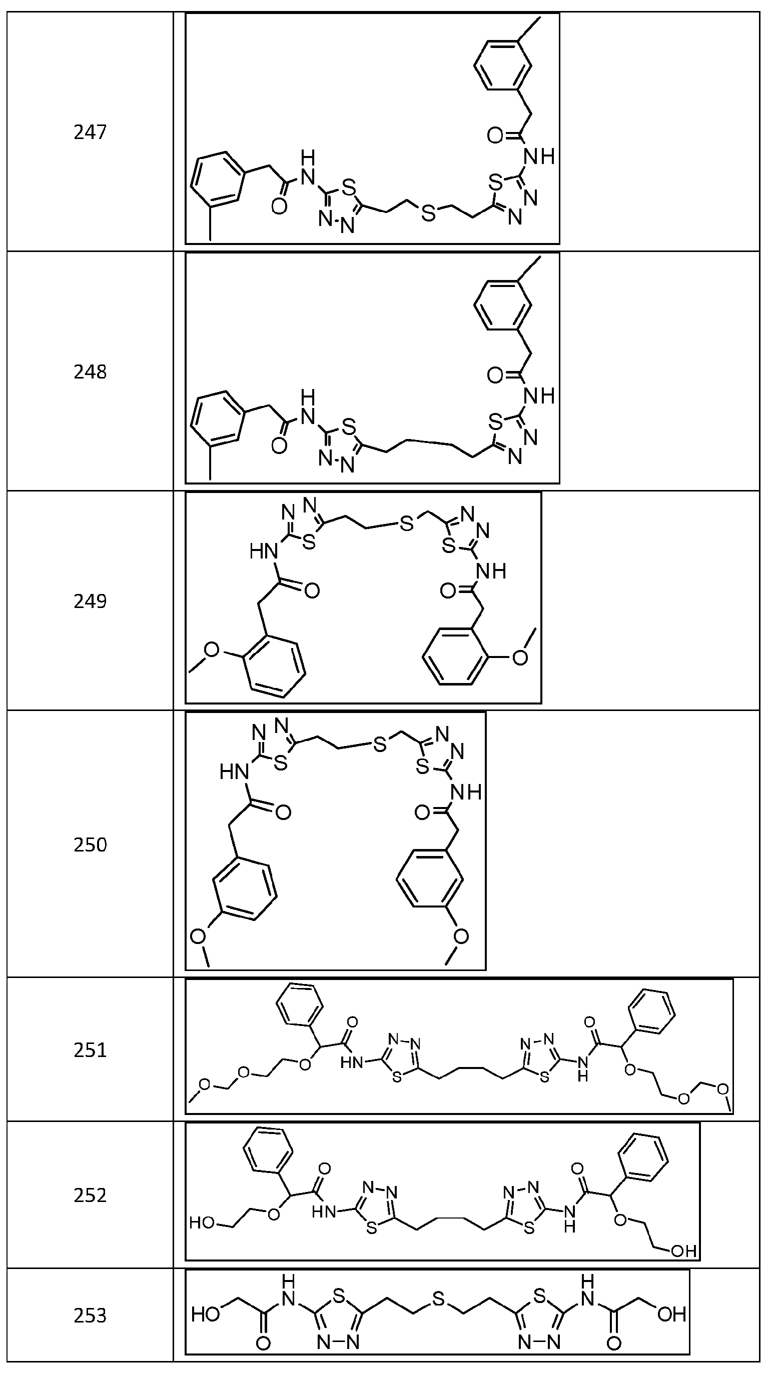

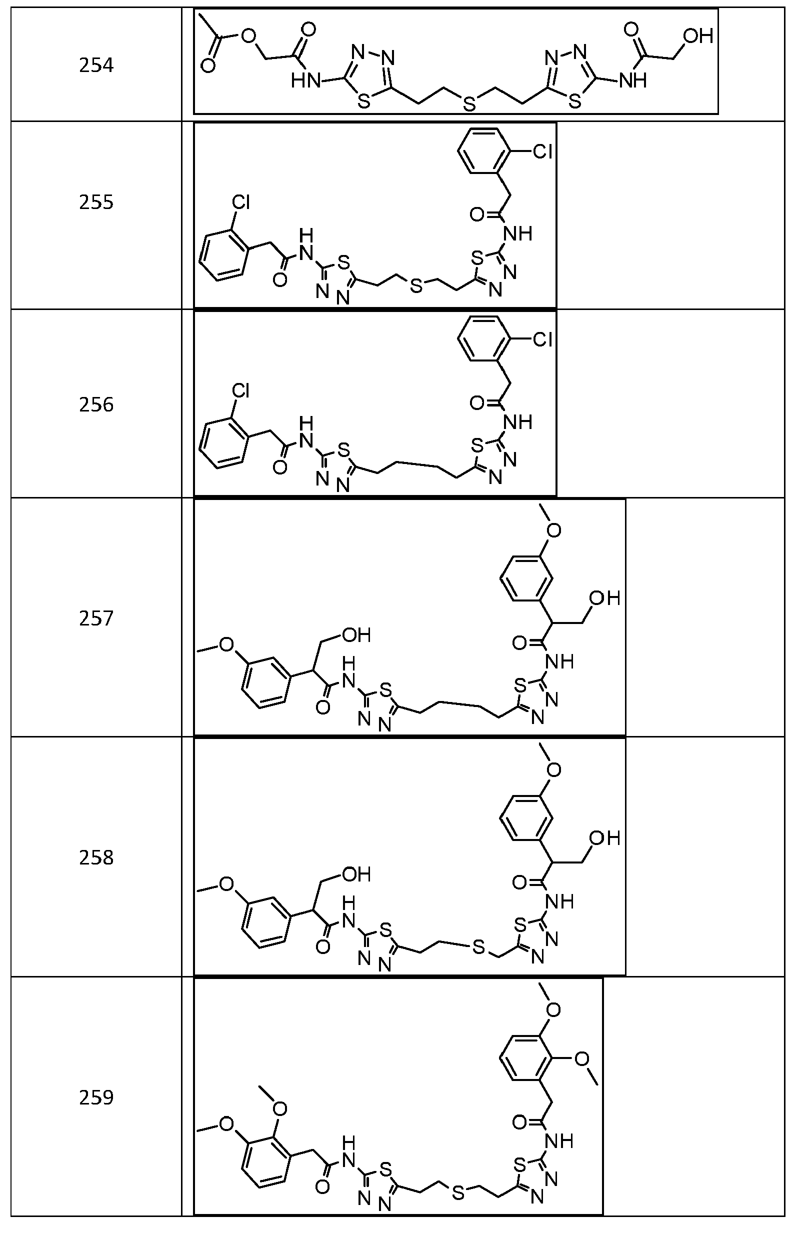

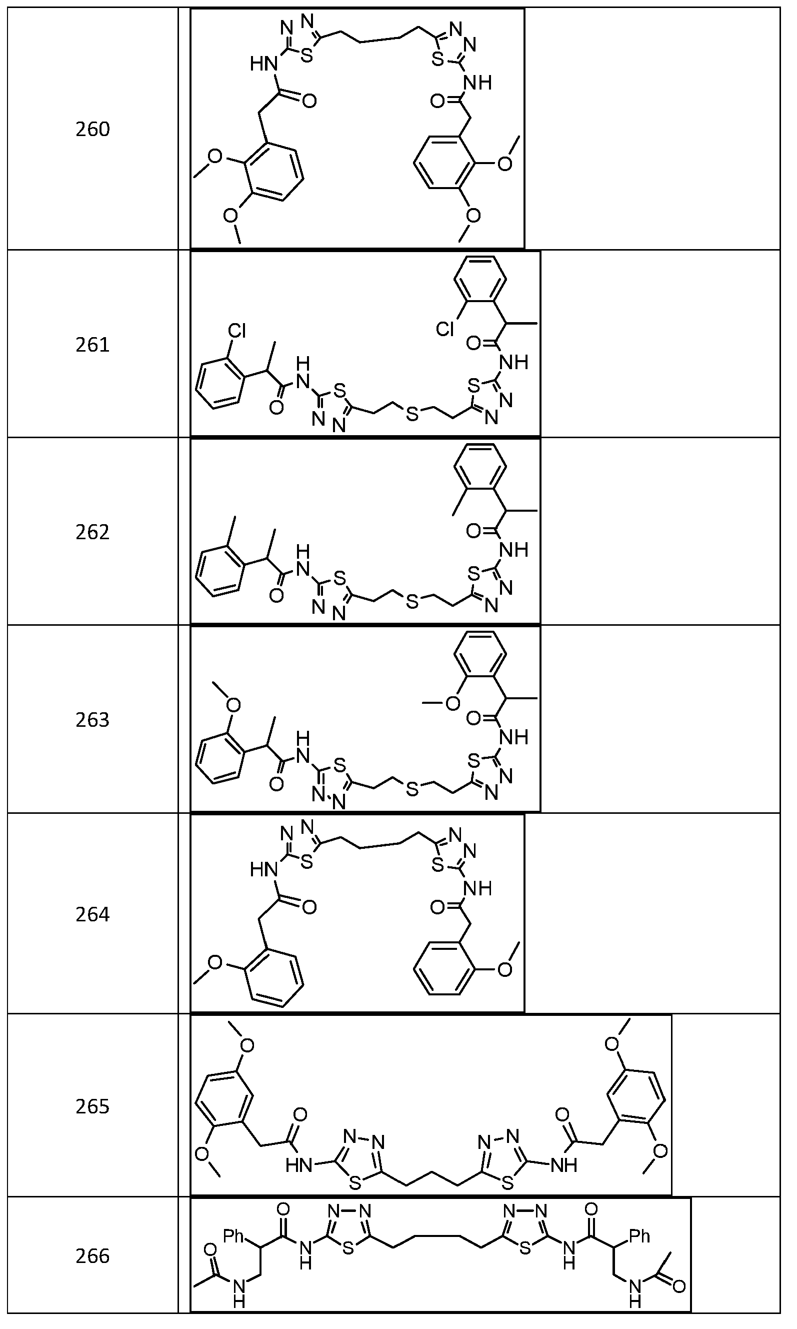

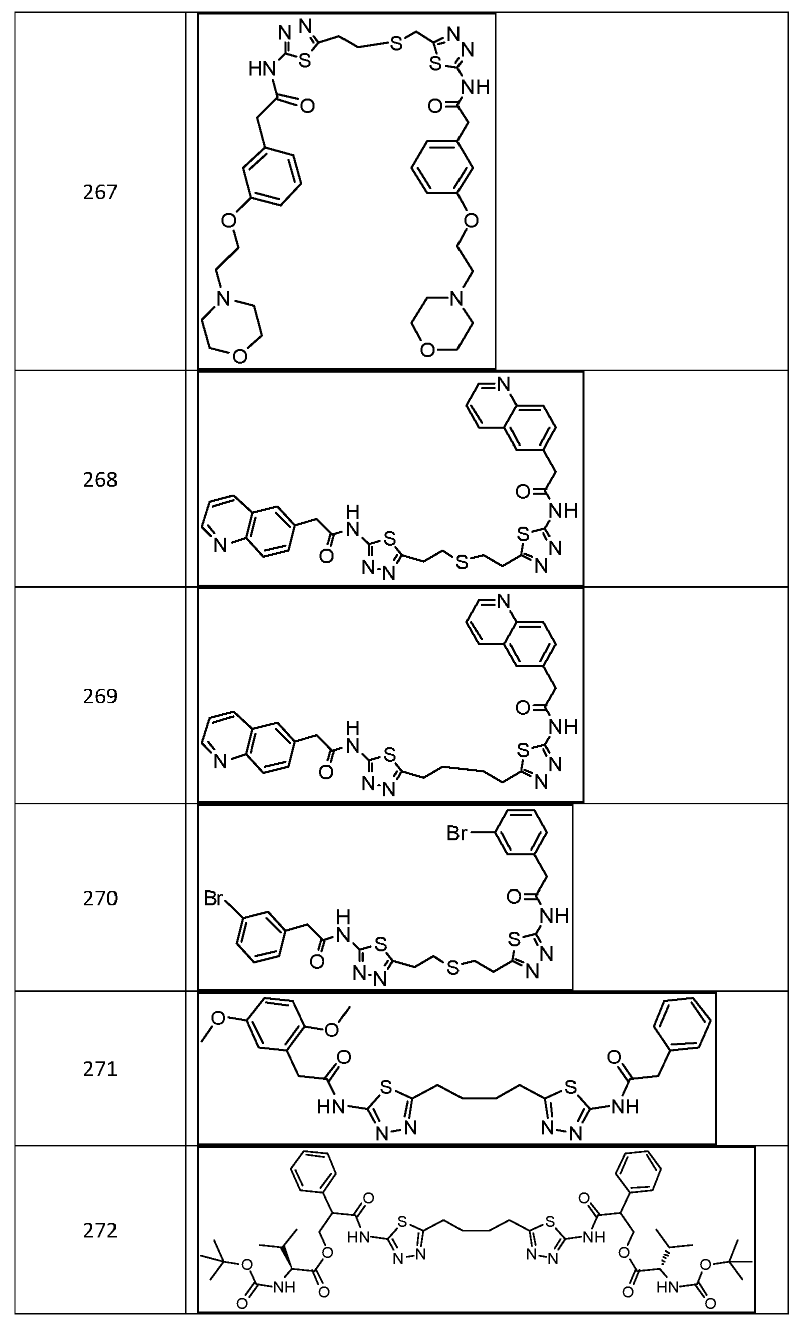

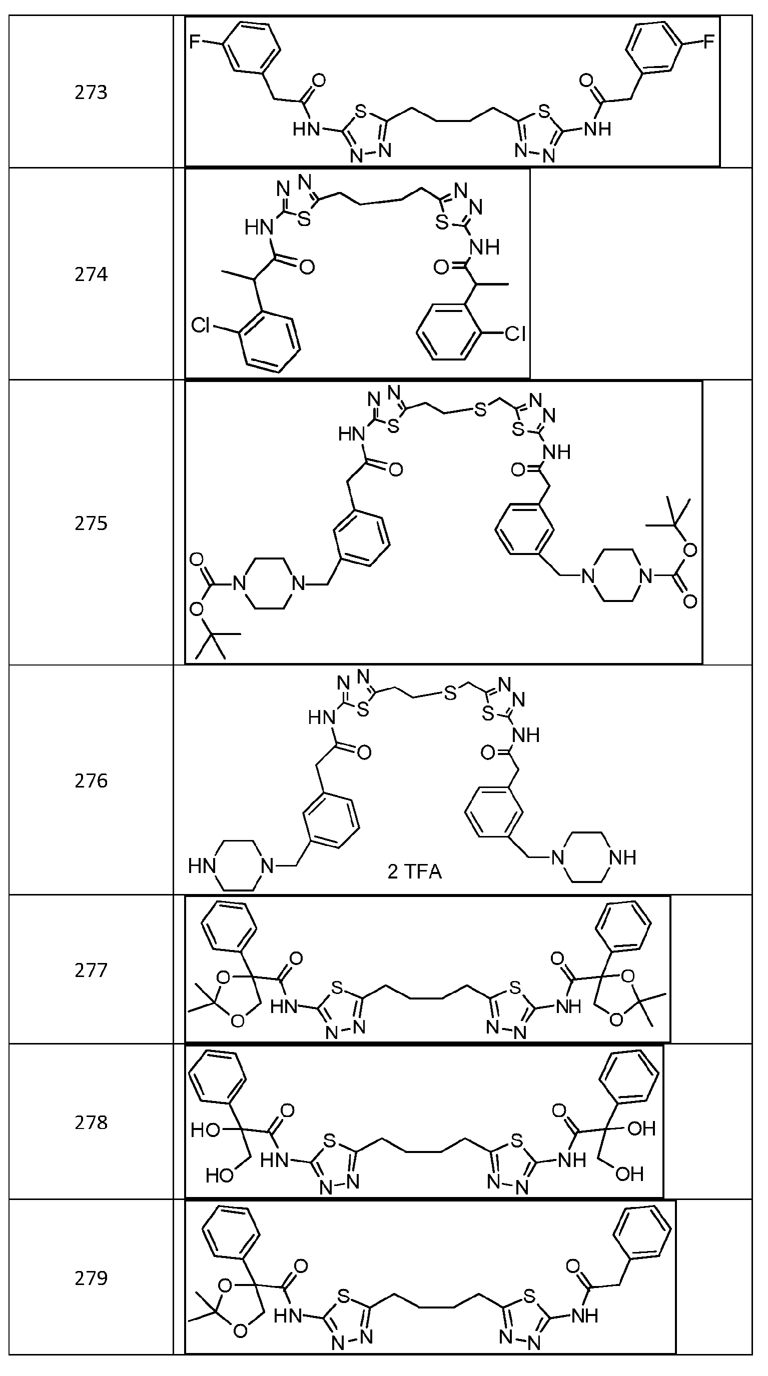

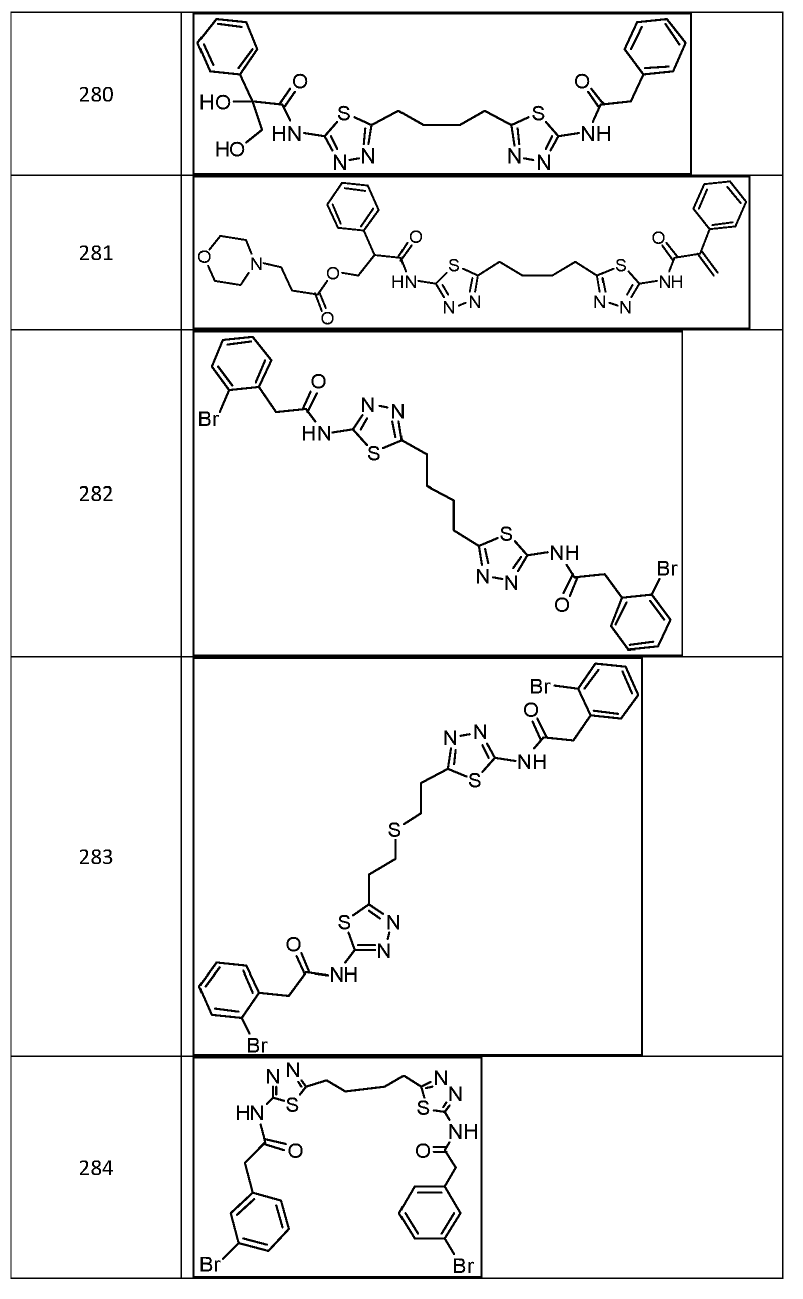

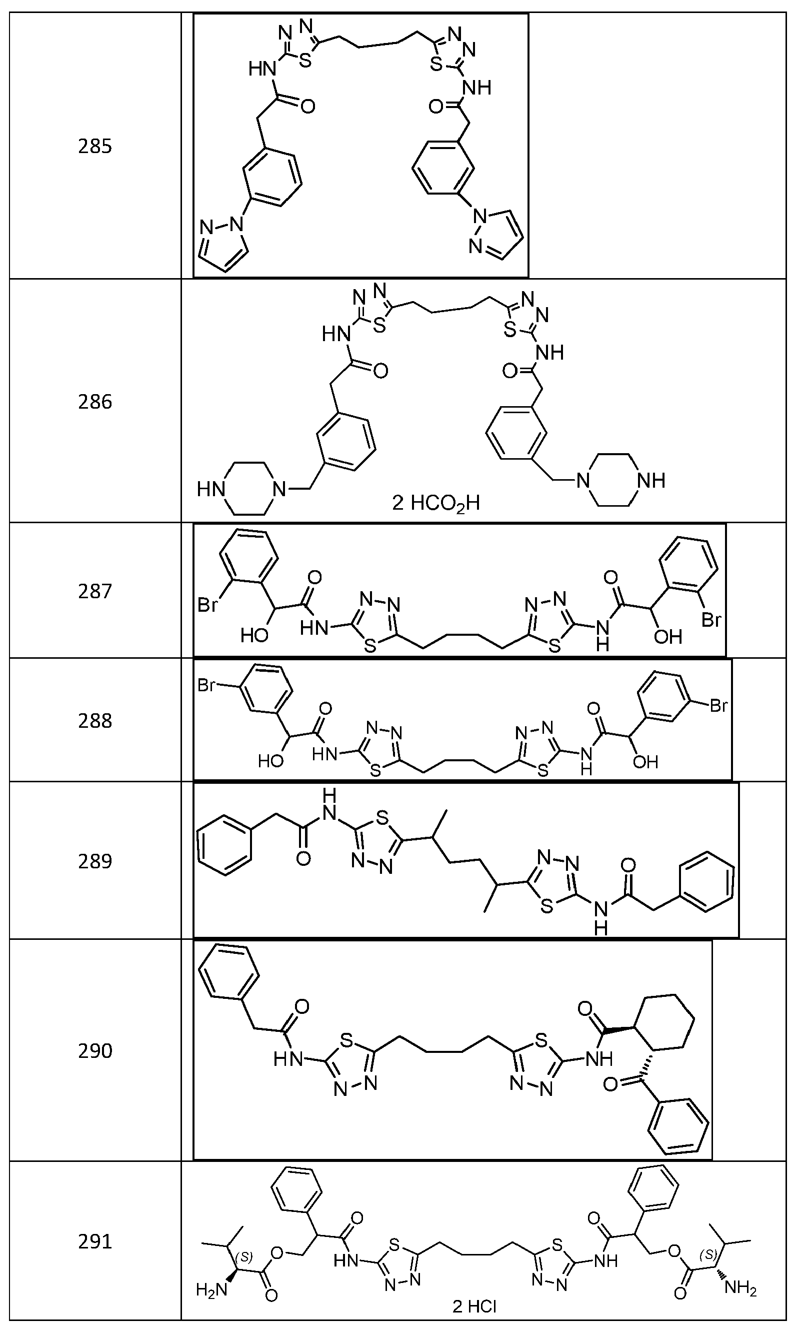

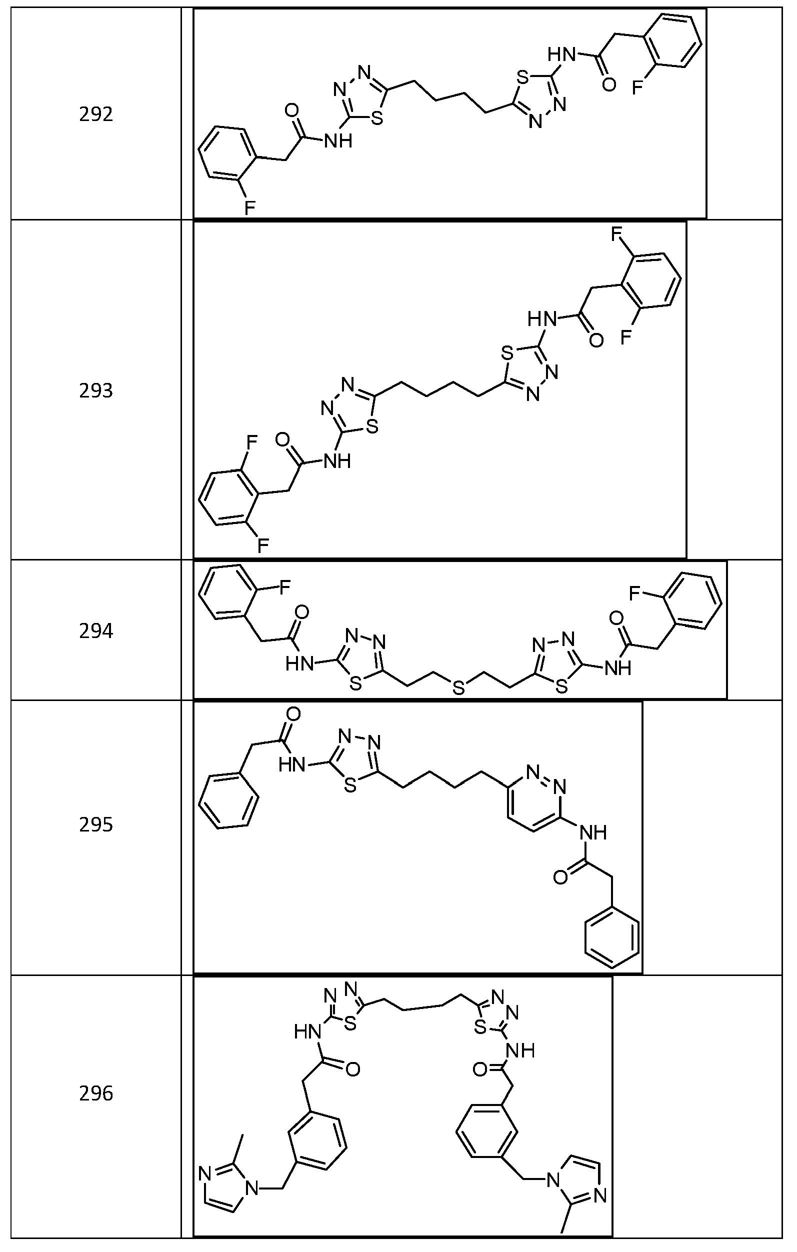

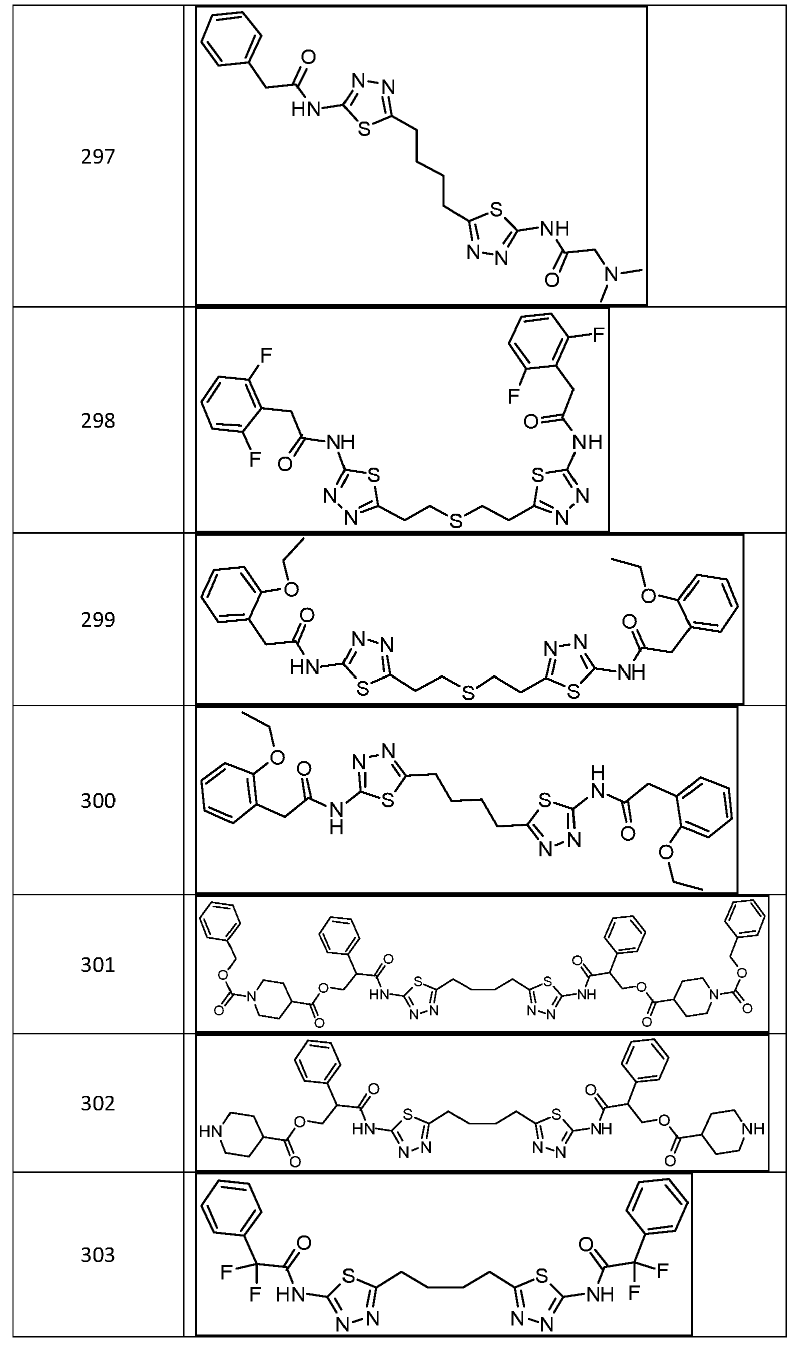

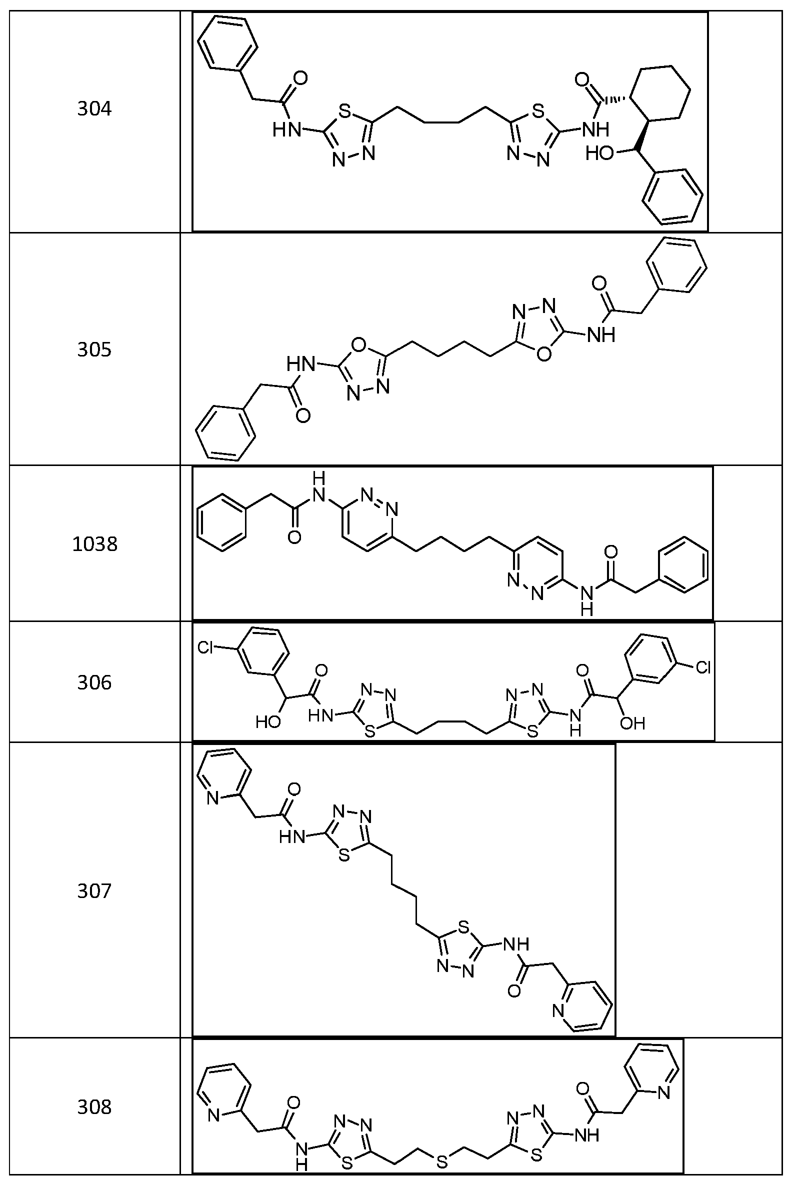

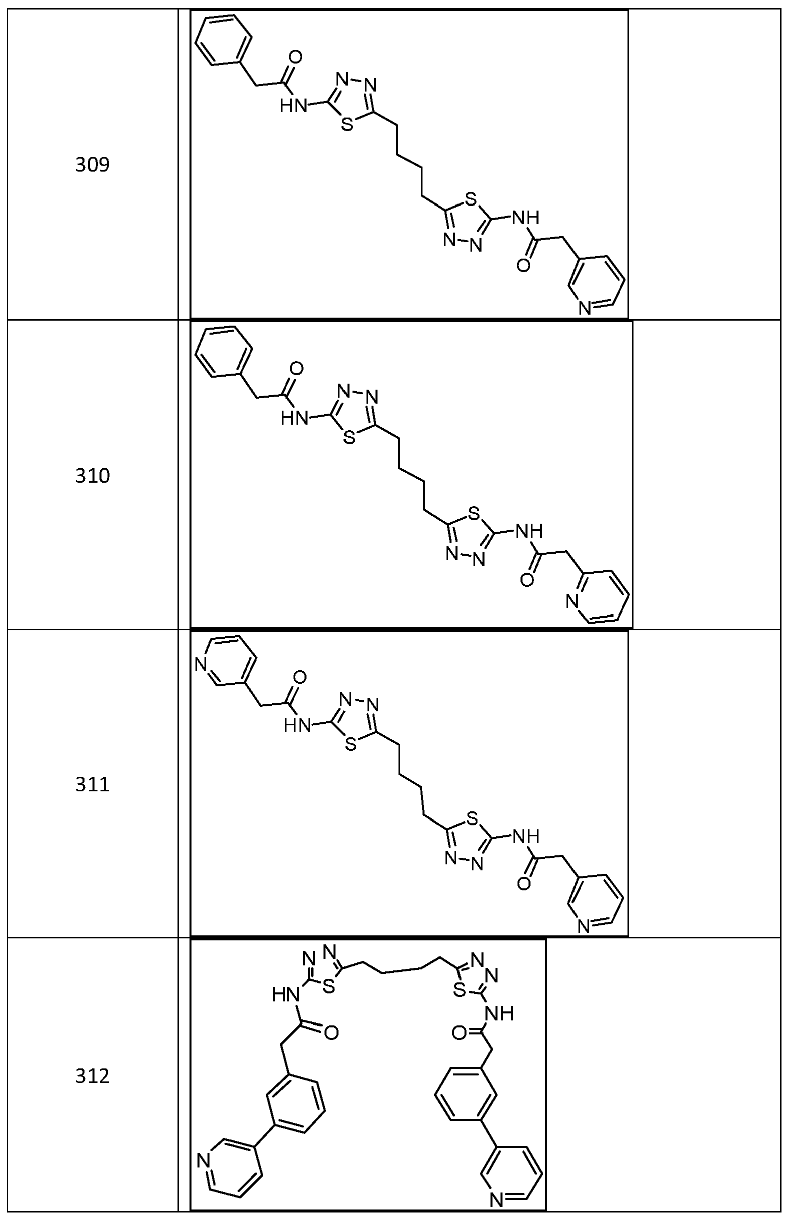

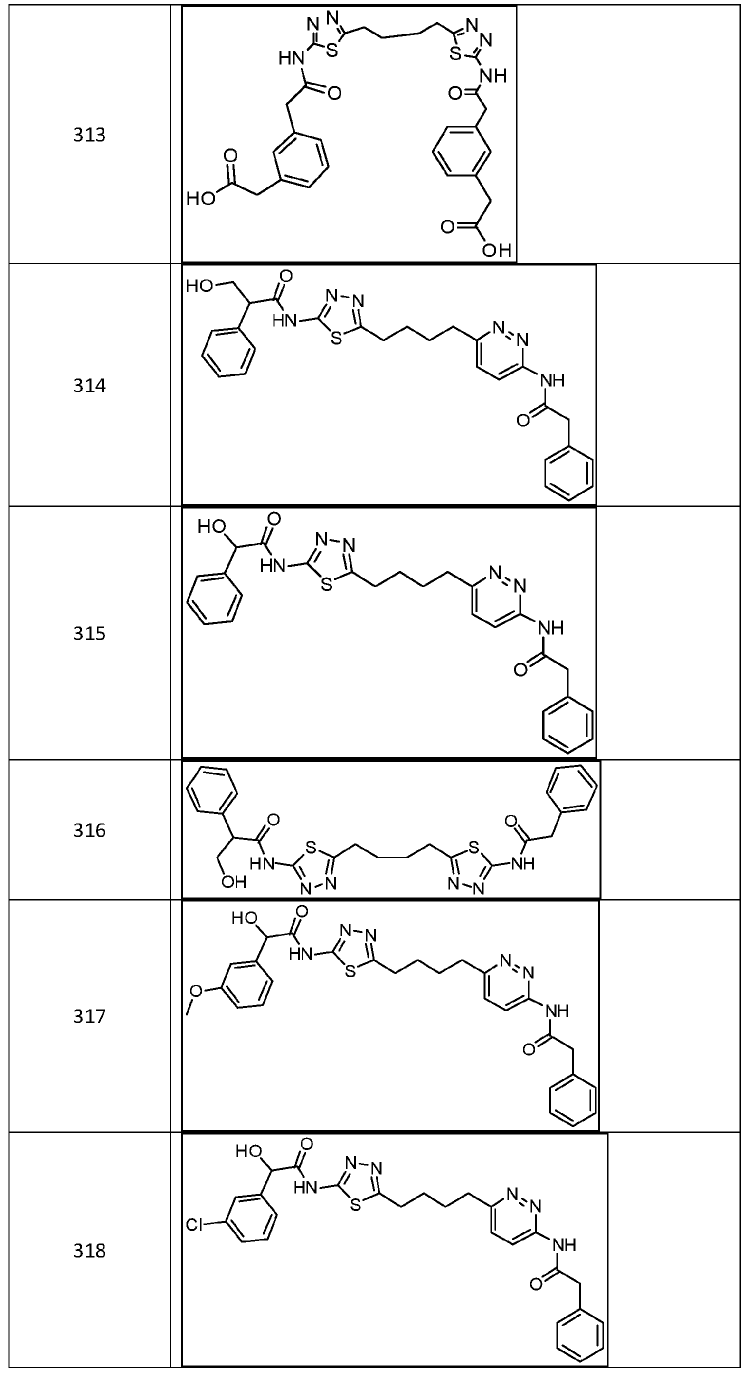

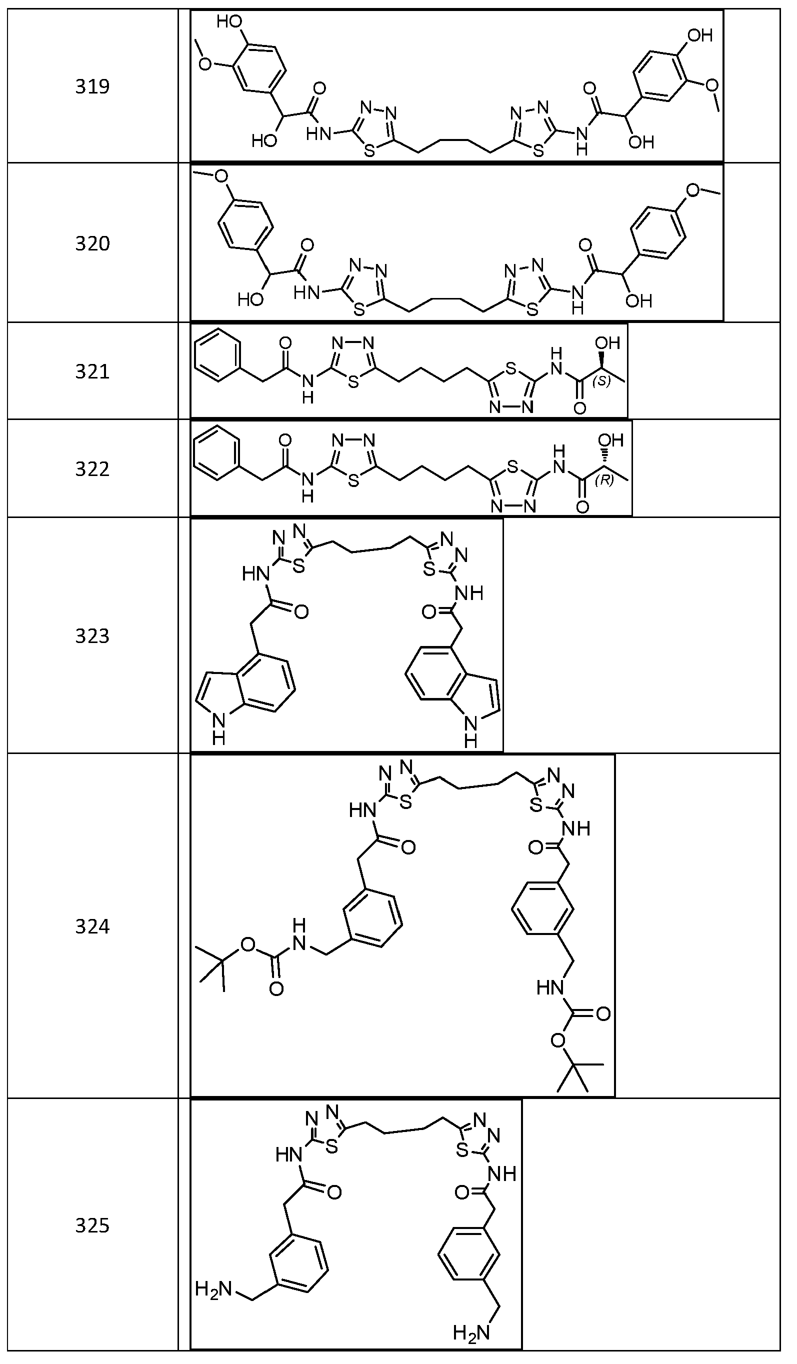

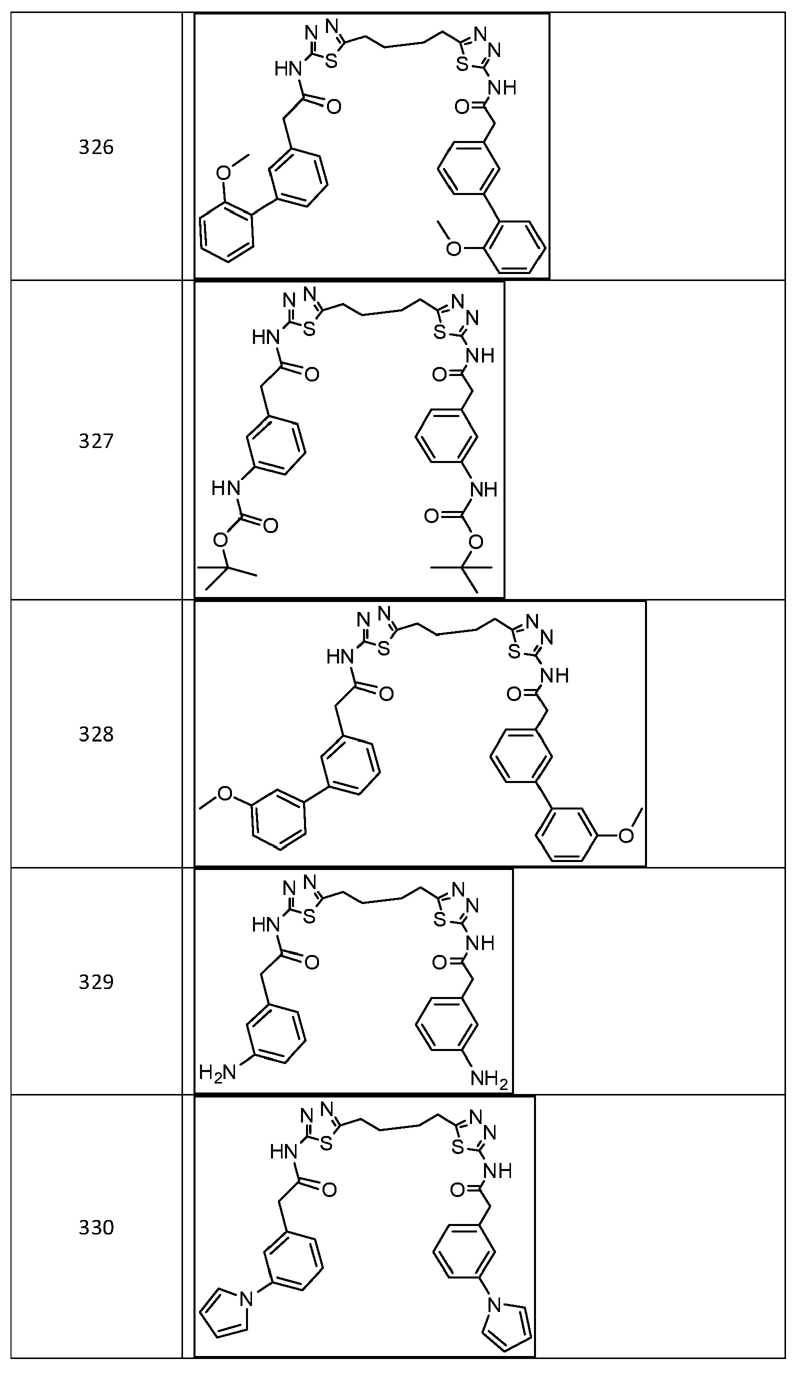

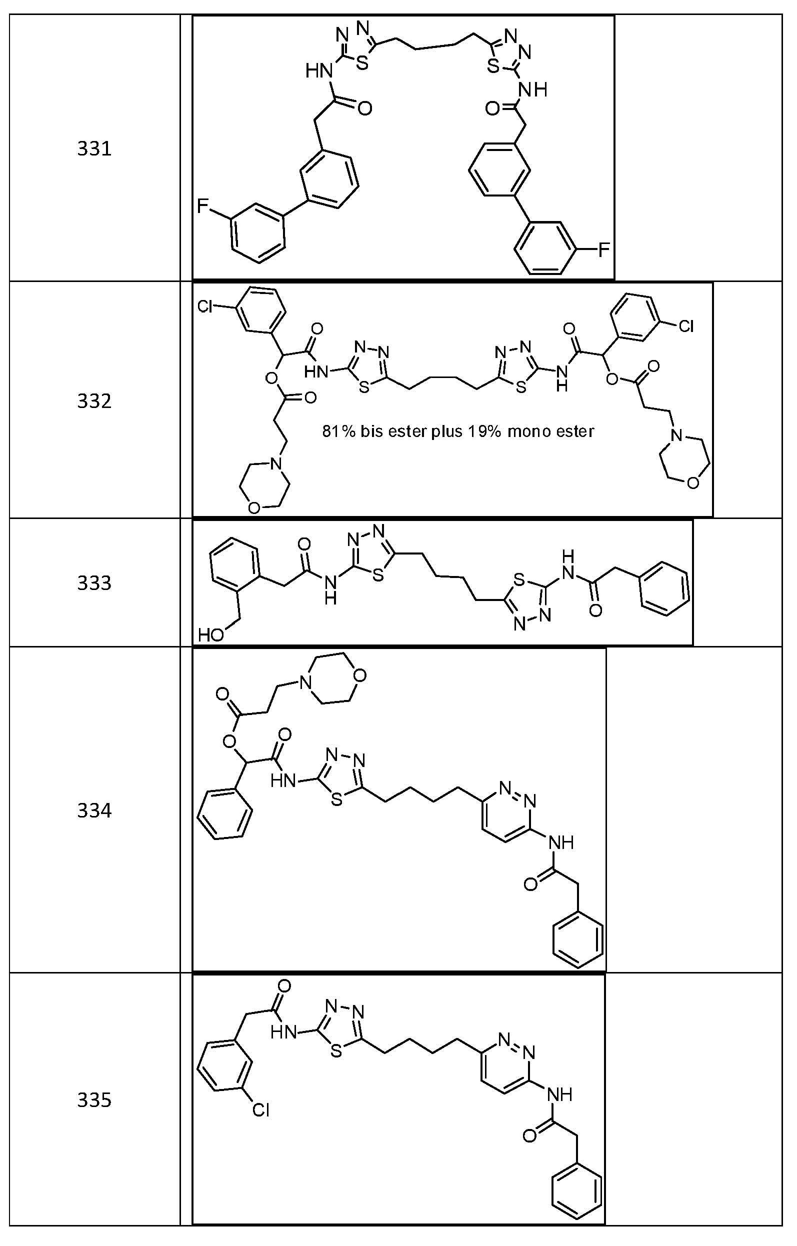

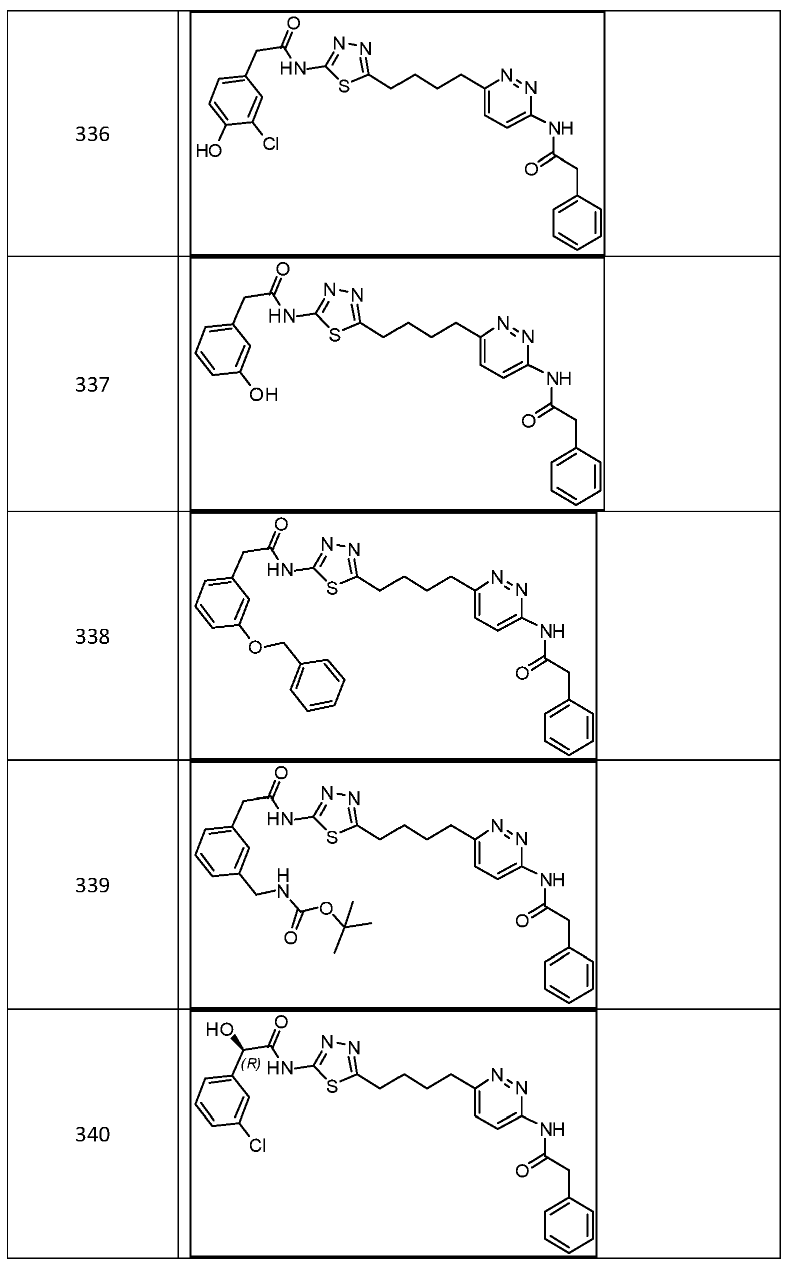

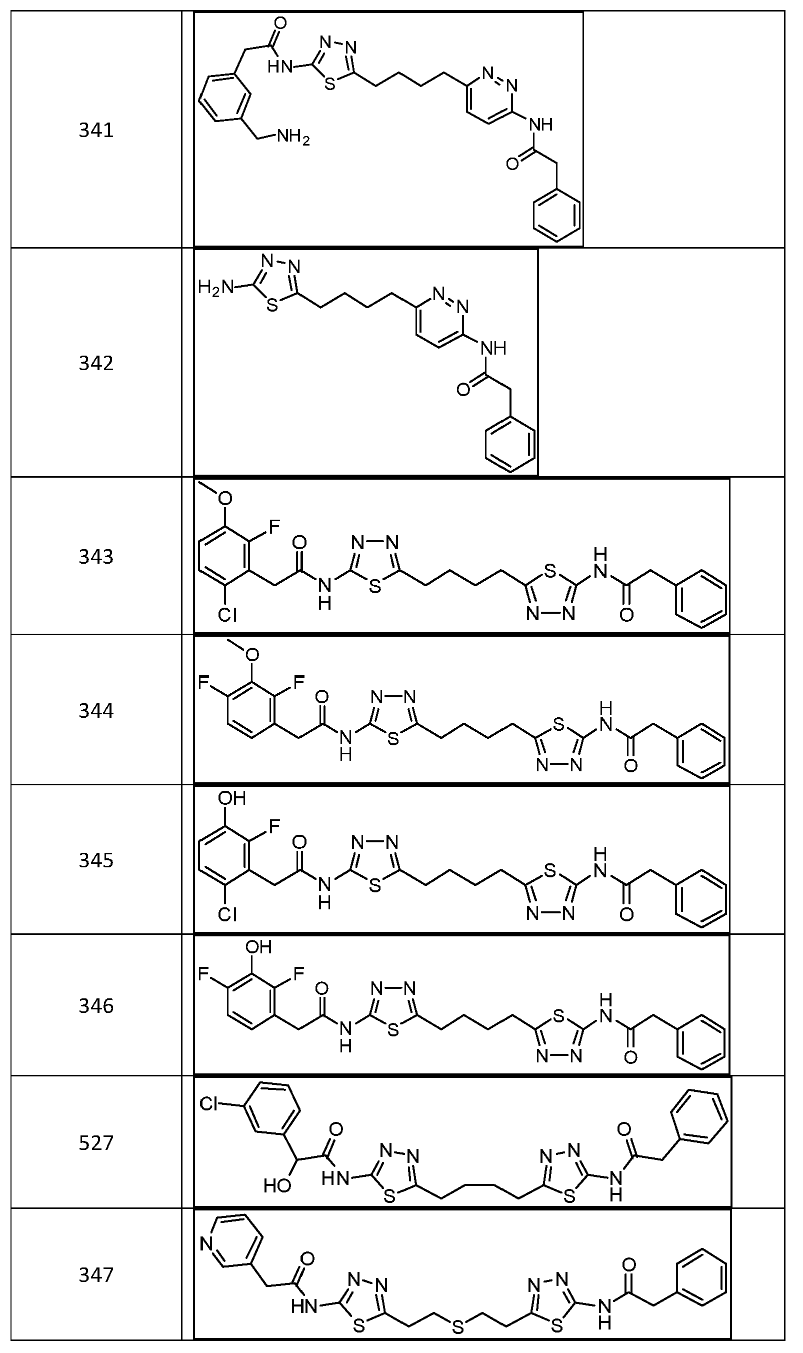

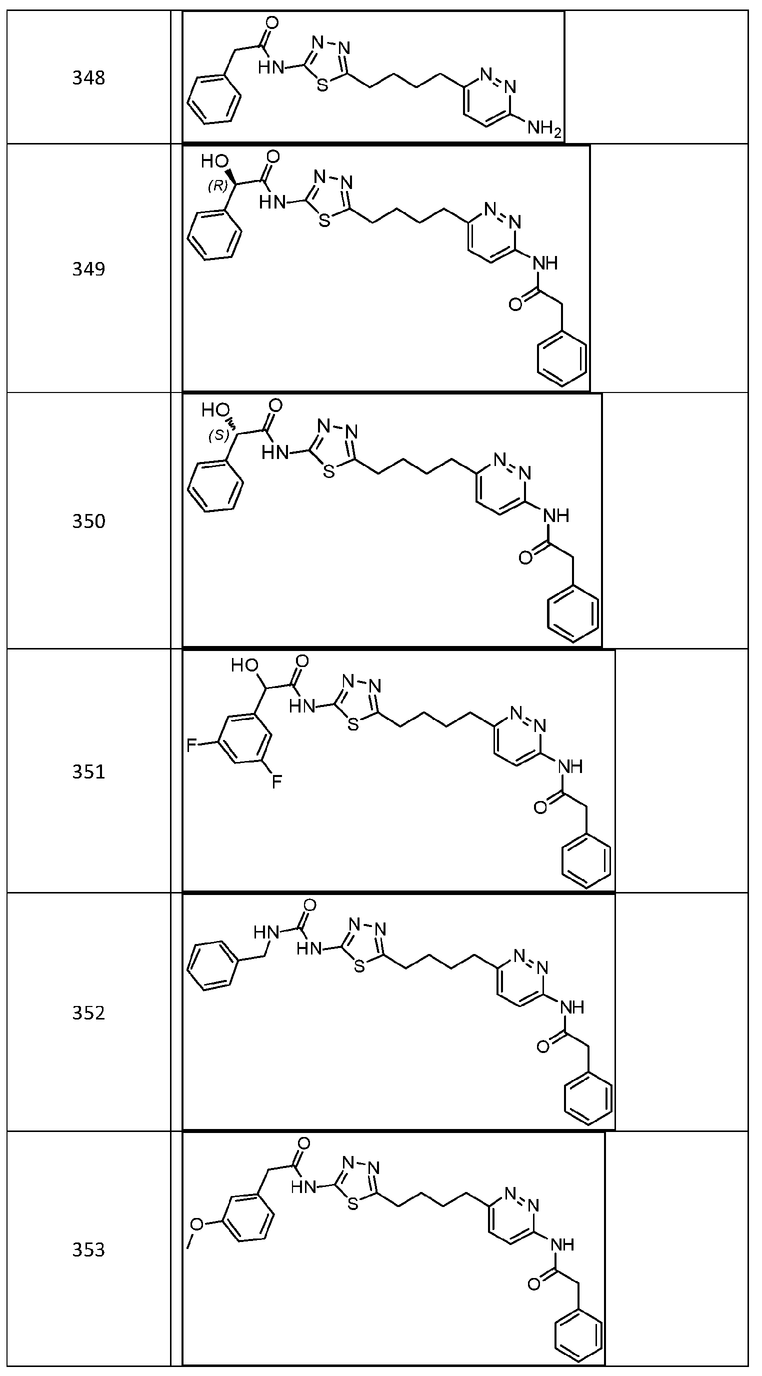

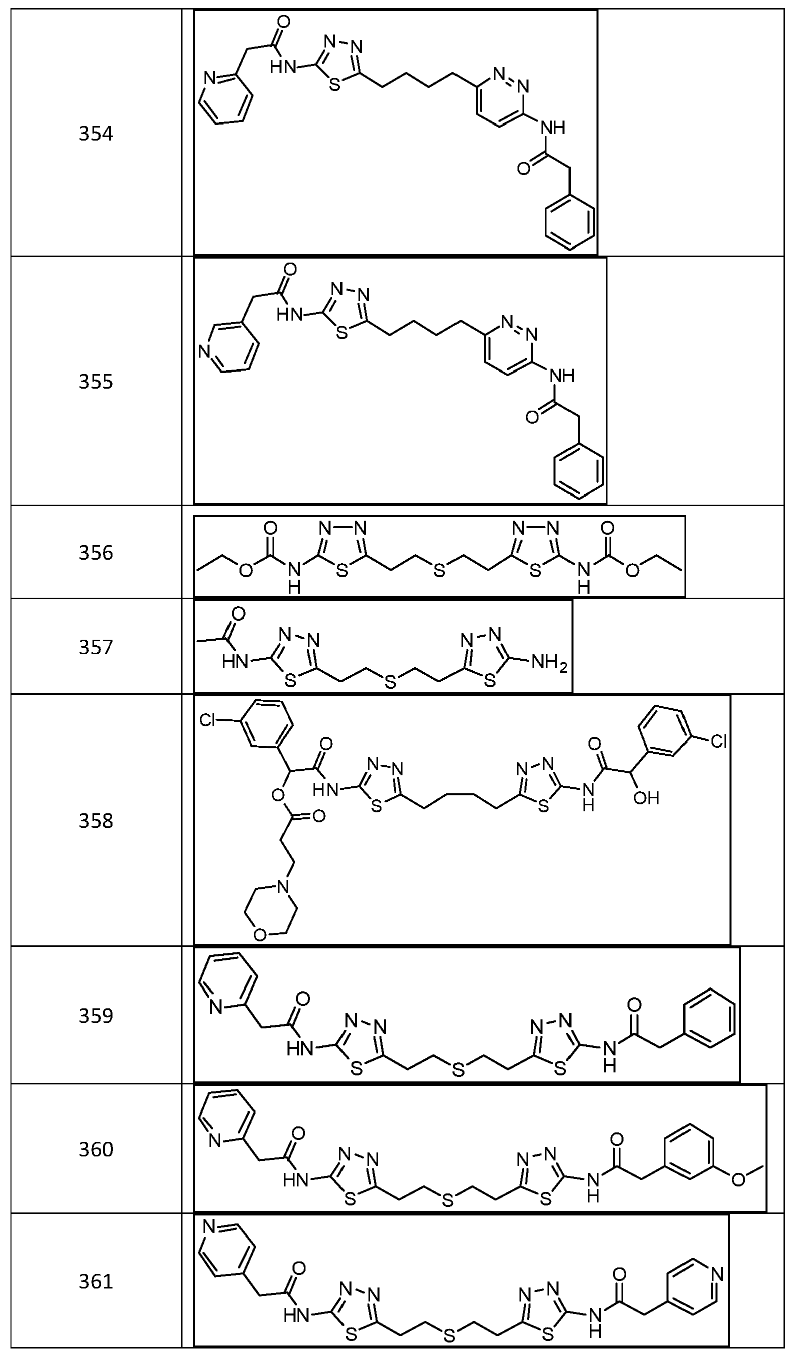

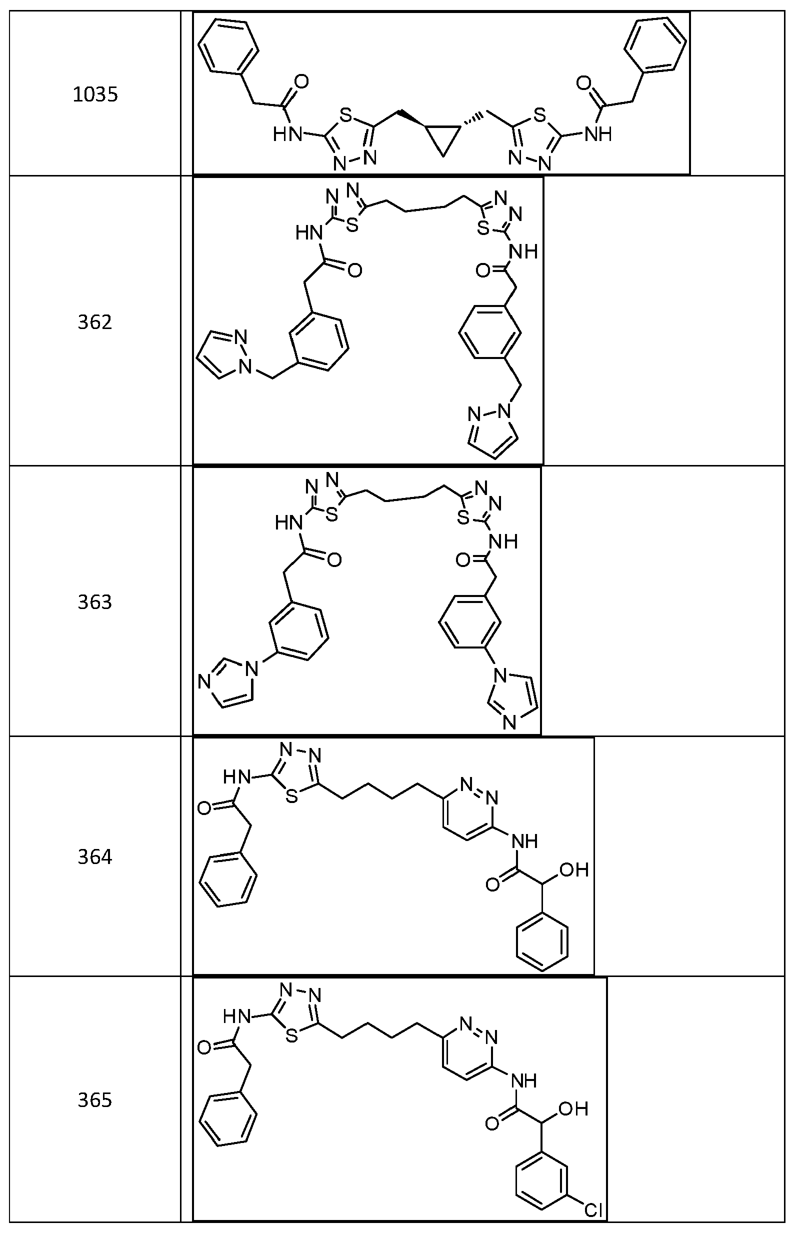

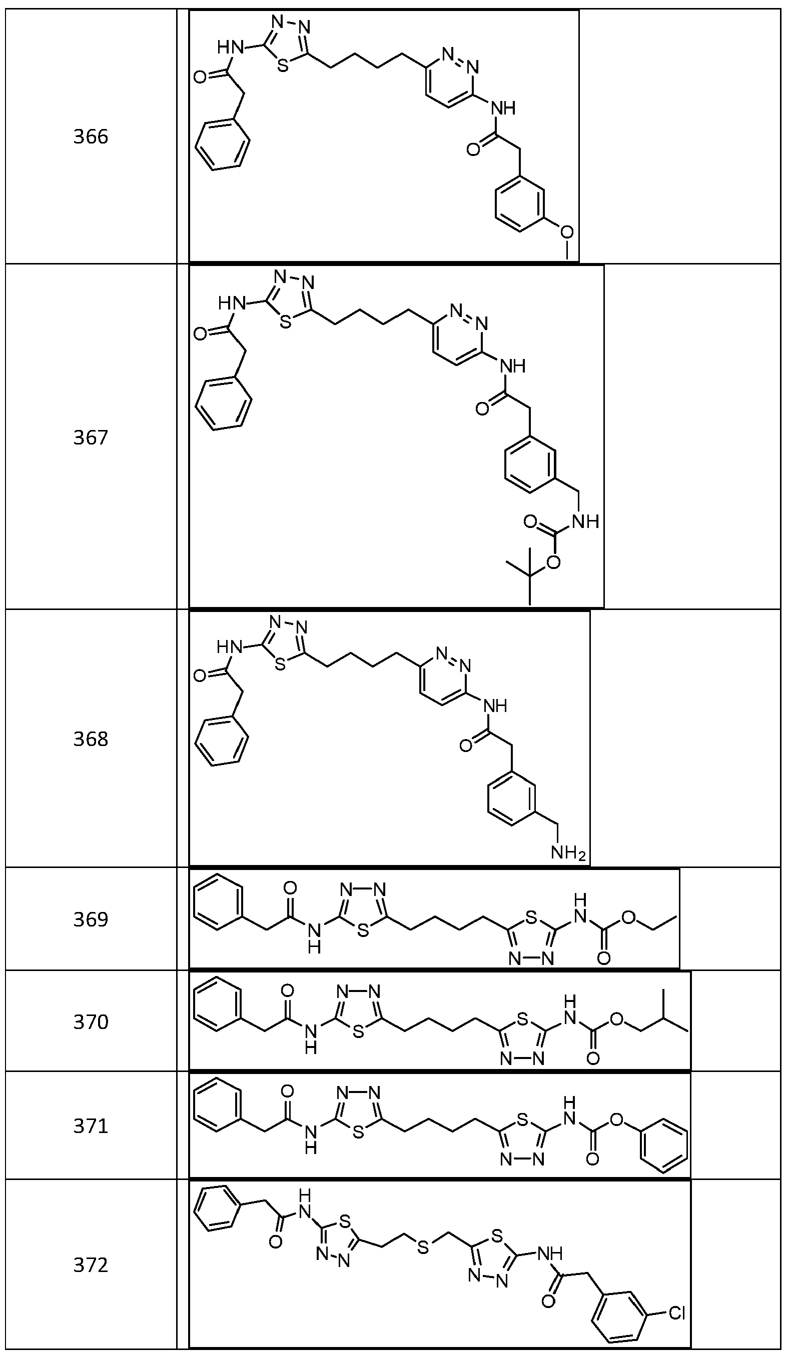

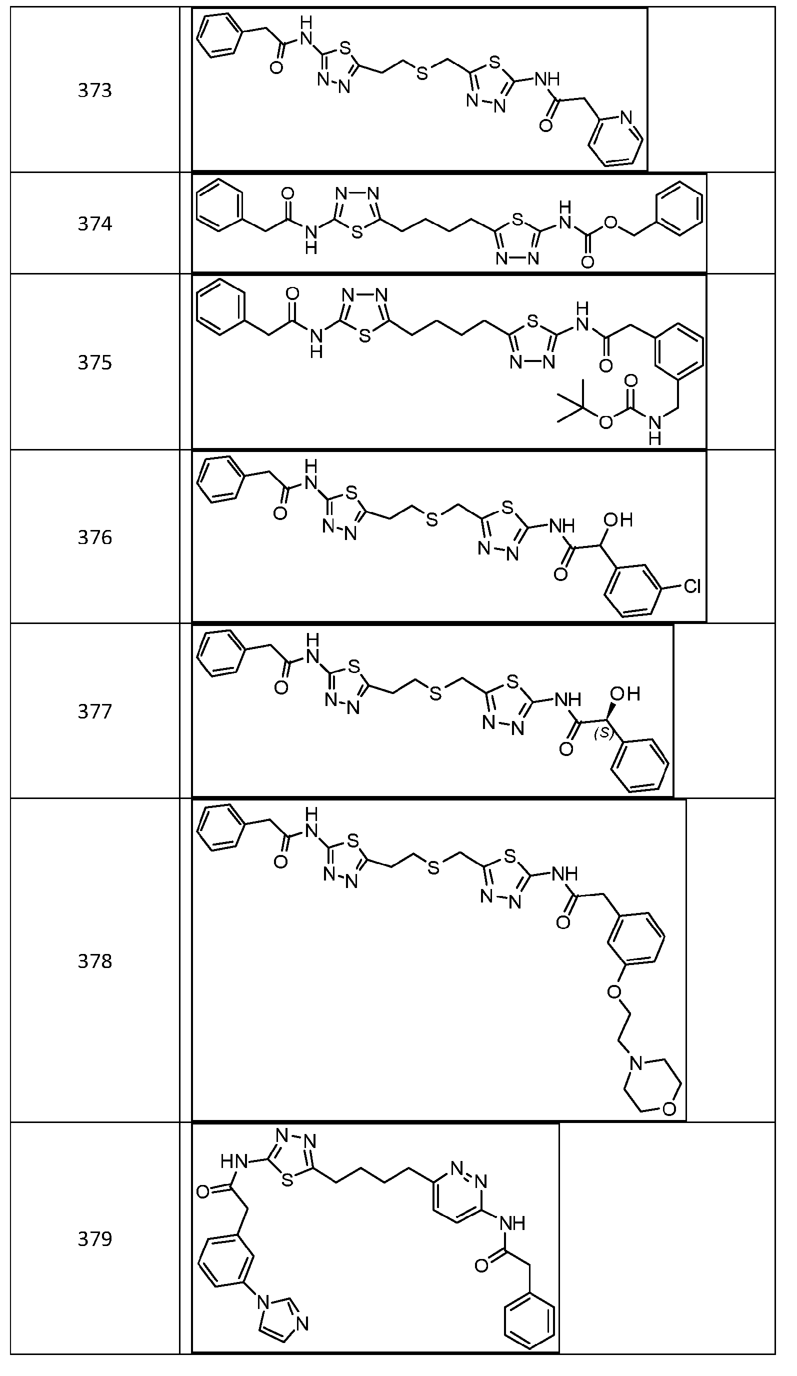

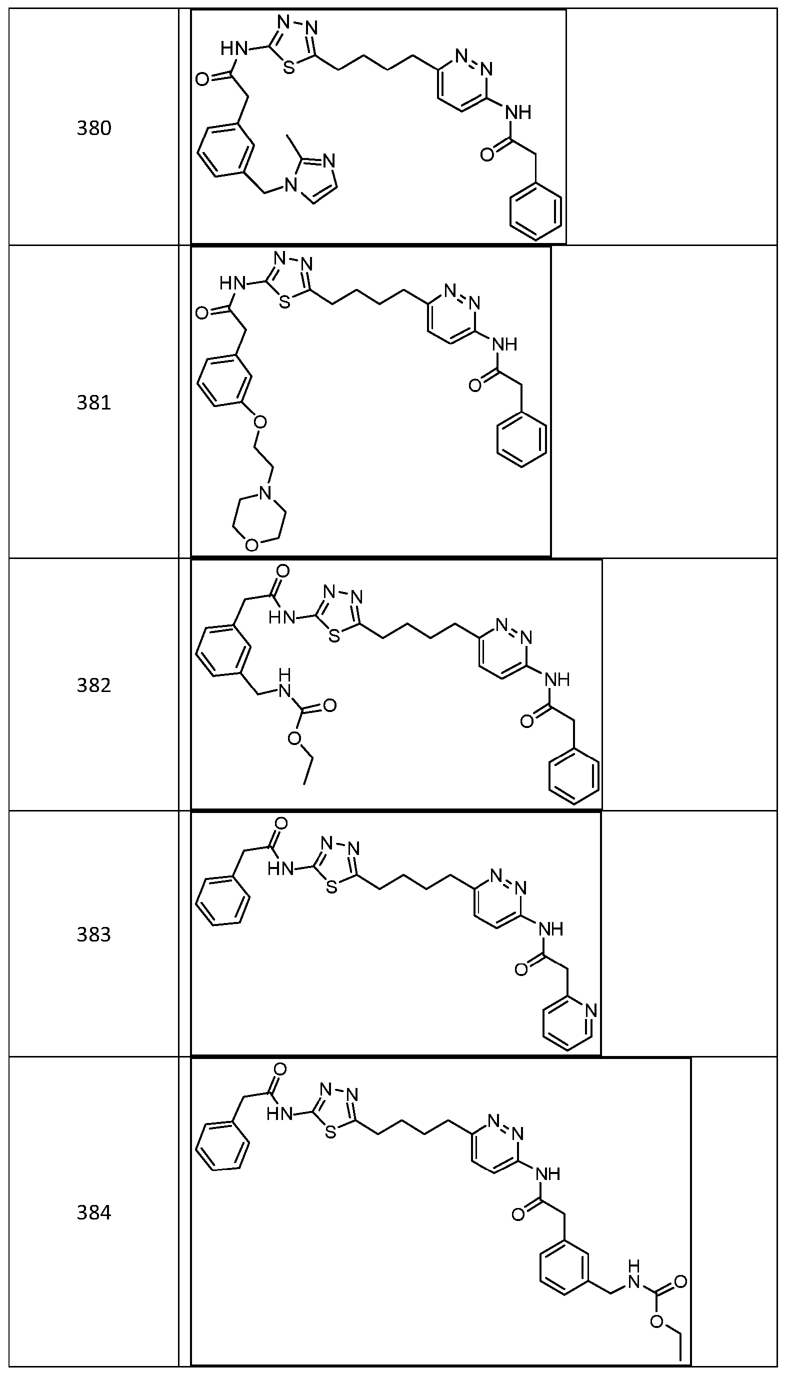

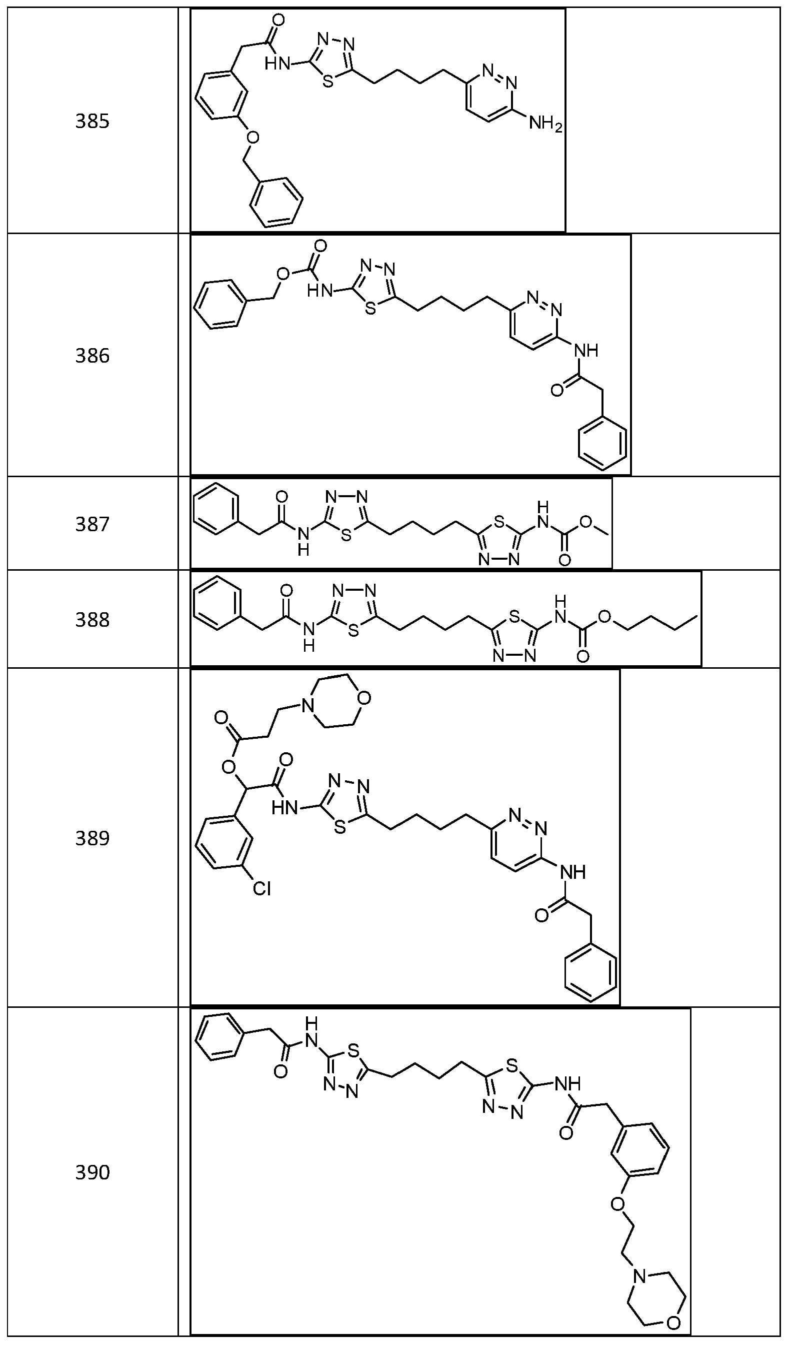

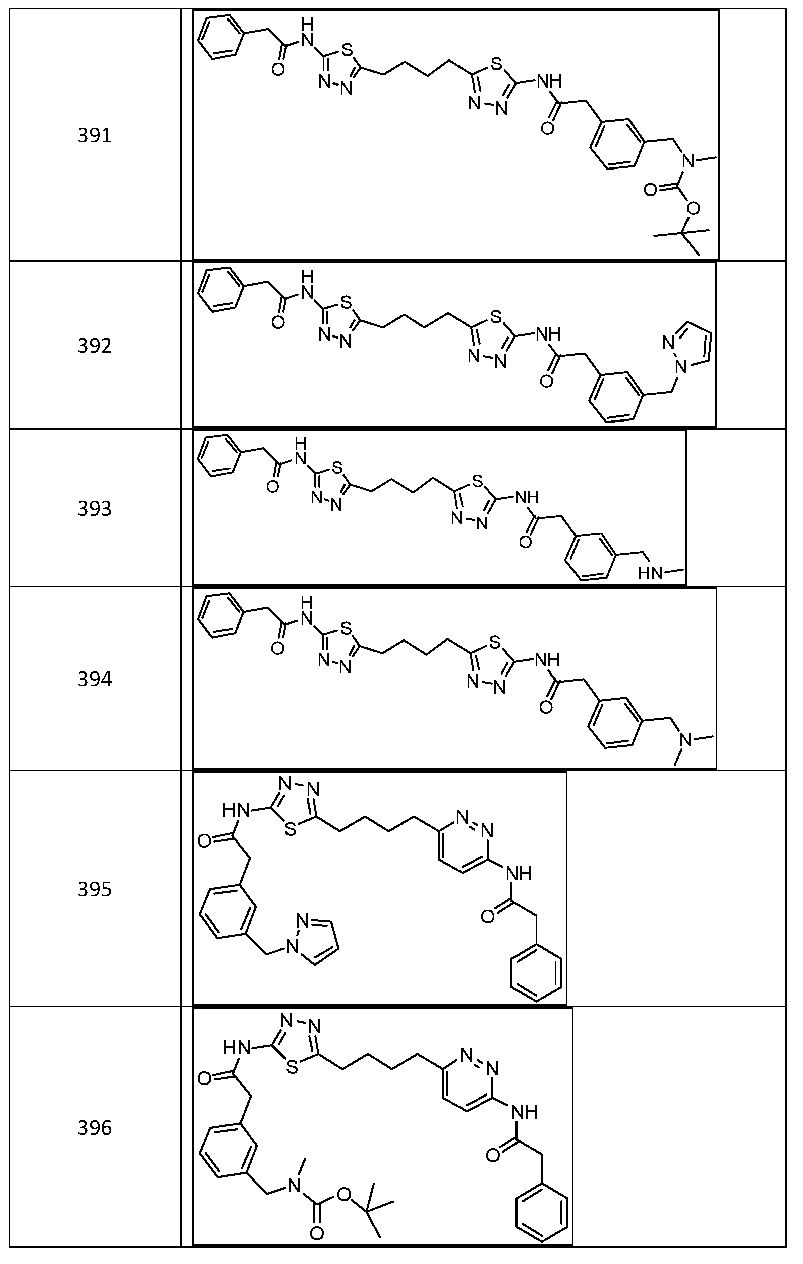

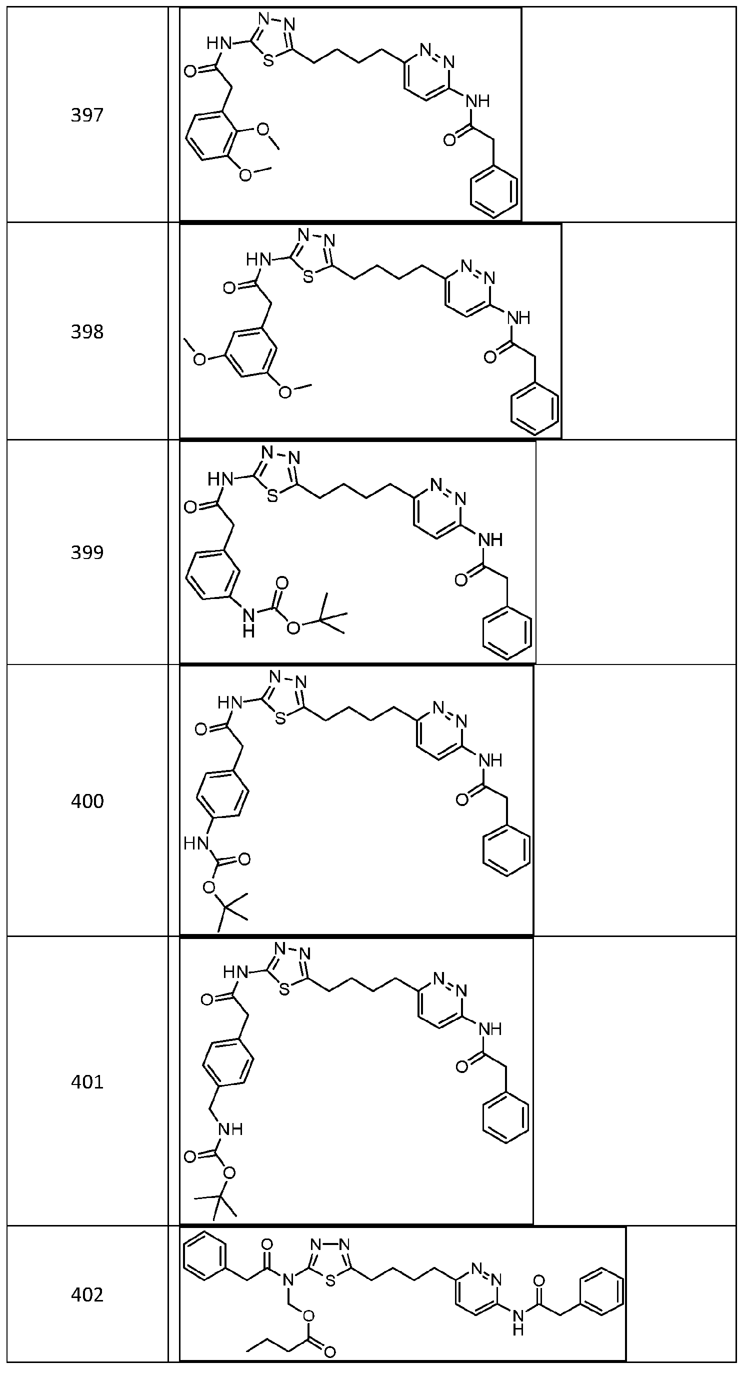

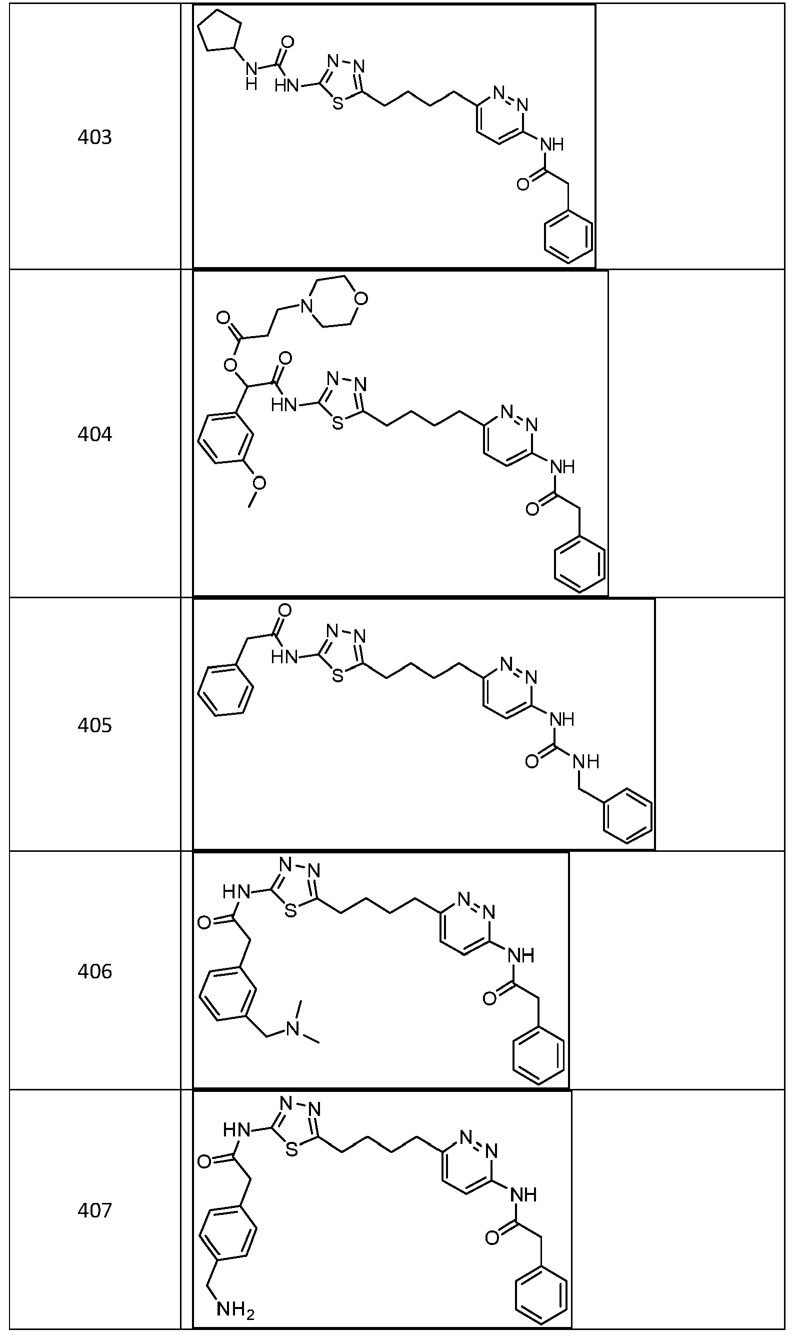

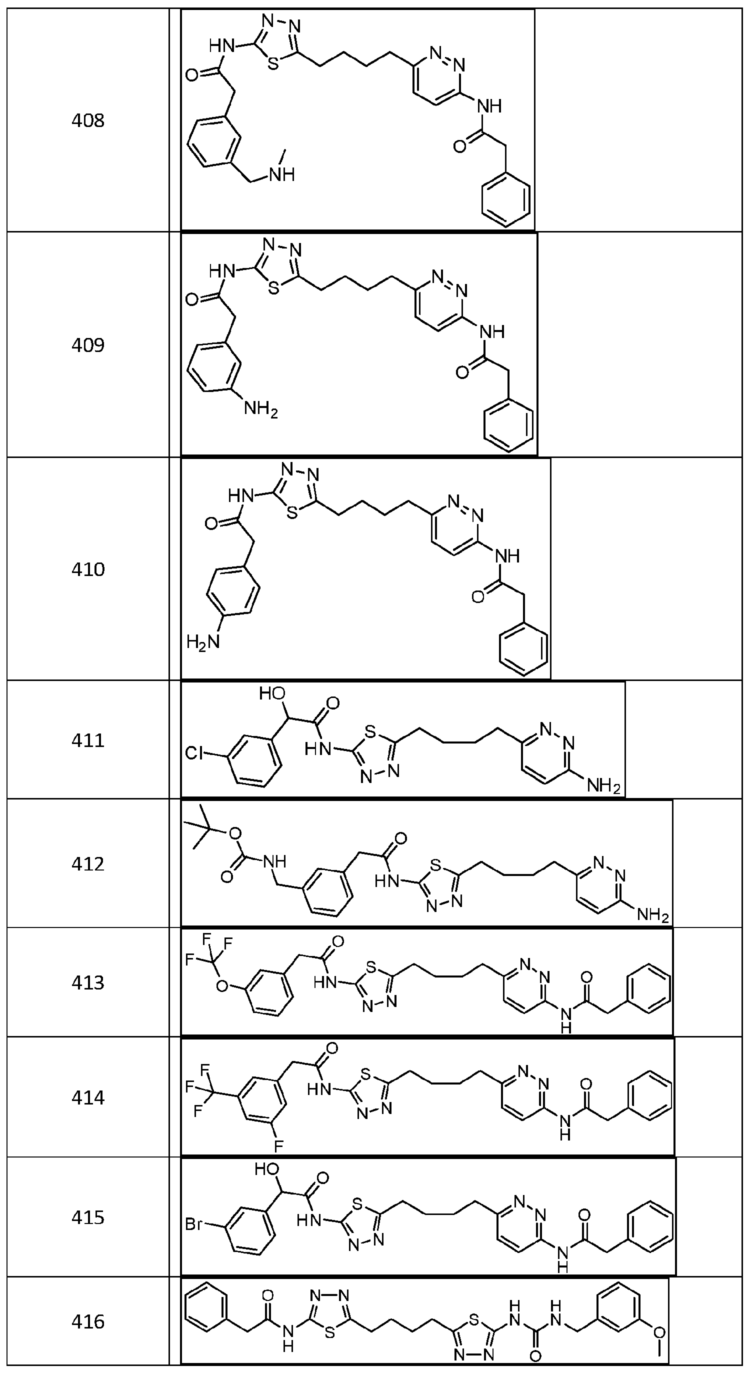

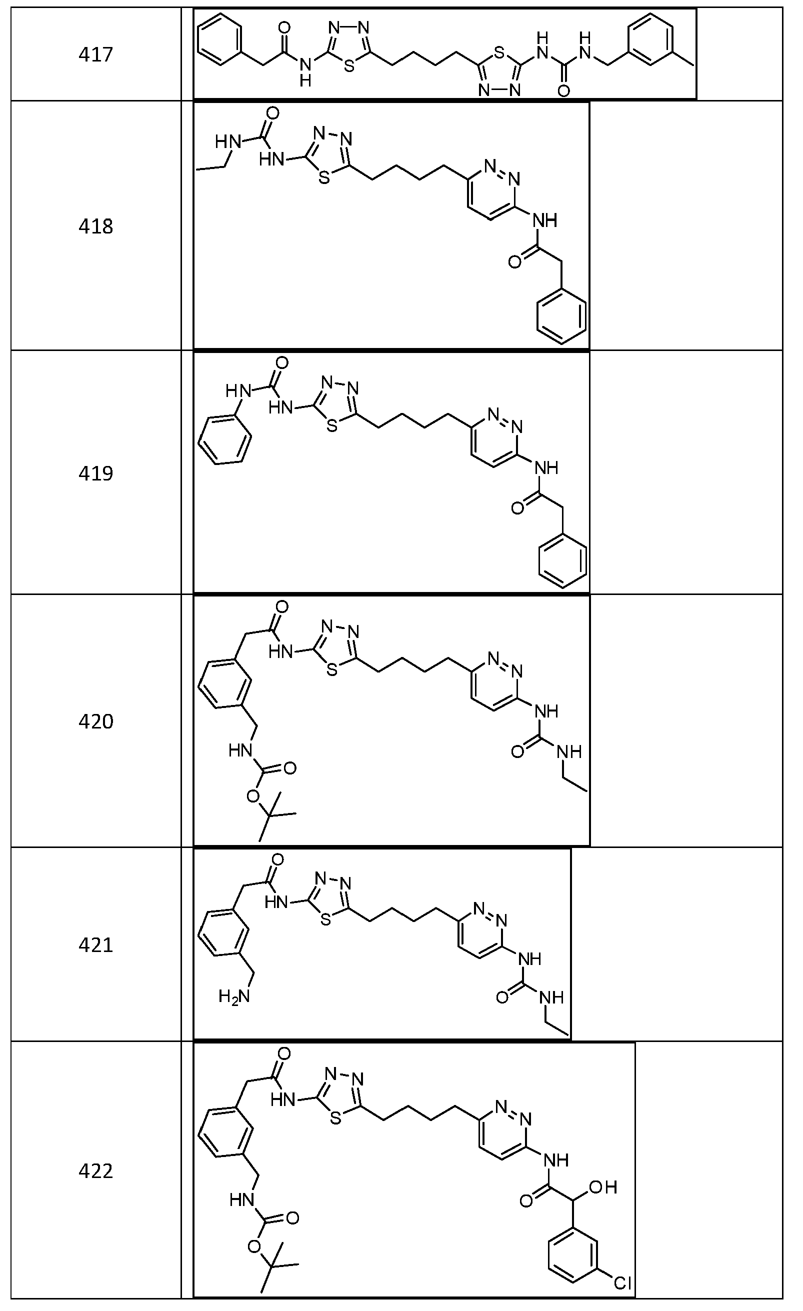

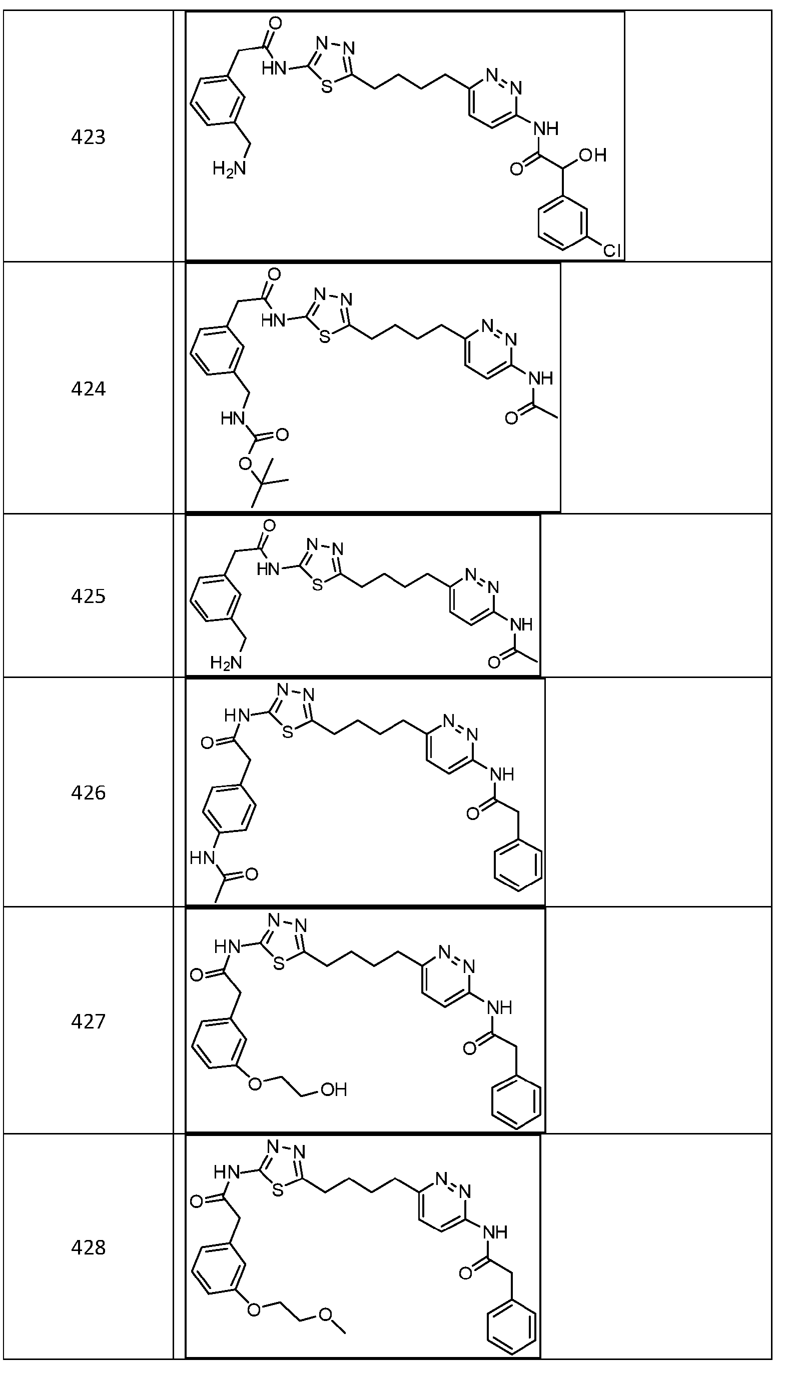

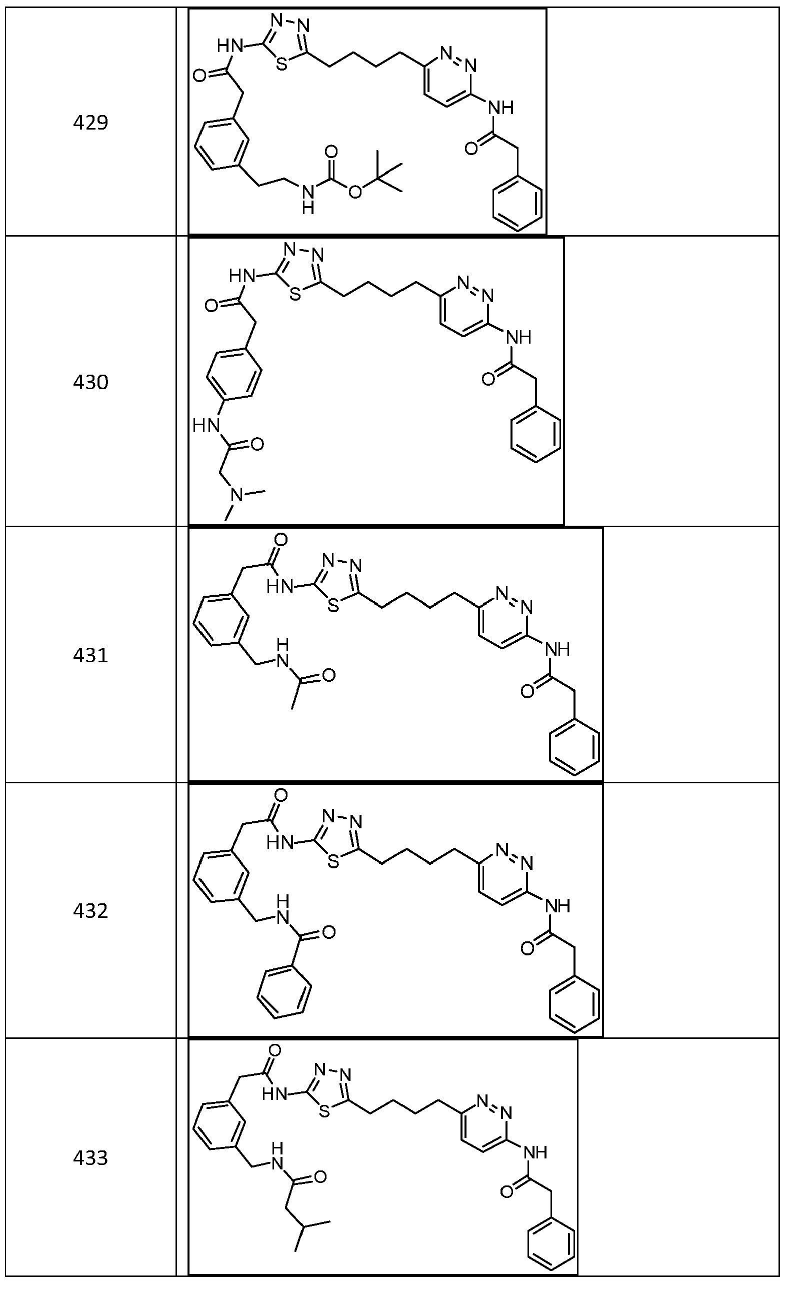

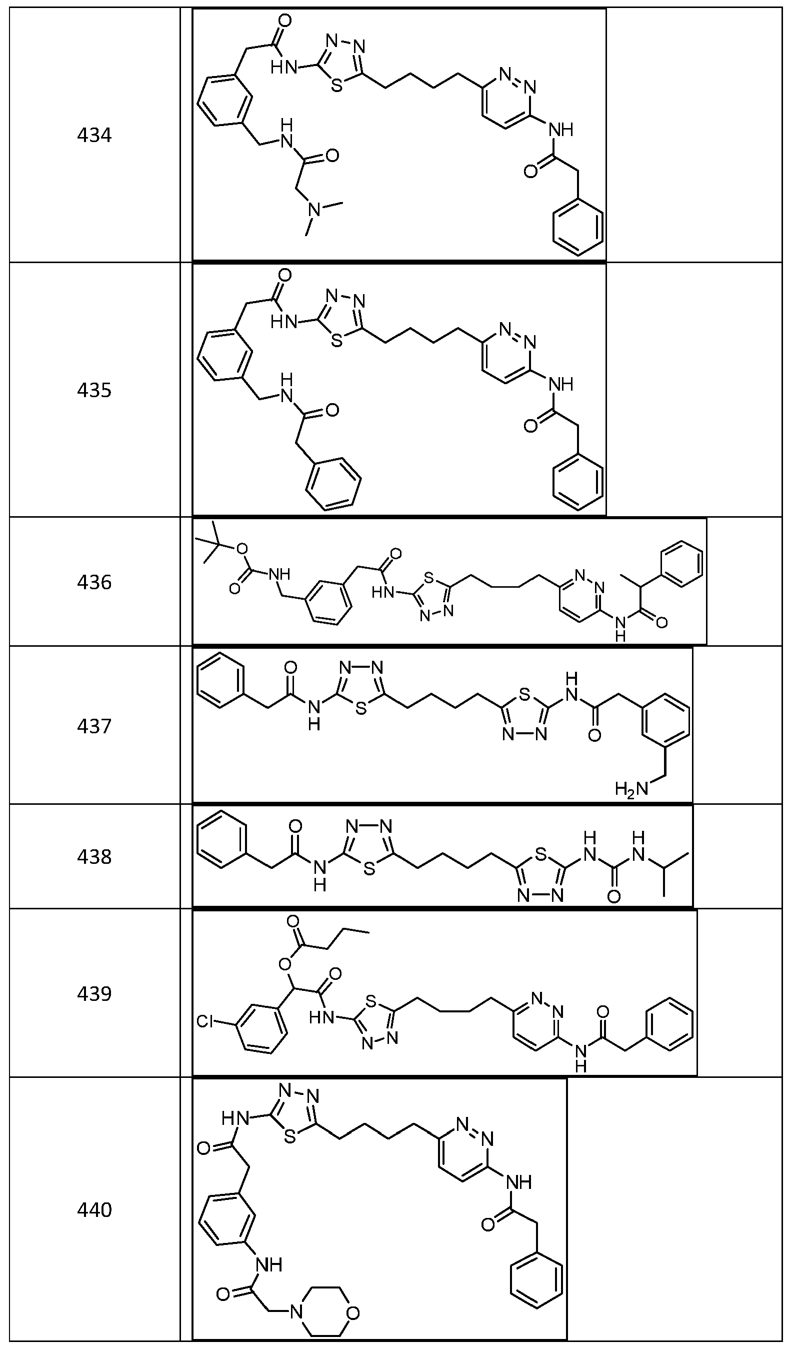

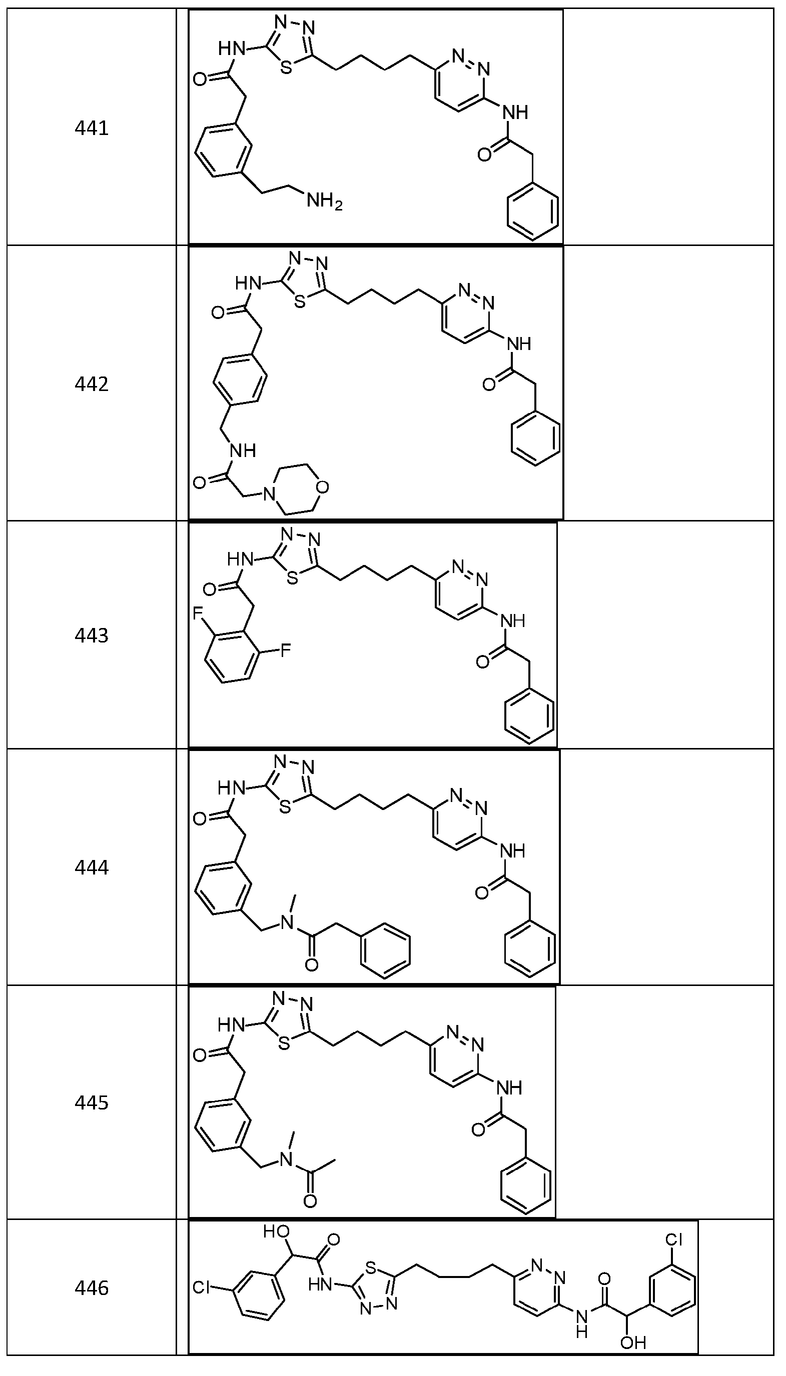

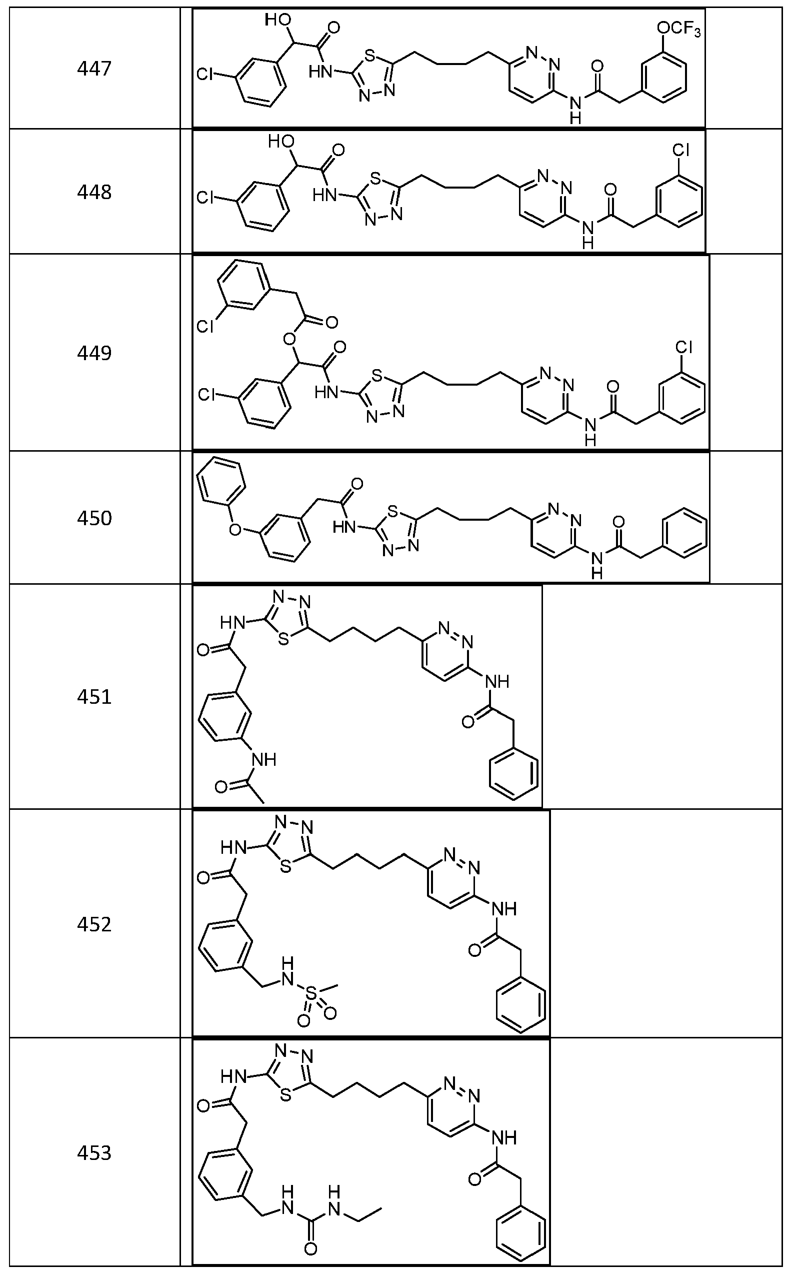

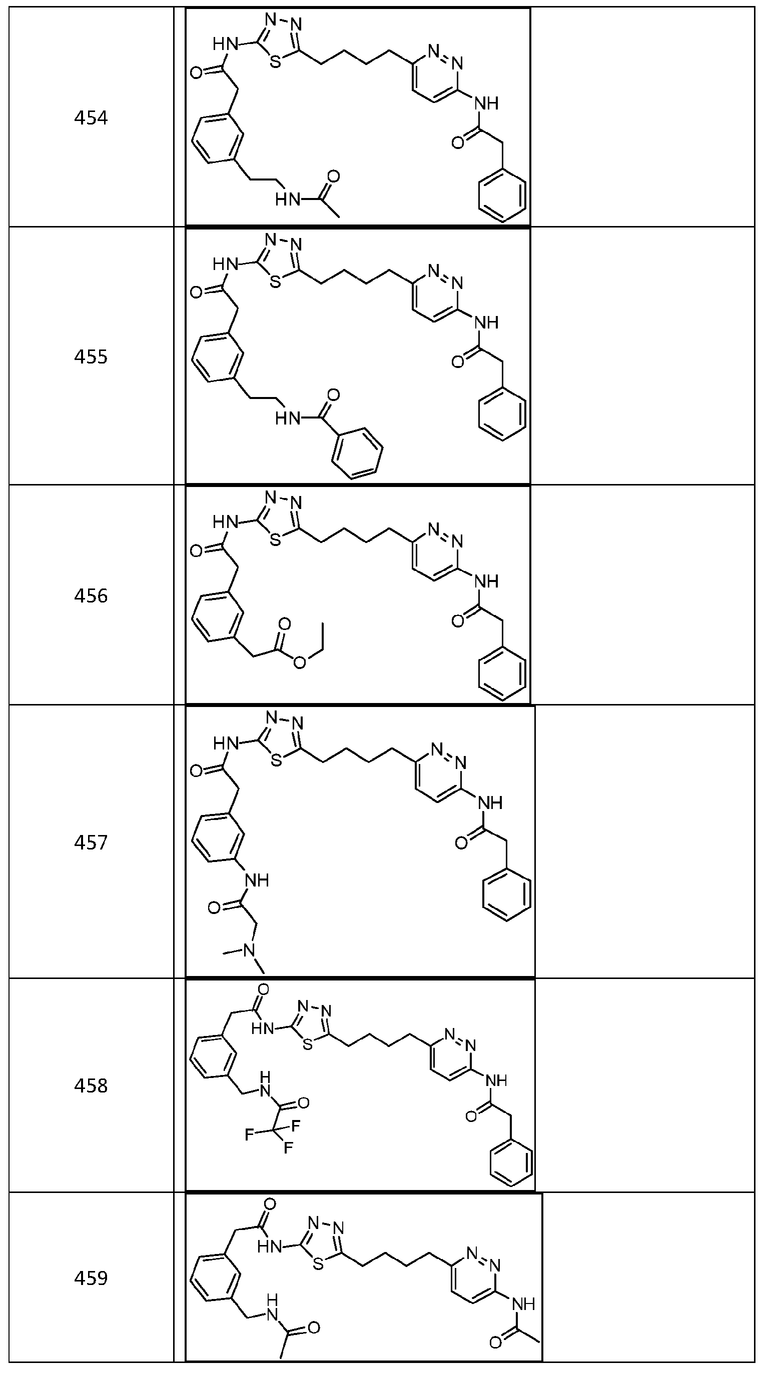

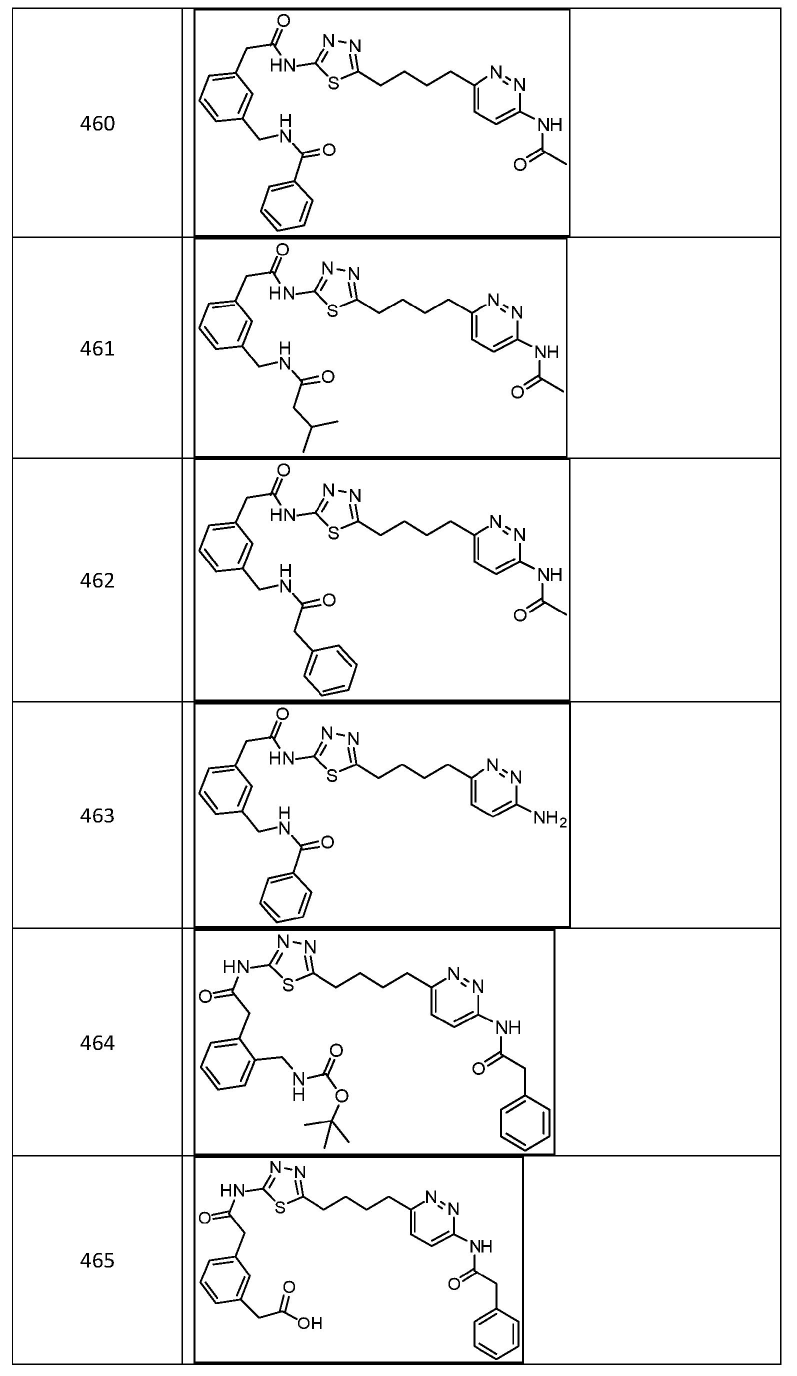

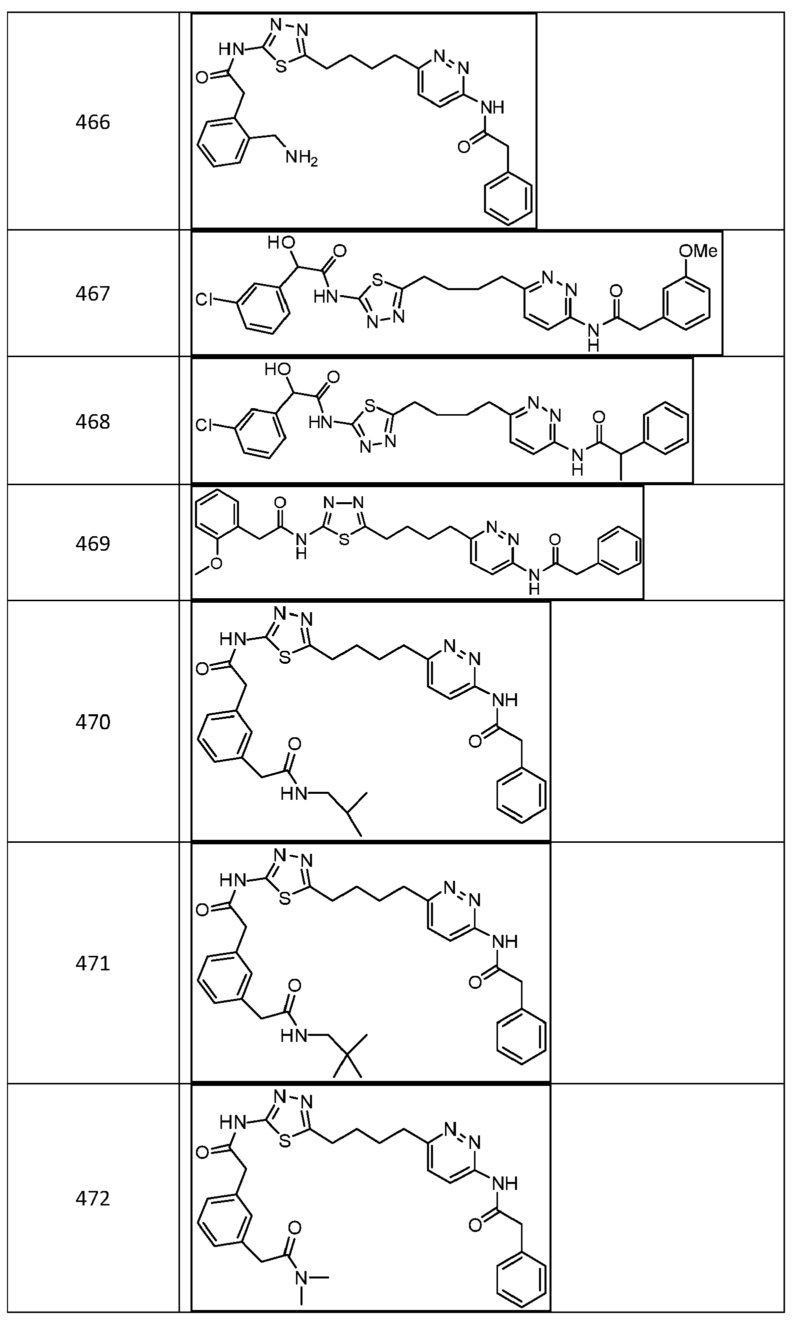

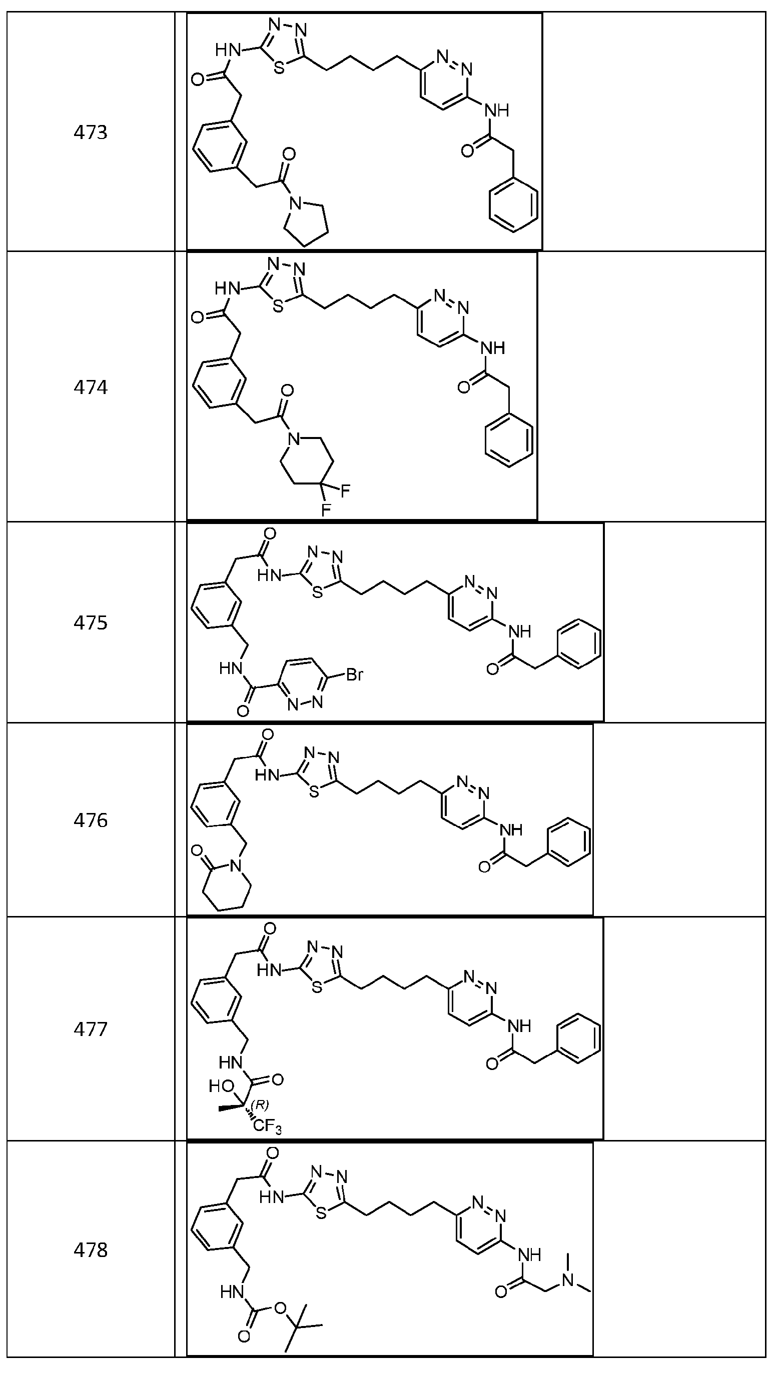

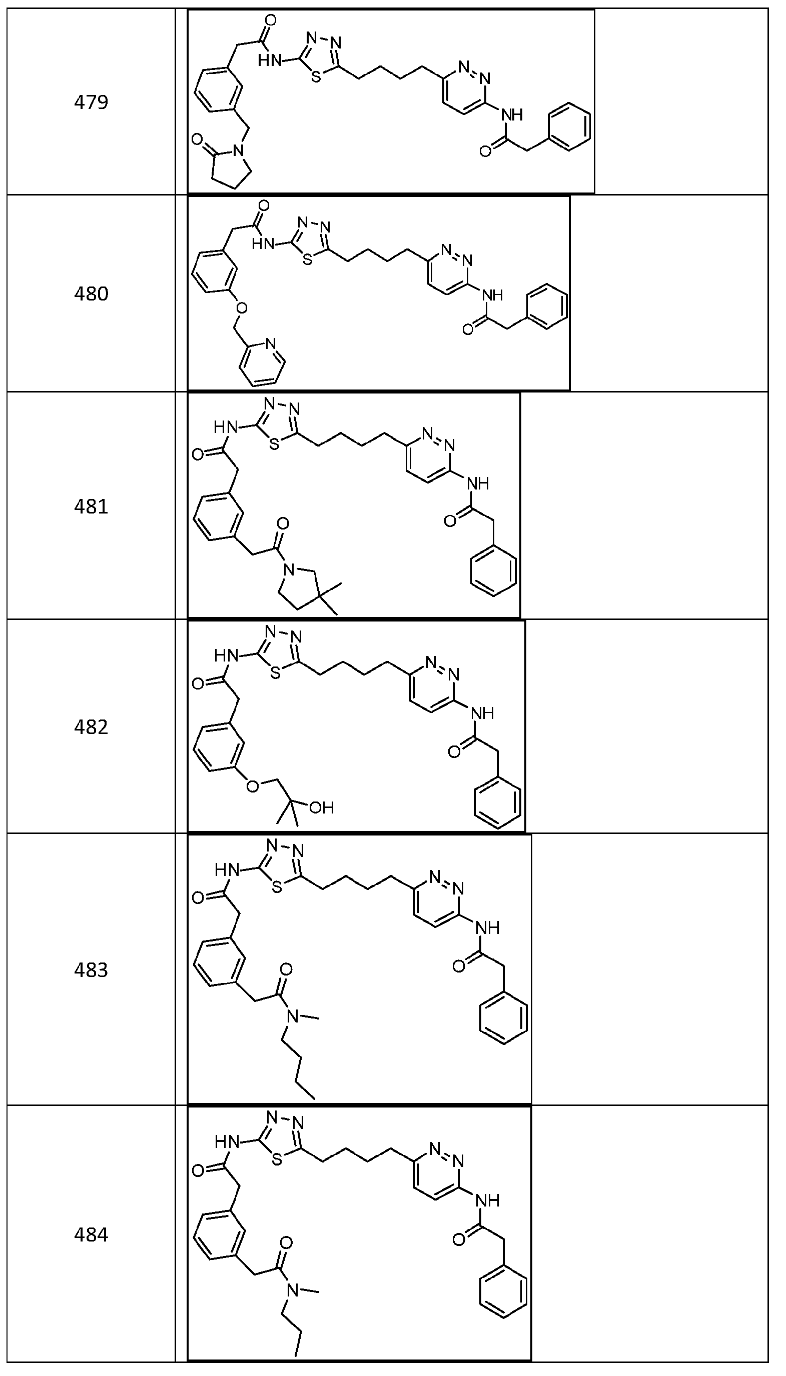

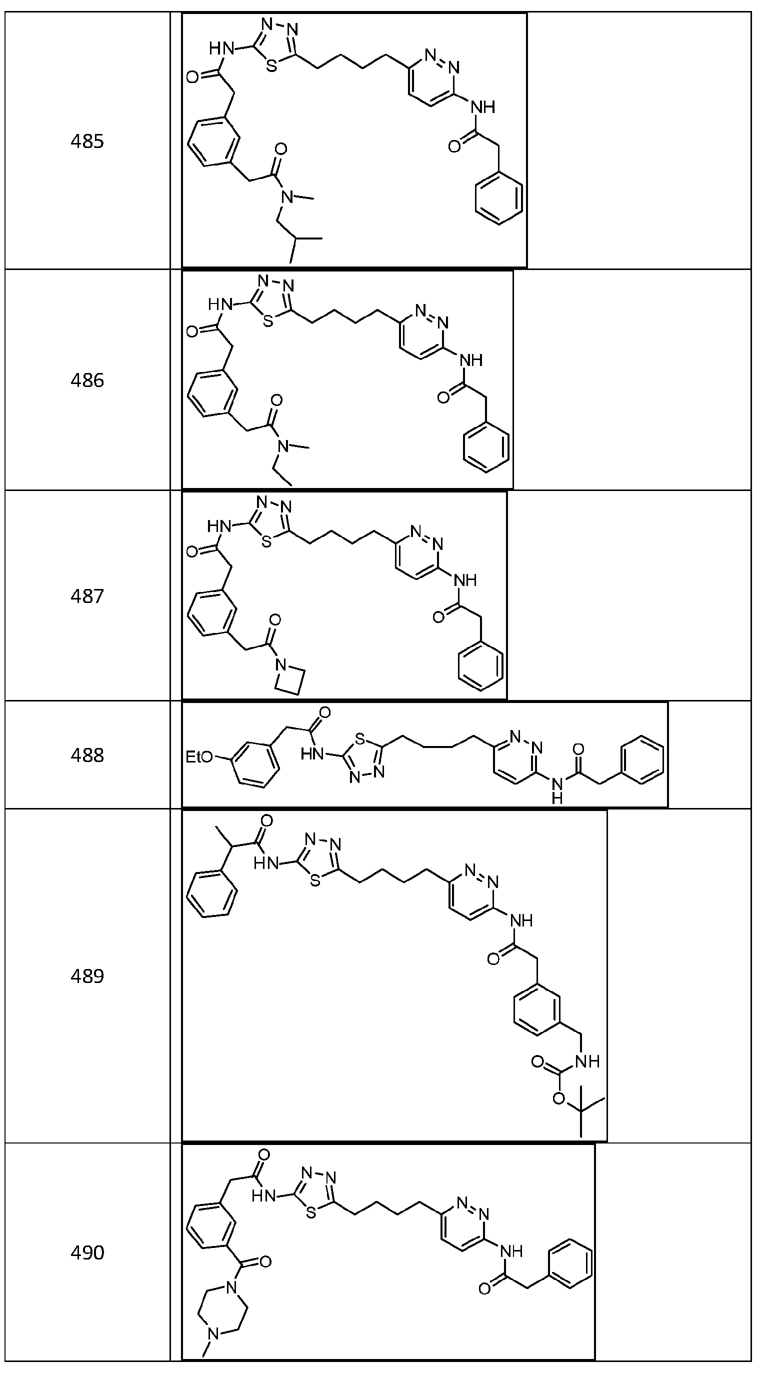

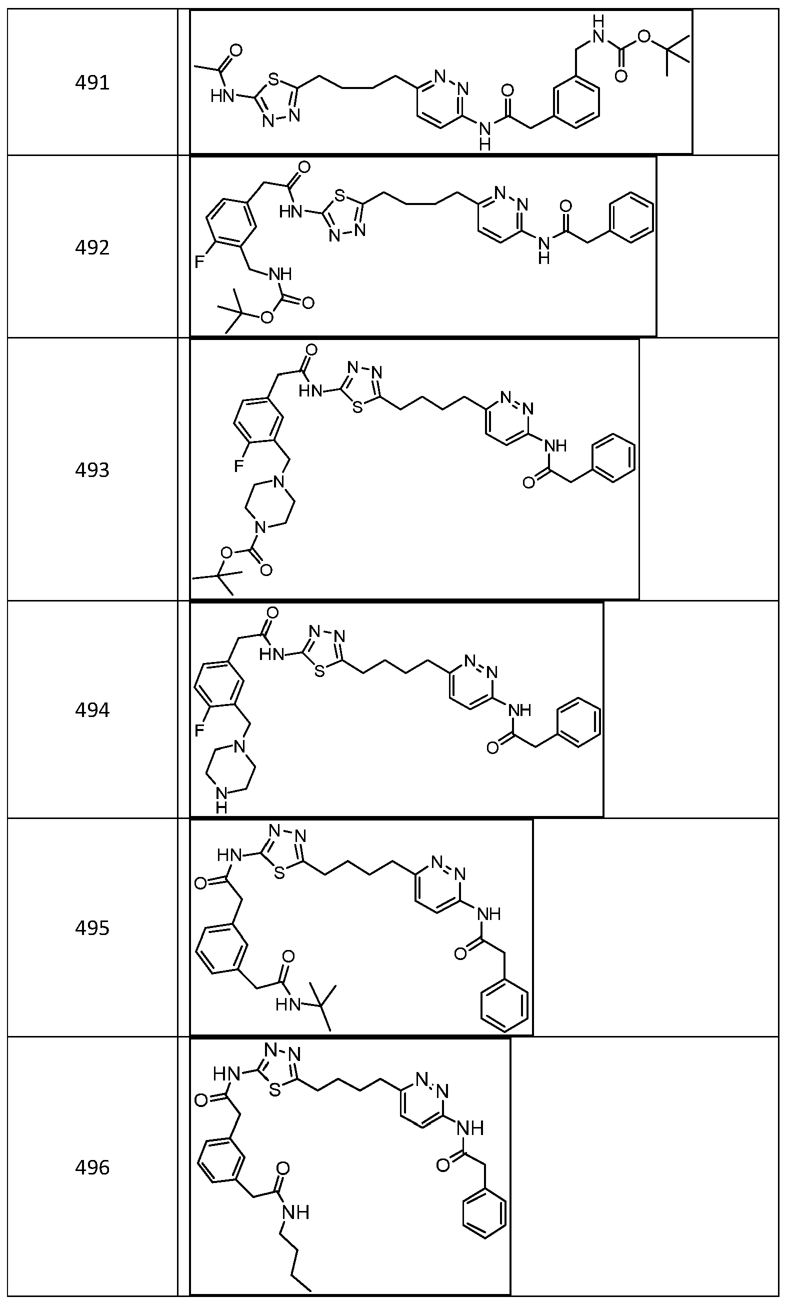

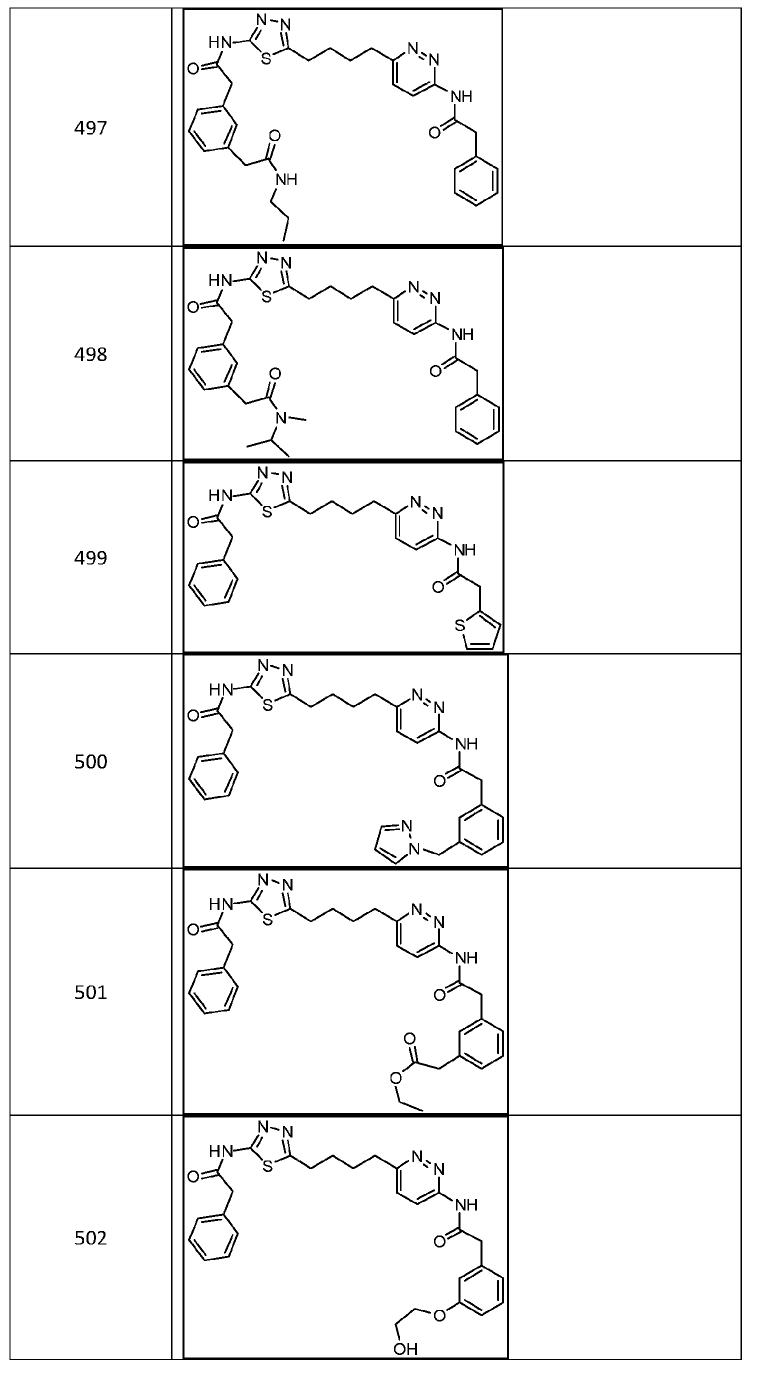

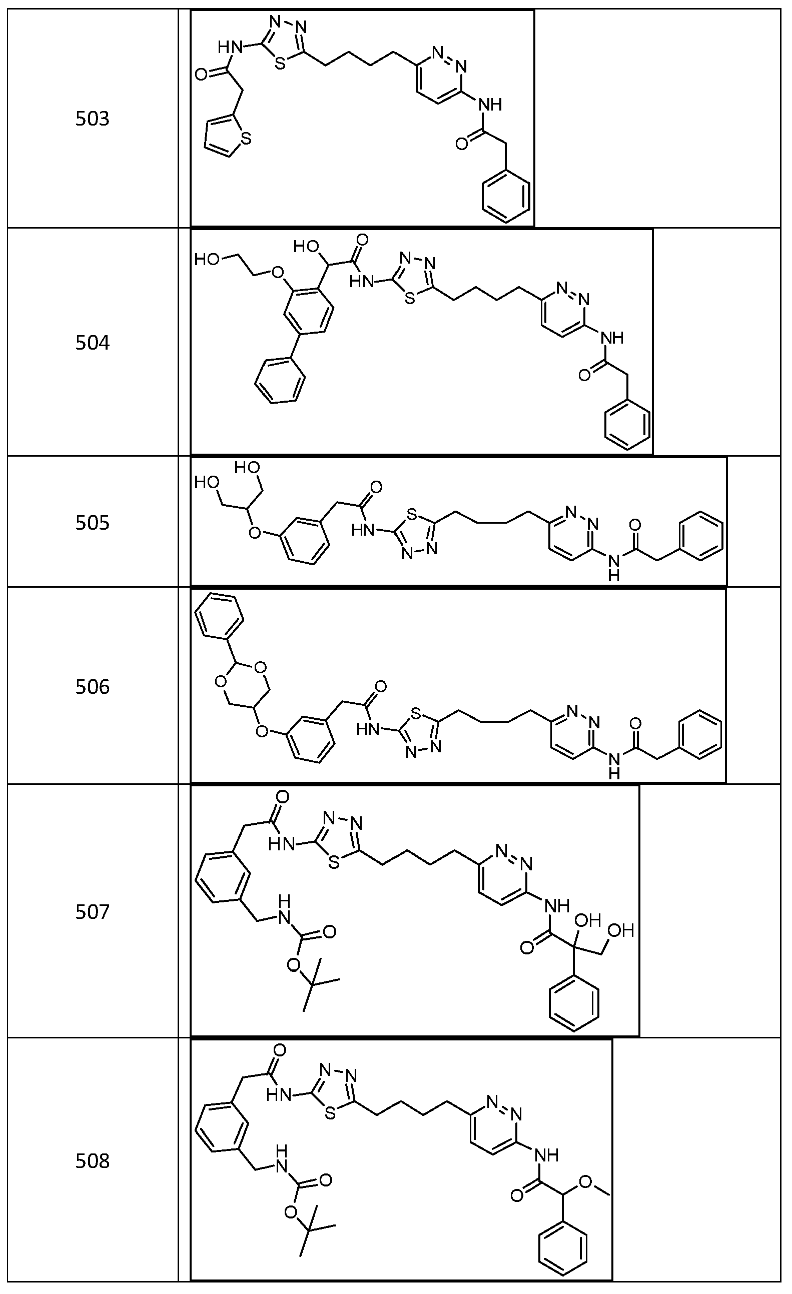

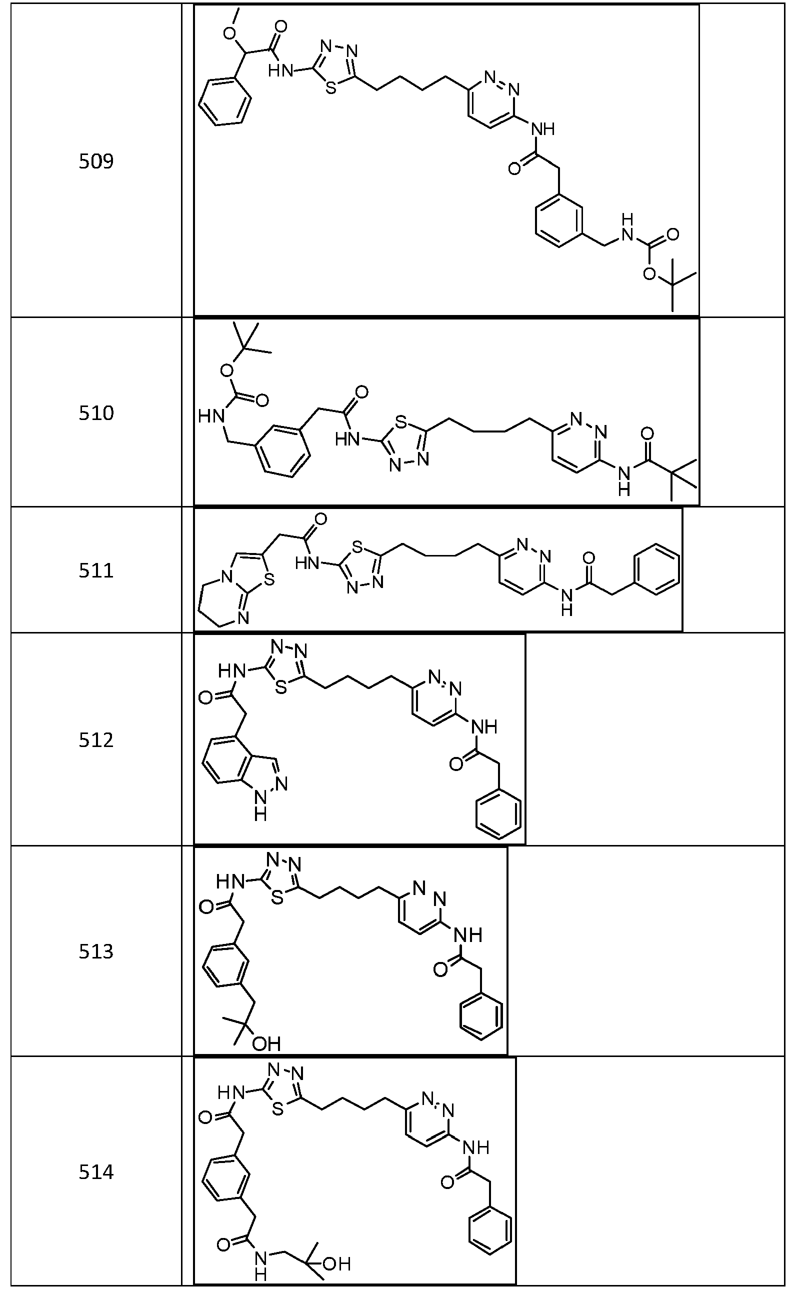

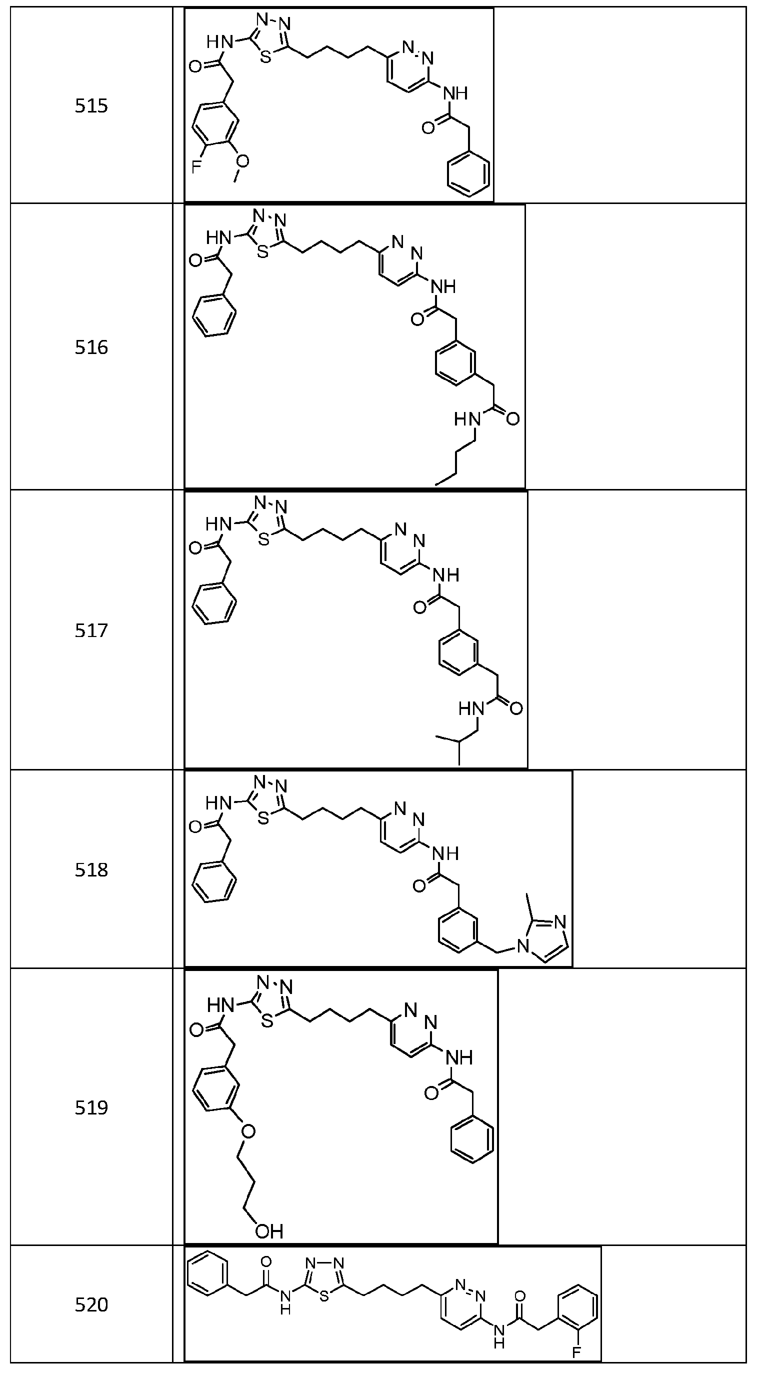

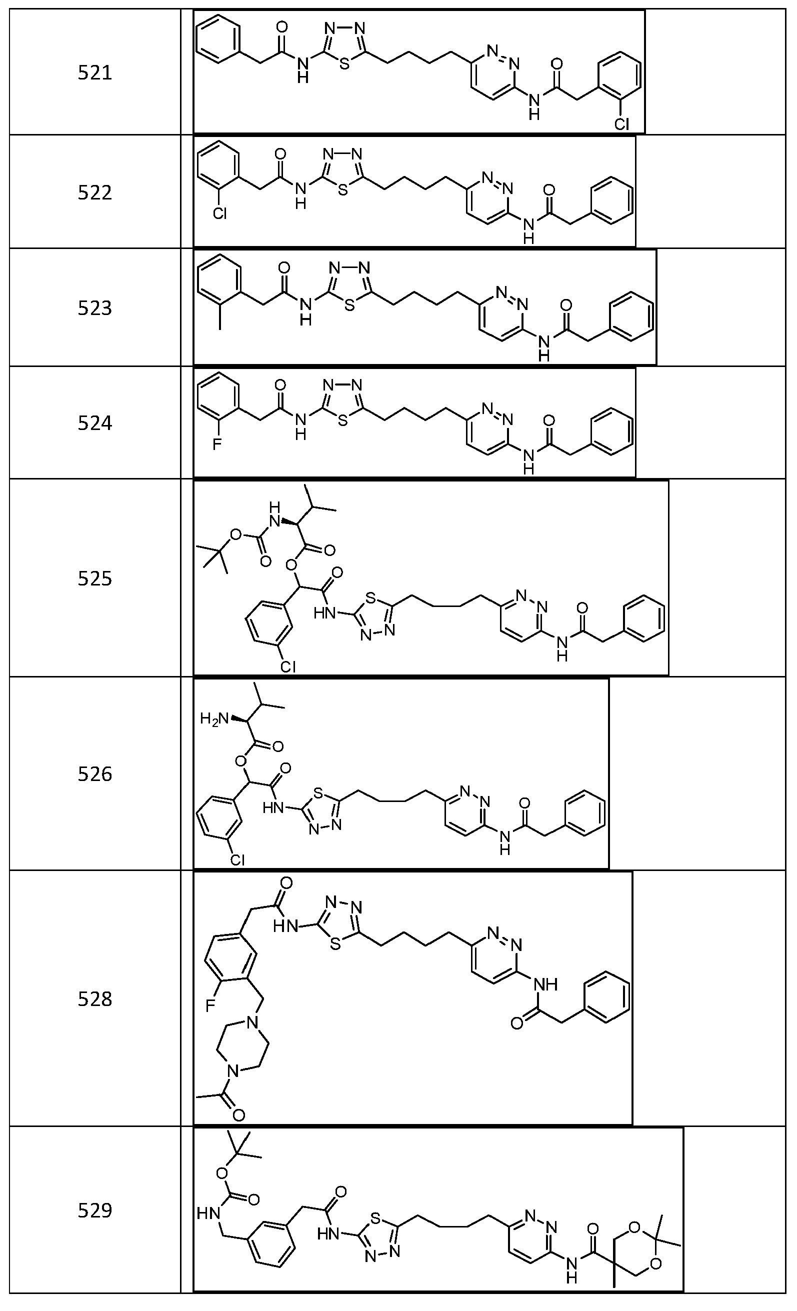

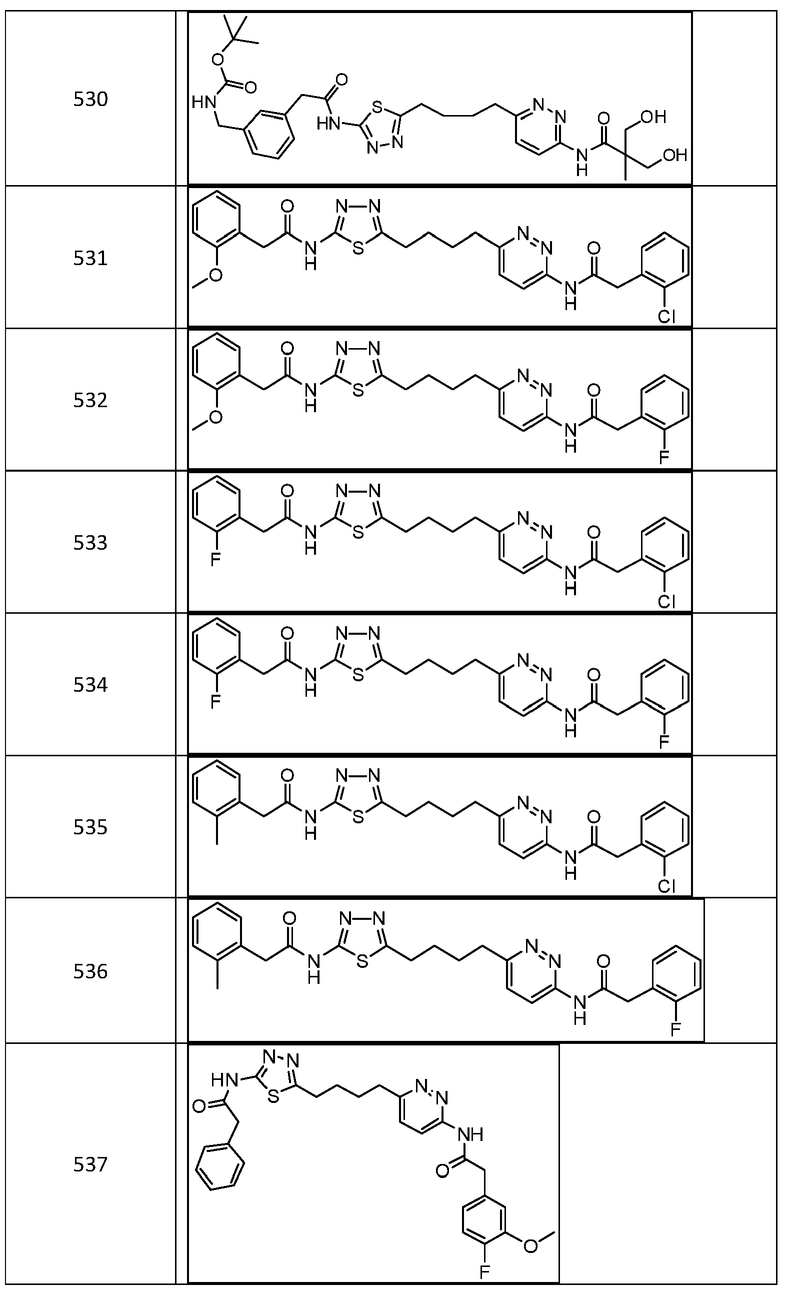

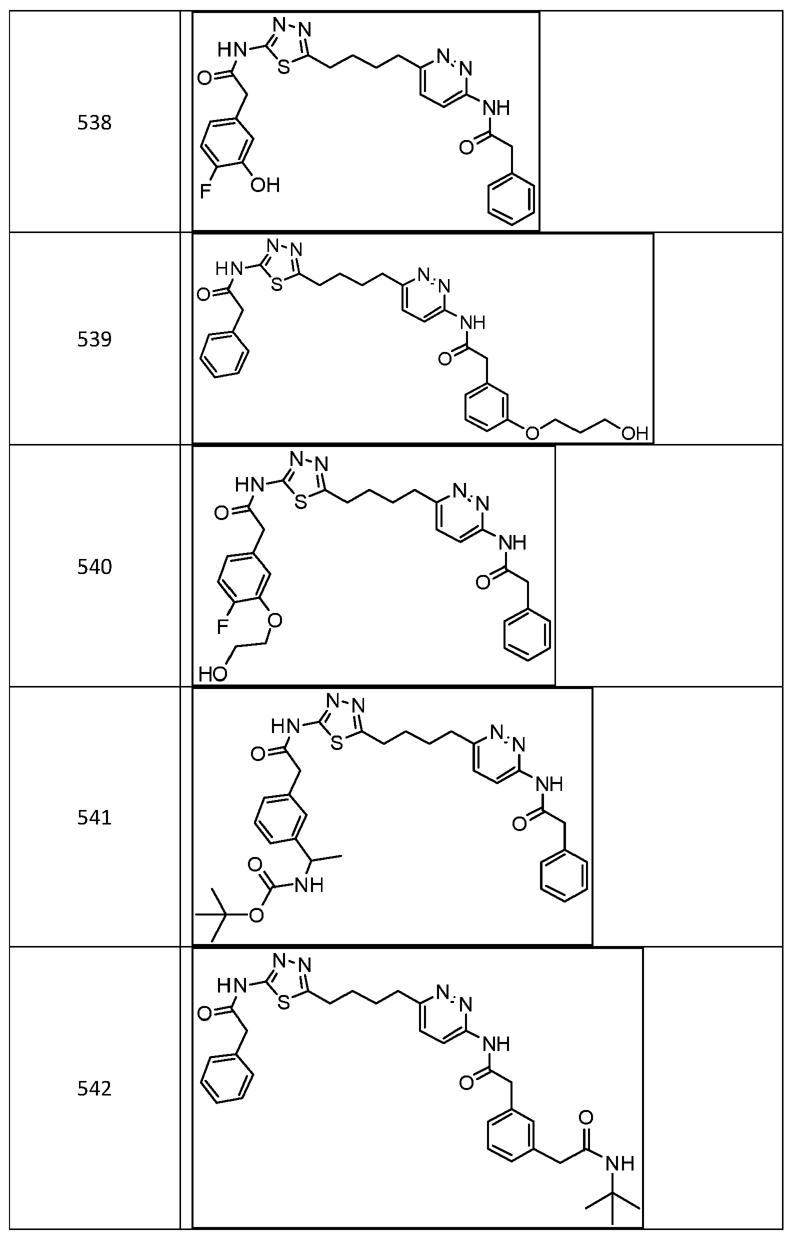

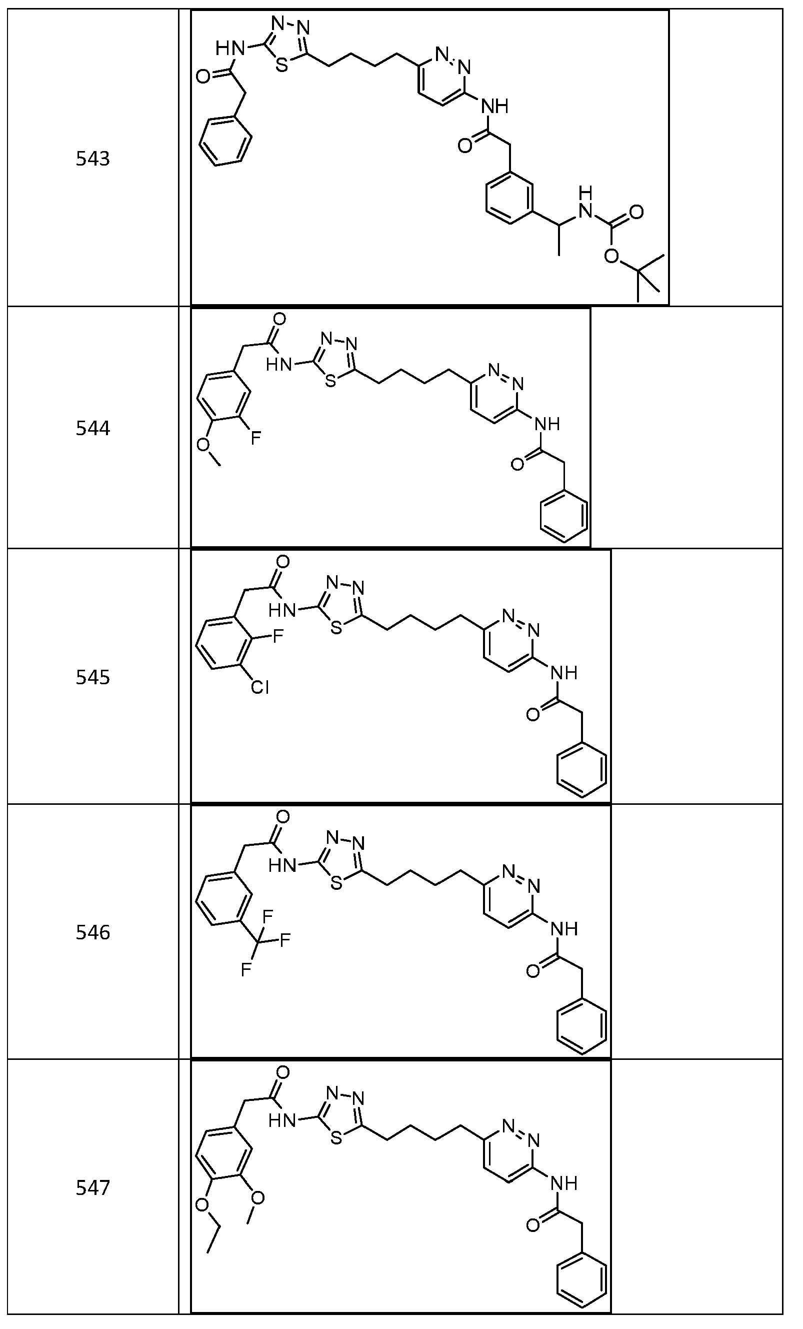

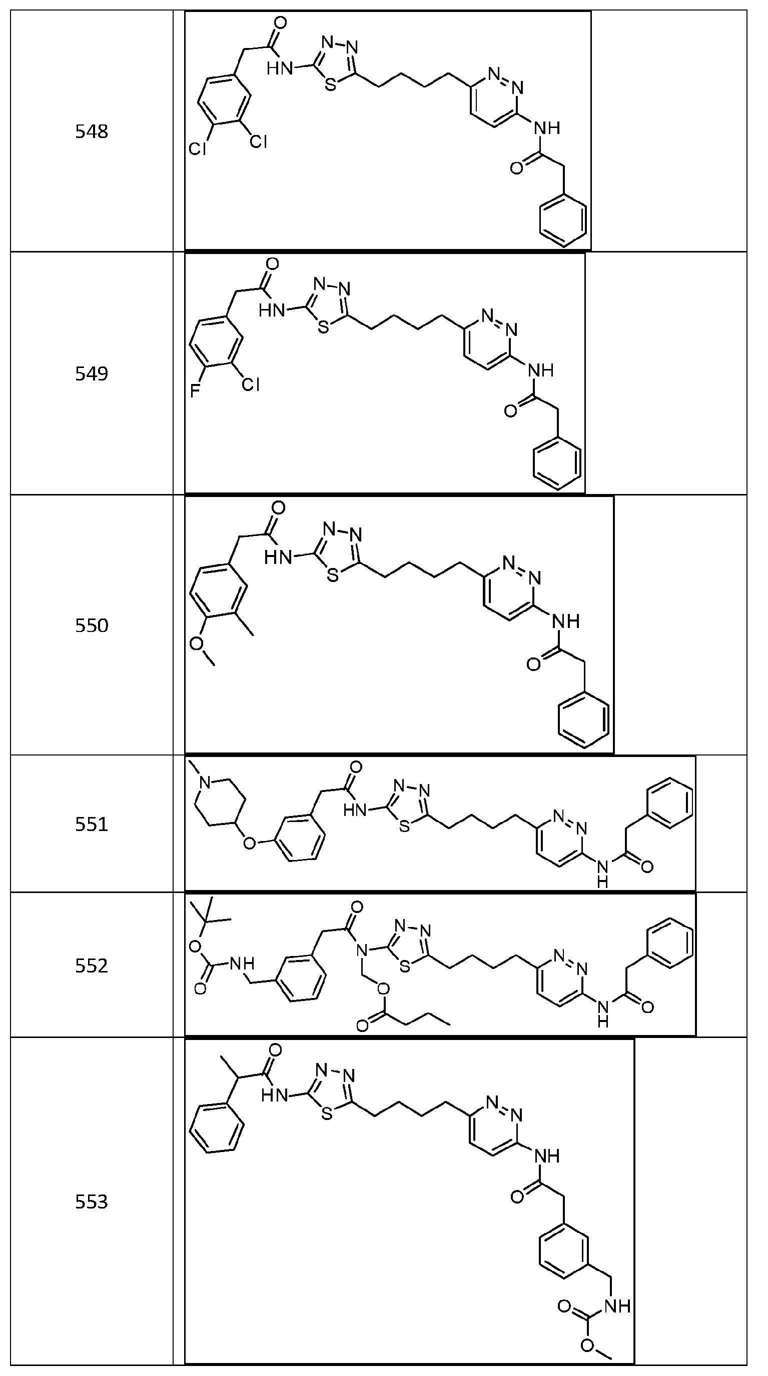

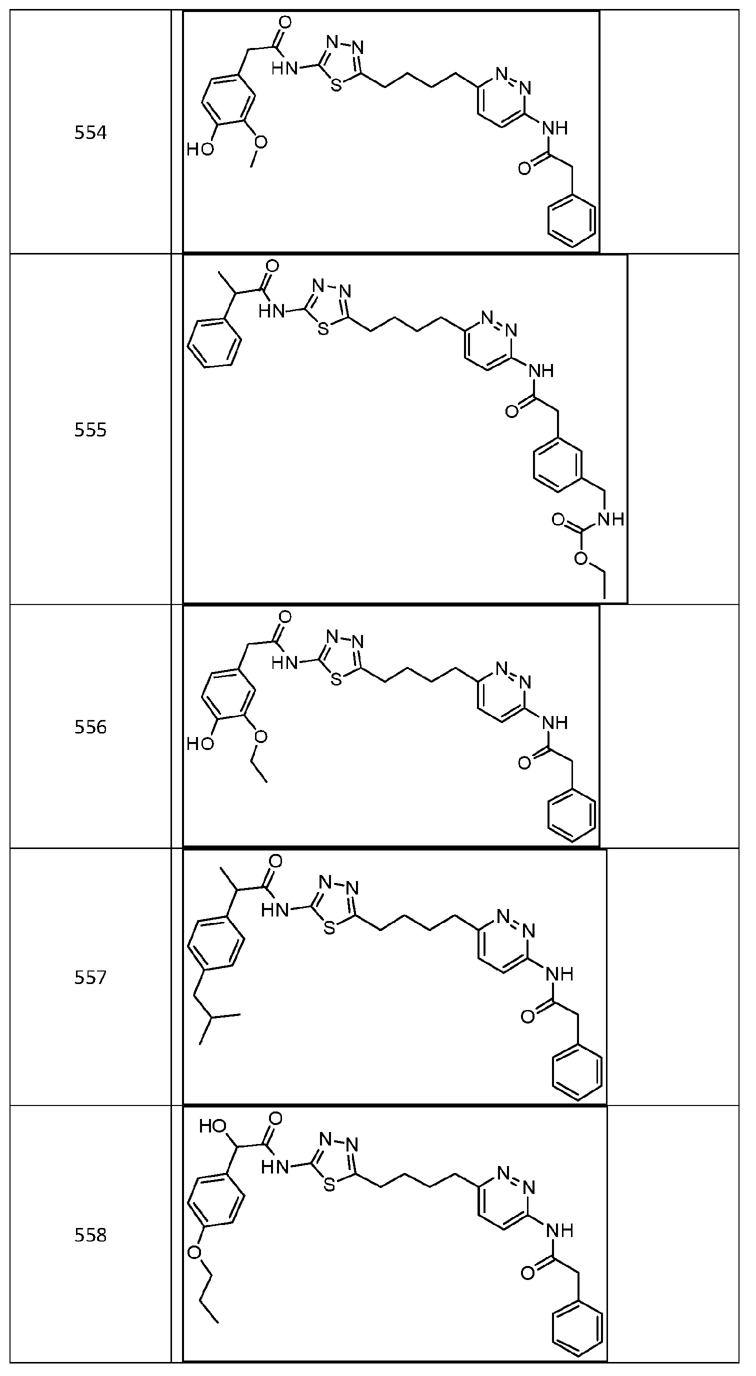

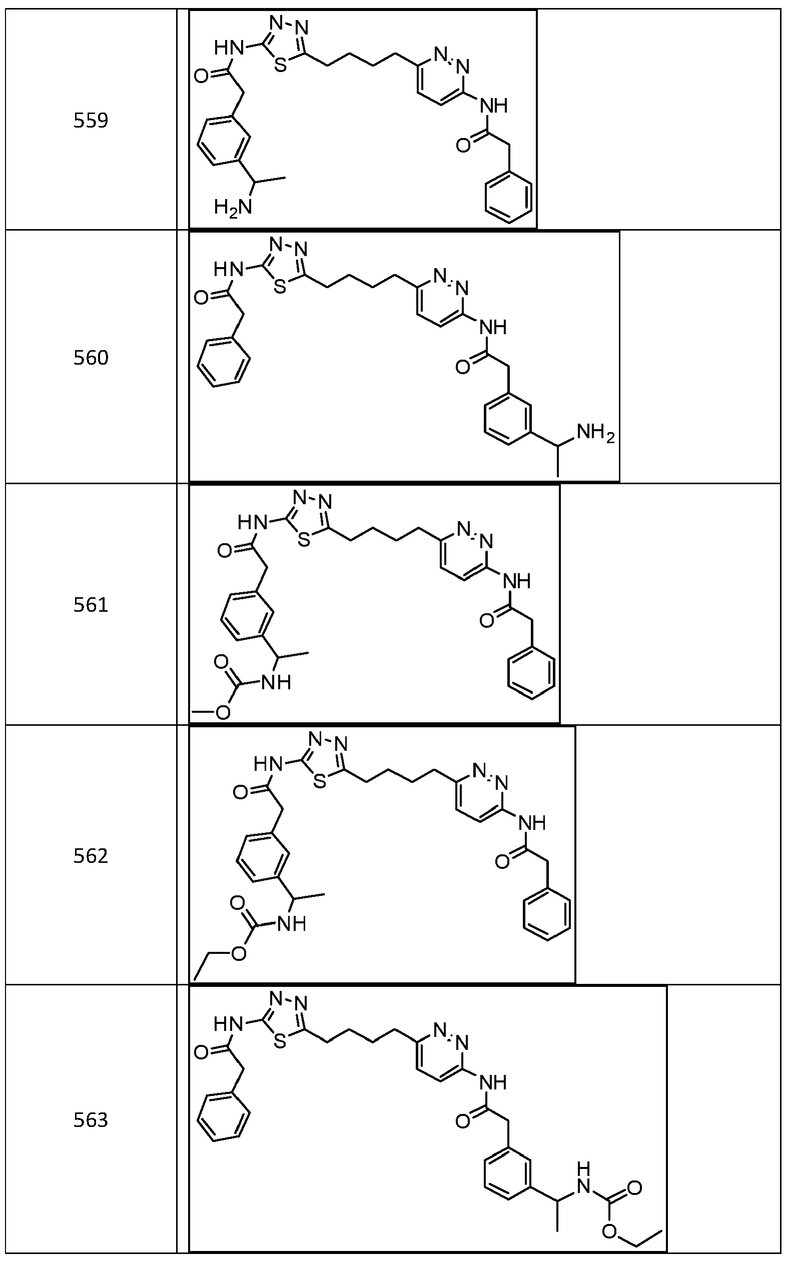

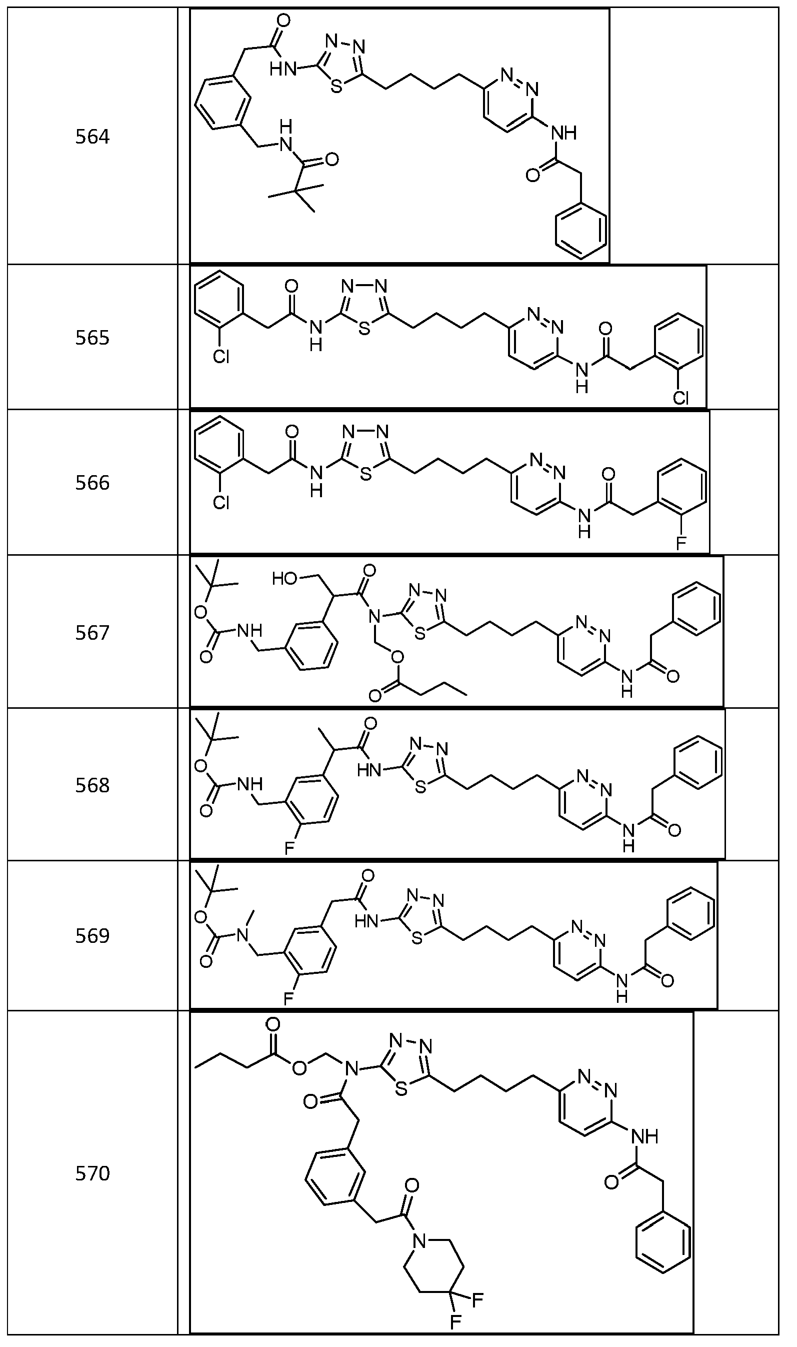

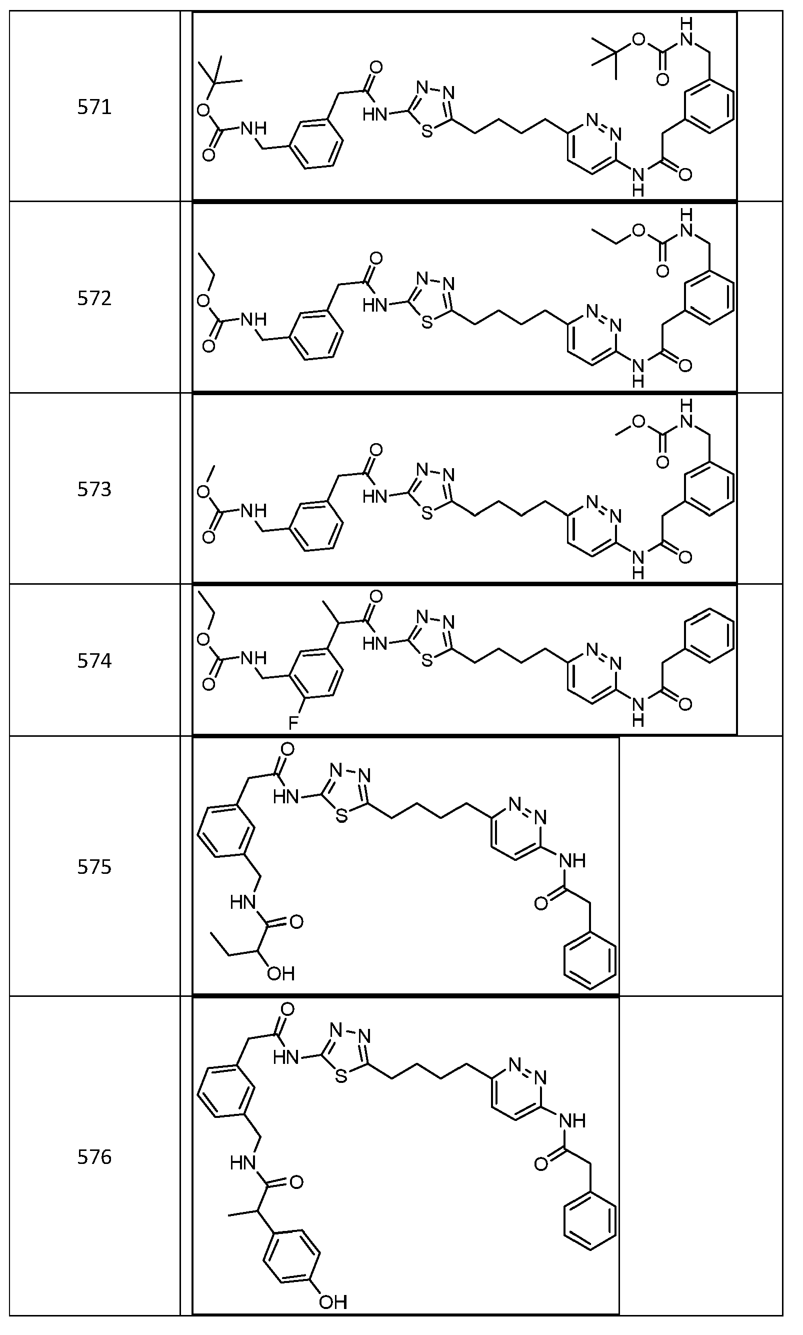

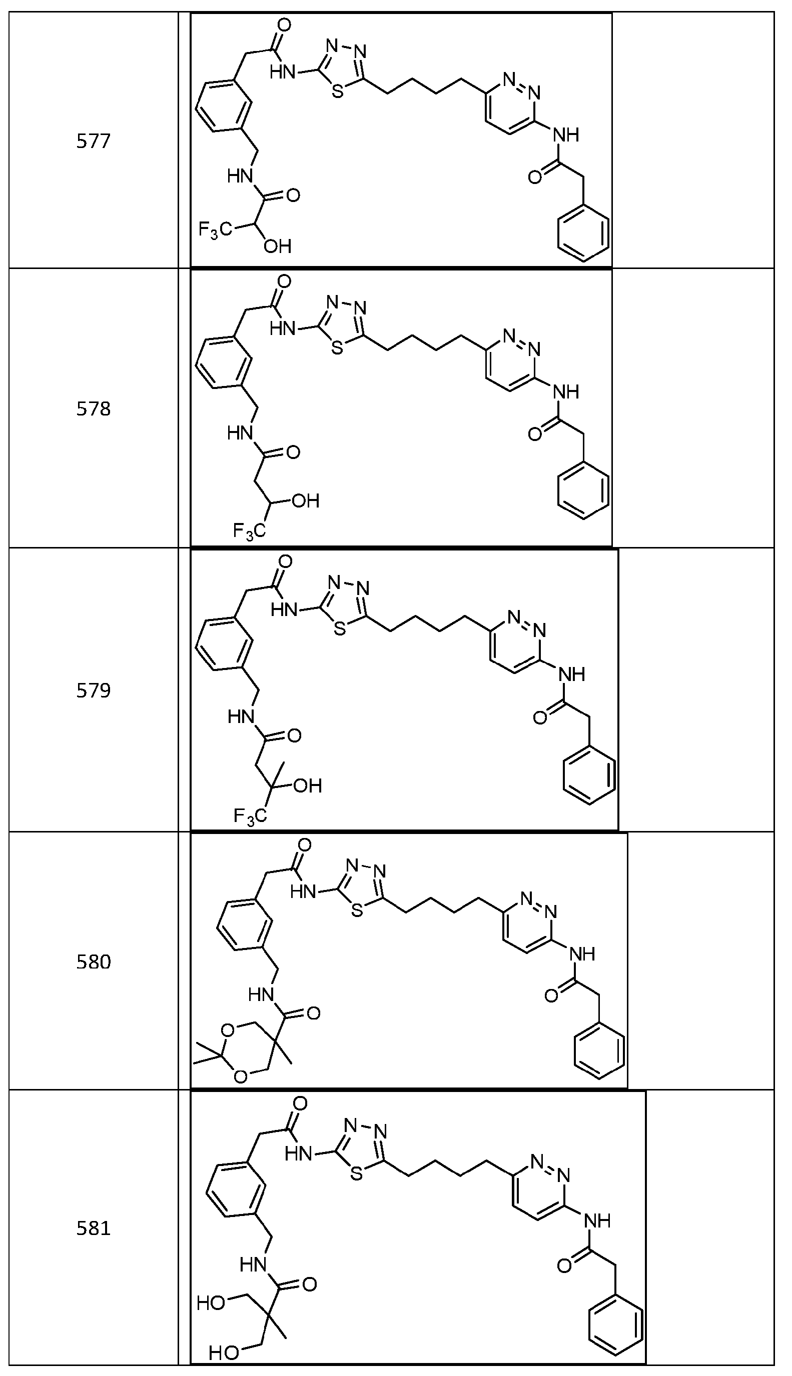

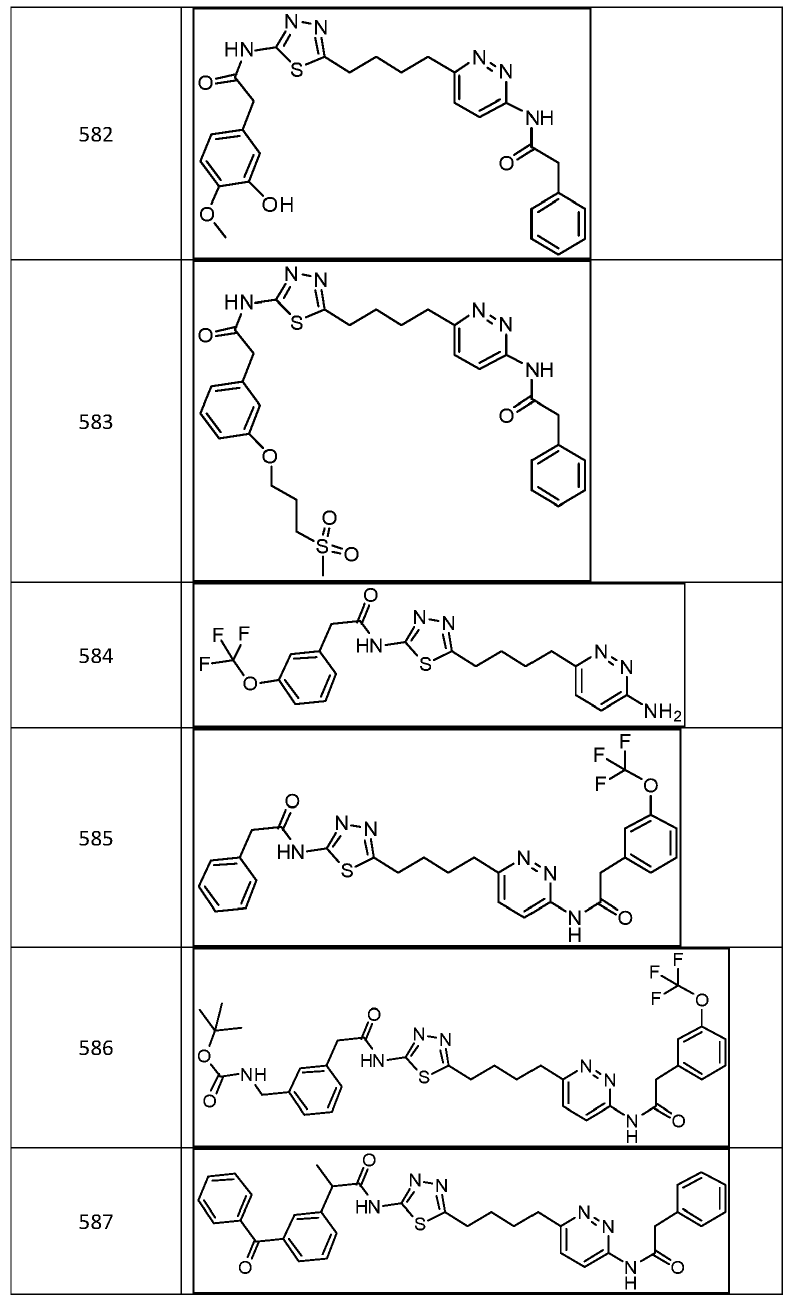

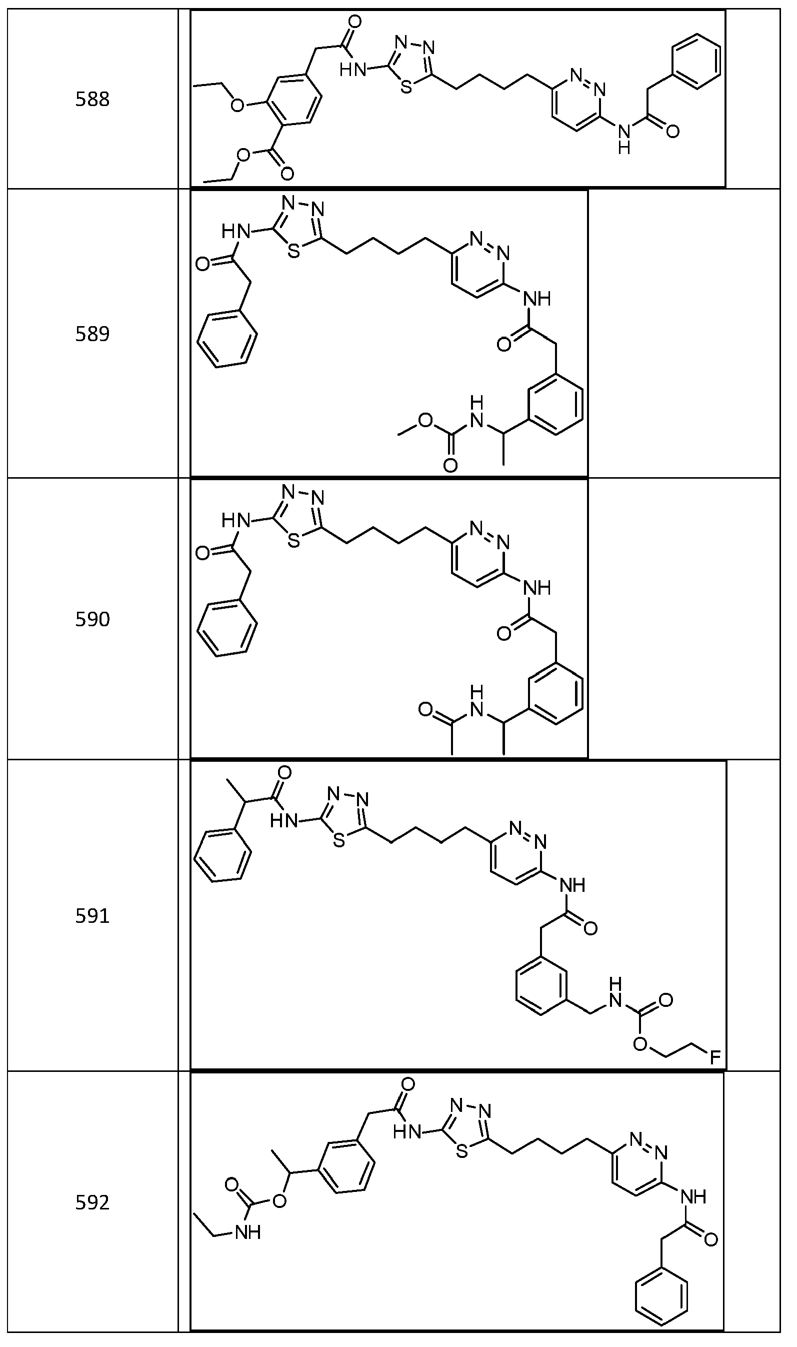

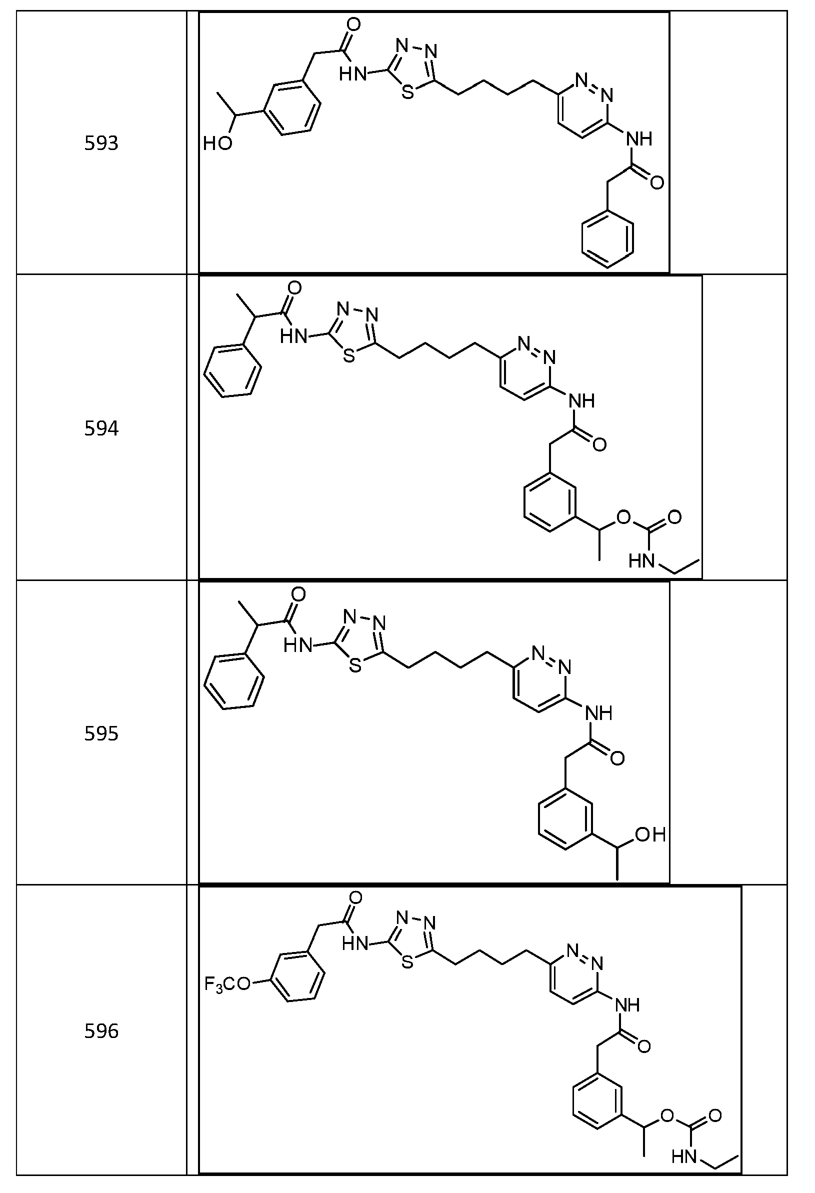

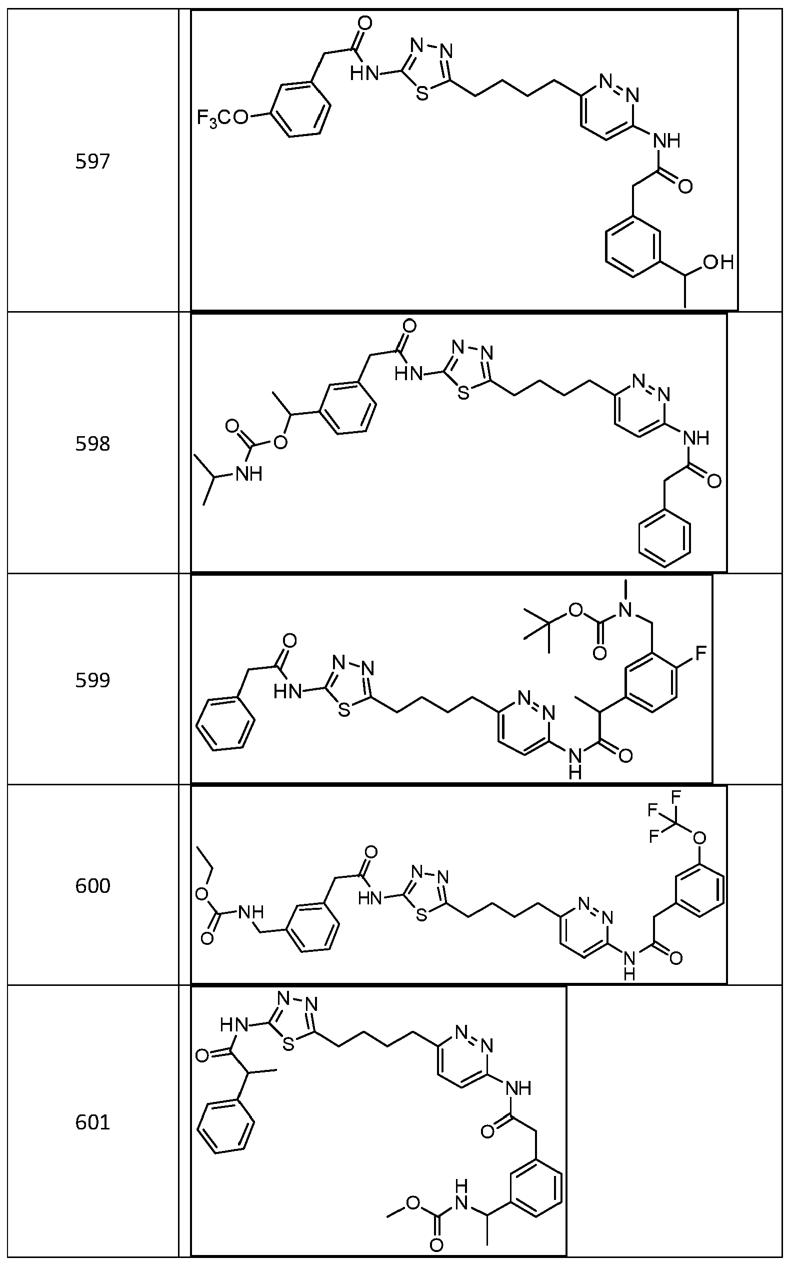

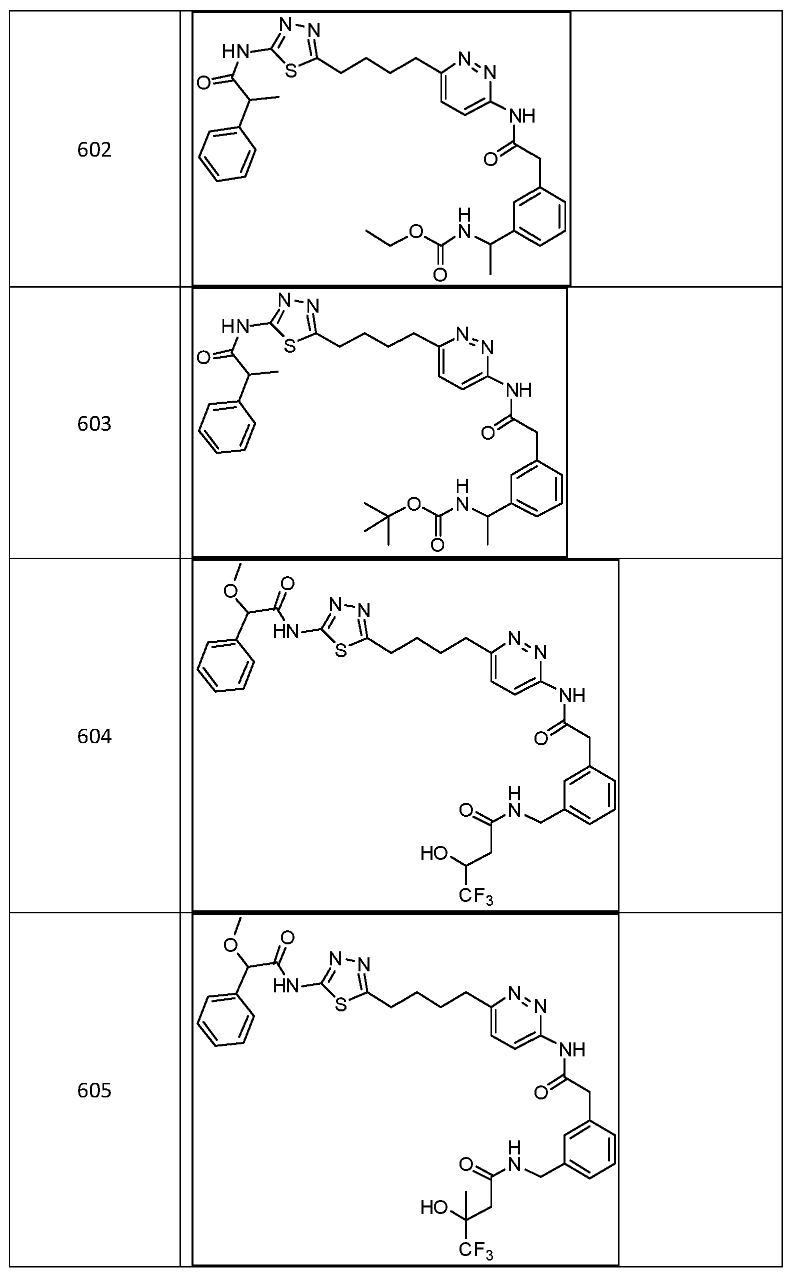

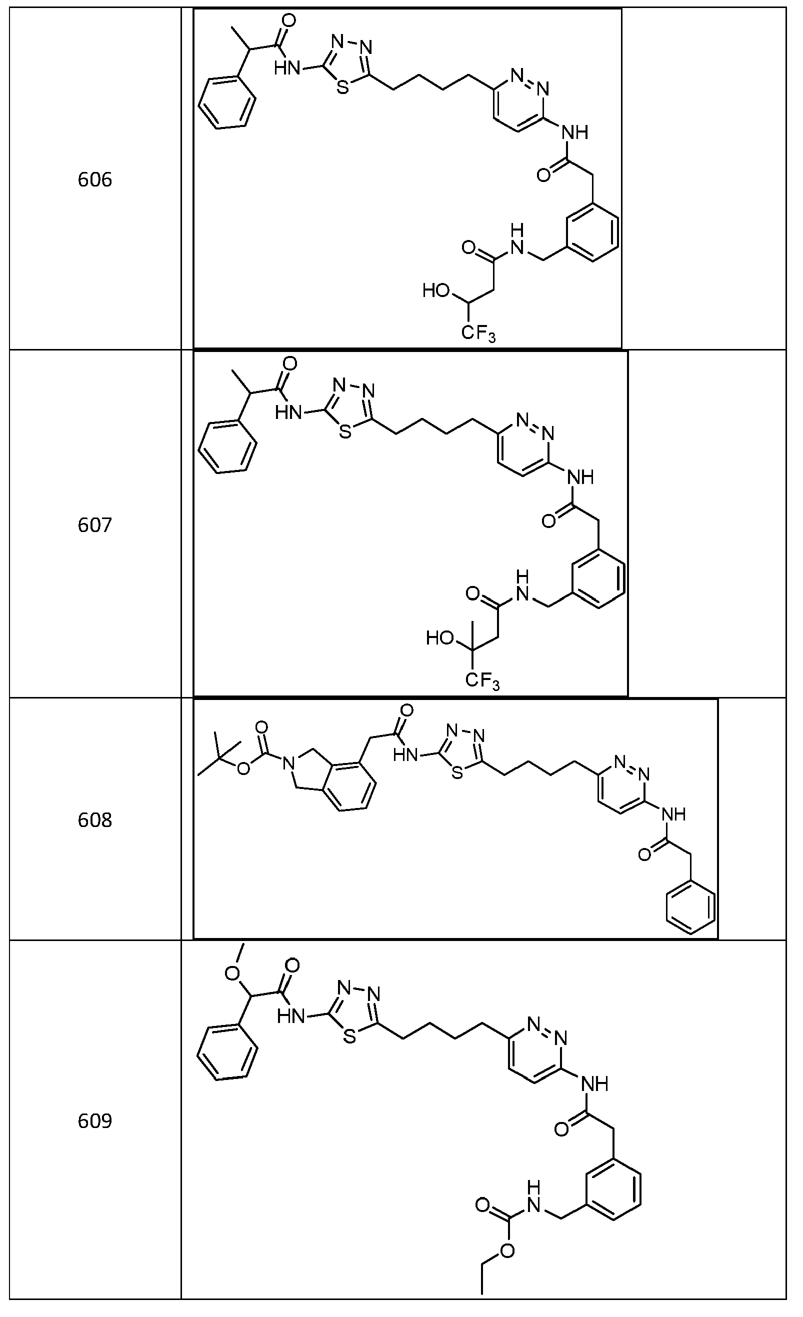

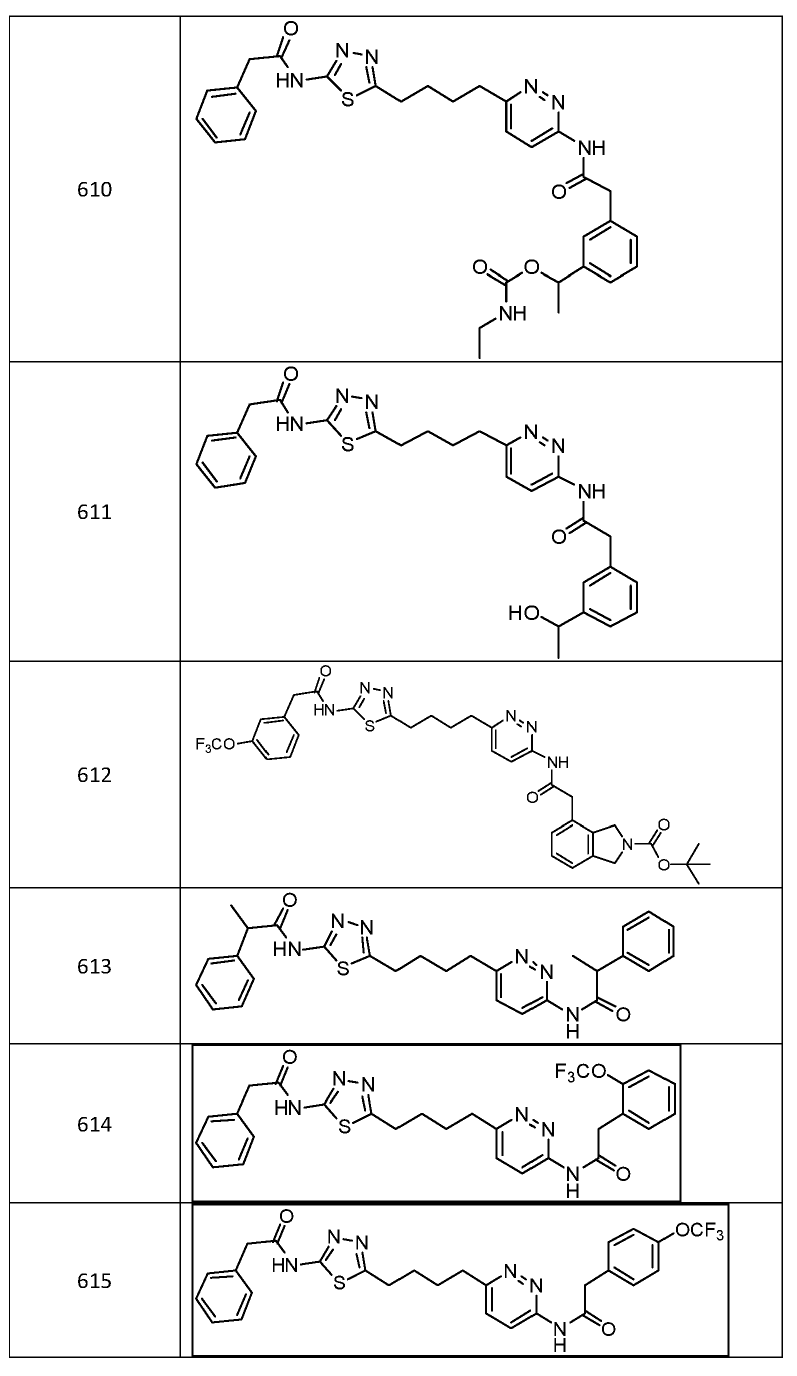

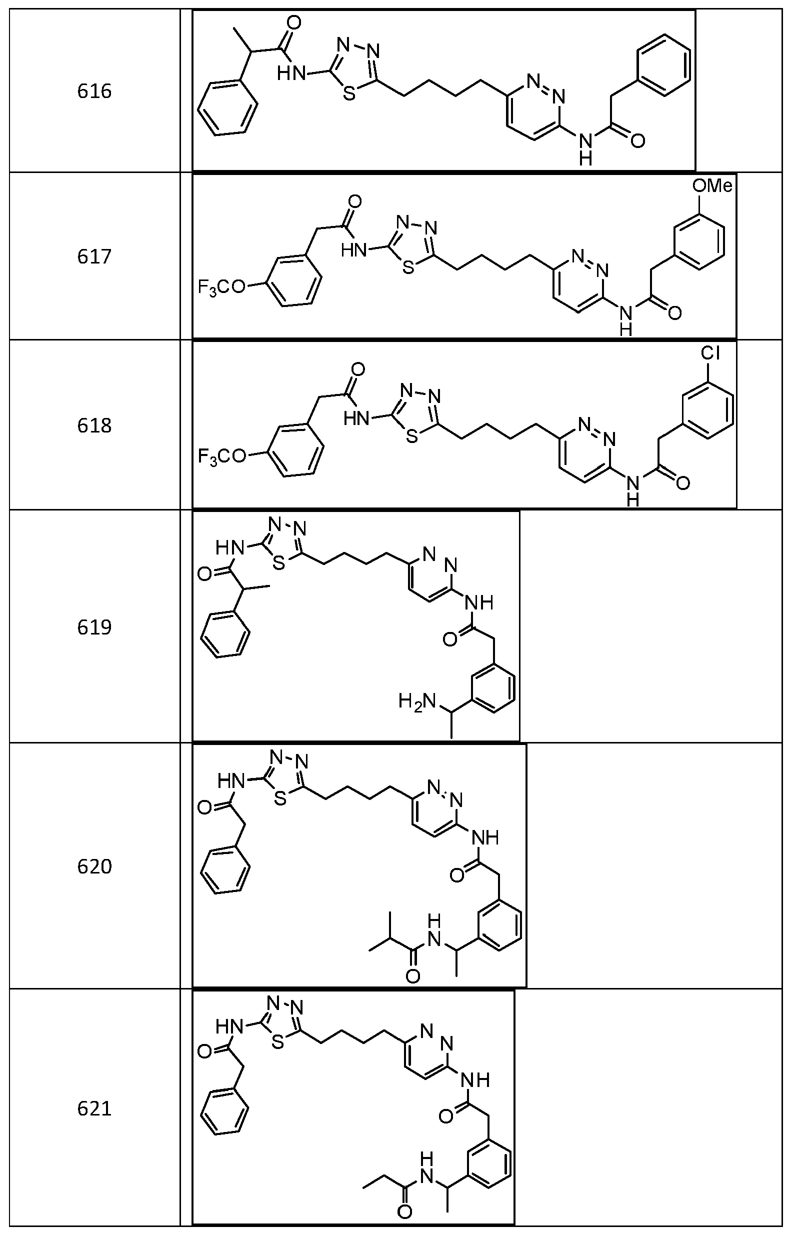

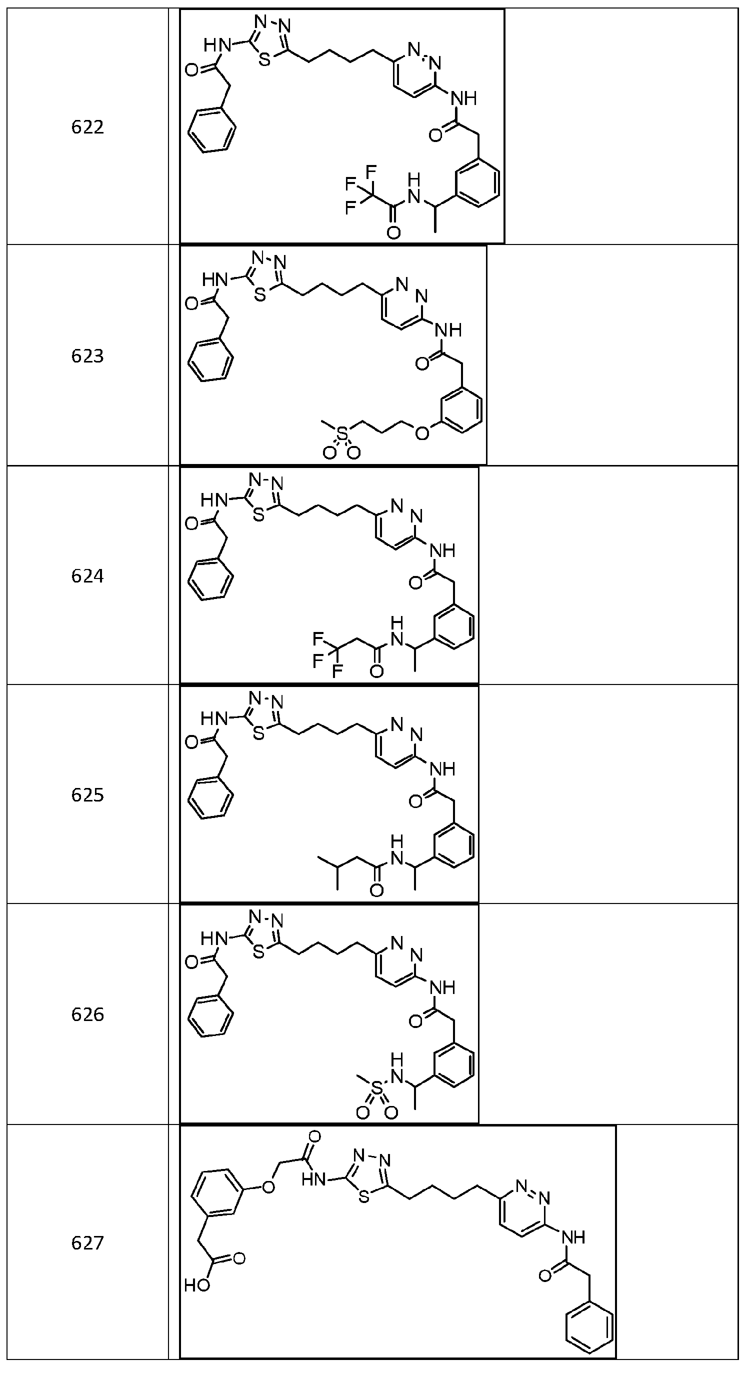

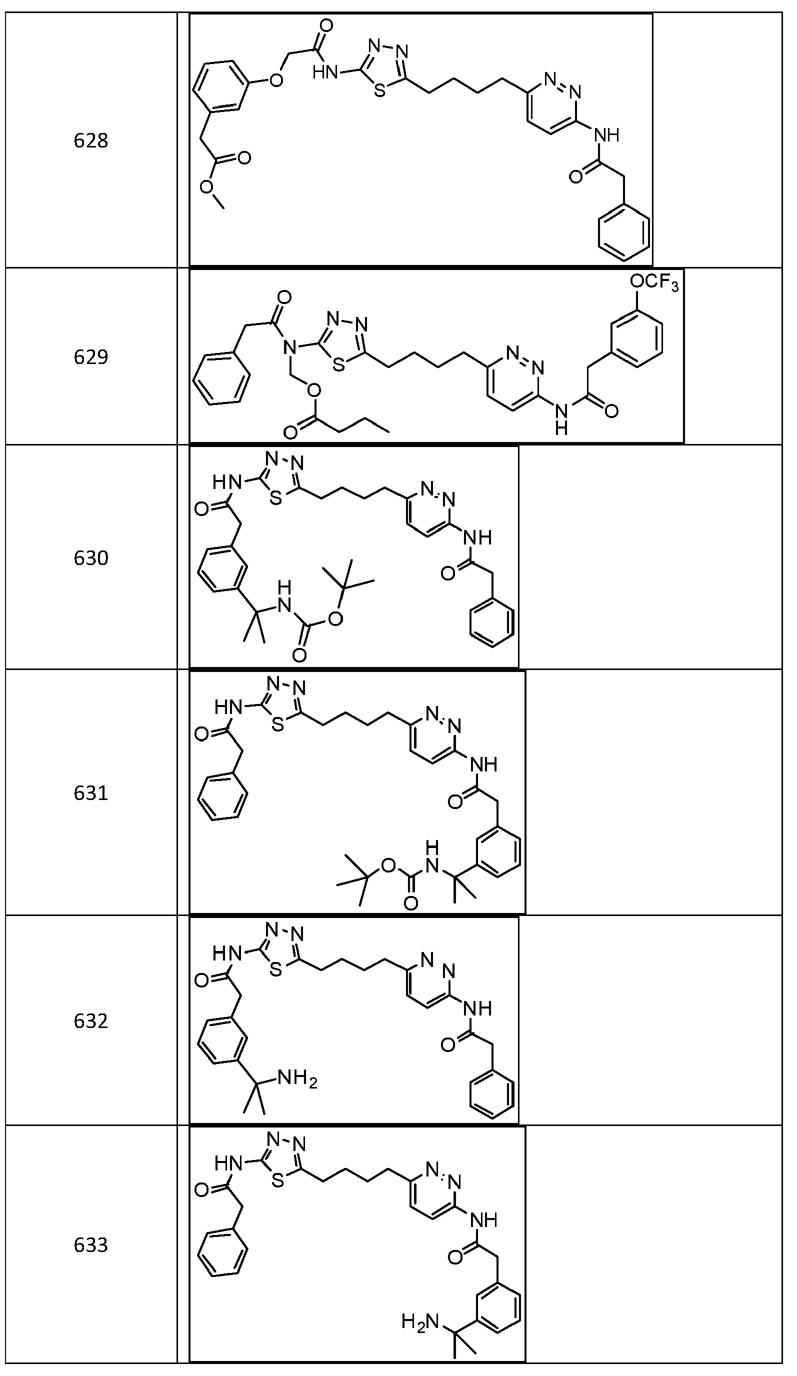

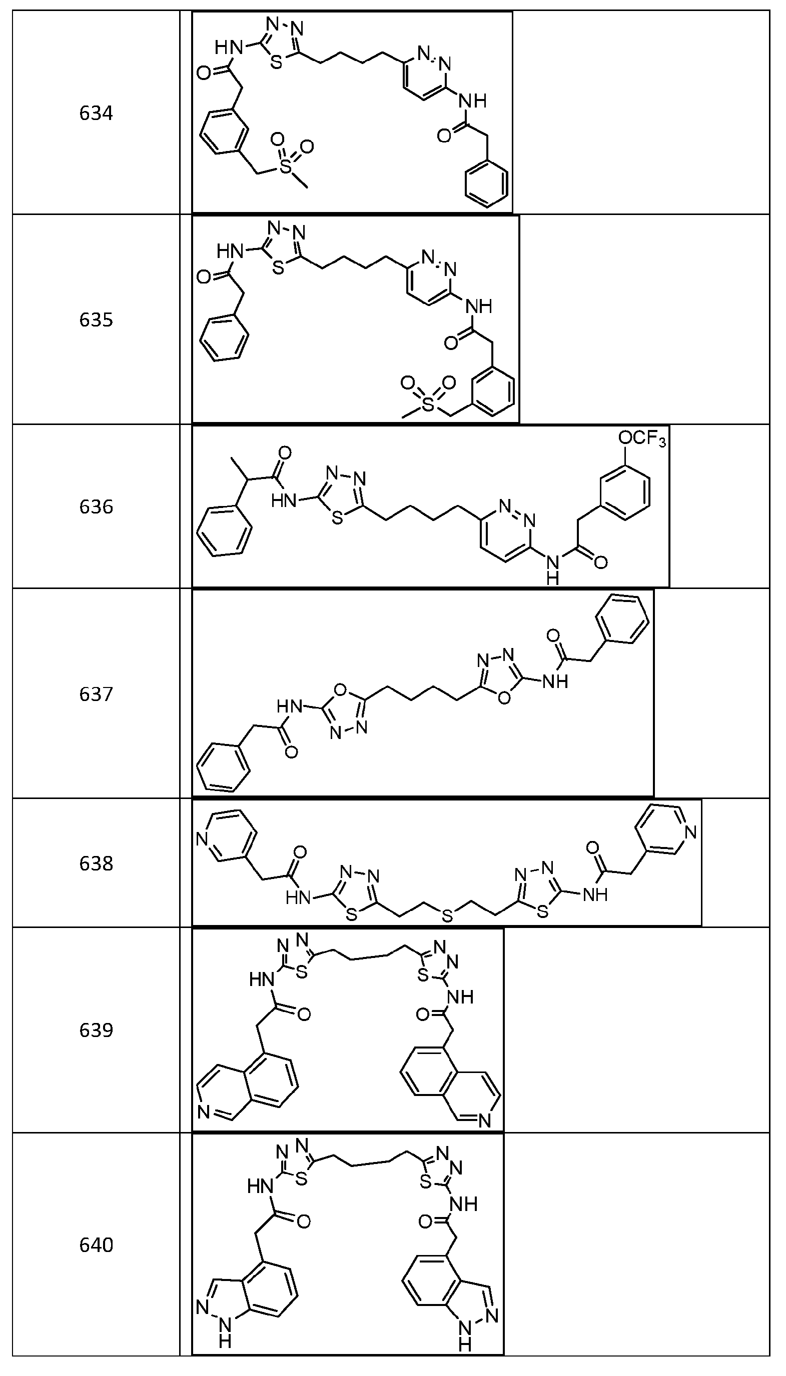

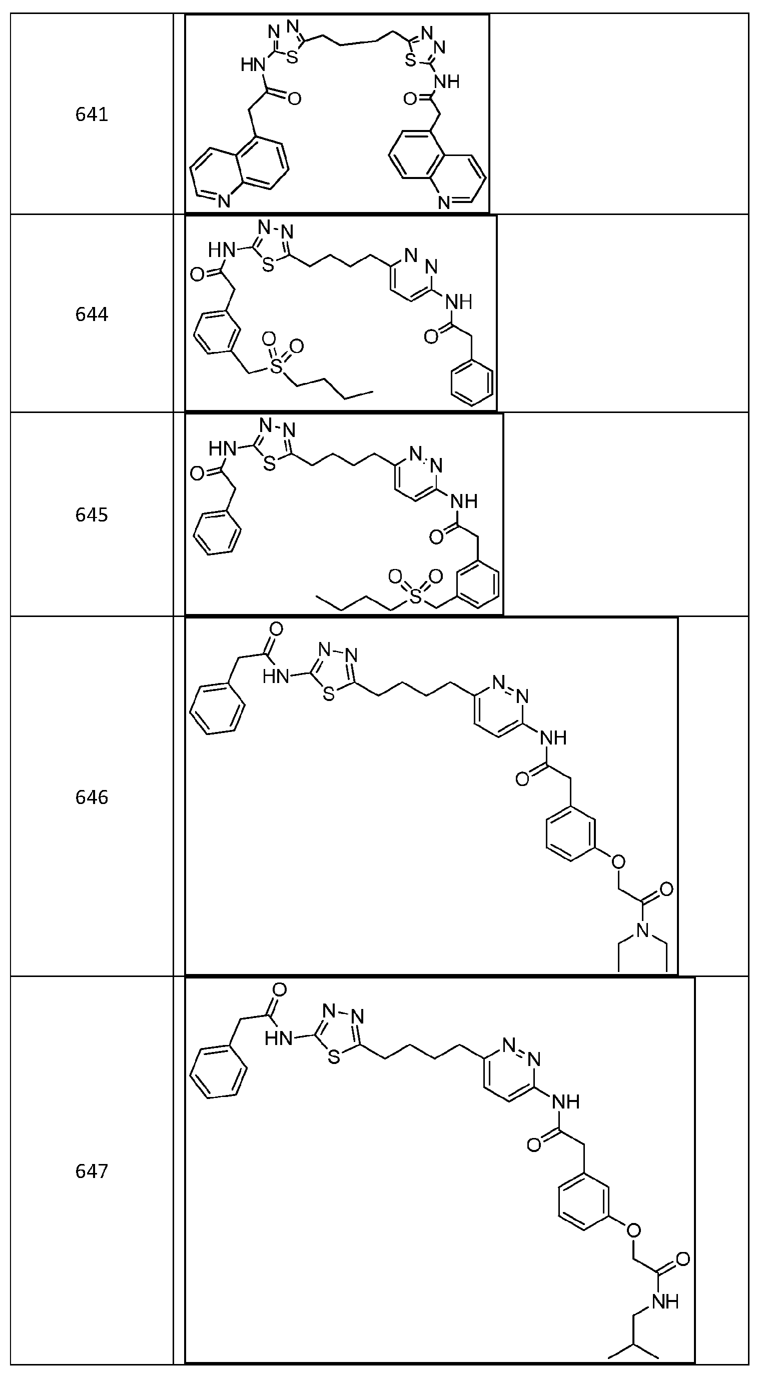

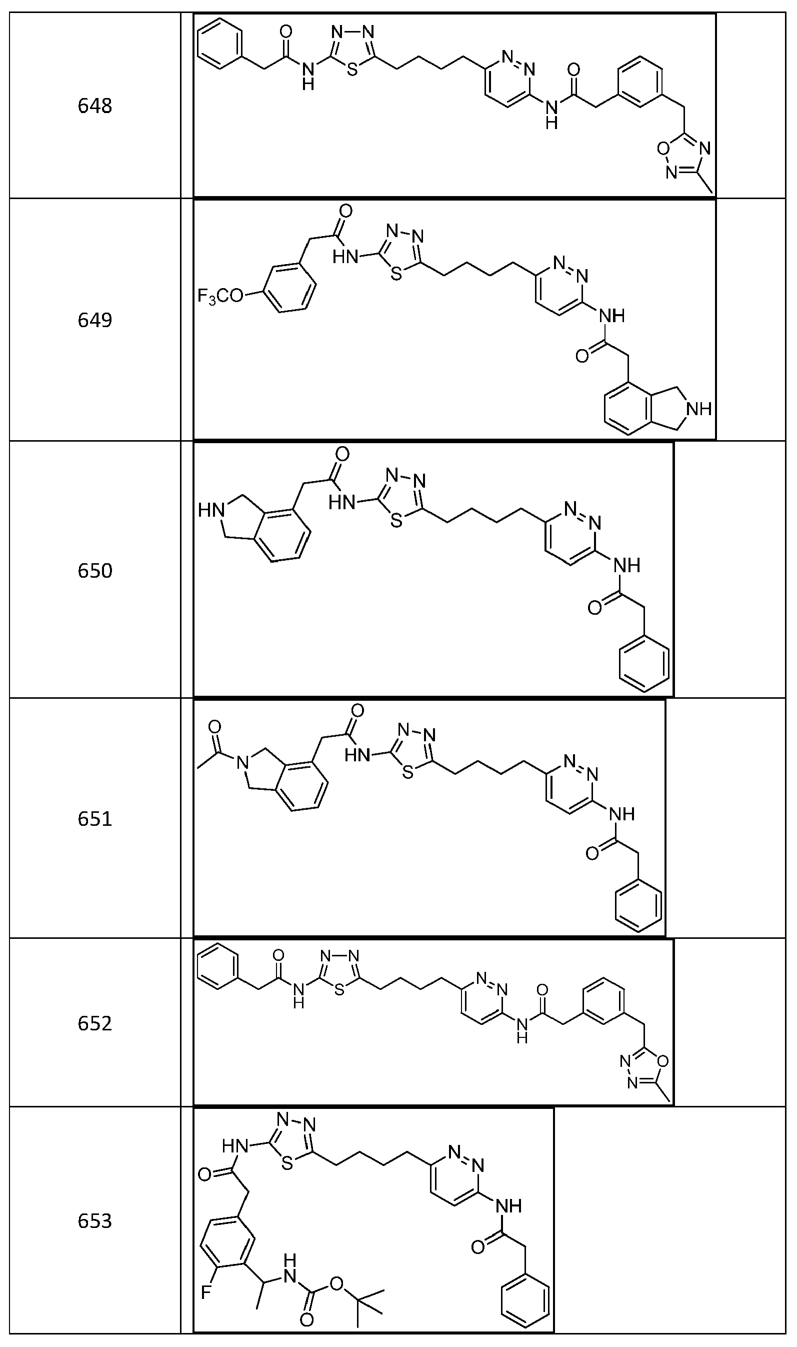

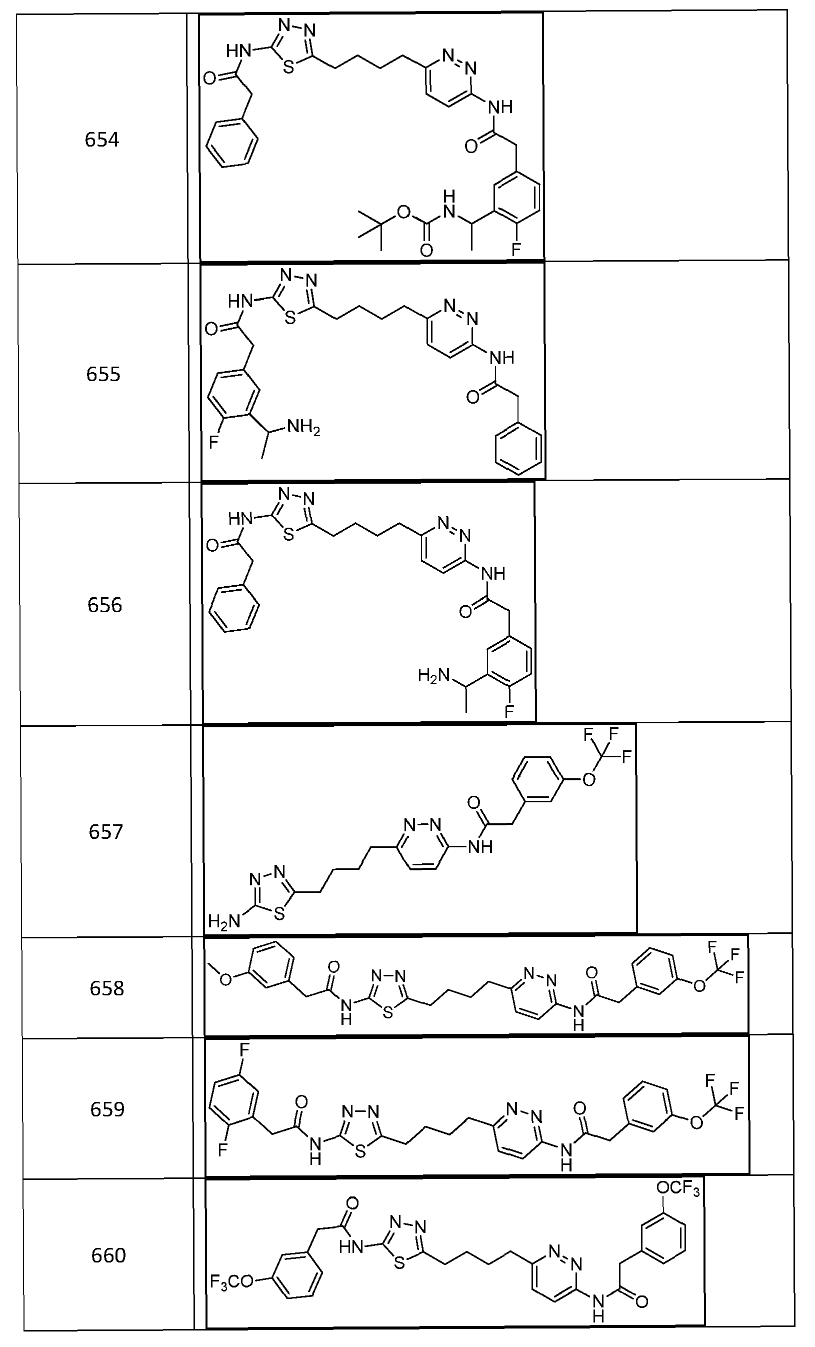

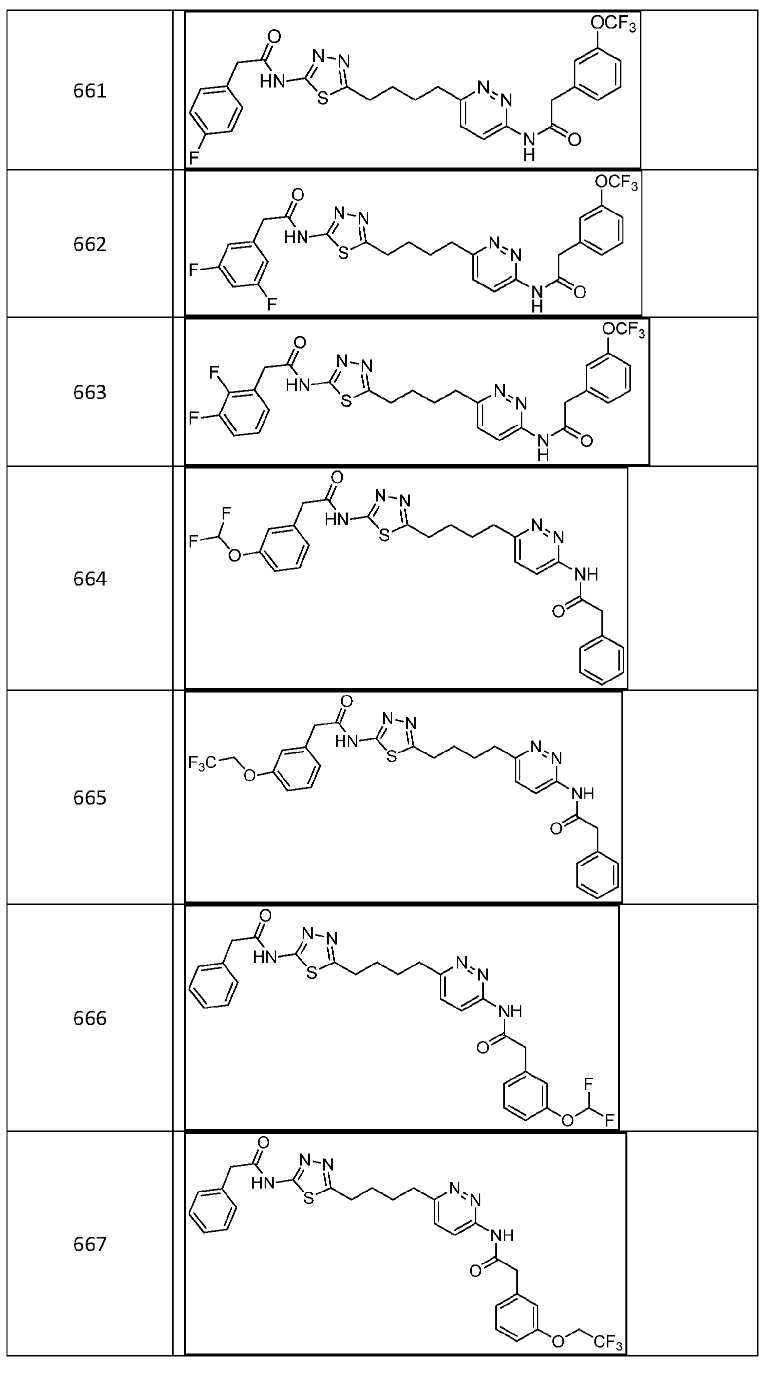

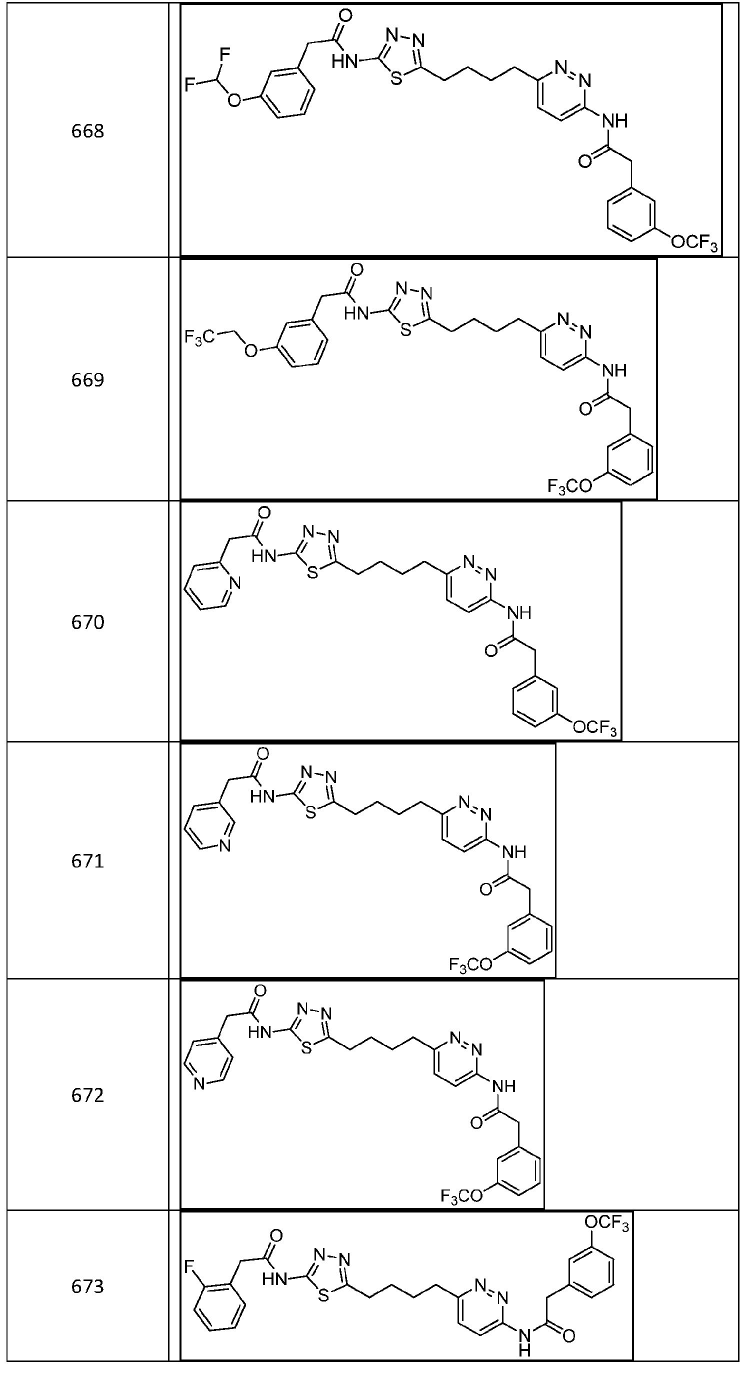

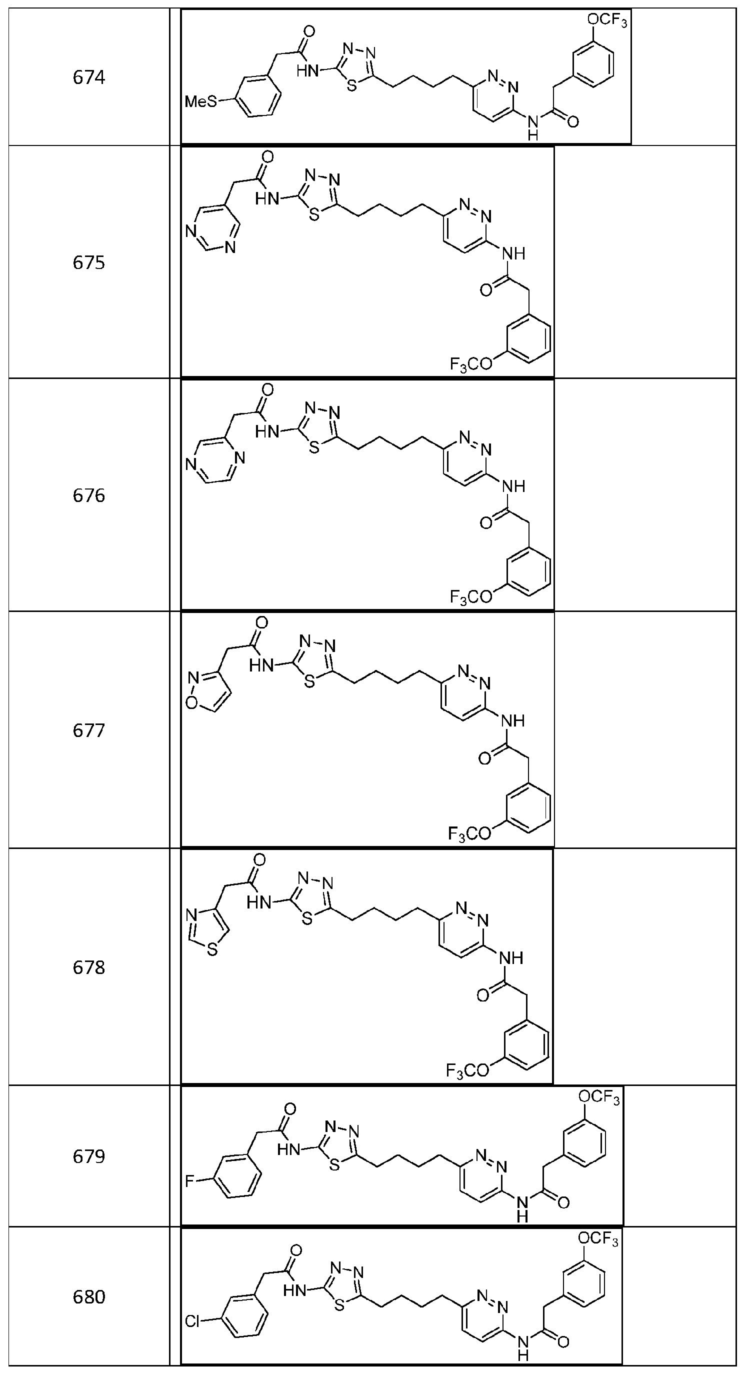

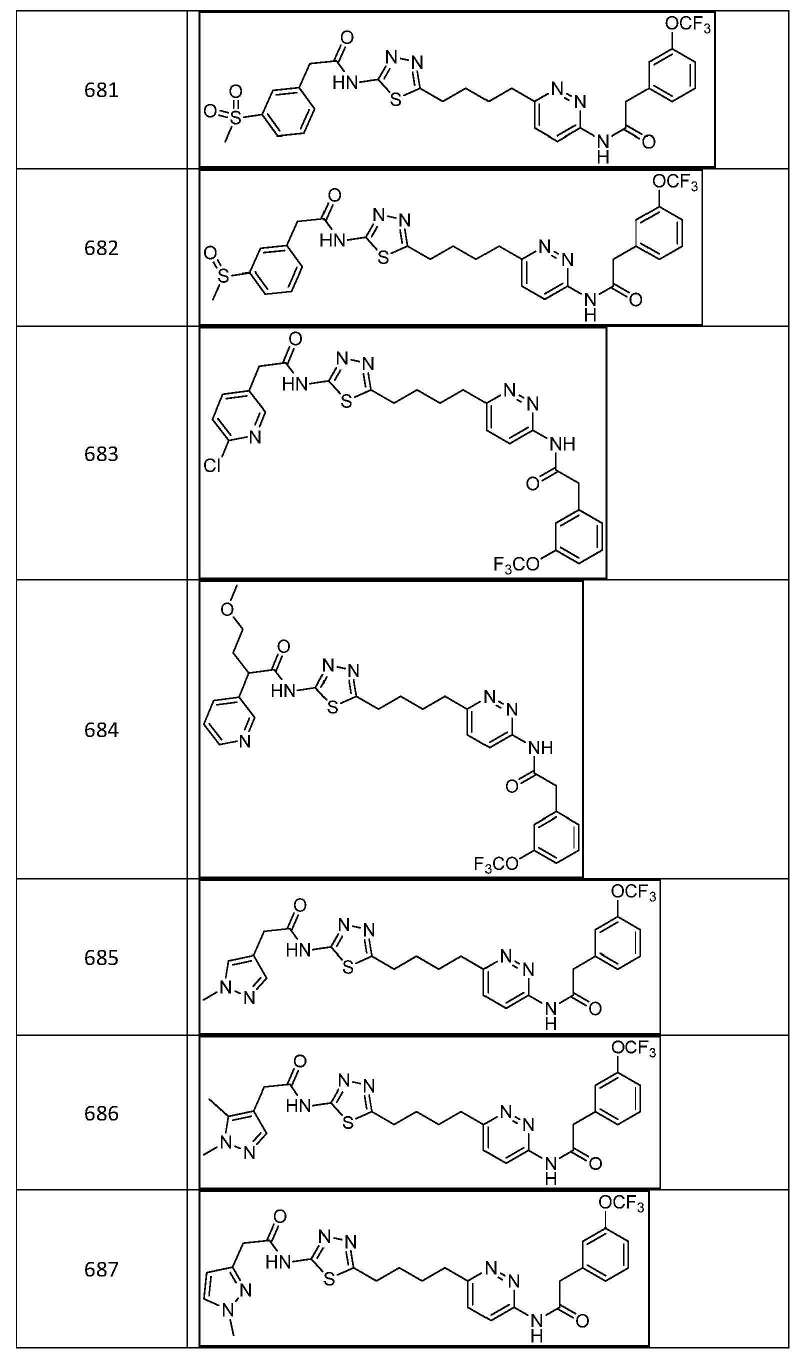

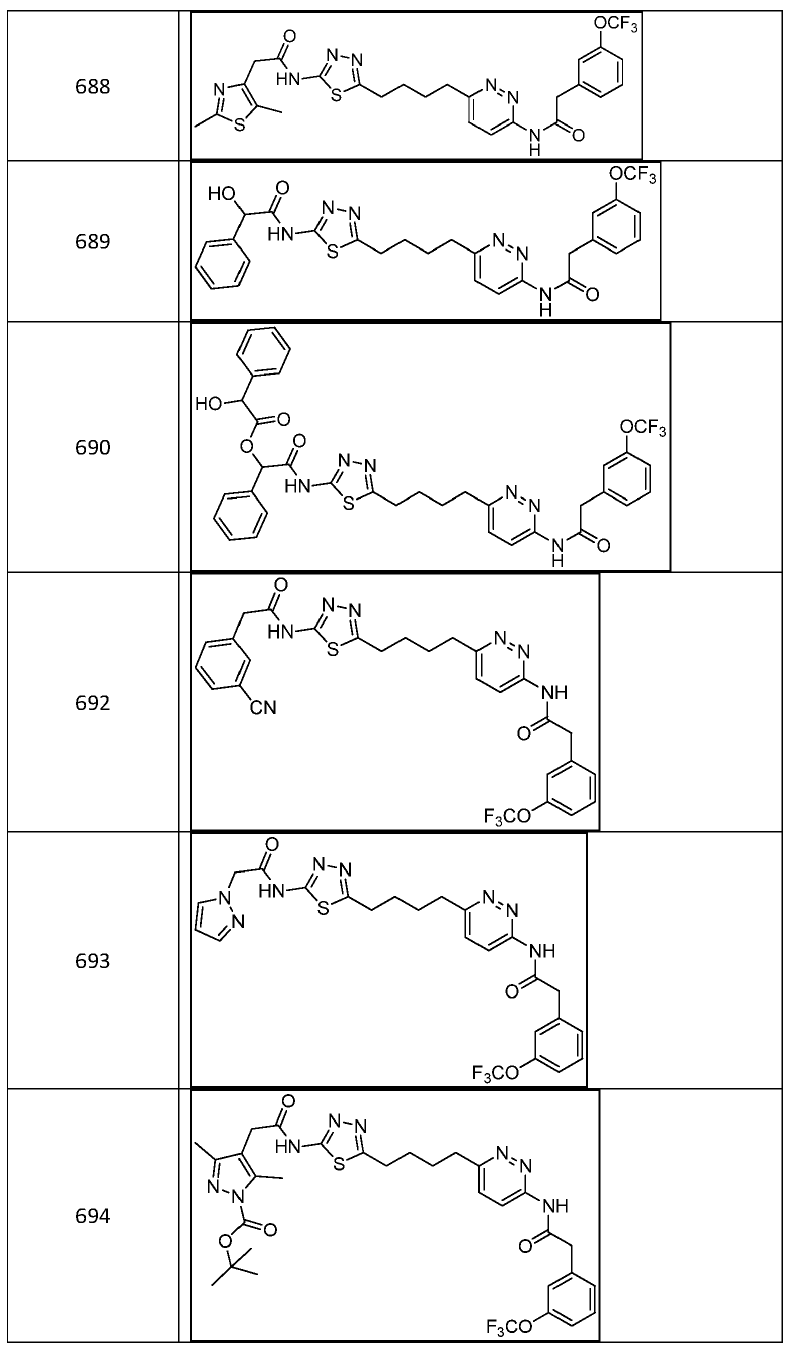

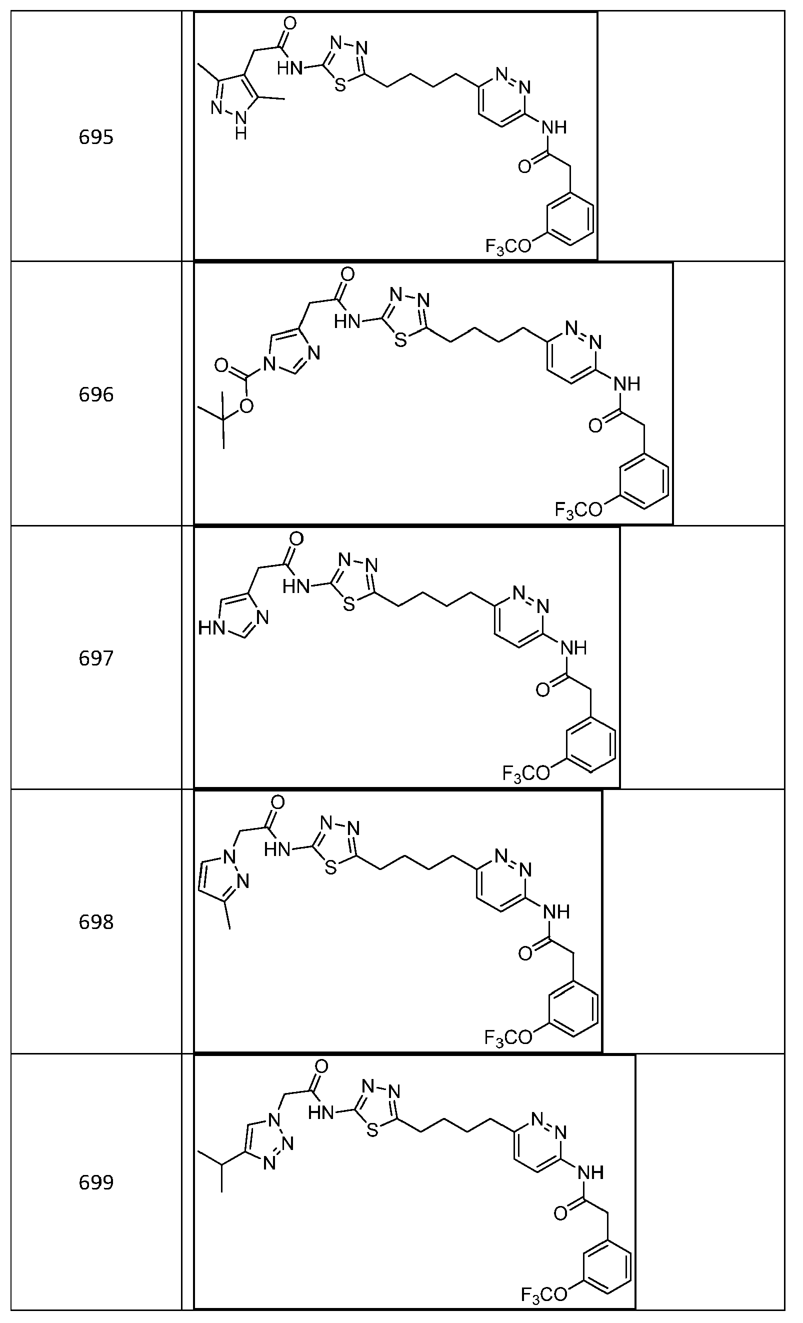

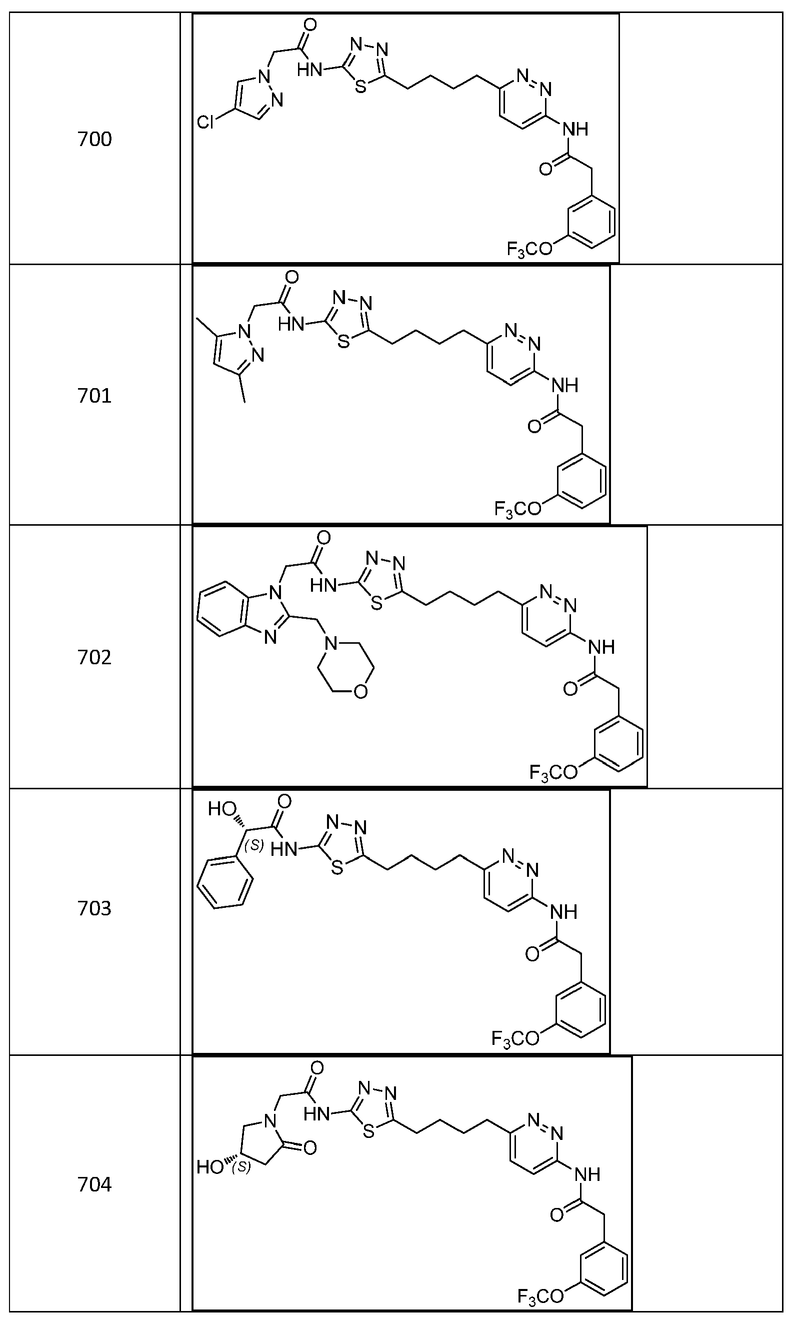

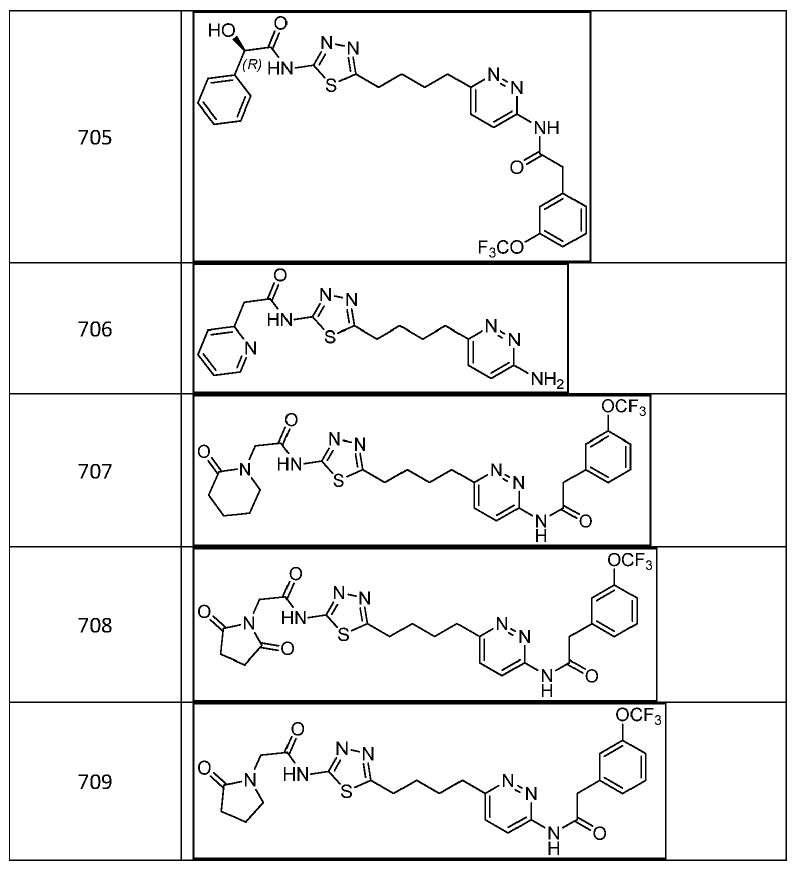

The present invention further provides methods of treating cancer, a myeloproliferative disease, an immunological disease, a neurological disease, or a viral infection comprising orally administering a compound of formula II, 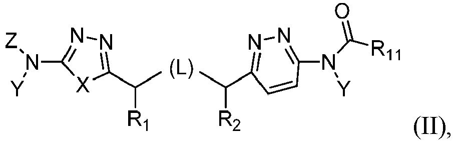 or a pharmaceutically acceptable salt thereof, wherein:
L represents CH₂SCH₂, CH₂CH₂, CH₂CH₂CH₂, CH₂, CH₂S, SCH₂, CH₂NHCH₂, CH=CH, or 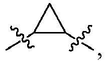 preferably CH₂CH₂, wherein any hydrogen atom of a CH or CH₂ unit may be replaced by alkyl or alkoxy, any hydrogen of an NH unit may be replaced by alkyl, and any hydrogen atom of a CH₂ unit of CH₂CH₂, CH₂CH₂CH₂ or CH₂ may be replaced by hydroxy;
X represents S, O or CH=CH, preferably S or CH=CH, wherein any hydrogen atom of a CH unit may be replaced by alkyl;
Y, independently for each occurrence, represents H or CH₂O(CO)R₇;
R₇, independently for each occurrence, represents H or substituted or unsubstituted alkyl, alkoxy, aminoalkyl, alkylaminoalkyl, heterocyclylalkyl, arylalkyl, or heterocyclylalkoxy;
Z represents H or R₃(CO);
R₁ and R₂ each independently represent H, alkyl, alkoxy or hydroxy;
R₃ represents substituted or unsubstituted alkyl, hydroxyalkyl, aminoalkyl, acylaminoalkyl, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, heteroaryloxyalkyl or C(R₈)(R₉)(R₁₀), N(R₄)(R₅) or OR₆, wherein any free hydroxyl group may be acylated to form C(O)R₇;
R₄ and R₅ each independently for each occurrence represent H or substituted or unsubstituted alkyl, hydroxyalkyl, acyl, aminoalkyl, acylaminoalkyl, alkenyl, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, wherein any free hydroxyl group may be acylated to form C(O)R₇;
R₆ represents substituted or unsubstituted alkyl, hydroxyalkyl, aminoalkyl, acylaminoalkyl, alkenyl, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, wherein any free hydroxyl group may be acylated to form C(O)R₇;
R₈, R₉ and R₁₀ each independently for each occurrence represent H or substituted or unsubstituted alkyl, hydroxy, hydroxyalkyl, amino, acylamino, aminoalkyl, acylaminoalkyl, alkoxycarbonyl, alkoxycarbonylamino, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, or R₈ and R₉ together with the carbon to which they are attached, form a carbocyclic or heterocyclic ring system, wherein any free hydroxyl group may be acylated to form C(O)R₇, and wherein at least two of R₈, R₉ and R₁₀ are not H;
R₁₁ represents aryl, arylalkyl, aryloxy, aryloxyalkyl, heteroaryl, heteroarylalkyl, heteroaryloxy, or heteroaryloxyalkyl, or R₁₁ represents C(R₁₂)(R₁₃)(R₁₄), N(R₄)(R₁₄) or OR₁₄, wherein any free hydroxyl group may be acylated to form C(O)R₇;
R₁₂ and R₁₃ each independently respresent H or substituted or unsubstituted alkyl, hydroxy, hydroxyalkyl, amino, acylamino, aminoalkyl, acylaminoalkyl, alkoxycarbonyl, alkoxycarbonylamino, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, wherein any free hydroxyl group may be acylated to form C(O)R₇, and wherein both of R₁₂ and R₁₃ are not H; and
R₁₄ represents aryl, arylalkyl, aryloxy, aryloxyalkyl, heteroaryl, heteroarylalkyl, heteroaryloxy, or heteroaryloxyalkyl;
preferably wherein the compound is administered with a meal.

In some embodiments, R₁₁ represents aryl, arylalkyl, aryloxy, aryloxyalkyl, heteroaryl, heteroarylalkyl, heteroaryloxy, or heteroaryloxyalkyl, wherein the aryl or heteroaryl ring is substituted with either -OCHF₂ or -OCF₃ and is optionally further substituted.

In some embodiments, R₁₄ represents aryl, arylalkyl, aryloxy, aryloxyalkyl, heteroaryl, heteroarylalkyl, heteroaryloxy, or heteroaryloxyalkyl, wherein the aryl or heteroaryl ring is substituted with either -OCHF₂ or -OCF₃ and is optionally further substituted.

In certain embodiments wherein alkyl, hydroxyalkyl, amino, acylamino, aminoalkyl, acylaminoalkyl, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl are substituted, they are substituted with one or more substituents selected from substituted or unsubstituted alkyl, such as perfluoroalkyl (*e.g.,* trifluoromethyl), alkenyl, alkoxy, alkoxyalkyl, aryl, aralkyl, arylalkoxy, aryloxy, aryloxyalkyl, hydroxyl, halo, alkoxy, such as perfluoroalkoxy (*e.g.,* trifluoromethoxy), alkoxyalkoxy, hydroxyalkyl, hydroxyalkylamino, hydroxyalkoxy, amino, aminoalkyl, alkylamino, aminoalkylalkoxy, aminoalkoxy, acylamino, acylaminoalkyl, such as perfluoro acylaminoalkyl (*e.g.,* trifluoromethylacylaminoalkyl), acyloxy, cycloalkyl, cycloalkylalkyl, cycloalkylalkoxy, heterocyclyl, heterocyclylalkyl, heterocyclyloxy, heterocyclylalkoxy, heteroaryl, heteroarylalkyl, heteroarylalkoxy, heteroaryloxy, heteroaryloxyalkyl, heterocyclylaminoalkyl, heterocyclylaminoalkoxy, amido, amidoalkyl, amidine, imine, oxo, carbonyl (such as carboxyl, alkoxycarbonyl, formyl, or acyl, including perfluoroacyl (*e.g.,* C(O)CF₃)), carbonylalkyl (such as carboxyalkyl, alkoxycarbonylalkyl, formylalkyl, or acylalkyl, including perfluoroacylalkyl (*e.g.,* -alkylC(O)CF₃)), carbamate, carbamatealkyl, urea, ureaalkyl, sulfate, sulfonate, sulfamoyl, sulfone, sulfonamide, sulfonamidealkyl, cyano, nitro, azido, sulfhydryl, alkylthio, thiocarbonyl (such as thioester, thioacetate, or thioformate), phosphoryl, phosphate, phosphonate or phosphinate.

In certain embodiments, R₁₁ represents arylalkyl, such as benzyl, wherein the aryl group is substituted with -OCF₃, such as meta-substituted with -OCF₃. In certain such embodiments, the aryl ring is not further substituted. In certain embodiments, R₁₁ represents trifluoromethoxybenzyl, such as

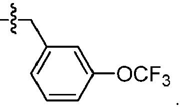

In certain embodiments, L represents CH₂SCH₂, CH₂CH₂, CH₂CH₂CH₂, CH₂, CH₂S, SCH₂, or CH₂NHCH₂, wherein any hydrogen atom of a CH₂ unit may be replaced by alkyl or alkoxy, and any hydrogen atom of a CH₂ unit of CH₂CH₂, CH₂CH₂CH₂ or CH₂ may be replaced by hydroxyl. In certain embodiments, L represents CH₂SCH₂, CH₂CH₂, CH₂S or SCH₂. In certain embodiments, L represents CH₂CH₂. In certain embodiments, L is not CH₂SCH₂.

In certain embodiments, Y represents H.

In certain embodiments, X represents S or CH=CH. In certain embodiments, X represents S.

In certain embodiments, Z represents R₃(CO). In certain embodiments wherein Z is R₃(CO), R₃ and R₁₁ are not identical (*e.g.,* the compound of formula II is not symmetrical).

In certain embodiments, R₁ and R₂ each represent H.

In certain embodiments, Z represents R₃(CO) and R₃ represents arylalkyl, heteroarylalkyl, cycloalkyl or heterocycloalkyl. In certain embodiments, Z represents R₃(CO) and R₃ represents heteroarylalkyl, such as pyridylalkyl (*e.g*., pyridylmethyl). In certain such embodiments, Z represents 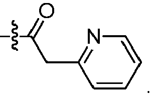 In certain embodiments, Z represents R₃(CO) and R₃ represents C(R₈)(R₉)(R₁₀), wherein R₈ represents aryl, arylalkyl, heteroaryl or heteroaralkyl, such as aryl, arylalkyl or heteroaryl, R₉ represents H, and R₁₀ represents hydroxy, hydroxyalkyl, alkoxy or alkoxyalkyl, such as hydroxy, hydroxyalkyl or alkoxy.

In certain embodiments, L represents CH₂SCH₂, CH₂CH₂, CH₂S or SCH₂, such as CH₂CH₂, Y represents H, X represents S, Z represents R₃(CO), R₁ and R₂ each represent H, R₃ represents arylalkyl, heteroarylalkyl, cycloalkyl or heterocycloalkyl, such as heteroarylalkyl (*e.g*., pyridylalkyl), and R₁₁ represents arylalkyl, such trifluoromethoxybenzyl (*e.g.*, 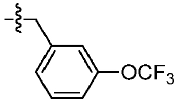 ). In certain such embodiments, Z represents R₃(CO) and R₃ represents pyridylmethyl, such as wherein Z represents

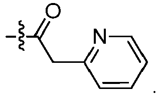

In certain embodiments, L represents CH₂SCH₂, CH₂CH₂, CH₂S or SCH₂, such as CH₂CH₂, Y represents H, X represents S, Z represents R₃(CO), R₁ and R₂ each represent H, and each R₃ represents C(R₈)(R₉)(R₁₀), wherein R₈ represents aryl, arylalkyl, heteroaryl or heteroaralkyl, such as aryl, arylalkyl or heteroaryl, R₉ represents H, and R₁₀ represents hydroxy, hydroxyalkyl, alkoxy or alkoxyalkyl, such as hydroxy, hydroxyalkyl or alkoxy, and R₁₁ represents arylalkyl, such trifluoromethoxybenzyl (*e.g.*, 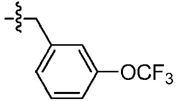 ).

In certain embodiments, L represents CH₂CH₂, Y represents H, X represents S or CH=CH, such as S, Z represents R₃(CO), R₁ and R₂ each represent H, R₃ represents substituted or unsubstituted arylalkyl, heteroarylalkyl, cycloalkyl or heterocycloalkyl, such as heteroarylalkyl (*e.g*., pyridylalkyl), and R₁₁ represents arylalkyl, such trifluoromethoxybenzyl (*e.g.*, 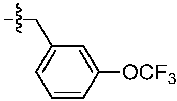 ). In certain such embodiments, Z represents R₃(CO) and R₃ represents pyridylmethyl, such as wherein Z represents

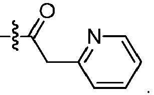

In certain embodiments, L represents CH₂CH₂, Y represents H, X represents S, Z represents R₃(CO), R₁ and R₂ each represent H, h R₃ represents C(R₈)(R₉)(R₁₀), wherein R₈ represents aryl, arylalkyl or heteroaryl, R₉ represents H, and R₁₀ represents hydroxy, hydroxyalkyl or alkoxy, and R₁₁ represents arylalkyl, such trifluoromethoxybenzyl (*e.g.*, 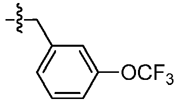 ). In certain such embodiments, R₈ represents aryl and R₁₀ represents hydroxyalkyl.

In certain embodiments, the compound is selected from any one of the compounds disclosed in Tables 1 and 2. In certain embodiments, the compound is selected from compound 447, 585, 586, 600, 614, 615, 629, 636, 657, 658, 659, 660, 661, 662, 663, 666, 668, 669, 670, 671, 672, 673, 674, 675, 676, 677, 678, 679, 680, 681, 682, 683, 684, 685, 686, 687, 688, 689, 690, 692, 693, 694, 695, 696, 697, 698, 699, 700, 701, 702, 703, 704, 705, 706, 707, 708, 709, 715, 716, 717, 718, 719, 720, 721, 722, 723, 724, 725, 726, 727, 728, 729, or 730. In certain embodiments, the compound is selected from compound 657, 658, 659, 660, 661, 662, 663, 666, 668, 669, 670, 671, 672, 673, 674, 675, 676, 677, 678, 679, 680, 681, 682, 683, 684, 685, 686, 687, 688, 689, 690, 692, 693, 694, 695, 696, 697, 698, 699, 700, 701, 702, 703, 704, 705, 706, 707, 708, 709, 715, 716, 717, 718, 719, 720, 721, 722, 723, 724, 725, 726, 727, 728, 729, or 730.

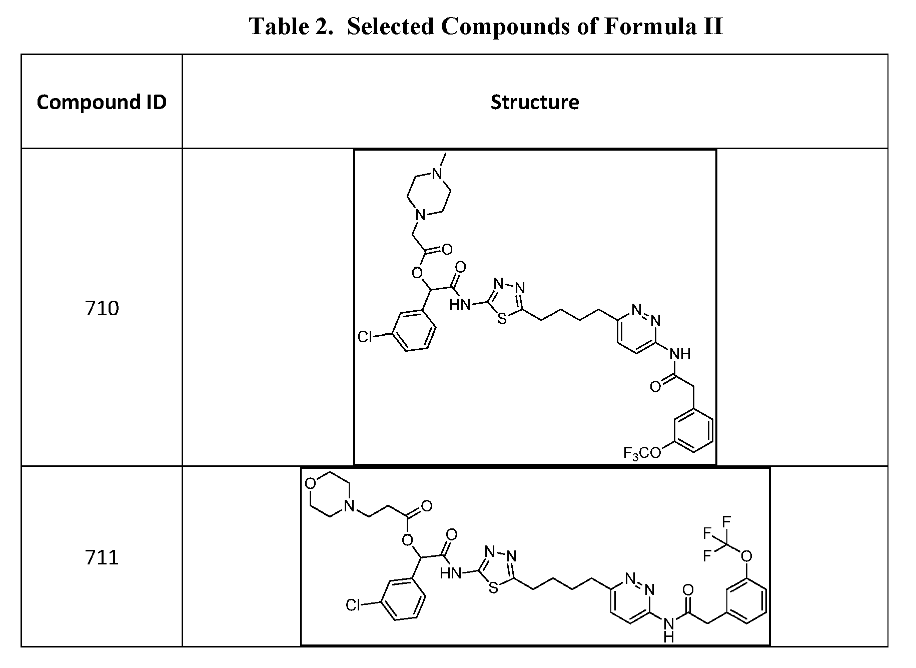

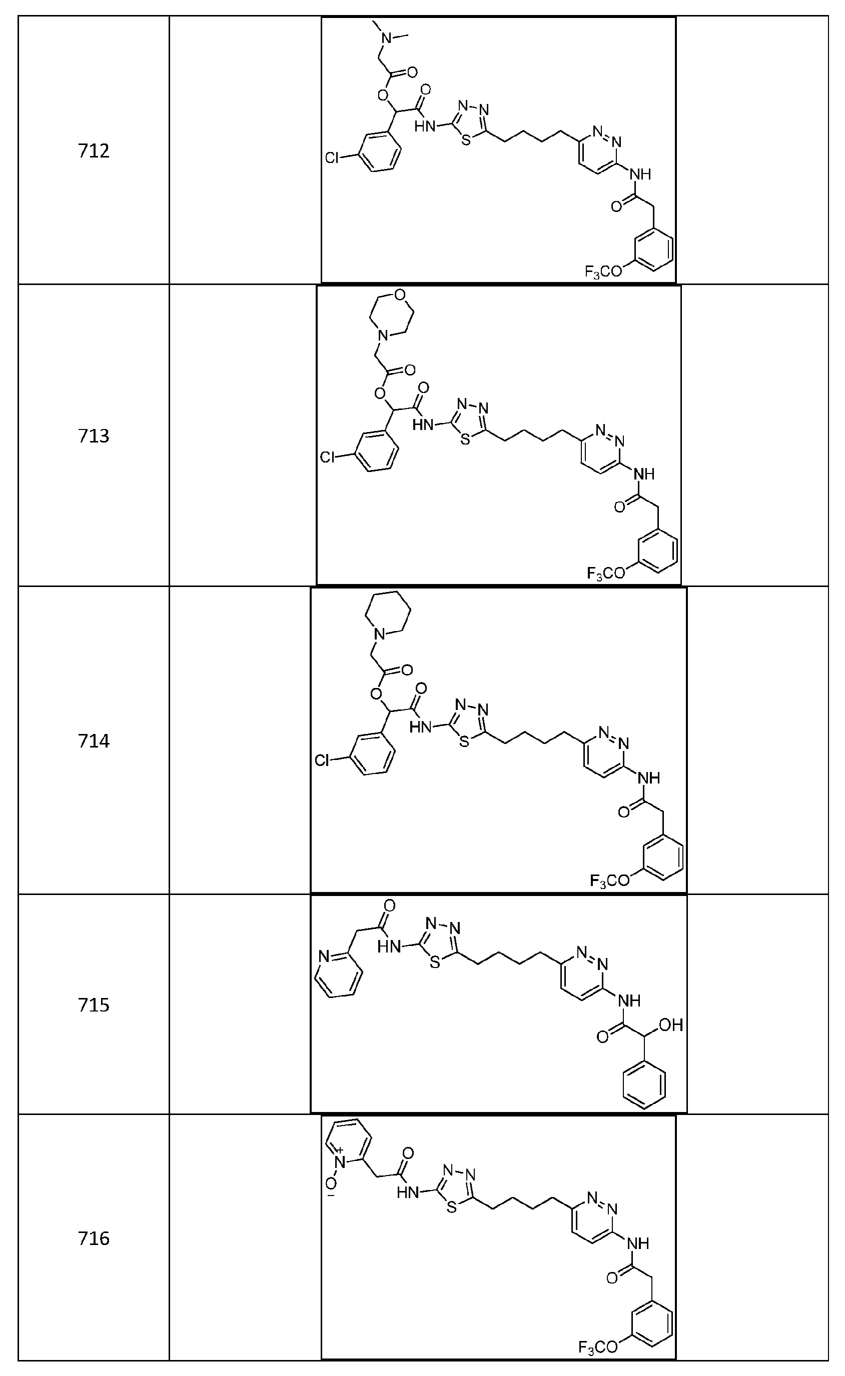

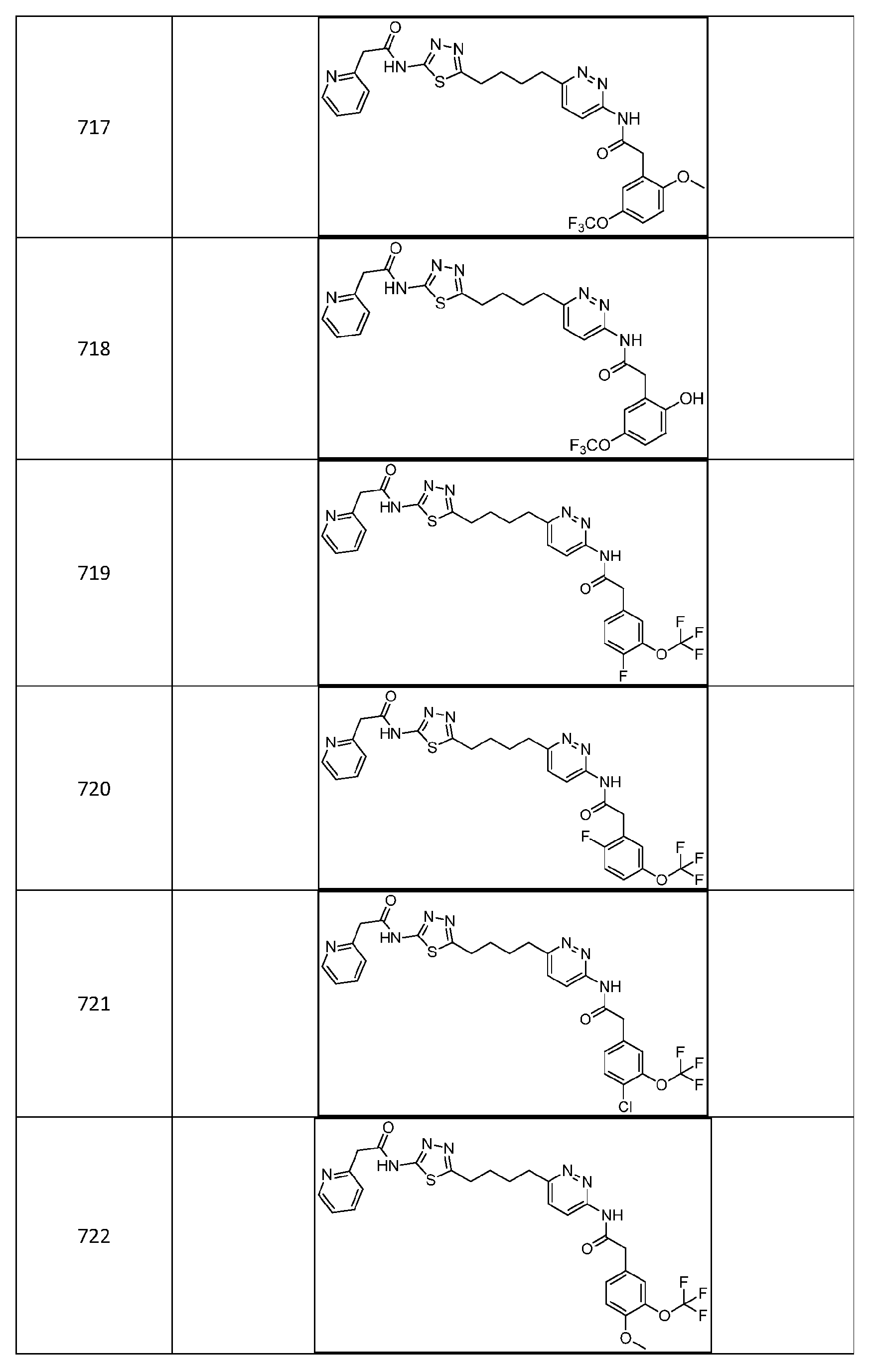

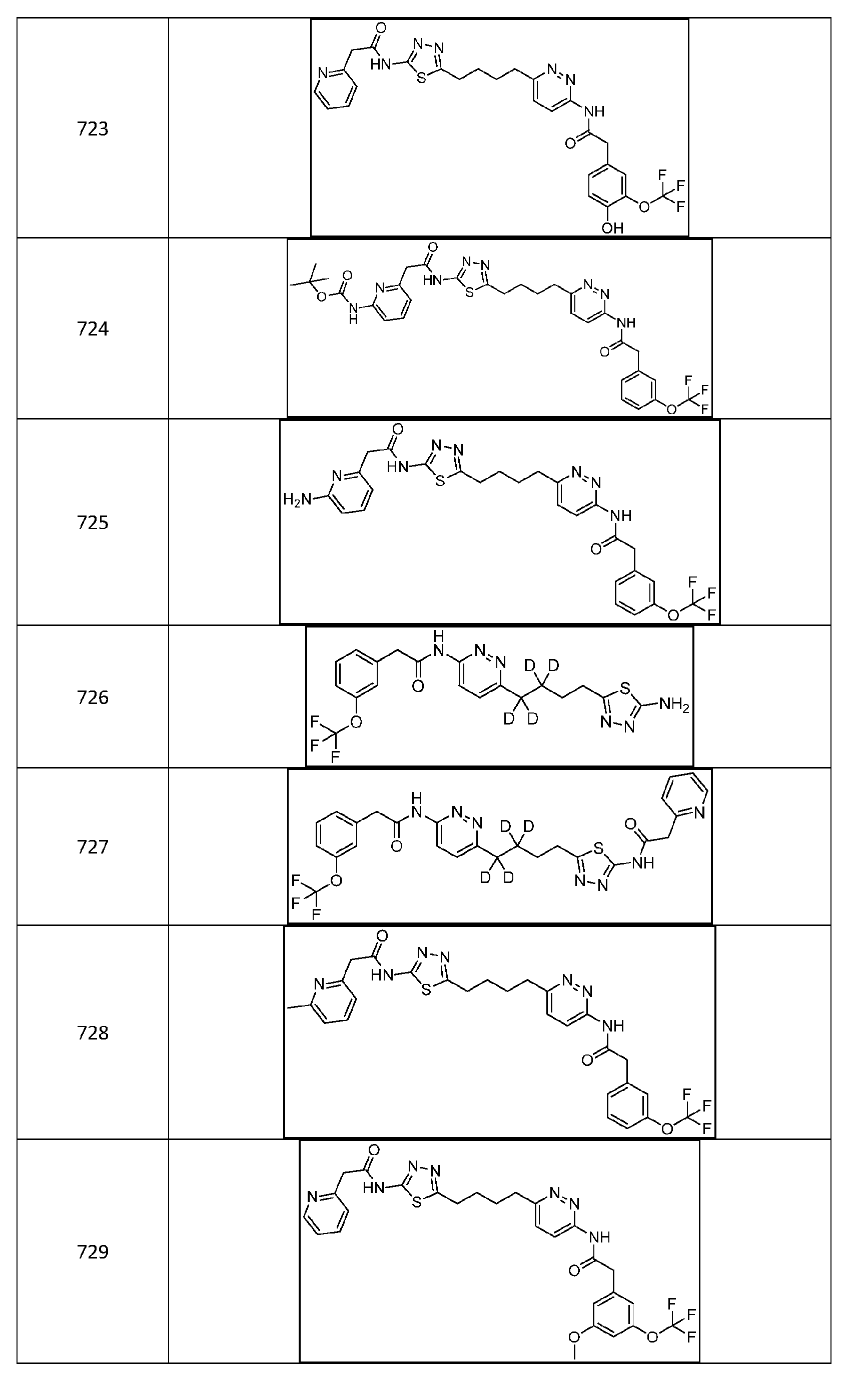

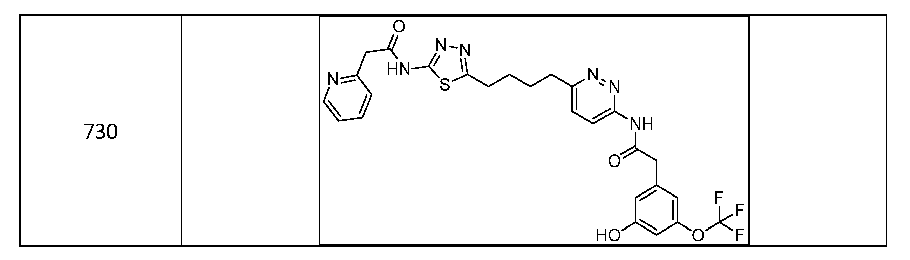

In certain embodiments of the methods described herein, the compound used in the methods of the invention is a compound having the structure of Formula (III): 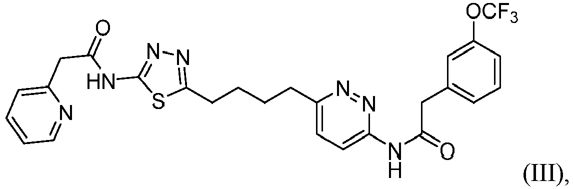 or a pharmaceutically acceptable salt thereof.

Compounds of any of Formulae (I), (Ia), (II), or (III) are alternatively referred to herein as "glutaminase inhibitors."

In certain embodiments, compounds of the invention may be prodrugs of the compounds of formulas I, II or III *e.g*., wherein a hydroxyl in the parent compound is presented as an ester or a carbonate, or carboxylic acid present in the parent compound is presented as an ester. In certain such embodiments, the prodrug is metabolized to the active parent compound in vivo (*e.g.,* the ester is hydrolyzed to the corresponding hydroxyl, or carboxylic acid).

In certain embodiments, compounds of the invention may be racemic. In certain embodiments, compounds of the invention may be enriched in one enantiomer. For example, a compound of the invention may have greater than 30% ee, 40% ee, 50% ee, 60% ee, 70% ee, 80% ee, 90% ee, or even 95% or greater ee. In certain embodiments, compounds of the invention may have more than one stereocenter. In certain such embodiments, compounds of the invention may be enriched in one or more diastereomer. For example, a compound of the invention may have greater than 30% de, 40% de, 50% de, 60% de, 70% de, 80% de, 90% de, or even 95% or greater de.

In certain embodiments, the therapeutic preparation may be enriched to provide predominantly one enantiomer of a compound (*e.g.,* of formulas I-III). An enantiomerically enriched mixture may comprise, for example, at least 60 mol percent of one enantiomer, or more preferably at least 75, 90, 95, or even 99 mol percent. In certain embodiments, the compound enriched in one enantiomer is substantially free of the other enantiomer, wherein substantially free means that the substance in question makes up less than 10%, or less than 5%, or less than 4%, or less than 3%, or less than 2%, or less than 1% as compared to the amount of the other enantiomer, *e.g*., in the composition or compound mixture. For example, if a composition or compound mixture contains 98 grams of a first enantiomer and 2 grams of a second enantiomer, it would be said to contain 98 mol percent of the first enantiomer and only 2% of the second enantiomer.

In certain embodiments, the therapeutic preparation may be enriched to provide predominantly one diastereomer of a compound (*e.g.,* of formulas I-III). A diastereomerically enriched mixture may comprise, for example, at least 60 mol percent of one diastereomer, or more preferably at least 75, 90, 95, or even 99 mol percent.

In certain embodiments, the present invention provides a pharmaceutical preparation suitable for oral administration to a human patient, comprising any of the compounds shown above (*e.g.,* a glutaminase inhibitor, such as a compound of formulas I-III). and one or more pharmaceutically acceptable excipients.

### II. USE OF COMPOUNDS

Glutamine plays an important role as a carrier of nitrogen, carbon, and energy. It is used for hepatic urea synthesis, for renal ammoniagenesis, for gluconeogenesis, and as respiratory fuel for many cells. The conversion of glutamine into glutamate is initated by the mitochondrial enzyme, glutaminase ("GLS"). There are two major forms of the enzyme, K-type and L-type, which are distinguished by their Km values for glutamine and response to glutamate, wherein the Km value, or Michaelis constant, is the concentration of substrate required to reach half the maximal velocity. The L-type, also known as "liver-type" or GLS2, has a high Km for glutamine and is glutamate resistant. The K-type, also known as "kidney-type or GLS1, has a low Km for glutamine and is inhibited by glutamate. An alternative splice form of GLS1, referred to as glutmainase C or "GAC", has been identified recently and has similar activity characteristics of GLS1. In certain embodiments, the compounds may selectively inhibit GLS1, GLS2 and GAC. In certain preferred embodiments, the compounds selectively inhibit GLS1 and GAC.

In addition to serving as the basic building blocks of protein synthesis, amino acids have been shown to contribute to many processes critical for growing and dividing cells, and this is particularly true for cancer cells. Nearly all definitions of cancer include reference to dysregulated proliferation. Numerous studies on glutamine metabolism in cancer indicate that many tumors are avid glutamine consumers. Accordingly, in certain embodiments, the invention provides methods for treating or preventing cancer, a myeloproliferative disease, an immunological disease, a neurological disease, or a viral infection comprising orally administering a glutaminase inhibitor (*e.g.,* a compound of any of formulas I-III or a pharmaceutically acceptable salt thereof), wherein the compound is administered with a meal.

In certain embodiments, the cancer may be one or a variant of Acute Lymphoblastic Leukemia (ALL), Acute Myeloid Leukemia (AML), Adrenocortical Carcinoma, AIDS-Related Cancers (Kaposi Sarcoma and Lymphoma), Anal Cancer, Appendix Cancer, Atypical Teratoid/Rhabdoid Tumor, Basal Cell Carcinoma, Bile Duct Cancer (including Extrahepatic), Bladder Cancer, Bone Cancer (including Osteosarcoma and Malignant Fibrous Histiocytoma), Brain Tumor (such as Astrocytomas, Brain and Spinal Cord Tumors, Brain Stem Glioma, Central Nervous System Atypical Teratoid/Rhabdoid Tumor, Central Nervous System Embryonal Tumors, Craniopharyngioma, Ependymoblastoma, Ependymoma, Medulloblastoma, Medulloepithelioma, Pineal Parenchymal Tumors of Intermediate Differentiation, Supratentorial Primitive Neuroectodermal Tumors and Pineoblastoma), Breast Cancer, Bronchial Tumors, Burkitt Lymphoma, Basal Cell Carcinoma, Bile Duct Cancer (including Extrahepatic), Bladder Cancer, Bone Cancer (including Osteosarcoma and Malignant Fibrous Histiocytoma), Carcinoid Tumor, Carcinoma of Unknown Primary, Central Nervous System (such as Atypical Teratoid/Rhabdoid Tumor, Embryonal Tumors and Lymphoma), Cervical Cancer, Childhood Cancers, Chordoma, Chronic Lymphocytic Leukemia (CLL), Chronic Myelogenous Leukemia (CML), Chronic Myeloproliferative Disorders, Colon Cancer, Colorectal Cancer, Craniopharyngioma, Cutaneous T-Cell Lymphoma (Mycosis Fungoides and Sezary Syndrome), Duct, Bile (Extrahepatic), Ductal Carcinoma In Situ (DCIS), Embryonal Tumors (Central Nervous System), Endometrial Cancer, Ependymoblastoma, Ependymoma, Esophageal Cancer, Esthesioneuroblastoma, Ewing Sarcoma Family of Tumors, Extracranial Germ Cell Tumor, Extragonadal Germ Cell Tumor, Extrahepatic Bile Duct Cancer, Eye Cancer (like Intraocular Melanoma, Retinoblastoma), Fibrous Histiocytoma of Bone (including Malignant and Osteosarcoma) Gallbladder Cancer, Gastric (Stomach) Cancer, Gastrointestinal Carcinoid Tumor, Gastrointestinal Stromal Tumors (GIST), Germ Cell Tumor (Extracranial, Extragonadal, Ovarian), Gestational Trophoblastic Tumor, Glioma, Hairy Cell Leukemia, Head and Neck Cancer, Heart Cancer, Hepatocellular (Liver) Cancer, Histiocytosis, Langerhans Cell, Hodgkin Lymphoma, Hypopharyngeal Cancer, Intraocular Melanoma, Islet Cell Tumors (Endocrine, Pancreas), Kaposi Sarcoma, Kidney (including Renal Cell), Langerhans Cell Histiocytosis, Laryngeal Cancer, Leukemia (including Acute Lymphoblastic (ALL), Acute Myeloid (AML), Chronic Lymphocytic (CLL), Chronic Myelogenous (CML), Hairy Cell), Lip and Oral Cavity Cancer, Liver Cancer (Primary), Lobular Carcinoma In Situ (LCIS), Lung Cancer (Non-Small Cell and Small Cell), Lymphoma (AIDS-Related, Burkitt, Cutaneous T-Cell (Mycosis Fungoides and Sezary Syndrome), Hodgkin, Non-Hodgkin, Primary Central Nervous System (CNS), Macroglobulinemia, Waldenstrom, Male Breast Cancer, Malignant Fibrous Histiocytoma of Bone and Osteosarcoma, Medulloblastoma, Medulloepithelioma, Melanoma (including Intraocular (Eye)), Merkel Cell Carcinoma, Mesothelioma (Malignant), Metastatic Squamous Neck Cancer with Occult Primary, Midline Tract Carcinoma Involving *NUT* Gene, Mouth Cancer, Multiple Endocrine Neoplasia Syndromes, Multiple Myeloma/Plasma Cell Neoplasm, Mycosis Fungoides, Myelodysplastic Syndromes, Myelodysplastic/Myeloproliferative Neoplasms, Myelogenous Leukemia, Chronic (CML), Myeloid Leukemia, Acute (AML), Myeloma and Multiple Myeloma, Myeloproliferative Disorders (Chronic), Nasal Cavity and Paranasal Sinus Cancer, Nasopharyngeal Cancer, Neuroblastoma, Non-Hodgkin Lymphoma, Non-Small Cell Lung Cancer, Oral Cancer, Oral Cavity Cancer, Lip and,Oropharyngeal Cancer, Osteosarcoma and Malignant Fibrous Histiocytoma of Bone, Ovarian Cancer (such as Epithelial, Germ Cell Tumor, and Low Malignant Potential Tumor), Pancreatic Cancer (including Islet Cell Tumors), Papillomatosis, Paraganglioma, Paranasal Sinus and Nasal Cavity Cancer, Parathyroid Cancer, Penile Cancer, Pharyngeal Cancer, Pheochromocytoma, Pineal Parenchymal Tumors of Intermediate Differentiation, Pineoblastoma and Supratentorial Primitive Neuroectodermal Tumors, Pituitary Tumor, Plasma Cell Neoplasm/Multiple Myeloma, Pleuropulmonary Blastoma, Pregnancy and Breast Cancer, Primary Central Nervous System (CNS) Lymphoma, Prostate Cancer, Rectal Cancer, Renal Cell (Kidney) Cancer, Renal Pelvis and Ureter, Transitional Cell Cancer, Retinoblastoma, Rhabdomyosarcoma, Salivary Gland Cancer, Sarcoma (like Ewing Sarcoma Family of Tumors, Kaposi, Soft Tissue, Uterine), Sezary Syndrome, Skin Cancer (such as Melanoma, Merkel Cell Carcinoma,Nonmelanoma), Small Cell Lung Cancer, Small Intestine Cancer, Soft Tissue Sarcoma, Squamous Cell Carcinoma, Squamous Neck Cancer with Occult Primary, Metastatic, Stomach (Gastric) Cancer, Supratentorial Primitive Neuroectodermal Tumors, T-Cell Lymphoma(Cutaneous, Mycosis Fungoides and Sezary Syndrome), Testicular Cancer, Throat Cancer, Thymoma and Thymic Carcinoma, Thyroid Cancer, Transitional Cell Cancer of the Renal Pelvis and Ureter, Trophoblastic Tumor (Gestational), Unknown Primary, Unusual Cancers of Childhood, Ureter and Renal Pelvis, Transitional Cell Cancer, Urethral Cancer, Uterine Cancer, Endometrial, Uterine Sarcoma, Waldenstrom Macroglobulinemia and Wilms Tumor.

In some instances, oncogenic mutations promote glutamine metabolism. Cells expressing oncogenic K-Ras exhibit increased utilization of glutamine. In certain embodiments, the cancer cells have a mutated K-Ras gene. In certain embodiments, the cancer is associated with tissue of the bladder, bone marrow, breast, colon, kidney, liver, lung, ovary, pancreas, prostate, skin or thyroid. The c-Myc gene is known to be altered in numerous cancers. Increased Myc protein expression has been correlated with increased expression of glutaminase, leading to up-regulation of glutamine metabolism. In certain embodiments, the cancer cells have an oncogenic c-Myc gene or elevated Myc protein expression. In some embodiments, the cancer is associated with tissue of the bladder, bone, bowel, breast, central nervous system (like brain), colon, gastric system (such as stomach and intestine), liver, lung, ovary, prostate, muscle, and skin.

For example, the most common type of renal cell carcinoma (RCC), clear cell type (ccRCC), is closely associated with von Hippel-Lindau (VHL) gene mutations. VHL-deficient cell lines have been shown to have an increased requirement for glutamine due to a loss of ability to make fatty acids from glucose (Metallo et al, Nature 2013). This dependency on glutamine makes the cells susceptible to glutaminase inhibitors (Gameiro et al., Cell Metab. 2013). Certain embodiments of the invention relate to the use of the compounds described herein for the treatment of VHL-deficient carcinomas. In certain embodiments the cancer is RCC. In certain embodiments the cancer is ccRCC.

EGFR (Epidermal growth factor receptor) is the cell-surface receptor for members of the epidermal growth factor (EGF) family of extracellular protein ligands. Mutations associated with EGFR overexpression have been associated with certain cancers, including lung cancers. Approximately 10% of non-small cell lung cancer patients in the United States, and approximately 35% of nsclc patients in East Asia have tumors associated with an EGFR mutation. Typically the EGFR mutation occurs in a region of the gene that encodes a portion of the EGFR kinase domain. Usually, such mutations result in gene amplification, increased kinase activity of EGFR, and hyperactivation of downstream pro-survival signaling pathways. See A. Kuykendall, et al. ("Advanced EGFR Mutation-Positive Non-Small Cell Lung Cancer: Case Report, Literature Review, and Treatment Recommendations" Cancer Control, 2014, V. 21, No. 1, 67-73) for a review about NSCLC and EGFR mutations.

Glutaminase inhibition may also be effective in certain rare cancers that have mutations or deletions of the TCA cycle enzymes including fumarate hydratase (FH), succinate dehydrogenase (SDH), and isocitrate dehydrogenase (IDH). Glutamate feeds into the TCA cycle upstream of where these mutations or deletions occur. Published studies indicate that glutamine metabolism is important in the synthesis of fumarate and succinate. In addition to FH and SDH, there is evidence that glutamine contributes to the production of 2-hydroxyglutatrate, another driver of tumor formation that accumulates in patients with tumors harboring mutations in the enzyme isocitrate dehydrogenase. Thus, inhibitors of glutaminase may block the effect of these mutations or deletions by limiting the availability of upstream starting materials. Rare mutations in FH lead to the development of hereditary leiomyomatosis and renal cell cancer (HLRCC), where patients can develop tumors of the skin, uterus and kidneys. Some gastrointestinal stromal tumors (GIST), arise from the lack of expression of SDH, and are often hereditary. Other SDH-loss-of-function mutations are found in patients exhibiting a rare head and neck cancer known as paraganglioma, and a rare adrenal or extra-adrenal cancer known as pheochromocytoma, and a rare subset clear cell RCC. Some patients with glioma, a form of brain cancer, chondrosarcoma, a rare bone cancer, cholangiocarcinoma, a rare bile duct tumor, AML, or high risk myeldysplasia/myeloproliferative disorders, a group of blood disorders, have IDH1 or IDH2 driver mutations.

In certain embodiments of the invention, compounds described herein can be used for the treatment of disease identified with a FH, SDH or IDH (1 and 2) mutation. For example, in certain embodiments, the disease is an isocitrate dehydrogenase (IDH)-mutant solid tumor. In certain embodiments the disease is hereditary leiomyomatosis or renal cell cancer (HLRCC). In certain embodiments the disease is GIST (e.g., SDH-deficient GIST), paraganglioma, pheochromocytoma, or clear cell RCC. In certain embodiments, the disease is glioma, chondrosarcoma, cholangiocarcinoma, acute myeloid leukemia (AML), or myelodysplasia/myeloproliferative disorder. In certain embodiments, the disease is mesothelioma. In certain embodiments, the disease is multiple myeloma.

In certain embodiments, the cancer is a non-small cell lung cancer having a KRAS or EGFR mutation.

While many cancer cells depend on exogenous glutamine for survival, the degree of glutamine dependence among tumor cell subtypes may make a population of cells more susceptible to the reduction of glutamine. As an example, gene expression analysis of breast cancers has identified five intrinsic subtypes (luminal A, luminal B, basal, HER2+, and normal-like). Although glutamine deprivation has an impact on cell growth and viability, basal-like cells appear to be more sensitive to the reduction of exogenous glutamine. This supports the concept that glutamine is a very important energy source in basal-like breast cancer cell lines, and suggests that inhibition of the glutaminase enzyme would be beneficial in the treatment of breast cancers comprised of basal-like cells. Triple-negative breast cancer (TNBC) is characterized by a lack of estrogen receptor, progesterone receptor and human epidermal growth factor receptor 2 expression. It has a higher rate of relapse following chemotherapy, and a poorer prognosis than with the other breast cancer subtypes. Interestingly, there appears to be significant similarities in metabolic profiling between TNBC cells and basal-like breast cancer cells. Therefore, an embodiment of the invention is the use of the compounds described herein for the treatment of TNBC, basal-type breast cancers, or claudin-low breast cancers.

In certain embodiments, the invention provides methods for treating colorectal cancer. In certain embodiments, the invention provides methods for treating endocrine cancer, such as adrenal cortex adenoma, adrenal cortex carcinoma, adrenal gland pheochromocytoma, and parathyroid gland adenoma.

In certain embodiments, the cancer is melanoma.

In certain embodiments, the method of treating or preventing cancer, may comprise orally administering a compound of the invention, *e.g.,* a compound of any of formulas I-III or a pharmaceutically acceptable salt thereof, *e.g.,* with a meal, conjointly with a chemotherapeutic agent. Chemotherapeutic agents that may be conjointly administered with compounds of the invention include: ABT-263, aminoglutethimide, amsacrine, anastrozole, asparaginase, azacitidine, AZD5363, Bacillus Calmette-Guerin vaccine (bcg), bicalutamide, bleomycin, bortezomib, buserelin, busulfan, campothecin, capecitabine, carboplatin, carfilzomib, carmustine, chlorambucil, chloroquine, cisplatin, cladribine, clodronate, cobimetinib, colchicine, cyclophosphamide, cyproterone, cytarabine, dacarbazine, dactinomycin, daunorubicin, demethoxyviridin, dexamethasone, dichloroacetate, dienestrol, diethylstilbestrol, docetaxel, doxorubicin, epirubicin, eribulin, erlotinib, estradiol, estramustine, etoposide, everolimus, exemestane, filgrastim, fludarabine, fludrocortisone, fluorouracil (e.g., 5-fluorouracil), fluoxymesterone, flutamide, gemcitabine, genistein, goserelin, hydroxyurea, idarubicin, ifosfamide, imatinib, interferon, irinotecan, ixabepilone, lenalidomide, letrozole, leucovorin, leuprolide, levamisole, lomustine, lonidamine, mechlorethamine, medroxyprogesterone, megestrol, melphalan, mercaptopurine, mesna, metformin, methotrexate, miltefosine, mitomycin, mitotane, mitoxantrone, MK-2206, nilutamide, nocodazole, octreotide, oxaliplatin, olaparib, paclitaxel, pamidronate, pazopanib, pentostatin, perifosine, PF-04691502, plicamycin, pomalidomide, porfimer, procarbazine, raltitrexed, rituximab, romidepsin, rucaparib, selumetinib, sorafenib, streptozocin, sunitinib, suramin, talazoparib, tamoxifen, temozolomide, temsirolimus, teniposide, testosterone, thioguanine, thalidomide, thiotepa, titanocene dichloride, topotecan, trametinib, trastuzumab, tretinoin, veliparib, vinblastine, vincristine, vindesine, vinorelbine and vorinostat (SAHA).

In certain embodiments, the one or more additional chemotherapeutic agents are selected from azacitidine, bortezomib, capecitabine, carboplatin, carfilzomib, cyclophosphamide, daunorubicin, dexamethasone, docetaxel, doxorubicin, epirubicin, eribulin, erlotinib, everolimus, fluorouracil, gemcitabine, ixabepilone, lenalidomide, methotrexate, mitoxantrone, mutamycin, paclitaxel, pomalidomide, rituximab, thiotepa, vincristine, and vinorelbine.

In certain embodiments, the one or more additional chemotherapeutic agents are selected from azacitidine, dexamethasone, docetaxel, erlotinib, everolimus, paclitaxel and pomalidomide.

Many combination therapies have been developed for the treatment of cancer. In certain embodiments, compounds of the invention may be conjointly administered with a combination therapy. Examples of combination therapies with which compounds of the invention may be conjointly administered are included in Table 3.

**Table 3: Exemplary combinatorial therapies for the treatment of cancer.**

| **Name** | **Therapeutic agents** |
|---|---|
| ABV | Doxorubicin, Bleomycin, Vinblastine |
| ABVD | Doxorubicin, Bleomycin, Vinblastine, Dacarbazine |
| AC (Breast) | Doxorubicin, Cyclophosphamide |
| AC (Sarcoma) | Doxorubicin, Cisplatin |
| AC (Neuroblastoma) | Cyclophosphamide, Doxorubicin |
| ACE | Cyclophosphamide, Doxorubicin, Etoposide |
| ACe | Cyclophosphamide, Doxorubicin |
| AD | Doxorubicin, Dacarbazine |
| AP | Doxorubicin, Cisplatin |
| ARAC-DNR | Cytarabine, Daunorubicin |
| B-CAVe | Bleomycin, Lomustine, Doxorubicin, Vinblastine |
| BCVPP | Carmustine, Cyclophosphamide, Vinblastine, Procarbazine, Prednisone |
| BEACOPP | Bleomycin, Etoposide, Doxorubicin, Cyclophosphamide, Vincristine, Procarbazine, Prednisone, Filgrastim |
| BEP | Bleomycin, Etoposide, Cisplatin |
| BIP | Bleomycin, Cisplatin, Ifosfamide, Mesna |
| BOMP | Bleomycin, Vincristine, Cisplatin, Mitomycin |
| CA | Cytarabine, Asparaginase |
| CABO | Cisplatin, Methotrexate, Bleomycin, Vincristine |
| CAF | Cyclophosphamide, Doxorubicin, Fluorouracil |
| CAL-G | Cyclophosphamide, Daunorubicin, Vincristine, Prednisone, Asparaginase |
| CAMP | Cyclophosphamide, Doxorubicin, Methotrexate, Procarbazine |
| CAP | Cyclophosphamide, Doxorubicin, Cisplatin |
| CaT | Carboplatin, Paclitaxel |
| CAV | Cyclophosphamide, Doxorubicin, Vincristine |
| CAVE ADD | CAV and Etoposide |
| CA-VP16 | Cyclophosphamide, Doxorubicin, Etoposide |
| CC | Cyclophosphamide, Carboplatin |
| CDDP/VP-16 | Cisplatin, Etoposide |
| CEF | Cyclophosphamide, Epirubicin, Fluorouracil |
| CEPP(B) | Cyclophosphamide, Etoposide, Prednisone, with or without/Bleomycin |
| CEV | Cyclophosphamide, Etoposide, Vincristine |
| CF | Cisplatin, Fluorouracil or Carboplatin Fluorouracil |
| CHAP | Cyclophosphamide or Cyclophosphamide, Altretamine, Doxorubicin, Cisplatin |
| ChlVPP | Chlorambucil, Vinblastine, Procarbazine, Prednisone |
| CHOP | Cyclophosphamide, Doxorubicin, Vincristine, Prednisone |
| CHOP-BLEO | Add Bleomycin to CHOP |
| CISCA | Cyclophosphamide, Doxorubicin, Cisplatin |
| CLD-BOMP | Bleomycin, Cisplatin, Vincristine, Mitomycin |
| CMF | Methotrexate, Fluorouracil, Cyclophosphamide |
| CMFP | Cyclophosphamide, Methotrexate, Fluorouracil, Prednisone |
| CMFVP | Cyclophosphamide, Methotrexate, Fluorouracil, Vincristine, Prednisone |
| CMV | Cisplatin, Methotrexate, Vinblastine |
| CNF | Cyclophosphamide, Mitoxantrone, Fluorouracil |
| CNOP | Cyclophosphamide, Mitoxantrone, Vincristine, Prednisone |
| COB | Cisplatin, Vincristine, Bleomycin |
| CODE | Cisplatin, Vincristine, Doxorubicin, Etoposide |
| COMLA | Cyclophosphamide, Vincristine, Methotrexate, Leucovorin, Cytarabine |
| COMP | Cyclophosphamide, Vincristine, Methotrexate, Prednisone |
| Cooper Regimen | Cyclophosphamide, Methotrexate, Fluorouracil, Vincristine, Prednisone |
| COP | Cyclophosphamide, Vincristine, Prednisone |
| COPE | Cyclophosphamide, Vincristine, Cisplatin, Etoposide |
| COPP | Cyclophosphamide, Vincristine, Procarbazine, Prednisone |
| CP(Chronic lymphocytic leukemia) | Chlorambucil, Prednisone |
| CP (Ovarian Cancer) | Cyclophosphamide, Cisplatin |
| CT | Cisplatin, Paclitaxel |
| CVD | Cisplatin, Vinblastine, Dacarbazine |
| CVI | Carboplatin, Etoposide, Ifosfamide, Mesna |
| CVP | Cyclophosphamide, Vincristine, Prednisome |
| CVPP | Lomustine, Procarbazine, Prednisone |
| CYVADIC | Cyclophosphamide, Vincristine, Doxorubicin, Dacarbazine |
| DA | Daunorubicin, Cytarabine |
| DAT | Daunorubicin, Cytarabine, Thioguanine |
| DAV | Daunorubicin, Cytarabine, Etoposide |
| DCT | Daunorubicin, Cytarabine, Thioguanine |
| DHAP | Cisplatin, Cytarabine, Dexamethasone |
| DI | Doxorubicin, Ifosfamide |
| DTIC/Tamoxifen | Dacarbazine, Tamoxifen |
| DVP | Daunorubicin, Vincristine, Prednisone |
| EAP | Etoposide, Doxorubicin, Cisplatin |
| EC | Etoposide, Carboplatin |
| EFP | Etoposie, Fluorouracil, Cisplatin |
| ELF | Etoposide, Leucovorin, Fluorouracil |
| EMA 86 | Mitoxantrone, Etoposide, Cytarabine |
| EP | Etoposide, Cisplatin |
| EVA | Etoposide, Vinblastine |
| FAC | Fluorouracil, Doxorubicin, Cyclophosphamide |
| FAM | Fluorouracil, Doxorubicin, Mitomycin |
| FAMTX | Methotrexate, Leucovorin, Doxorubicin |
| FAP | Fluorouracil, Doxorubicin, Cisplatin |
| F-CL | Fluorouracil, Leucovorin |
| FEC | Fluorouracil, Cyclophosphamide, Epirubicin |
| FED | Fluorouracil, Etoposide, Cisplatin |
| FL | Flutamide, Leuprolide |
| FZ | Flutamide, Goserelin acetate implant |
| HDMTX | Methotrexate, Leucovorin |
| Hexa-CAF | Altretamine, Cyclophosphamide, Methotrexate, Fluorouracil |
| ICE-T | Ifosfamide, Carboplatin, Etoposide, Paclitaxel, Mesna |
| IDMTX/6-MP | Methotrexate, Mercaptopurine, Leucovorin |
| IE | Ifosfamide, Etoposie, Mesna |
| IfoVP | Ifosfamide, Etoposide, Mesna |
| IPA | Ifosfamide, Cisplatin, Doxorubicin |
| M-2 | Vincristine, Carmustine, Cyclophosphamide, Prednisone, Melphalan |
| MAC-III | Methotrexate, Leucovorin, Dactinomycin, Cyclophosphamide |
| MACC | Methotrexate, Doxorubicin, Cyclophosphamide, Lomustine |
| MACOP-B | Methotrexate, Leucovorin, Doxorubicin, Cyclophosphamide, Vincristine, Bleomycin, Prednisone |
| MAID | Mesna, Doxorubicin, Ifosfamide, Dacarbazine |
| m-BACOD | Bleomycin, Doxorubicin, Cyclophosphamide, Vincristine, Dexamethasone, Methotrexate, Leucovorin |
| MBC | Methotrexate, Bleomycin, Cisplatin |
| MC | Mitoxantrone, Cytarabine |
| MF | Methotrexate, Fluorouracil, Leucovorin |
| MICE | Ifosfamide, Carboplatin, Etoposide, Mesna |
| MINE | Mesna, Ifosfamide, Mitoxantrone, Etoposide |
| mini-BEAM | Carmustine, Etoposide, Cytarabine, Melphalan |
| MOBP | Bleomycin, Vincristine, Cisplatin, Mitomycin |
| MOP | Mechlorethamine, Vincristine, Procarbazine |
| MOPP | Mechlorethamine, Vincristine, Procarbazine, Prednisone |
| MOPP/ABV | Mechlorethamine, Vincristine, Procarbazine, Prednisone, Doxorubicin, Bleomycin, Vinblastine |
| MP (multiple myeloma) | Melphalan, Prednisone |
| MP (prostate cancer) | Mitoxantrone, Prednisone |
| MTX/6-MO | Methotrexate, Mercaptopurine |
| MTX/6-MP/VP | Methotrexate, Mercaptopurine, Vincristine, Prednisone |
| MTX-CDDPAdr | Methotrexate, Leucovorin, Cisplatin, Doxorubicin |
| MV (breast cancer) | Mitomycin, Vinblastine |
| MV (acute myelocytic leukemia) | Mitoxantrone, Etoposide |
| M-VAC Methotrexate | Vinblastine, Doxorubicin, Cisplatin |
| MVP Mitomycin | Vinblastine, Cisplatin |
| MVPP | Mechlorethamine, Vinblastine, Procarbazine, Prednisone |
| NFL | Mitoxantrone, Fluorouracil, Leucovorin |
| NOVP | Mitoxantrone, Vinblastine, Vincristine |
| OPA | Vincristine, Prednisone, Doxorubicin |
| OPPA | Add Procarbazine to OPA. |
| PAC | Cisplatin, Doxorubicin |
| PAC-I | Cisplatin, Doxorubicin, Cyclophosphamide |
| PA-CI | Cisplatin, Doxorubicin |
| PC | Paclitaxel, Carboplatin or Paclitaxel, Cisplatin |
| PCV | Lomustine, Procarbazine, Vincristine |
| PE | Paclitaxel, Estramustine |
| PFL | Cisplatin, Fluorouracil, Leucovorin |
| POC | Prednisone, Vincristine, Lomustine |
| ProMACE | Prednisone, Methotrexate, Leucovorin, Doxorubicin, Cyclophosphamide, Etoposide |
| ProMACE/cytaBOM | Prednisone, Doxorubicin, Cyclophosphamide, Etoposide, Cytarabine, Bleomycin, Vincristine, Methotrexate, Leucovorin, Cotrimoxazole |
| PRoMACE/MOPP | Prednisone, Doxorubicin, Cyclophosphamide, Etoposide, Mechlorethamine, Vincristine, Procarbazine, Methotrexate, Leucovorin |
| Pt/VM | Cisplatin, Teniposide |
| PVA | Prednisone, Vincristine, Asparaginase |
| PVB | Cisplatin, Vinblastine, Bleomycin |
| PVDA | Prednisone, Vincristine, Daunorubicin, Asparaginase |
| SMF | Streptozocin, Mitomycin, Fluorouracil |
| TAD | Mechlorethamine, Doxorubicin, Vinblastine, Vincristine, Bleomycin, Etoposide, Prednisone |
| TCF | Paclitaxel, Cisplatin, Fluorouracil |
| TIP | Paclitaxel, Ifosfamide, Mesna, Cisplatin |
| TTT | Methotrexate, Cytarabine, Hydrocortisone |
| Topo/CTX | Cyclophosphamide, Topotecan, Mesna |
| VAB-6 | Cyclophosphamide, Dactinomycin, Vinblastine, Cisplatin, Bleomycin |
| VAC | Vincristine, Dactinomycin, Cyclophosphamide |
| VACAdr | Vincristine, Cyclophosphamide, Doxorubicin, Dactinomycin, Vincristine |
| VAD | Vincristine, Doxorubicin, Dexamethasone |
| VATH | Vinblastine, Doxorubicin, Thiotepa, Flouxymesterone |
| VBAP | Vincristine, Carmustine, Doxorubicin, Prednisone |
| VBCMP | Vincristine, Carmustine, Melphalan, Cyclophosphamide, Prednisone |
| VC | Vinorelbine, Cisplatin |
| VCAP | Vincristine, Cyclophosphamide, Doxorubicin, Prednisone |
| VD | Vinorelbine, Doxorubicin |
| VelP | Vinblastine, Cisplatin, Ifosfamide, Mesna |
| VIP | Etoposide, Cisplatin, Ifosfamide, Mesna |
| VM | Mitomycin, Vinblastine |
| VMCP | Vincristine, Melphalan, Cyclophosphamide, Prednisone |
| VP | Etoposide, Cisplatin |
| V-TAD | Etoposide, Thioguanine, Daunorubicin, Cytarabine |
| 5+2 | Cytarabine, Daunorubicin, Mitoxantrone |
| 7+3 | Cytarabine with/, Daunorubicin or Idarubicin or Mitoxantrone |
| "8 in 1" | Methylprednisolone, Vincristine, Lomustine, Procarbazine, Hydroxyurea, Cisplatin, Cytarabine, Dacarbazine |

In certain embodiments, the compounds of the invention may be conjointly administered with an immunomodulatory agent. Examples of immunomodulatory agents with which the compounds of the invention may be administered in a combination therapy include granulocyte colony-stimulating factor (G-CSF), interferons, imiquimod, IL-2, IL-7, IL-12, various chemokines, synthetic cytosine phosphate-guanosine (CpG) oligodeoxynucleotides, glucans, and synthetic small molecules such as apremilast, CC-122, CC-11006, CC-10015, lenalidomide, pomalidomide, and thalidomide. In certain embodiments, the immunomodulatory agent is a thalidomide analog, such as those disclosed in WO 1999/46258, WO 2008/033567, WO 2010/093434, WO 2010/093605, WO 2011/100380, and WO 2012/097116.

In certain embodiments, the compounds of the invention may be conjointly administered with an anticancer agent selected from an enzyme inhibitor (such as a kinase inhibitor), a mitotic inhibitor, a DNA-modifying agent, and a cytidine analog. Examples of anticancer agents with which the compounds of the invention may be administered in a combination therapy include microtubule assembly inhibitors, AKT inhibitors, mTOR inhibitors, MEK inhibitors, RTK inhibitors, ATM inhibitors, ATR inhibitors, PI3K inhibitors, EGFR inhibitors, B-Raf inhibitors, C-kit inhibitors, DNA cross-linking agents, DNA intercalating agents, and cytidine analogs. In certain embodiments, the anticancer agent vincristine, carboplatin, cisplatin, gemcitabine, MK2206, everolimus, trametinib, sunitinib, sorafenib, BEZ235, paclitaxel, docetaxel, erlotinib, selumetinib, sirolimus, trametinib, temsirolimus, pazopanib, or GSK1120212.

The proliferation of cancer cells requires lipid synthesis. Normally, acetyl-coA used for lipid synthesis is formed from a mitochondrial pool of pyruvate that is derived from glycolysis. Yet under hypoxic conditions, such as those normally found in a tumor environment, the conversion of pyruvate to acetyl-coA within the mitochondria is downregulated. Recent studies revealed that under such hypoxic conditions, cells instead largely switch to using a pathway involving the reductive carboxylation of alpha-ketoglutarate to make acetyl-coA for lipid synthesis. The first step in this pathway involves converting glutamine to glutamate via glutaminase enzymes. Subsequently, glutamate is converting to alpha-ketoglutarate, and the resulting alpha-ketoglutarate is converted to isocitrate in a reductive carboxylation step mediated by the isocitrate dehydrogenase enzymes. A switch to this reductive carboxylation pathway also occurs in some renal carcinoma cell lines that contain either impaired mitochondria or an impaired signal for induction of the enzyme responsible for converting glycolytic pyruvate to acetyl-coA. A similar switch occurs in cells exposed to mitochondrial respiratory chain inhibitors such as metformin, rotenone, and antimycin. Therefore, in some embodiments of this invention, we propose using combinations of mitochondrial respiratory chain inhibitors and glutaminase inhibitors to simultaneously increase cancer cells' dependence on glutaminase-dependent pathways for lipid synthesis while inhibiting those very pathways.

The increased dependence on glycolysis in tumor cells is likely because the hypoxic tumor environment impairs mitochondrial respiration. Furthermore, depletion of glucose induces apoptosis in cells transformed with the MYC oncogene. These findings suggest that inhibiting glycolysis would have a therapeutic value in preventing cancer cell proliferation. There are currently many documented glycolytic inhibitors. However, available glycolytic inhibitors are generally not very potent, and thus, high doses are required, which may cause high levels of systemic toxicity. Since cancer cells typically use both glucose and glutamine at higher levels than normal cells, impairing utilization of each of those metabolites will likely have a synergistic effect. Therefore, in some embodiments of this invention, we propose using combinations of glycolytic pathway inhibitors and glutaminase inhibitors. Such glycolytic inhibitors include 2-deoxyglucose, lonidamine, 3-bromopyruvate, imatinib, oxythiamine, rapamycin, and their pharmacological equivalents. Glycolysis can be inhibited indirectly by depleting NAD+ via DNA damage induced by DNA alkylating agents through a pathway activated by poly(ADP-ribose) polymerase. Therefore, in one embodiment of this invention, we propose using a combination of DNA alkylating agents and glutaminase inhibitors. Cancer cells use the pentose phosphate pathway along with the glycolytic pathway to create metabolic intermediates derived from glucose. Therefore, in another embodiment of this invention, we propose using a combination of pentose phosphate inhibitors such as 6-aminonicotinamide along with glutaminase inhibitors.

In certain embodiments, a compound of the invention may be conjointly administered (*e.g.,* orally administered, with a meal) with non-chemical methods of cancer treatment. In certain embodiments, a compound of the invention may be conjointly administered with radiation therapy. In certain embodiments, a compound of the invention may be conjointly administered with surgery, with thermoablation, with focused ultrasound therapy, with cryotherapy, or with any combination of these.

In certain embodiments, different compounds of the invention may be conjointly administered with one or more other compounds of the invention. Moreover, such combinations may be conjointly administered with other therapeutic agents, such as other agents suitable for the treatment of cancer, immunological or neurological diseases, such as the agents identified above.

In certain embodiments, the method of treating or preventing cancer, may comprise orally administering a compound of the invention, *e.g.,* a glutaminase inhibitor of any of formulas I-III or a pharmaceutically acceptable salt thereof, *e.g.,* with a meal, conjointly with an immunomodulatory agent.

In certain embodiments, conjointly administering the immunomodulatory agent and a compound of the invention (i.e., a glutaminase inhibitor) provides improved efficacy relative to individual administration of the immunomodulatory agent or glutaminase inhibitor as a single agent.

In certain embodiments, the conjoint administration of the immunomodulatory agent and glutaminase inhibitor provides an additive effect.

In certain embodiments, the conjoint administration of the immunomodulatory agent and glutaminase inhibitor provides a synergistic effect.

In certain embodiments of the invention, the immunomodulatory agent is administered simultaneously with the glutaminase inhibitor. In certain embodiments the immunomodulatory agent is administered within about 5 minutes to within about 168 hours prior or after of the glutaminase inhibitor.

In certain embodiments, the immunomodulatory agent has a structure of Formula X: 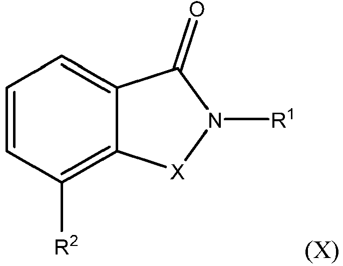 or a pharmaceutically acceptable salt, prodrug, and/or stereoisomer thereof, wherein:
X is C=O or CH₂;
R¹ is heterocyclyl, such as 2,6-dioxopiperidin-3-yl, or aralkyl, such as a sulfonyl-substituted aralkyl, and
R² is independently a hydrogen, an amino group, an acylamino group, an alkylamino group, or is one of the following moieties:
   a) 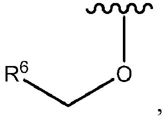 wherein R⁶ is substituted or unsubstituted phenyl, aryl or heteroaryl, or
   b) 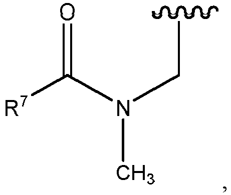 wherein R⁷ is C₁-C₆ alkyl, cycloalkyl, NH-Ar, where Ar is phenyl or substituted phenyl, or NR⁸R⁹, where R⁸ and R⁹ may be independently H or C₁-C₆-alkyl.

In certain embodiments, the immunomodulatory agent is apremilast, lenalidomide, pomalidomide, thalidomide, CC-11006, or CC-10015.

In certain embodiments, the cancer being treated by the methods of the invention is resistant to an immunodulatory agent. In certain embodiments, the cancer is resistant to a compound having the structure of formula (X). In certain embodiments, the cancer is resistant to apremilast, lenalidomide, pomalidomide, thalidomide, CC-11006, or CC-10015.

In certain embodiments, the invention provides methods for treating a myeloproliferative disease, comprising orally administering to a subject a glutaminase inhibitor with a meal, wherein the glutaminase inhibitors are described above.

In certain embodiments, the myeloproliferative disease is selected from chronic eosinophilic leukemia, chronic myelogenous leukemia (CML), chronic neutrophilic leukemia, essential thrombocythemia, polycythemia vera, and myelofibrosis.

In certain embodiments, the myeloproliferative disease being treated by the methods of the invention is resistant to an immunodulatory agent. In certain embodiments, the myeloproliferative disease is resistant to a compound having the structure of formula (X). In certain embodiments, the myeloproliferative disease is resistant to apremilast, lenalidomide, pomalidomide, thalidomide, CC-11006, or CC-10015.

The methods of treating or preventing cancer, a myeloproliferative disease, can further comprise administration of one or more additional chemotherapeutic agents, described above.

In certain preferred embodiments, the additional chemotherapeutic agent is dexamethasone.

### III. KITS

In certain embodiments, the present invention provides a kit comprising: a) one or more single dosage forms of a compound of the invention; b) one or more single dosage forms of a chemotherapeutic agent as mentioned above; and c) instructions for the administration of the compound of the invention and the chemotherapeutic agent. The instructions may state that the compound be taken with food. For example, the instructions may state that the compound should be taken after a meal. The instructions may state that the compound should be taken once, twice, or three times a day, *e.g.,* with meals or after meals.

The present invention provides a kit comprising:
a) a pharmaceutical formulation (*e.g.,* one or more single dosage forms) comprising a compound of the invention; and
b) instructions for the administration of the pharmaceutical formulation, *e.g.,* for treating or preventing any of the conditions discussed above, wherein the instructions state that the compound should be taken with food or after a meal.

In certain embodiments, the kit further comprises instructions for the administration of the pharmaceutical formulation comprising a compound of the invention conjointly with a chemotherapeutic agent as mentioned above. In certain embodiments, the kit further comprises a second pharmaceutical formulation (*e.g.,* as one or more single dosage forms) comprising a chemotherapeutic agent as mentioned above.

### IV. PHARMACEUTICAL COMPOSITIONS

The compositions and methods of the present invention may be utilized to treat an individual in need thereof. In certain embodiments, the individual is a mammal such as a human, or a non-human mammal. When administered to an animal, such as a human, the composition or the compound is preferably administered as a pharmaceutical composition comprising, for example, a compound of the invention and a pharmaceutically acceptable carrier. Pharmaceutically acceptable carriers are well known in the art and include, for example, aqueous solutions such as water or physiologically buffered saline or other solvents or vehicles such as glycols, glycerol, oils such as olive oil, or organic esters. The excipients can be chosen, for example, to effect delayed release of an agent. The pharmaceutical composition can be in dosage unit form such as tablet, capsule (including sprinkle capsule and gelatin capsule), granule, lyophile for reconstitution, powder, solution, syrup, or the like.

A pharmaceutically acceptable carrier can contain physiologically acceptable agents that act, for example, to stabilize, increase solubility or to increase the absorption of a compound such as a compound of the invention. Such physiologically acceptable agents include, for example, carbohydrates, such as glucose, sucrose or dextrans, antioxidants, such as ascorbic acid or glutathione, chelating agents, low molecular weight proteins or other stabilizers or excipients.

A pharmaceutical composition (preparation) may be administered to a patient orally (for example, drenches as in aqueous or non-aqueous solutions or suspensions, tablets, capsules (including sprinkle capsules and gelatin capsules), boluses, powders, granules, pastes). In certain embodiments, a compound may be simply dissolved or suspended in sterile water. Details of appropriate routes of administration and compositions suitable for same can be found in, for example, U.S. Pat. Nos. 6,110,973, 5,763,493, 5,731,000, 5,541,231, 5,427,798, 5,358,970 and 4,172,896, as well as in patents cited therein.

The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient that can be combined with a carrier material to produce a single dosage form will generally be that amount of the compound which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association an active compound, such as a compound of the invention, with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules (including sprinkle capsules and gelatin capsules), cachets, pills, tablets, lozenges (using a flavored basis, usually sucrose and acacia or tragacanth), lyophile, powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. Compositions or compounds may also be administered as a bolus, electuary or paste.

To prepare solid dosage forms for oral administration (capsules (including sprinkle capsules and gelatin capsules), tablets, pills, dragees, powders, granules and the like), the active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; (10) complexing agents, such as, modified and unmodified cyclodextrins; and (11) coloring agents. In the case of capsules (including sprinkle capsules and gelatin capsules), tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions, such as dragees, capsules (including sprinkle capsules and gelatin capsules), pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions that can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Liquid dosage forms useful for oral administration include pharmaceutically acceptable emulsions, lyophiles for reconstitution, microemulsions, solutions, suspensions, syrups, and elixirs. In addition to the active ingredient, the liquid dosage forms may contain inert diluents commonly used in the art, such as, for example, water or other solvents, cyclodextrins and derivatives thereof, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Examples of suitable aqueous and nonaqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents that delay absorption such as aluminum monostearate and gelatin.

For use in the methods of this invention, active compounds can be given per se or as a pharmaceutical composition containing, for example, 0.1 to 99.5% (more preferably, 0.5 to 90%) of active ingredient in combination with a pharmaceutically acceptable carrier.

Actual dosage levels of the active ingredients in the pharmaceutical compositions may be varied so as to obtain an amount of the active ingredient that is effective to achieve the desired therapeutic response for a particular patient, composition, and mode of administration, without being toxic to the patient.

The selected dosage level will depend upon a variety of factors including the activity of the particular compound or combination of compounds employed, or the ester, salt or amide thereof, the route of administration, the time of administration, the rate of excretion of the particular compound(s) being employed, the duration of the treatment, other drugs, compounds and/or materials used in combination with the particular compound(s) employed, the age, sex, weight, condition, general health and prior medical history of the patient being treated, and like factors well known in the medical arts.

A physician or veterinarian having ordinary skill in the art can readily determine and prescribe the therapeutically effective amount of the pharmaceutical composition required. For example, the physician or veterinarian could start doses of the pharmaceutical composition or compound at levels lower than that required in order to achieve the desired therapeutic effect and gradually increase the dosage until the desired effect is achieved.

In general, a suitable daily dose of an active compound used in the compositions and methods of the invention will be that amount of the compound that is the lowest dose effective to produce a therapeutic effect. Such an effective dose will generally depend upon the factors described above.

If desired, the effective daily dose of the active compound may be administered as one, two, three, four, five, six or more sub-doses administered separately at appropriate intervals throughout the day, optionally, in unit dosage forms. In certain embodiments of the present invention, the active compound may be administered two or three times daily. In preferred embodiments, the active compound will be administered once daily.

The patient receiving this treatment is any animal in need, including primates, in particular humans, and other mammals such as equines, cattle, swine and sheep; and poultry and pets in general.

In certain embodiments, compounds of the invention may be used alone or conjointly administered with another type of therapeutic agent.

This invention includes the use of pharmaceutically acceptable salts of compounds of the invention in the compositions and methods of the present invention. In certain embodiments, contemplated salts of the invention include, but are not limited to, alkyl, dialkyl, trialkyl or tetra-alkyl ammonium salts. In certain embodiments, contemplated salts of the invention include, but are not limited to, L-arginine, benenthamine, benzathine, betaine, calcium hydroxide, choline, deanol, diethanolamine, diethylamine, 2-(diethylamino)ethanol, ethanolamine, ethylenediamine, N-methylglucamine, hydrabamine, 1H-imidazole, lithium, L-lysine, magnesium, 4-(2-hydroxyethyl)morpholine, piperazine, potassium, 1-(2-hydroxyethyl)pyrrolidine, sodium, triethanolamine, tromethamine, and zinc salts. In certain embodiments, contemplated salts of the invention include, but are not limited to, Na, Ca, K, Mg, Zn or other metal salts.

The pharmaceutically acceptable acid addition salts can also exist as various solvates, such as with water, methanol, ethanol, dimethylformamide, and the like. Mixtures of such solvates can also be prepared. The source of such solvate can be from the solvent of crystallization, inherent in the solvent of preparation or crystallization, or adventitious to such solvent.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: (1) water-soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal-chelating agents, such as citric acid, ethylenediamine tetraacetic acid (EDTA), sorbitol, tartaric acid, phosphoric acid, and the like.

### V. METHODS

In some aspects, the invention relates to the claimed compounds for use in a method of treating cancer, a myeloproliferative disease, comprising orally administering a compound of formula I, formula II, formula III, wherein the compound is administered with a meal. The compound may be, for example, any one of the compounds listed in tables 1 or 2, or in Appendix A.

In some aspects, the invention relates to the claimed compounds for use in a method of treating cancer, a myeloproliferative disease, comprising orally administering a compound of formula I, formula II, formula III, wherein the compound is administered with food. The compound may be, for example, any one of the compounds listed in tables 1 or 2, or in Appendix A.

In some embodiments, the invention relates to the claimed compounds for use in a method of treating cancer, a myeloproliferative disease, comprising orally administering a compound of formula I, formula II, formula III, wherein the compound is administered to a subject in fed mode. The compound may be, for example, any one of the compounds listed in tables 1 or 2, or in Appendix A.

In certain embodiments, the subject is a mammal. In certain preferred embodiments, the subject is a human.

In some embodiments, the compound is administered orally between 30 minutes prior to the subject (e.g., a human) ingesting food to 6 hours after ingesting food, such as between 30 minutes prior to ingesting food to 5 hours after ingesting food, between 30 minutes prior to ingesting food to 4 hours after ingesting food, between 30 minutes prior to ingesting food to 3 hours after ingesting food, between 30 minutes prior to ingesting food to 2 hours after ingesting food, or between 30 minutes prior to ingesting food to 1 hours after ingesting food. Preferably, the compound is administered between 30 minutes prior to the subject ingesting food to 90 minutes after ingesting food, such as between 20 minutes prior to ingesting food to 90 minutes after ingesting food, between 20 minutes prior to ingesting food to 60 minutes after ingesting food, between 10 minutes prior to ingesting food to 60 minutes after ingesting food, between 5 minutes prior to ingesting food to 60 minutes after ingesting food, or between 5 minutes prior to ingesting food to 30 minutes after ingesting food.

In some embodiments, the method comprises orally administering a glutaminase inhibitor (e.g., preferably a compound of formula III) to a subject (e.g., a human), preferably in the fed mode, wherein between 100 mg and 10 g of the compound is administered orally per day. For example, the daily oral dose of the compound may be from 100 mg to 5000 mg, e.g., 200 mg to 4000 mg, 300 mg to 3000 mg, 600 mg to 2400 mg, 800 mg to 2200 mg, 1000 mg to 2000 mg, or 1200 mg to 1800 mg, or about 1600 mg.

In some embodiments, the method comprises orally administering the compound of formula III, and 100 mg to 10 g of the compound is administered orally per day. For example, 100 mg to 5000 mg of the compound may be administered orally per day, such as 200 mg to 4000 mg, 300 mg to 3000 mg, 600 mg to 2400 mg, 800 mg to 2200 mg, 1000 mg to 2000 mg, 1200 mg to 1800 mg, or about 1600 mg.

In some embodiments, an aggregate dose equivalent to between 100 mg and 10 g of the compound of formula III is administered orally per day. The term "aggregate dose" refers to the total amount of the compound administered, e.g., per day. For example, if a 600 mg dose of the compound is administered two times per day, then the aggregate dose is 1200 mg per day. The term "equivalent to an amount of the compound of formula III" refers to the administration of an amount of a compound that has the same efficacy as an amount of the compound of formula III. For example, if a first compound, such as a compound of formula I, or II has the same efficacy as the compound of formula III, then an equivalent of the first compound is equal to the same amount of the compound of formula III, *e.g.,* 600 mg of the first compound is equivalent to 600 mg of the compound of formula III. Similarly, if a second compound has, for example, twice the efficacy of the compound of formula III, then an equivalent of the second compound is equal to half the amount of the compound of formula III, *e.g.,* 300 mg of the second compound is equivalent to 600 mg of the compound of formula III.

Preferably, the glutaminase inhibitor is administered to the subject with a meal (i.e., the subject is in the fed mode).

In some embodiments, an aggregate dose equivalent to between about 100 mg and about 5000 mg of a glutaminase inhibitor (e.g., preferably a compound of formula III) is administered to a subject (e.g., a human) orally per day. In exemplary embodiments, an aggregate dose is equivalent to between about 200 mg and about 4000 mg, about 300 mg and about 3000 mg, about 400 mg and about 2800 mg, about 600 mg and about 2400 mg, about 800 mg and about 2200 mg, about 1000 mg and about 2000 mg, about 1000 mg and about 1800 mg, about 1200 mg and about 1800 mg, about 1200 mg and about 1600 mg. In certain preferred embodiments, a compound of formula III is delivered orally to a human subject twice daily for an aggregate dose of 1600 mg. Preferably, the human subject is in the fed mode. In certain preferred embodiments, the compound is administered with a meal.

In some embodiments, an aggregate dose equivalent to between about 100 mg and about 5000 mg of the compound of formula III is administered to a subject (e.g., a human) orally per day. In exemplary embodiments, an aggregate dose is equivalent to between about 200 mg and about 4000 mg, about 300 mg and about 3000 mg, about 400 mg and about 2800 mg, about 600 mg and about 2400 mg, about 800 mg and about 2200 mg, about 1000 mg and about 2000 mg, about 1000 mg and about 1800 mg, about 1200 mg and about 1800 mg, about 1200 mg and about 1600 mg. In certain preferred embodiments, a compound of formula III is delivered orally to a human subject twice daily for an aggregate dose of 1600 mg. Preferably, the human subject is in the fed mode, e.g., the compound is administered with a meal.

In some embodiments, between 100 mg and 10 g of the compound is administered daily. For example, 100 mg, 150 mg, 200 mg, 250 mg, 300 mg, 350 mg, mg, 400 mg, 450 mg, 500 mg, 550 mg, 600 mg, 650 mg, 700 mg, 750 mg, 800 mg, 850 mg, 900 mg, 950 mg, 1000 mg, 1100 mg, 1200 mg, 1300 mg, 1350 mg, 1400 mg, 1500 mg, 1600 mg, 1650 mg, 1700 mg, 1800 mg, 1900 mg, 1950 mg, 2000 mg, 2100 mg, 2200 mg, 2250 mg, 2300 mg, 2400 mg, 2500 mg, 2550 mg, 2600 mg, 2700 mg, 2800 mg, 2850 mg, 2900 mg, 3000 mg, 4000 mg, 5000 mg, 6000 mg, 7000 mg, 8000 mg, 9000 mg, or 10,000 mg may be administered daily. In certain preferred embodiments, 1200 mg of the compound is administered per day, *e.g.,* with two doses of 600 mg each. In some embodiments, 1800 mg of the compound is administered per day, *e.g.,* with three doses of 600 mg each. In other preferred embodiments, 1600 mg of the compound is administered to a subject (e.g., a human) per day, *e.g.,* with two doses of 800 mg each. Preferably, each administration includes a meal.

In some embodiments, the compound is administered once per day, two times per day, three times per day, or four times per day. In preferred embodiments, the compound is administered two times per day or three times per day, *e.g.,* each time with food. In more preferred embodiments, the compound is administered two times per day, *e.g.,* each time with food.

### Examples

### Example 1: Comparision of various dose sizes

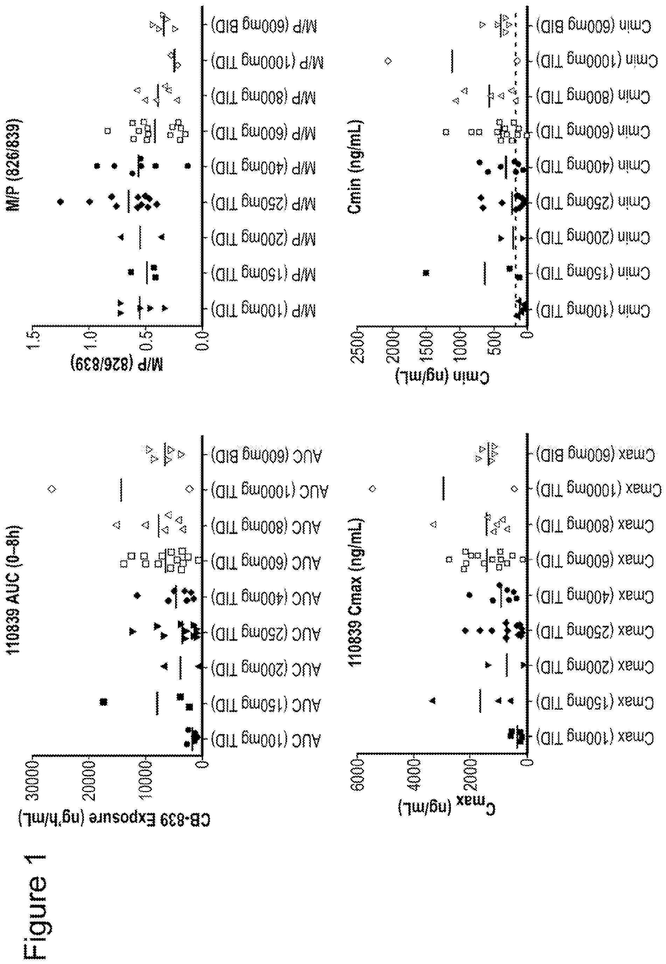

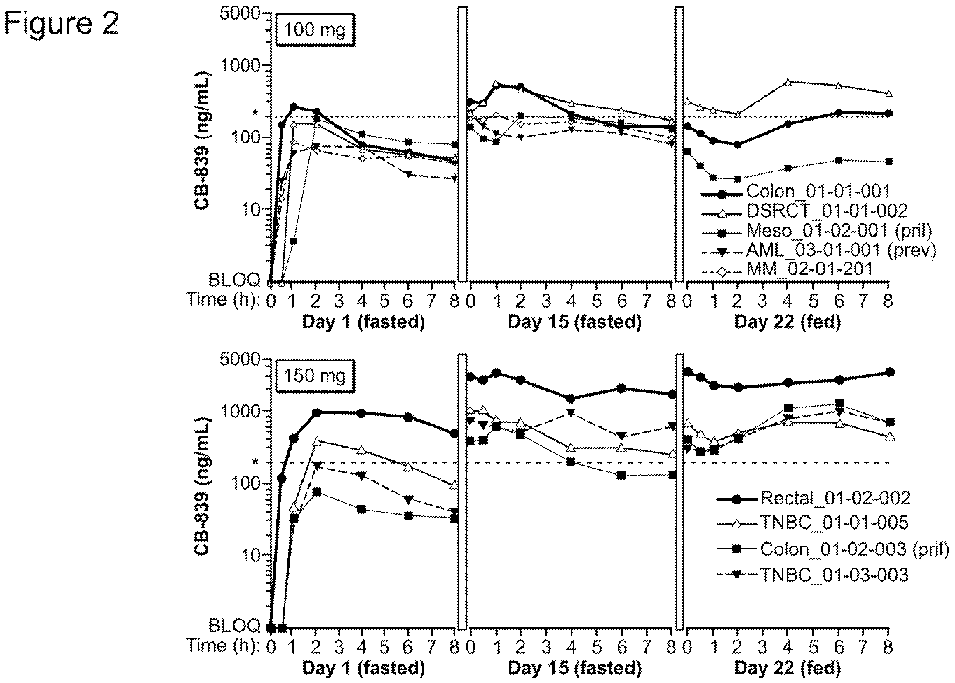

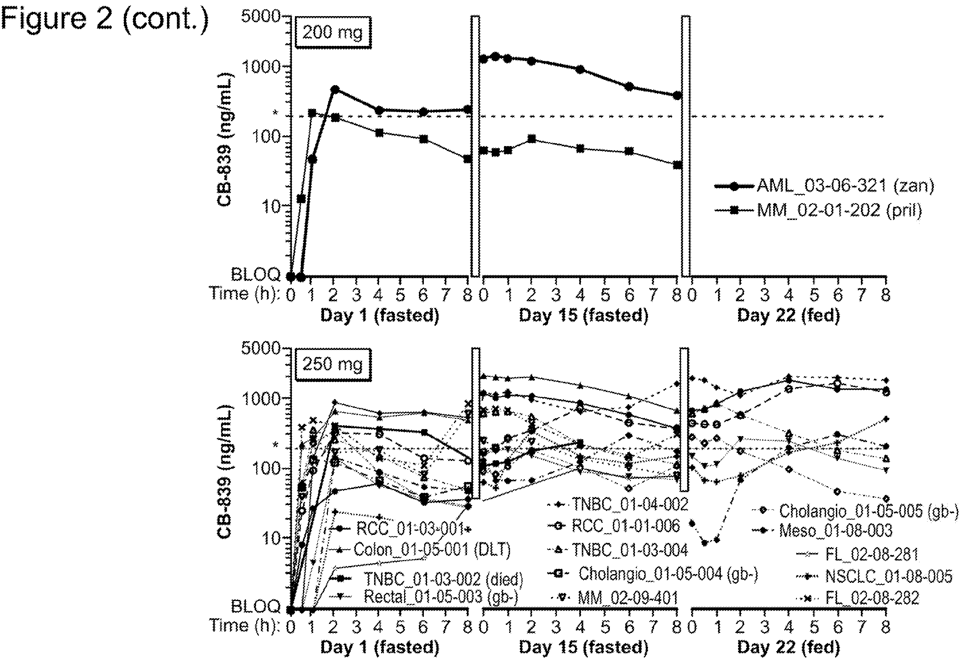

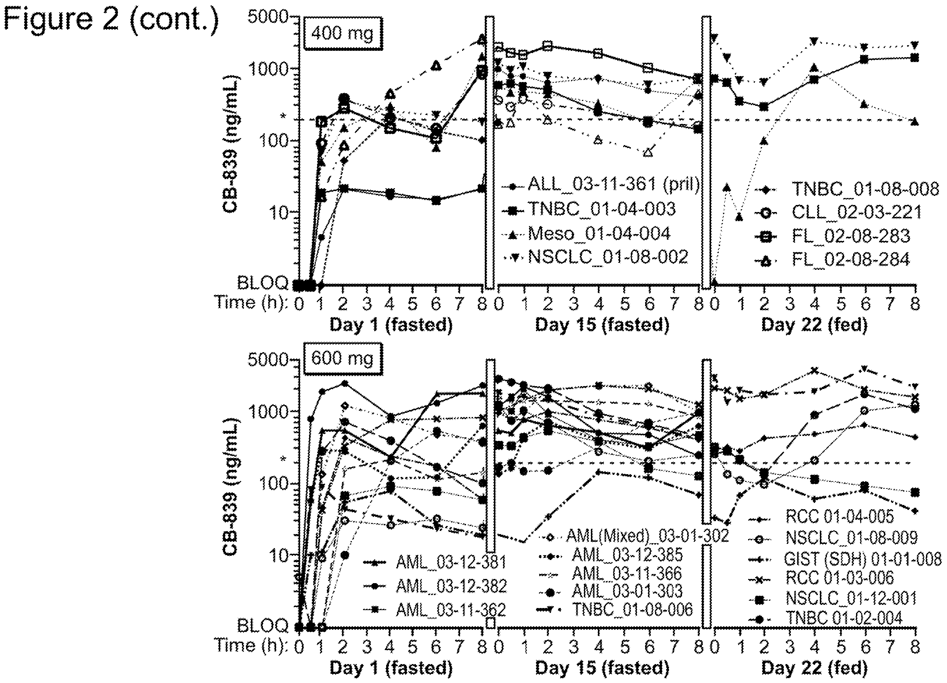

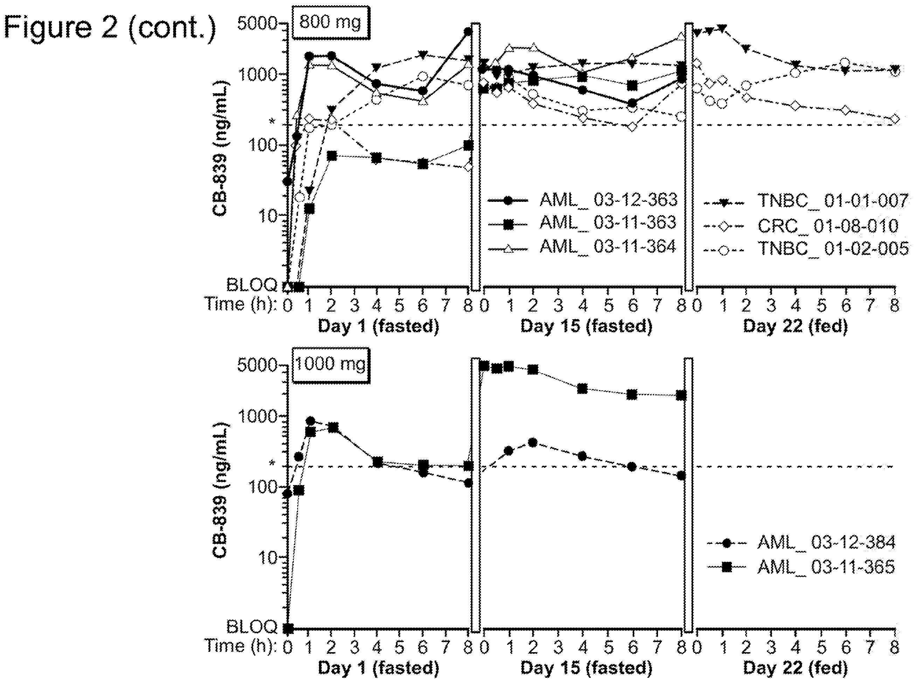

The compound of formula III (CB-839) was administered, orally, to fifteen human subjects with acute leukemia for 22 days. The compound was administered three times per day ("TID") at doses ranging from 100 mg per dose to 1000 mg per dose (*i.e.,* 300 mg to 3000 mg total compound per day). Plasma levels of the compound were monitored on days 1, 15, and 22. Subjects received the compound in a fasted state on days 1 and 15 (*e.g.,* without a meal as defined herein) and in a fed state on day 22 (*e.g.,* with a meal as defined herein). Administration of the compound in a fasted state consisted of oral administration of a first dose 1 hour before breakfast, oral administration of a second dose at 3 PM, and oral administration of a third dose prior to bedtime. An increase in exposure was demonstrated with increasing dose (Figures 1 & 2). The steady state plasma concentration of CB-839 on Day 15 was found to be above 250 nM, continuously, in most patients receiving doses of 600 mg three times per day and higher (Figure 2), which is a plasma concentration that has previously been shown to be therapeutically effective. Peripheral blood mononuclear cells (PBMCs) from three patients treated with doses of 600, 800, and 1000 mg three times per day were found to have between 10 and 58% leukemic blast counts and showed >94% inhibition of glutaminase activity. When CB-839 was administered at 600 mg twice a day with food, the Cmax was reached in 2-6 hours, and plasma levels of the compound exceeded 450 nM in all subjects, suggesting that the fed state resulted in increased drug exposure (Figure 2).

### Example 2: Comparision of administration in fed and fasted states

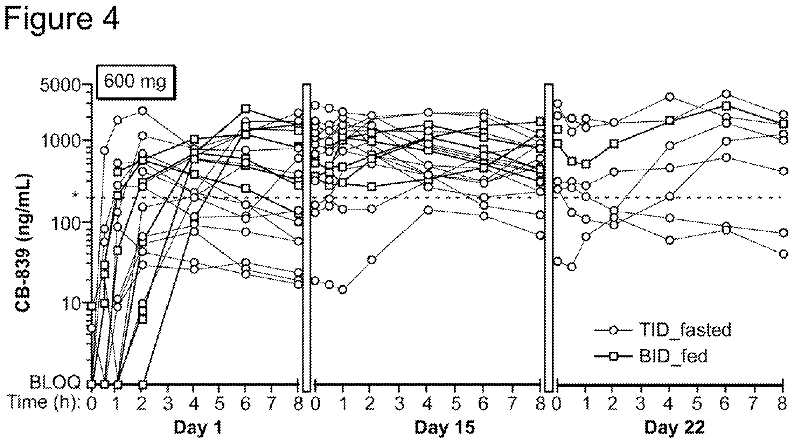

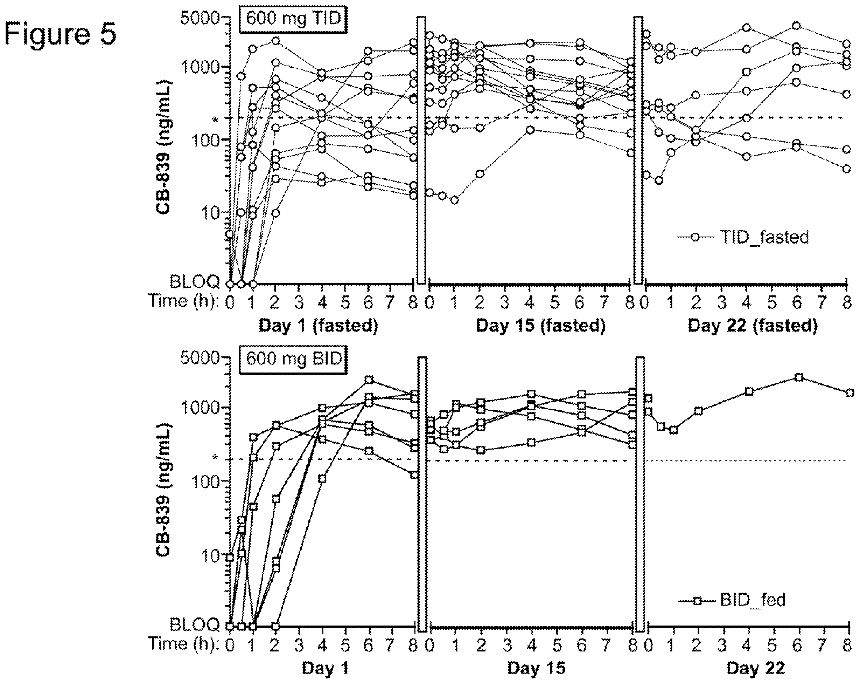

Each subject from Example 1 who remained enrolled in the trial were administered 600 mg of the compound of formula III orally, twice a day ("BID"), with food, each day after day 22 of the trial (*i.e.,* 1200 mg of the compound per day). Plasma levels of the compound were monitored on days 1, 15, and 22 of the BID dosing regimen for comparision with the results of Example 1. Pharmacokinetics data was compared for subjects receiving 600 mg of the compound three times per day in a fasted state (*i.e.,* 1800 mg of the compound per day, without meals as defined herein) and subjects receiving 600 mg of the compound two times per day in a fed state (*i.e.,* 1200 mg of the compound per day, with meals as defined herein). This data suggested that each group had the same amount of drug exposure despite the fed group receiving less compound per day than the fasted group (Figures 4 & 5).

### Example 3: Outcomes

No dose-limiting toxicity was identified in Examples 1 and 2, and treatment-related adverse events that occurred in greater than 10% of the subjects consisted of increased transaminase levels (in 4 subjects) and increased bilirubin levels (in 2 subjects). No grade 3 or higher adverse events were considered treatment-related in more than 10% of the subjects. Stable disease for 4-10 cycles was observed in 5 (33%) of 15 efficacy-evaluable subjects across all dose levels, with subjects remaining on the study drug for an average of 134 days (*i.e.,* greater than 6 cycles; 1 cycle = 21 days). One subject achieved a complete response in the bone marrow with incomplete recovery of peripheral counts after 6 cycles of dosing. All subjects with stable disease or better were older than 65 years of age and ineligible for high-dose therapy.

### Example 4: Pharmacokinetics

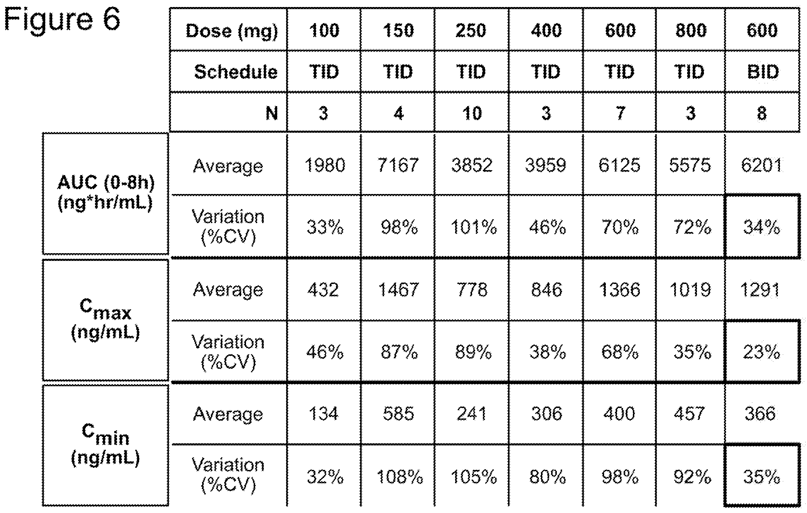

CB-839 was administered to cancer patients according to the dosing schedule in Figure 6. The half-life of CB-839 is approximately 4 hours. Exposure generally increases with dose.

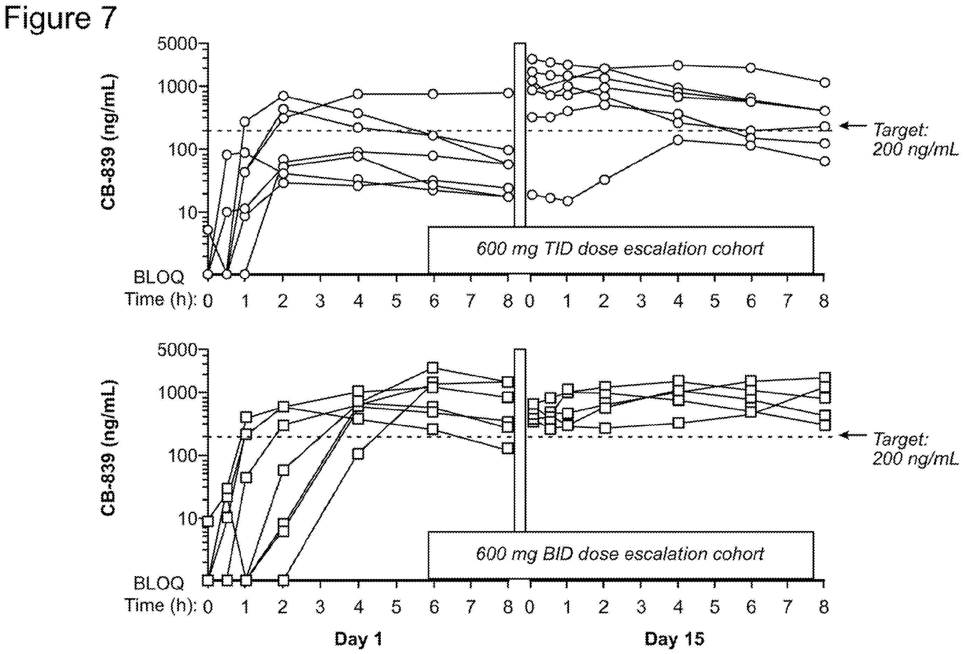

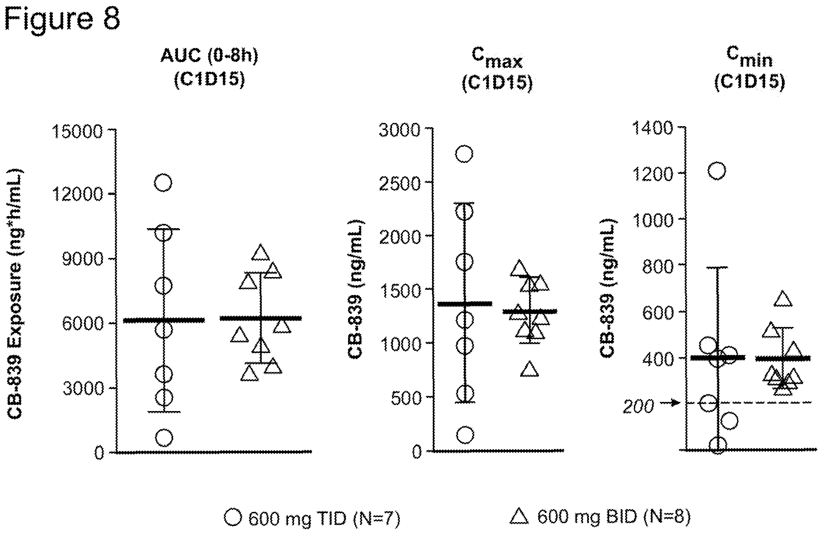

As shown in Figure 6-8, target CB-839 concentrations are maintained with PK variability is reduced with BID Fed dosing regimen.

## Claims

1. A compound of formula I, or a pharmaceutically acceptable salt thereof, for use in the treatment of cancer, 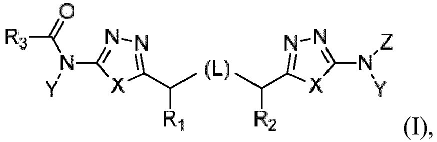 wherein:
L represents CH₂SCH₂, CH₂CH₂, CH₂CH₂CH₂, CH₂, CH₂S, SCH₂, CH₂NHCH₂, CH=CH, or 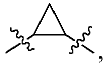 wherein any hydrogen atom of a CH or CH₂ unit may be replaced by alkyl or alkoxy, any hydrogen of an NH unit may be replaced by alkyl, and any hydrogen atom of a CH₂ unit of CH₂CH₂, CH₂CH₂CH₂ or CH₂ may be replaced by hydroxy;
X, independently for each occurrence, represents S, O or CH=CH, wherein any hydrogen atom of a CH unit may be replaced by alkyl;
Y, independently for each occurrence, represents H or CH₂O(CO)R_{7;}
R₇, independently for each occurrence, represents H or substituted or unsubstituted alkyl, alkoxy, aminoalkyl, alkylaminoalkyl, heterocyclylalkyl, or heterocyclylalkoxy;
Z represents H or R₃(CO);
R₁ and R₂ each independently represent H, alkyl, alkoxy or hydroxy;
R₃, independently for each occurrence, represents substituted or unsubstituted alkyl, hydroxyalkyl, aminoalkyl, acylaminoalkyl, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, heteroaryloxyalkyl or C(R₈)(R₉)(R₁₀), N(R₄)(R₅) or OR₆, wherein any free hydroxyl group may be acylated to form C(O)R₇;
R₄ and R₅ each independently represent H or substituted or unsubstituted alkyl, hydroxyalkyl, acyl, aminoalkyl, acylaminoalkyl, alkenyl, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, wherein any free hydroxyl group may be acylated to form C(O)R₇;
R₆, independently for each occurrence, represents substituted or unsubstituted alkyl, hydroxyalkyl, aminoalkyl, acylaminoalkyl, alkenyl, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, wherein any free hydroxyl group may be acylated to form C(O)R₇;
R₈, R₉ and R₁₀ each independently represent H or substituted or unsubstituted alkyl, hydroxy, hydroxyalkyl, amino, acylamino, aminoalkyl, acylaminoalkyl, alkoxycarbonyl, alkoxycarbonylamino, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, or R₈ and R₉ together with the carbon to which they are attached, form a carbocyclic or heterocyclic ring system, wherein any free hydroxyl group may be acylated to form C(O)R₇; and
wherein the compound is to be administered orally with a meal.

2. A compound of formula II, or a pharmaceutically acceptable salt thereof, for use in the treatment of cancer, 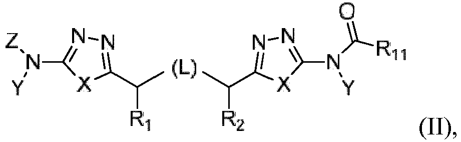 wherein:
L represents CH₂SCH₂, CH₂CH₂, CH₂CH₂CH₂, CH₂, CH₂S, SCH₂, CH₂NHCH₂, CH=CH, or 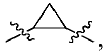 wherein any hydrogen atom of a CH or CH₂ unit may be replaced by alkyl or alkoxy, any hydrogen of an NH unit may be replaced by alkyl, and any hydrogen atom of a CH₂ unit of CH₂CH₂, CH₂CH₂CH₂ or CH₂ may be replaced by hydroxy;
X, independently for each occurrence, represents S, O or CH=CH, wherein any hydrogen atom of a CH unit may be replaced by alkyl;
Y, independently for each occurrence, represents H or CH₂O(CO)R₇;
R₇, independently for each occurrence, represents H or substituted or unsubstituted alkyl, alkoxy, aminoalkyl, alkylaminoalkyl, heterocyclylalkyl, arylalkyl, or heterocyclylalkoxy;
Z represents H or R₃(CO);
R₁ and R₂ each independently represent H, alkyl, alkoxy or hydroxy;
R₃ represents substituted or unsubstituted alkyl, hydroxyalkyl, aminoalkyl, acylaminoalkyl, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, heteroaryloxyalkyl or C(R₈)(R₉)(R₁₀), N(R₄)(R₅) or OR₆, wherein any free hydroxyl group may be acylated to form C(O)R₇;
R₄ and R₅ each independently for each occurrence represent H or substituted or unsubstituted alkyl, hydroxyalkyl, acyl, aminoalkyl, acylaminoalkyl, alkenyl, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, wherein any free hydroxyl group may be acylated to form C(O)R₇;
R₆ represents substituted or unsubstituted alkyl, hydroxyalkyl, aminoalkyl, acylaminoalkyl, alkenyl, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, wherein any free hydroxyl group may be acylated to form C(O)R₇;
R₈, R₉ and R₁₀ each independently for each occurrence represent H or substituted or unsubstituted alkyl, hydroxy, hydroxyalkyl, amino, acylamino, aminoalkyl, acylaminoalkyl, alkoxycarbonyl, alkoxycarbonylamino, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, or R₈ and R₉ together with the carbon to which they are attached, form a carbocyclic or heterocyclic ring system, wherein any free hydroxyl group may be acylated to form C(O)R₇, and wherein at least two of R₈, R₉ and R₁₀ are not H;
R₁₁ represents aryl, arylalkyl, aryloxy, aryloxyalkyl, heteroaryl, heteroarylalkyl, heteroaryloxy, or heteroaryloxylalkyl or R₁₁ represents C(R₁₂)(R₁₃)(R₁₄), N(R₄)(R₁₄) or OR₁₄, wherein any free hydroxyl group may be acylated to form C(O)R₇;
R₁₂ and R₁₃ each independently respresent H or substituted or unsubstituted alkyl, hydroxy, hydroxyalkyl, amino, acylamino, aminoalkyl, acylaminoalkyl, alkoxycarbonyl, alkoxycarbonylamino, alkenyl, alkoxy, alkoxyalkyl, aryl, arylalkyl, aryloxy, aryloxyalkyl, cycloalkyl, cycloalkylalkyl, heterocyclyl, heterocyclylalkyl, heteroaryl, heteroarylalkyl, heteroaryloxy, or heteroaryloxyalkyl, wherein any free hydroxyl group may be acylated to form C(O)R₇, and wherein both of R₁₂ and R₁₃ are not H;
R₁₄ represents aryl, arylalkyl, aryloxy, aryloxyalkyl, heteroaryl, heteroarylalkyl, heteroaryloxy, or heteroaryloxyalkyl; and
wherein the compound is to be administered orally with a meal.

3. A compound for use according to claim 2, wherein
(a) R₁₁ represents aryl, arylalkyl, aryloxy, aryloxyalkyl, heteroaryl, heteroarylalkyl, heteroaryloxy, or heteroaryloxyalkyl, and the aryl or heteroaryl ring is substituted with either -OCHF₂ or -OCF₃ and is optionally further substituted; or
(b) R₁₁ represents arylalkyl, wherein the aryl ring is substituted with -OCF₃; or
(c) R₁₁ represents trifluoromethoxybenzyl; or
(d) R₁₁ represents

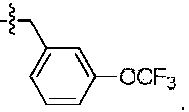

4. A compound for use according to claim 3, wherein R₁₄ represents aryl, arylalkyl, aryloxy, aryloxyalkyl, heteroaryl, heteroarylalkyl, heteroaryloxy, or heteroaryloxyalkyl, and the aryl or heteroaryl ring is substituted with either -OCHF₂ or -OCF₃ and is optionally further substituted.

5. A compound for use according to any one of claims 2-4, wherein
(a) L represents CH₂SCH₂, CH₂CH₂, CH₂S or SCH₂; preferably L represents CH₂CH₂; and/or
(b) each Y represents H; and/or
(c) X represents S or CH=CH, wherein any hydrogen atom of a CH unit may be replaced by alkyl; and/or
(d) Z represents R₃(CO); and/or
(e) R₁ and R₂ each represent H.

6. A compound for use according to claim 5, wherein Z represents R₃(CO) and R₃ and R₁₁ are not identical.

7. A compound for use according to any one of claims 2-6, wherein:
(a) Z represents R₃(CO) and R₃ represents substituted or unsubstituted arylalkyl, heteroarylalkyl, cycloalkyl or heterocycloalkyl; or
(b) Z represents R₃(CO) and R₃ represents substituted or unsubstituted heteroarylalkyl; or
(c) Z represents R₃(CO) and R₃ represents substituted or unsubstituted pyridylalkyl.

8. A compound for use according to any one of claims 3 and 4, wherein L represents CH₂CH₂, each Y represents H, X represents S, Z represents R₃(CO), R₁ and R₂ each represent H, and R₃ represents arylalkyl, heteroarylalkyl, cycloalkyl or heterocycloalkyl, preferably R₃ represents heteroarylalkyl.

9. A compound for use according to any one of claims 1 to 8, wherein the cancer is selected from breast cancer, colorectal cancer, endocrine cancer, lung cancer, melanoma, mesothelioma, renal cancer, and a B cell malignancy.

10. A compound for use according to claim 9, wherein the cancer is breast cancer, suitably wherein the breast cancer comprises basal-type breast cancer cells, triple-negative breast cancer cells or claudin-low breast cancer cells.

11. A compound for use according to claim 9, wherein the cancer is colorectal cancer.

12. A compound for use according to claim 9, wherein the cancer is endocrine cancer, suitably wherein the endocrine cancer is selected from adrenal cortex adenoma, adrenal cortex carcicnoma, adrenal gland pheochromocytoma, and parathyroid gland adenoma.

13. A compound for use according to claim 9, wherein the cancer is melanoma.

14. A compound for use according to claim 9, wherein the cancer is renal cancer.

15. A compound for use according to claim 9, wherein the cancer is a B cell malignancy, suitably wherein:
(a) the B cell malignancy is multiple myeloma, or
(b) the B cell malignancy is leukemia, preferably selected from acute lymphoblastic leukemia, and chronic lymphoblastic leukemia; or
(c) the B cell malignancy is lymphoma, preferably selected from Burkitt's lymphoma, Diffuse large B cell lymphoma, follicular lymphoma, and Hodgkin's lymphoma.

16. A compound for use according to any one of the preceding claims, wherein the compound is the compound of formula III, 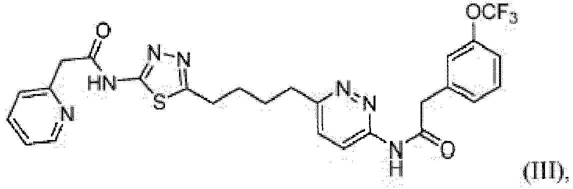 and 300 mg to 3000 mg of the compound is administered per day.

17. A compound for use according to any one of the preceding claims, wherein the compound is for administration two or three times per day.

## Patentansprüche

1. Verbindung der Formel I oder ein pharmazeutisch unbedenkliches Salz davon zur Verwendung bei der Behandlung von Krebs, 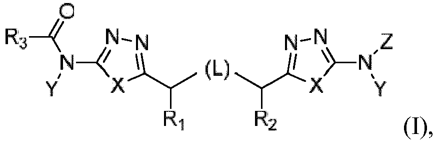 wobei:
L CH₂SCH₂, CH₂CH₂, CH₂CH₂CH₂, CH₂, CH₂S, SCH₂, CH₂NHCH₂, CH=CH oder 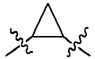 darstellt, wobei jedes Wasserstoffatom einer CH- oder CH₂-Einheit durch Alkyl oder Alkoxy ersetzt werden kann, jeder Wasserstoff einer NH-Einheit durch Alkyl ersetzt werden kann und jedes Wasserstoffatom einer CH₂-Einheit von CH₂CH₂, CH₂CH₂CH₂ oder CH₂ durch Hydroxy ersetzt werden kann;
X unabhängig für jedes Vorkommen S, O oder CH=CH darstellt, wobei jedes Wasserstoffatom einer CH-Einheit durch Alkyl ersetzt werden kann;
Y unabhängig für jedes Auftreten H oder CH₂O(CO)R₇ darstellt;
R₇ unabhängig für jedes Vorkommen H oder substituiertes oder unsubstituiertes Alkyl, Alkoxy, Aminoalkyl, Alkylaminoalkyl, Heterocyclylalkyl oder Heterocyclylalkoxy darstellt;
Z H oder R₃(CO) darstellt;
R₁ und R₂ jeweils unabhängig voneinander H, Alkyl, Alkoxy oder Hydroxy darstellen;
R₃ unabhängig für jedes Vorkommen substituiertes oder unsubstituiertes Alkyl, Hydroxyalkyl, Aminoalkyl, Acylaminoalkyl, Alkenyl, Alkoxy, Alkoxyalkyl, Aryl, Arylalkyl, Aryloxy, Aryloxyalkyl, Cycloalkyl, Cycloalkylalkyl, Heterocyclyl, Heterocyclylalkyl, Heteroaryl, Heteroarylalkyl, Heteroaryloxy, Heteroaryloxyalkyl oder C(R₈)(R₉)(R₁₀), N(R₄)(R₅) oder OR₆ darstellt, wobei jede freie Hydroxylgruppe akyliert werden kann, um C(O)R₇ auszubilden;
R₄ und R₅ jeweils unabhängig H oder substituiertes oder unsubstituiertes Alkyl, Hydroxyalkyl, Acyl, Aminoalkyl, Acylaminoalkyl, Alkenyl, Alkoxyalkyl, Aryl, Arylalkyl, Aryloxy, Aryloxyalkyl, Cycloalkyl, Cycloalkylalkyl, Heterocyclyl, Heterocyclylalkyl, Heteroaryl, Heteroarylalkyl, Heteroaryloxy oder Heteroaryloxyalkyl darstellen, wobei jede freie Hydroxylgruppe acyliert werden kann, um C(O)R₇ auszubilden;
R₆ unabhängig für jedes Vorkommen substituiertes oder unsubstituiertes Alkyl, Hydroxyalkyl, Aminoalkyl, Acylaminoalkyl, Alkenyl, Alkoxyalkyl, Aryl, Arylalkyl, Aryloxy, Aryloxyalkyl, Cycloalkyl, Cycloalkylalkyl, Heterocyclyl, Heterocyclylalkyl, Heteroaryl, Heteroarylalkyl, Heteroaryloxy oder Heteroaryloxyalkyl darstellt, wobei jede freie Hydroxylgruppe acyliert werden kann, um C(O)R₇ auszubilden;
R₈, R₉ und R₁₀ jeweils unabhängig H oder substituiertes oder unsubstituiertes Alkyl, Hydroxy, Hydroxyalkyl, Amino, Acylamino, Aminoalkyl, Acylaminoalkyl, Alkoxycarbonyl, Alkoxycarbonylamino, Alkenyl, Alkoxy, Alkoxyalkyl, Aryl, Arylalkyl, Aryloxyl, Aryloxyalkyl, Cycloalkyl, Cycloalkylalkyl, Heterocyclyl, Heterocyclylalkyl, Heteroaryl, Heteroarylalkyl, Heteroaryloxy oder Heteroaryloxyalkyl darstellen, oder R₈ und R₉ zusammen mit dem Kohlenstoff, an den sie gebunden sind, ein carbocyclisches oder heterocyclisches Ringsystem ausbilden, wobei jede freie Hydroxylgruppe akyliert werden kann, um C(O)R₇ auszubilden; und
wobei die Verbindung oral mit einer Mahlzeit verabreicht werden soll.

2. Verbindung der Formel II oder ein pharmazeutisch unbedenkliches Salz davon zur Verwendung bei der Behandlung von Krebs, 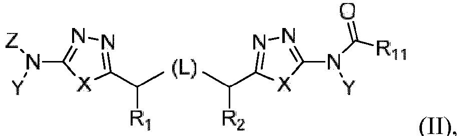 wobei:
L CH₂SCH₂, CH₂CH₂, CH₂CH₂CH₂, CH₂, CH₂S, SCH₂, CH₂NHCH₂, CH=CH oder 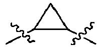 darstellt, wobei jedes Wasserstoffatom einer CH- oder CH₂-Einheit durch Alkyl oder Alkoxy ersetzt werden kann, jeder Wasserstoff einer NH-Einheit durch Alkyl ersetzt werden kann und jedes Wasserstoffatom einer CH₂-Einheit von CH₂CH₂, CH₂CH₂CH₂ oder CH₂ durch Hydroxy ersetzt werden kann;
X unabhängig für jedes Vorkommen S, O oder CH=CH darstellt, wobei jedes Wasserstoffatom einer CH-Einheit durch Alkyl ersetzt werden kann;
Y unabhängig für jedes Vorkommen H oder CH₂O(CO)R₇ darstellt;
R₇ unabhängig für jedes Vorkommen H oder substituiertes oder unsubstituiertes Alkyl, Alkoxy, Aminoalkyl, Alkylaminoalkyl, Heterocyclylalkyl, Arylalkyl oder Heterocyclylalkoxy darstellt;
Z H oder R₃(CO) darstellt;
R₁ und R₂ jeweils unabhängig voneinander H, Alkyl, Alkoxy oder Hydroxy darstellen;
R₃ substituiertes oder unsubstituiertes Alkyl, Hydroxyalkyl, Aminoalkyl, Acylaminoalkyl, Alkenyl, Alkoxy, Alkoxyalkyl, Aryl, Arylalkyl, Aryloxy, Aryloxyalkyl, Cycloalkyl, Cycloalkylalkyl, Heterocyclyl, Heterocyclylalkyl, Heteroaryl, Heteroarylalkyl, Heteroaryloxy, Heteroaryloxyalkyl oder C(R₈)(R₉)(R₁₀), N(R₄)(R₅) oder OR₆ darstellt, wobei jede freie Hydroxylgruppe akyliert werden kann, um C(O)R₇ auszubilden;
R₄ und R₅ jeweils unabhängig für jedes Vorkommen H oder substituiertes oder unsubstituiertes Alkyl, Hydroxyalkyl, Acyl, Aminoalkyl, Acylaminoalkyl, Alkenyl, Alkoxyalkyl, Aryl, Arylalkyl, Aryloxy, Aryloxyalkyl, Cycloalkyl, Cycloalkylalkyl, Heterocyclyl, Heterocyclylaryl, Heteroaryl, Heteroarylalkyl, Heteroaryloxy oder Heteroaryloxyalkyl darstellen, wobei jede freie Hydroxylgruppe akyliert werden kann, um C(O)R₇ auszubilden;
R₆ substituiertes oder unsubstituiertes Alkyl, Hydroxyalkyl, Aminoalkyl, Acylaminoalkyl, Alkenyl, Alkoxyalkyl, Aryl, Arylalkyl, Aryloxy, Aryloxyalkyl, Cycloalkyl, Cycloalkylalkyl, Heterocyclyl, Heterocyclylaryl, Heteroaryl, Heteroarylalkyl, Heteroaryloxy oder Heteroaryloxyalkyl darstellt, wobei jede freie Hydroxylgruppe akyliert werden kann, um C(O)R₇ auszubilden;
R₈, R₉ und R₁₀ jeweils unabhängig für jedes Vorkommen H oder substituiertes oder unsubstituiertes Alkyl, Hydroxy, Hydroxyalkyl, Amino, Acylamino, Aminoalkyl, Acylaminoalkyl, Alkoxycarbonyl, Alkoxycarbonylamino, Alkenyl, Alkoxy, Alkoxyalkyl, Aryl, Arylalkyl, Aryloxyl, Aryloxyalkyl, Cycloalkyl, Cycloalkylalkyl, Heterocyclyl, Heterocyclylalkyl, Heteroaryl, Heteroarylalkyl, Heteroaryloxy oder Heteroaryloxyalkyl darstellen oder R₈ und R₉ zusammen mit dem Kohlenstoff, an den sie gebunden sind, ein carbocyclisches oder heterocyclisches Ringsystem ausbilden, wobei jede freie Hydroxylgruppe akyliert werden kann, um C(O)R₇ auszubilden, und wobei wenigstens zwei von R₈, R₉ und R₁₀ nicht H sind;
R₁₁ Aryl, Arylalkyl, Aryloxy, Aryloxyalkyl, Heteroaryl, Heteroarylalkyl, Heteroaryloxy oder Heteroaryloxylalkyl darstellt oder
R₁₁ C(R₁₂)(R₁₃)(R₁₄), N(R₄)(R₁₄) oder OR₁₄ darstellt, wobei jede freie Hydroxylgruppe akyliert werden kann, um C(O)R₇ auszubilden;
R₁₂ und R₁₃ jeweils unabhängig voneinander H oder substituiertes oder unsubstituiertes Alkyl, Hydroxy, Hydroxyalkyl, Amino, Acylamino, Aminoalkyl, Acylaminoalkyl, Alkoxycarbonyl, Alkoxycarbonylamino, Alkenyl, Alkoxy, Alkoxyalkyl, Aryl, Arylalkyl, Aryloxy, Aryloxyalkyl, Cycloalkyl, Cycloalkylalkyl, Heterocyclyl, Heterocyclylalkyl, Heteroaryl, Heteroarylalkyl, Heteroaryloxy oder Heteroaryloxyalkyl darstellen, wobei jede freie Hydroxylgruppe akyliert werden kann, um C(O)R₇ auszubilden, und wobei sowohl R₁₂ als auch R₁₃ nicht H sind;
R₁₄ Aryl, Arylalkyl, Aryloxy, Aryloxyalkyl, Heteroaryl, Heteroarylalkyl, Heteroaryloxy oder Heteroaryloxyalkyl darstellt; und
wobei die Verbindung oral mit einer Mahlzeit verabreicht werden soll.

3. Verbindung nach Anspruch 2, wobei
(a) R₁₁ Aryl, Arylalkyl, Aryloxy, Aryloxyalkyl, Heteroaryl, Heteroarylalkyl, Heteroaryloxy oder Heteroaryloxyalkyl darstellt, und der Aryl- oder Heteroarylring entweder mit -OCHF₂ oder -OCF₃ substituiert ist und optional weiter substituiert ist; oder
(b) R₁₁ Arylalkyl darstellt, wobei der Arylring mit -OCF₃ substituiert ist; oder
(c) R₁₁ Trifluormethoxybenzyl darstellt; oder
(d) 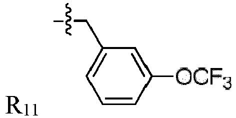 darstellt.

4. Verbindung zur Verwendung nach Anspruch 3, wobei R₁₄ Aryl, Arylalkyl, Aryloxy, Aryloxyalkyl, Heteroaryl, Heteroarylalkyl, Heteroaryloxy oder Heteroaryloxyalkyl darstellt, und der Aryl- oder Heteroarylring entweder mit -OCHF₂ oder -OCF₃ substituiert ist und optional weiter substituiert ist.

5. Verbindung zur Verwendung nach einem der Ansprüche 2-4, wobei
(a) L CH₂SCH₂, CH₂CH₂, CH₂S oder SCH₂ darstellt; vorzugsweise L CH₂CH₂ darstellt; und/oder
(b) jedes Y H darstellt; und/oder
(c) X S oder CH=CH darstellt, wobei jedes Wasserstoffatom einer CH-Einheit durch Alkyl ersetzt werden kann; und/oder
(d) Z R₃(CO) darstellt; und/oder
(e) R₁ und R₂ jeweils H darstellen.

6. Verbindung zur Verwendung nach Anspruch 5, wobei Z R₃(CO) darstellt und R₃ und R₁₁ nicht identisch sind.

7. Verbindung zur Verwendung nach einem der Ansprüche 2-6, wobei:
(a) Z R₃(CO) darstellt und R₃ substituiertes oder unsubstituiertes Arylalkyl, Heteroarylalkyl, Cycloalkyl oder Heterocycloalkyl darstellt; oder
(b) Z R₃(CO) darstellt und R₃ substituiertes oder unsubstituiertes Heteroarylalkyl darstellt; oder
(c) Z R₃(CO) darstellt und R₃ substituiertes oder unsubstituiertes Pyridylalkyl darstellt.

8. Verbindung zur Verwendung nach einem der Ansprüche 3 und 4, wobei L CH₂CH₂ darstellt, jedes Y H darstellt, X S darstellt, Z R₃(CO) darstellt, R₁ und R₂ jeweils H darstellen und R₃ Arylalkyl, Heteroarylalkyl, Cycloalkyl oder Heterocycloalkyl darstellt, vorzugsweise R₃ Heteroarylalkyl darstellt.

9. Verbindung zur Verwendung nach einem der Ansprüche 1 bis 8, wobei der Krebs aus Brustkrebs, Darmkrebs, endokrinem Krebs, Lungenkrebs, Melanom, Mesotheliom, Nierenkrebs und einer B-Zell-Malignität ausgewählt ist.

10. Verbindung zur Verwendung nach Anspruch 9, wobei der Krebs Brustkrebs ist, geeigneterweise wobei der Brustkrebs Brustkrebszellen des Basaltyps, dreifach negative Brustkrebszellen oder Claudin-niedrige Brustkrebszellen umfasst.

11. Verbindung zur Verwendung nach Anspruch 9, wobei der Krebs Darmkrebs ist.

12. Verbindung zur Verwendung nach Anspruch 9, wobei der Krebs endokriner Krebs ist, geeigneterweise wobei der endokrine Krebs aus Nebennierenrindenadenom, Nebennierenrindenkarziknom, Nebennieren-Phäochromozytom und Nebenschilddrüsenadenom ausgewählt ist.

13. Verbindung zur Verwendung nach Anspruch 9, wobei der Krebs ein Melanom ist.

14. Verbindung zur Verwendung nach Anspruch 9, wobei der Krebs Nierenkrebs ist.

15. Verbindung zur Verwendung nach Anspruch 9, wobei der Krebs eine B-Zell-Malignität ist, geeigneterweise wobei:
(a) die B-Zell-Malignität multiples Myelom ist oder
(b) die B-Zell-Malignität Leukämie ist, vorzugsweise ausgewählt aus akuter lymphoblastischer Leukämie und chronischer lymphoblastischer Leukämie, oder
(c) die B-Zell-Malignität ein Lymphom ist, vorzugsweise ausgewählt aus Burkitt-Lymphom, diffusem großzelligem B-Zell-Lymphom, follikulärem Lymphom und Hodgkin-Lymphom.

16. Verbindung zur Verwendung nach einem der vorhergehenden Ansprüche, wobei die Verbindung die Verbindung der Formel III ist, 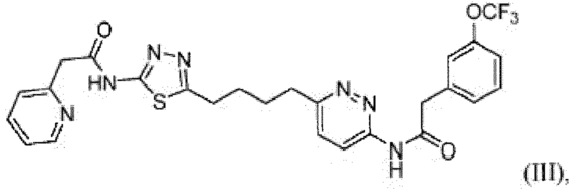 und 300 mg bis 3000 mg der Verbindung pro Tag verabreicht werden.

17. Verbindung zur Verwendung nach einem der vorhergehenden Ansprüche, wobei die Verbindung für die zwei- oder dreimal tägliche Verabreichung dient.

## Revendications

1. Composé de la formule I, ou sel pharmaceutiquement acceptable de celui-ci, pour utilisation dans le traitement du cancer, 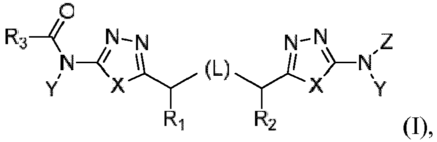 dans lequel :
L représente CH₂SCH₂, CH₂CH₂, CH₂CH₂CH₂, CH₂, CH₂S, SCH₂, CH₂NHCH₂, CH=CH, ou 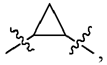 dans lequel tout atome d'hydrogène d'une unité CH ou CH₂ peut être remplacé par alkyle ou alkoxy, tout hydrogène d'une unité NH peut être remplacé par alkyle, et tout atome d'hydrogène d'une unité CH₂ de CH₂CH₂, CH₂CH₂CH₂ ou CH₂ peut être remplacé par hydroxy ;
X, indépendamment pour chaque occurrence, représente S, O ou CH=CH, tout atome d'hydrogène d'une unité CH pouvant être remplacé par alkyle ;
Y, indépendamment pour chaque occurrence, représente H ou CH₂O(CO)R₇ ;
R₇, indépendamment pour chaque occurrence, représente H ou alkyle, alkoxy, aminoalkyle, alkylaminoalkyle, hetérocyclylalkyle, ou hétérocyclylalkoxy substitué ou non substitué ;
Z représente H ou R₃(CO) ;
R₁ et R₂ représentent chacun indépendamment H, alkyle, alkoxy ou hydroxy ;
R₃, indépendamment pour chaque occurrence, représente alkyle, hydroxyalkyle, aminoalkyle, acylaminoalkyle, alkényle, alkoxy, alkoxyalkyle, aryle, arylalkyle, aryloxy, aryloxyalkyle, cycloalkyle, cycloalkylalkyle, hétérocyclyle, hétérocyclylalkyle, hétéroaryle, hétéroarylalkyle, hétéroaryloxy, hétéroaryloxyalkyle substitué ou non substitué ou C(R₈)(R₉)(R₁₀), N(R₄)(R₅) ou OR₆, où tout groupe hydroxyle libre peut être acylé pour former C(O)R₇;
R₄ et R₅ représentent chacun indépendamment H ou alkyle, hydroxyalkyle, acyle, aminoalkyle, acylaminoalkyle, alkényle, alkoxyalkyle, aryle, arylalkyle, aryloxy, aryloxyalkyle, cycloalkyle, cycloalkylalkyle, hétérocyclyle, hétérocyclylalkyle, hétéroaryle, hétéroarylalkyle, hétéroaryloxy, ou hétéroaryloxyalkyle substitué ou non substitué, où tout groupe hydroxyle libre peut être acylé pour former C(O)R₇ ;
R₆ indépendamment pour chaque occurrence représente alkyle, hydroxyalkyle, aminoalkyle, acylaminoalkyle, alkényle, alkoxyalkyle, aryle, arylalkyle, aryloxy, aryloxyalkyle, cycloalkyle, cycloalkylalkyle, hétérocyclyle, hétérocyclylalkyle, hétéroaryle, hétéroarylalkyle, hétéroaryloxy, ou hétéroaryloxyalkyle substitué ou non substitué, où tout groupe hydroxyle libre peut être acylé pour former C(O)R₇ ;
R₈, R₉ et R₁₀ représentent chacun indépendamment H ou alkyle, hydroxy, hydroxyalkyle, amino, acylamino, aminoalkyle, acylaminoalkyle, alkoxycarbonyle, alkoxycarbonylamino, alkényle, alkoxy, alkoxyalkyle, aryle, arylalkyle, aryloxy, aryloxyalkyle, cycloalkyle, cycloalkylalkyle, hétérocyclyle, hétérocyclylalkyle, hétéroaryle, hétéroarylalkyle, héteroaryloxy, ou hétéroaryloxyalkyle substitué ou non substitué, ou R₈ et R₉ conjointement avec le carbone auquel ils sont fixés, forment un système cyclique carbocyclique ou hétérocyclique, où tout groupe hydroxyle libre peut être acylé pour former C(O)R₇ ; et
dans lequel le composé est destiné à être administré par voie orale avec un repas.

2. Composé de la formule II, ou sel pharmaceutiquement acceptable de celui-ci, pour utilisation dans le traitement du cancer, 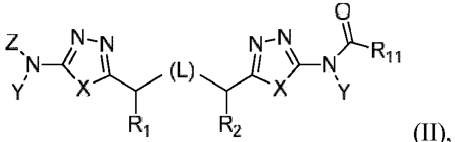 dans lequel:
L représente CH₂SCH₂, CH₂CH₂, CH₂CH₂CH₂, CH₂, CH₂S, SCH₂, CH₂NHCH₂, CH=CH, ou 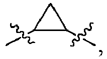 dans lequel tout atome d'hydrogène d'une unité CH ou CH₂ peut être remplacé par alkyle ou alkoxy, tout hydrogène d'une unité NH peut être remplacé par alkyle, et tout atome d'hydrogène d'une unité CH₂ de CH₂CH₂, CH₂CH₂CH₂ ou CH₂ peut être remplacé par hydroxy ;
X, indépendamment pour chaque occurrence, représente S, O ou CH=CH, tout atome d'hydrogène d'une unité CH pouvant être remplacé par alkyle ;
Y, indépendamment pour chaque occurrence, représente H ou CH₂O(CO)R₇ ;
R₇, indépendamment pour chaque occurrence, représente H ou alkyle, alkoxy, aminoalkyle, alkylaminoalkyle, hetérocyclylalkyle, arylalkyle, ou hétérocyclylalkoxy substitué ou non substitué ;
Z représente H ou R₃(CO) ;
R₁ et R₂ représentent chacun indépendamment H, alkyle, alkoxy ou hydroxy ;
R₃ représente alkyle, hydroxyalkyle, aminoalkyle, acylaminoalkyle, alkényle, alkoxy, alkoxyalkyle, aryle, arylalkyle, aryloxy, aryloxyalkyle, cycloalkyle, cycloalkylalkyle, hétérocyclyle, hétérocyclylalkyle, hétéroaryle, hétéroarylalkyle, hétéroaryloxy, hétéroaryloxyalkyle substitué ou non substitué ou C(R₈)(R₉)(R₁₀), N(R₄)(R₅) ou OR₆, où tout groupe hydroxyle libre peut être acylé pour former C(O)R₇ ;
R₄ et R₅ chacun indépendamment pour chaque occurrence représentent H ou alkyle, hydroxyalkyle, acyle, aminoalkyle acylaminoalkyle, alkényle, alkoxyalkyle, aryle, arylalkyle, aryloxy, aryloxyalkyle, cycloalkyle, cycloalkylalkyle, hétérocyclyle, hétérocyclylalkyle, hétéroaryle, héteroarylalkyle, hétéroaryloxy, ou hétéroaryloxyalkyle substitué ou non substitué, où tout groupe hydroxyle libre peut être acylé pour former C(O)R₇ ;
R₆ représente alkyle, hydroxyalkyle, aminoalkyle, acylaminoalkyle, alkényle, alkoxyalkyle, aryle, arylalkyle, aryloxy, aryloxyalkyle, cycloalkyle, cycloalkylalkyle, hétérocyclyle, hétérocyclylalkyle, hétéroaryle, hétéroarylalkyle, hétéroaryloxy, ou hétéroaryloxyalkyle substitué ou non substitué, où tout groupe hydroxyle libre peut être acylé pour former C(O)R₇ ;
R₈, R₉ et R₁₀ chacun indépendamment pour chaque occurrence représentent H ou alkyle, hydroxy, hydroxyalkyle, amino, acylamino, aminoalkyle, acylaminoalkyle, alkoxycarbonyle, alkoxycarbonylamino, alkényle, alkoxy, alkoxyalkyle, aryle, arylalkyle, aryloxy, aryloxyalkyle, cycloalkyle, cycloalkylalkyle, hétérocyclyle, hétérocyclylalkyle, hétéroaryle, hétéroarylalkyle, hétéroaryloxy, ou hétéroaryloxyalkyle substitué ou non substitué, ou R₈ et R₉ conjointement avec le carbone auquel ils sont fixés, forment un système cyclique carbocyclique ou hétérocyclique, où tout groupe hydroxyle libre peut être acylé pour former C(O)R₇, et où au moins deux parmi R₈, R₉ et R₁₀ ne sont pas H ;
R₁₁ représente aryle, arylalkyle, aryloxy, aryloxyalkyle, heteroaryle, hétéroarylalkyle, hétéroaryloxy, ou hétéroaryloxyalkyle ou
R₁₁ représente C(R₁₂)(R₁₃)(R₁₄), N(R₄)(R₁₄) ou
OR₁₄, où tout groupe hydroxyle libre peut être acylé pour former C(O)R₇ ;
R₁₂ et R₁₃ chacun indépendamment représentent H ou alkyle, hydroxy, hydroxyalkyle, amino, acylamino, aminoalkyle, acylaminoalkyle, alkoxycarbonyle, alkoxycarbonylamino, alkényle, alkoxy, alkoxyalkyle, aryle, arylalkyle, aryloxy, aryloxyalkyle, cycloalkyle, cycloalkylalkyle, hétérocyclyle, hétérocyclylalkyle, hétéroaryle, hétéroarylalkyle, hétéroaryloxy, ou hétéroaryloxyalkyle, où tout groupe hydroxyle libre peut être acylé pour former C(O)R₇, et où à la fois R₁₂ et R₁₃ ne sont pas H ;
R₁₄ représente aryle, arylalkyle, aryloxy, aryloxyalkyle, hétéroaryle, hétéroarylalkyle, hétéroaryloxy, ou hétéroaryloxyalkyle ; et
dans lequel le composé est destiné à être administré par voie orale avec un repas.

3. Composé pour utilisation selon la revendication 2, dans lequel
(a) R₁₁ représente aryle, arylalkyle, aryloxy, aryloxyalkyle, hétéroaryle, hétéroarylalkyle, hétéroaryloxy, ou hétéroaryloxyalkyle, et le cycle aryle ou hétéroaryle étant substitué par -OCHF₂ ou -OCF₃ et étant éventuellement en outre substitué ; ou
(b) R₁₁ représente arylalkyle, le cycle aryle étant substitué par -OCF₃ ; ou
(c) R₁₁ représente trifluorométhoxybenzyle ; ou
(d) R₁₁ représente

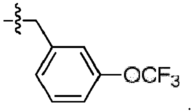

4. Composé pour utilisation selon la revendication 3, dans lequel R₁₄ représente aryle, arylalkyle, aryloxy, aryloxyalkyle, hétéroaryle, hétéroarylalkyle, hétéroaryloxy, ou hétéroaryloxyalkyle, et le cycle aryle ou hétéroaryle est substitué par -OCHF₂ ou -OCF₃ et est éventuellement en outre substitué.

5. Composé pour utilisation selon l'une quelconque des revendications 2 à 4, dans lequel
(a) L représente CH₂SCH₂, CH₂CH₂, CH₂S ou SCH₂ ; de préférence L représente CH₂CH₂ ; et/ou
(b) chaque Y représente H ; et/ou
(c) X représente S ou CH=CH, tout atome d'hydrogène d'une unité CH pouvant être remplacé par alkyle ; et/ou
(d) Z représente R₃(CO) ; et/ou
(e) R₁ et R₂ représentent chacun H.

6. Composé pour utilisation selon la revendication 5, dans lequel Z représente R₃(CO) et R₃ et R₁₁ ne sont pas identiques.

7. Composé pour utilisation selon l'une quelconque des revendications 2 à 6, dans lequel
(a) Z représente R₃(CO) et R₃ représente arylalkyle, hétéroarylalkyle, cycloalkyle ou hétérocycloalkyle substitué ou non substitué ; ou
(b) Z représente R₃(CO) et R₃ représente hétéroarylalkyle substitué ou non substitué ; ou
(c) Z représente R₃(CO) et R₃ représente pyridylalkyle substitué ou non substitué.

8. Composé pour utilisation selon l'une quelconque des revendications 3 et 4, dans lequel L représente CH₂CH₂, chaque Y représente H, X représente S, Z représente R₃(CO), R₁ et R₂ chacun représente H, et R₃ représente arylalkyle, hétéroarylalkyle, cycloalkyle ou hétérocycloalkyle, de préférence R₃ représente hétéroarylalkyle.

9. Composé pour utilisation selon l'une quelconque des revendications 1 à 8, dans lequel le cancer est choisi parmi cancer du sein, cancer colorectal, cancer du système endocrinien, cancer du poumon, mélanome, mésothéliome, cancer du rein, et une malignité des lymphocytes B.

10. Composé pour utilisation selon la revendication 9, dans lequel le cancer est le cancer du sein, de manière appropriée dans lequel le cancer du sein comprend des cellules du cancer du sein de type basal, des cellules du cancer du sein triple négatif ou des cellules du cancer du sein claudin-low.

11. Composé pour utilisation selon la revendication 9, dans lequel le cancer est le cancer colorectal.

12. Composé pour utilisation selon la revendication 9, dans lequel le cancer est le cancer du système endocrinien, de manière appropriée dans lequel le cancer du système endocrinien est choisi parmi adénome du cortex surrénalien, carcinome du cortex surrénalien, phéochromocytome des glandes surrénales, et adénome de la glande parathyroïde.

13. Composé pour utilisation selon la revendication 9, dans lequel le cancer est un mélanome.

14. Composé pour utilisation selon la revendication 9, dans lequel le cancer est le cancer du rein.

15. Composé pour utilisation selon la revendication 9, dans lequel le cancer est une malignité des lymphocytes B, de manière appropriée dans lequel :
(a) la malignité des lymphocytes B est un myélome multiple, ou
(b) la malignité des lymphocytes B est la leucémie, de préférence choisie parmi leucémie lymphoblastique aigüe, et leucémie lymphoblastique chronique ; ou
(c) la malignité des lymphocytes B est le lymphome, de préférence choisie parmi lymphome de Burkitt, lymphome diffus à grandes cellules B, lymphome folliculaire, et lymphome de Hodgkin.

16. Composé pour utilisation selon l'une quelconque des revendications précédentes, dans lequel le composé est de formule III, 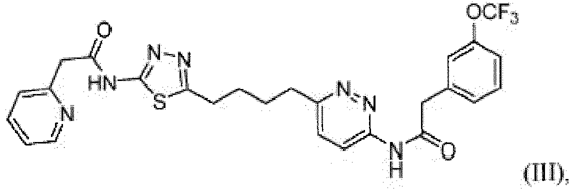 et 300 mg à 3000 mg du composé sont administrés par jour.

17. Composé pour utilisation selon l'une quelconque des revendications précédentes, dans lequel le composé est destiné à être administré deux ou trois fois par jour.
